# EUROPEAN PATENT SPECIFICATION

(11) **EP 2 350 313 B1**
(45) Date of publication and mention of the grant of the patent: **04.06.2014**
(21) Application number: 09744086.1
(22) Date of filing: 29.10.2009
(51) Int. Cl.: C12Q 1/68

(54) **SEQUENCING OF NUCLEIC ACID MOLECULES BY MASS SPECTROMETRY**
SEQUENZIERUNG VON NUKLEINSÄUREMOLEKÜLEN MITTELS MASSENSPEKTROMETRIE
SÉQUENÇAGE DE MOLÉCULES D ACIDES NUCLÉIQUES PAR SPECTROMÉTRIE DE MASSE

(30) Priority: 29.10.2008 EP 08018916
(43) Date of publication of application: 03.08.2011
(73) Proprietor: Noxxon Pharma AG, 10589 Berlin (DE)
(72) Inventor: TURNER, John, 13156 Berlin (DE); HOOS, Johannes, 13587 Berlin (DE); KLUSSMANN, Sven, 10585 Berlin (DE)
(74) Representative: Bohmann, Armin K.
(86) International application number: PCT/EP2009/007754
(87) International publication number: WO 2010/049156

(56) References cited:
- WO-A2-2004/097369
- US-B1- 6 566 059
- TOLEDANO M B ET AL: "Redox-dependent shift of OxyR-DNA contacts along an extended DNA-binding site: A mechanism for differential promoter selection" CELL, CELL PRESS, CAMBRIDGE, NA, US, vol. 78, no. 5, 9 September 1994 (1994-09-09), pages 897-909, XP024246465 ISSN: 0092-8674 [retrieved on 1994-09-09]
- PEATTIE D A: "DIRECT CHEMICAL METHOD FOR SEQUENCING RNA" PROCEEDINGS OF THE NATIONAL ACADEMY OF SCIENCES OF THE UNITED STATES OF AMERICA, vol. 76, no. 4, 1979, pages 1760-1764, XP002568905 ISSN: 0027-8424 cited in the application
- GLOVER ROBERT P ET AL: "Sequencing of oligonucleotides using high performance liquid chromatography and electrospray mass spectrometry" RAPID COMMUNICATIONS IN MASS SPECTROMETRY, vol. 9, no. 10, 1995, pages 897-901, XP008119074 ISSN: 0951-4198
- OHARA R ET AL: "A new solid-phase chemical DNA sequencing method which uses streptavidin-coated magnetic beads.", DNA RESEARCH : AN INTERNATIONAL JOURNAL FOR RAPID PUBLICATION OF REPORTS ON GENES AND GENOMES 30 JUN 1995, vol. 2, no. 3, 30 June 1995 (1995-06-30), pages 123-128, ISSN: 1340-2838
- ISOLA N R ET AL: "Chemical cleavage sequencing of DNA using matrix-assisted laser desorption/ionization time-of-flight mass spectrometry.", ANALYTICAL CHEMISTRY 1 JUL 1999, vol. 71, no. 13, 1 July 1999 (1999-07-01), pages 2266-2269, ISSN: 0003-2700

### SEQUENCE LISTING

<110> NOXXON Pharma AG
<120> Sequencing of nucleic acid molecules by mass spectrometry
<130> N 10074 PCT
<150> EP 08 018 916.0
   <151> 2008-10-29
<160> 146
<170> PatentIn version 3.3
<210> 1
   <211> 40
   <212> RNA
   <213> Artificial
<220>
   <223> Synthetic
<400> 1
   gcacgucccu caccggugca agugaagccg uggcucugcg 40
<210> 2
   <211> 40
   <212> RNA
   <213> Artificial
<220>
   <223> Synthetic
<400> 2
   gcacgucccu caccggugca agugaagccg uggcucugcg 40
<210> 3
   <211> 40
   <212> RNA
   <213> Artificial
<220>
   <223> Synthetic
<400> 3
   gcacgucccc uaccggugca agugaagccg ugguccugcg 40
<210> 4
   <211> 3
   <212> RNA
   <213> Artificial
<220>
   <223> Synthetic
<400> 4
   gcg 3
<210> 5
   <211> 5
   <212> RNA
   <213> Artificial
<220>
   <223> Synthetic
<400> 5
   cugcg 5
<210> 6
   <211> 9
   <212> RNA
   <213> Artificial
<220>
   <223> Synthetic
<400> 6
   ggcucugcg 9
<210> 7
   <211> 17
   <212> RNA
   <213> Artificial
<220>
   <223> Synthetic
<400> 7
   gaagccgugg cucugcg 17
<210> 8
   <211> 23
   <212> RNA
   <213> Artificial
<220>
   <223> Synthetic
<400> 8
   gcaagugaag ccguggcucu gcg 23
<210> 9
   <211> 29
   <212> RNA
   <213> Artificial
<220>
   <223> Synthetic
<400> 9
   caccggugca gugaagccgu ggcucugcg 29
<210> 10
   <211> 34
   <212> RNA
   <213> Artificial
<220>
   <223> Synthetic
<400> 10
   cccucaccgg ugcaagugaa gccguggcuc ugcg 34
<210> 11
   <211> 1
   <212> RNA
   <213> Artificial
<220>
   <223> Synthetic
<400> 11
   g 1
<210> 12
   <211> 2
   <212> RNA
   <213> Artificial
<220>
   <223> Synthetic
<400> 12
   gc 2
<210> 13
   <211> 3
   <212> RNA
   <213> Artificial
<220>
   <223> Synthetic
<400> 13
   gca 3
<210> 14
   <211> 4
   <212> RNA
   <213> Artificial
<220>
   <223> Synthetic
<400> 14
   gcac 4
<210> 15
   <211> 5
   <212> RNA
   <213> Artificial
<220>
   <223> Synthetic
<400> 15
   gcacg 5
<210> 16
   <211> 6
   <212> RNA
   <213> Artificial
<220>
   <223> Synthetic
<400> 16
   gcacgu 6
<210> 17
   <211> 7
   <212> RNA
   <213> Artificial
<220>
   <223> Synthetic
<400> 17
   gcacguc 7
<210> 18
   <211> 8
   <212> RNA
   <213> Artificial
<220>
   <223> Synthetic
<400> 18
   gcacgucc 8
<210> 19
   <211> 9
   <212> RNA
   <213> Artificial
<220>
   <223> Synthetic
<400> 19
   gcacguccc 9
<210> 20
   <211> 10
   <212> RNA
   <213> Artificial
<220>
   <223> Synthetic
<400> 20
   gcacgucccu 10
<210> 21
   <211> 11
   <212> RNA
   <213> Artificial
<220>
   <223> Synthetic
<400> 21
   gcacgucccu c 11
<210> 22
   <211> 12
   <212> RNA
   <213> Artificial
<220>
   <223> Synthetic
<400> 22
   gcacgucccu ca 12
<210> 23
   <211> 13
   <212> RNA
   <213> Artificial
<220>
   <223> Synthetic
<400> 23
   gcacgucccu cac 13
<210> 24
   <211> 14
   <212> RNA
   <213> Artificial
<220>
   <223> Synthetic
<400> 24
   gcacgucccu cacc 14
<210> 25
   <211> 15
   <212> RNA
   <213> Artificial
<220>
   <223> Synthetic
<400> 25
   gcacgucccu caccg 15
<210> 26
   <211> 16
   <212> RNA
   <213> Artificial
<220>
   <223> Synthetic
<400> 26
   gcacgucccu caccgg 16
<210> 27
   <211> 17
   <212> RNA
   <213> Artificial
<220>
   <223> Synthetic
<400> 27
   gcacgucccu caccggu 17
<210> 28
   <211> 18
   <212> RNA
   <213> Artificial
<220>
   <223> Synthetic
<400> 28
   gcacgucccu caccggug 18
<210> 29
   <211> 19
   <212> RNA
   <213> Artificial
<220>
   <223> Synthetic
<400> 29
   gcacgucccu caccggugc 19
<210> 30
   <211> 20
   <212> RNA
   <213> Artificial
<220>
   <223> Synthetic
<400> 30
   gcacgucccu caccggugca 20
<210> 31
   <211> 21
   <212> RNA
   <213> Artificial
<220>
   <223> Synthetic
<400> 31
   gcacgucccu caccggugca a 21
<210> 32
   <211> 22
   <212> RNA
   <213> Artificial
<220>
   <223> Synthetic
<400> 32
   gcacgucccu caccggugca ag 22
<210> 33
   <211> 23
   <212> RNA
   <213> Artificial
<220>
   <223> Synthetic
<400> 33
   gcacgucccu caccggugca agu 23
<210> 34
   <211> 24
   <212> RNA
   <213> Artificial
<220>
   <223> Synthetic
<400> 34
   gcacgucccu caccggugca agug 24
<210> 35
   <211> 25
   <212> RNA
   <213> Artificial
<220>
   <223> Synthetic
<400> 35
   gcacgucccu caccggugca aguga 25
<210> 36
   <211> 26
   <212> RNA
   <213> Artificial
<220>
   <223> Synthetic
<400> 36
   gcacgucccu caccggugca agugaa 26
<210> 37
   <211> 27
   <212> RNA
   <213> Artificial
<220>
   <223> Synthetic
<400> 37
   gcacgucccu caccggugca agugaag 27
<210> 38
   <211> 28
   <212> RNA
   <213> Artificial
<220>
   <223> Synthetic
<400> 38
   gcacgucccu caccggugca agugaagc 28
<210> 39
   <211> 29
   <212> RNA
   <213> Artificial
<220>
   <223> Synthetic
<400> 39
   gcacgucccu caccggugca agugaagcc 29
<210> 40
   <211> 30
   <212> RNA
   <213> Artificial
<220>
   <223> Synthetic
<400> 40
   gcacgucccu caccggugca agugaagccg 30
<210> 41
   <211> 31
   <212> RNA
   <213> Artificial
<220>
   <223> Synthetic
<400> 41
   gcacgucccu caccggugca agugaagccg u 31
<210> 42
   <211> 32
   <212> RNA
   <213> Artificial
<220>
   <223> Synthetic
<400> 42
   gcacgucccu caccggugca agugaagccg ug 32
<210> 43
   <211> 33
   <212> RNA
   <213> Artificial
<220>
   <223> Synthetic
<400> 43
   gcacgucccu caccggugca agugaagccg ugg 33
<210> 44
   <211> 34
   <212> RNA
   <213> Artificial
<220>
   <223> Synthetic
<400> 44
   gcacgucccu caccggugca agugaagccg uggc 34
<210> 45
   <211> 35
   <212> RNA
   <213> Artificial
<220>
   <223> Synthetic
<400> 45
   gcacgucccu caccggugca agugaagccg uggcu 35
<210> 46
   <211> 36
   <212> RNA
   <213> Artificial
<220>
   <223> Synthetic
<400> 46
   gcacgucccu caccggugca agugaagccg uggcuc 36
<210> 47
   <211> 37
   <212> RNA
   <213> Artificial
<220>
   <223> Synthetic
<400> 47
   gcacgucccu caccggugca agugaagccg uggcucu 37
<210> 48
   <211> 38
   <212> RNA
   <213> Artificial
<220>
   <223> Synthetic
<400> 48
   gcacgucccu caccggugca agugaagccg uggcucug 38
<210> 49
   <211> 39
   <212> RNA
   <213> Artificial
<220>
   <223> Synthetic
<400> 49
   gcacgucccu caccggugca agugaagccg uggcucugc 39
<210> 50
   <211> 40
   <212> RNA
   <213> Artificial
<220>
   <223> Synthetic
<400> 50
   gcacgucccu caccggugca agugaagccg uggcucugcg 40
<210> 51
   <211> 1
   <212> RNA
   <213> Artificial
<220>
   <223> Synthetic
<400> 51
   g 1
<210> 52
   <211> 2
   <212> RNA
   <213> Artificial
<220>
   <223> Synthetic
<400> 52
   gc 2
<210> 53
   <211> 3
   <212> RNA
   <213> Artificial
<220>
   <223> Synthetic
<400> 53
   gca 3
<210> 54
   <211> 4
   <212> RNA
   <213> Artificial
<220>
   <223> Synthetic
<400> 54
   gcac 4
<210> 55
   <211> 5
   <212> RNA
   <213> Artificial
<220>
   <223> Synthetic
<400> 55
   gcacg 5
<210> 56
   <211> 5
   <212> RNA
   <213> Artificial
<220>
   <223> Synthetic
<400> 56
   gcacg 5
<210> 57
   <211> 3
   <212> RNA
   <213> Artificial
<220>
   <223> Synthetic
<400> 57
   ccc 3
<210> 58
   <211> 6
   <212> RNA
   <213> Artificial
<220>
   <223> Synthetic
<400> 58
   caccgg 6
<210> 59
   <211> 5
   <212> RNA
   <213> Artificial
<220>
   <223> Synthetic
<400> 59
   gcaag 5
<210> 60
   <211> 7
   <212> RNA
   <213> Artificial
<220>
   <223> Synthetic
<400> 60
   gaagccg 7
<210> 61
   <211> 3
   <212> RNA
   <213> Artificial
<220>
   <223> Synthetic
<400> 61
   ggc 3
<210> 62
   <211> 3
   <212> RNA
   <213> Artificial
<220>
   <223> Synthetic
<400> 62
   gcg 3
<210> 63
   <211> 40
   <212> RNA
   <213> Artificial
<220>
   <223> Synthetic
<400> 63
   gcacgucccu caccggugca agugaagccg uggcucugcg 40
<210> 64
   <211> 45
   <212> RNA
   <213> Artificial
<220>
   <223> Synthetic
<400> 64
   gcguggugug aucuagaugu auuggcugau ccuagucagg uacgc 45
<210> 65
   <211> 45
   <212> RNA
   <213> Artificial
<220>
   <223> Synthetic
<400> 65
   gcguggugug aucuagaugu auuggcugau ccuagucagg uacgc 45
<210> 66
   <211> 6
   <212> RNA
   <213> Artificial
<220>
   <223> Synthetic
<400> 66
   gcacgu 6
<210> 67
   <211> 7
   <212> RNA
   <213> Artificial
<220>
   <223> Synthetic
<400> 67
   gcacguc 7
<210> 68
   <211> 8
   <212> RNA
   <213> Artificial
<220>
   <223> Synthetic
<400> 68
   gcacgucc 8
<210> 69
   <211> 9
   <212> RNA
   <213> Artificial
<220>
   <223> Synthetic
<400> 69
   gcacguccc 9
<210> 70
   <211> 10
   <212> RNA
   <213> Artificial
<220>
   <223> Synthetic
<400> 70
   gcacgucccu 10
<210> 71
   <211> 11
   <212> RNA
   <213> Artificial
<220>
   <223> Synthetic
<400> 71
   gcacgucccu c 11
<210> 72
   <211> 12
   <212> RNA
   <213> Artificial
<220>
   <223> Synthetic
<400> 72
   gcacgucccu ca 12
<210> 73
   <211> 13
   <212> RNA
   <213> Artificial
<220>
   <223> Synthetic
<400> 73
   gcacgucccu cac 13
<210> 74
   <211> 14
   <212> RNA
   <213> Artificial
<220>
   <223> Synthetic
<400> 74
   gcacgucccu cacc 14
<210> 75
   <211> 15
   <212> RNA
   <213> Artificial
<220>
   <223> Synthetic
<400> 75
   gcacgucccu caccg 15
<210> 76
   <211> 16
   <212> RNA
   <213> Artificial
<220>
   <223> Synthetic
<400> 76
   gcacgucccu caccgg 16
<210> 77
   <211> 17
   <212> RNA
   <213> Artificial
<220>
   <223> Synthetic
<400> 77
   gcacgucccu caccggu 17
<210> 78
   <211> 18
   <212> RNA
   <213> Artificial
<220>
   <223> Synthetic
<400> 78
   gcacgucccu caccggug 18
<210> 79
   <211> 19
   <212> RNA
   <213> Artificial
<220>
   <223> Synthetic
<400> 79
   gcacgucccu caccggugc 19
<210> 80
   <211> 20
   <212> RNA
   <213> Artificial
<220>
   <223> Synthetic
<400> 80
   gcacgucccu caccggugca 20
<210> 81
   <211> 21
   <212> RNA
   <213> Artificial
<220>
   <223> Synthetic
<400> 81
   gcacgucccu caccggugca a 21
<210> 82
   <211> 22
   <212> RNA
   <213> Artificial
<220>
   <223> Synthetic
<400> 82
   gcacgucccu caccggugca ag 22
<210> 83
   <211> 23
   <212> RNA
   <213> Artificial
<220>
   <223> Synthetic
<400> 83
   gcacgucccu caccggugca agu 23
<210> 84
   <211> 24
   <212> RNA
   <213> Artificial
<220>
   <223> Synthetic
<400> 84
   gcacgucccu caccggugca agug 24
<210> 85
   <211> 25
   <212> RNA
   <213> Artificial
<220>
   <223> Synthetic
<400> 85
   gcacgucccu caccggugca aguga 25
<210> 86
   <211> 26
   <212> RNA
   <213> Artificial
<220>
   <223> Synthetic
<400> 86
   gcacgucccu caccggugca agugaa 26
<210> 87
   <211> 27
   <212> RNA
   <213> Artificial
<220>
   <223> Synthetic
<400> 87
   gcacgucccu caccggugca agugaag 27
<210> 88
   <211> 28
   <212> RNA
   <213> Artificial
<220>
   <223> Synthetic
<400> 88
   gcacgucccu caccggugca agugaagc 28
<210> 89
   <211> 29
   <212> RNA
   <213> Artificial
<220>
   <223> Synthetic
<400> 89
   gcacgucccu caccggugca agugaagcc 29
<210> 90
   <211> 30
   <212> RNA
   <213> Artificial
<220>
   <223> Synthetic
<400> 90
   gcacgucccu caccggugca agugaagccg 30
<210> 91
   <211> 31
   <212> RNA
   <213> Artificial
<220>
   <223> Synthetic
<400> 91
   gcacgucccu caccggugca agugaagccg u 31
<210> 92
   <211> 32
   <212> RNA
   <213> Artificial
<220>
   <223> Synthetic
<400> 92
   gcacgucccu caccggugca agugaagccg ug 32
<210> 93
   <211> 33
   <212> RNA
   <213> Artificial
<220>
   <223> Synthetic
<400> 93
   gcacgucccu caccggugca agugaagccg ugg 33
<210> 94
   <211> 34
   <212> RNA
   <213> Artificial
<220>
   <223> Synthetic
<400> 94
   gcacgucccu caccggugca agugaagccg uggc 34
<210> 95
   <211> 35
   <212> RNA
   <213> Artificial
<220>
   <223> Synthetic
<400> 95
   gcacgucccu caccggugca agugaagccg uggcu 35
<210> 96
   <211> 36
   <212> RNA
   <213> Artificial
<220>
   <223> Synthetic
<400> 96
   gcacgucccu caccggugca agugaagccg uggcuc 36
<210> 97
   <211> 37
   <212> RNA
   <213> Artificial
<220>
   <223> Synthetic
<400> 97
   gcacgucccu caccggugca agugaagccg uggcucu 37
<210> 98
   <211> 38
   <212> RNA
   <213> Artificial
<220>
   <223> Synthetic
<400> 98
   gcacgucccu caccggugca agugaagccg uggcucug 38
<210> 99
   <211> 39
   <212> RNA
   <213> Artificial
<220>
   <223> Synthetic
<400> 99
   gcacgucccu caccggugca agugaagccg uggcucugc 39
<210> 100
   <211> 40
   <212> RNA
   <213> Artificial
<220>
   <223> Synthetic
<400> 100
   gcacgucccu caccggugca agugaagccg uggcucugcg 40
<210> 101
   <211> 1
   <212> RNA
   <213> Artificial
<220>
   <223> Synthetic
<400> 101
   g 1
<210> 102
   <211> 2
   <212> RNA
   <213> Artificial
<220>
   <223> Synthetic
<400> 102
   gc 2
<210> 103
   <211> 3
   <212> RNA
   <213> Artificial
<220>
   <223> Synthetic
<400> 103
   gcg 3
<210> 104
   <211> 4
   <212> RNA
   <213> Artificial
<220>
   <223> Synthetic
<400> 104
   gcgu 4
<210> 105
   <211> 5
   <212> RNA
   <213> Artificial
<220>
   <223> Synthetic
<400> 105
   gcgug 5
<210> 106
   <211> 6
   <212> RNA
   <213> Artificial
<220>
   <223> Synthetic
<400> 106
   gcgugg 6
<210> 107
   <211> 7
   <212> RNA
   <213> Artificial
<220>
   <223> Synthetic
<400> 107
   gcguggu 7
<210> 108
   <211> 8
   <212> RNA
   <213> Artificial
<220>
   <223> Synthetic
<400> 108
   gcguggug 8
<210> 109
   <211> 9
   <212> RNA
   <213> Artificial
<220>
   <223> Synthetic
<400> 109
   gcguggugu 9
<210> 110
   <211> 10
   <212> RNA
   <213> Artificial
<220>
   <223> Synthetic
<400> 110
   gcguggugug 10
<210> 111
   <211> 11
   <212> RNA
   <213> Artificial
<220>
   <223> Synthetic
<400> 111
   gcguggugug a 11
<210> 112
   <211> 12
   <212> RNA
   <213> Artificial
<220>
   <223> Synthetic
<400> 112
   gcguggugug au 12
<210> 113
   <211> 13
   <212> RNA
   <213> Artificial
<220>
   <223> Synthetic
<400> 113
   gcguggugug auc 13
<210> 114
   <211> 14
   <212> RNA
   <213> Artificial
<220>
   <223> Synthetic
<400> 114
   gcguggugug aucu 14
<210> 115
   <211> 15
   <212> RNA
   <213> Artificial
<220>
   <223> Synthetic
<400> 115
   gcguggugug aucua 15
<210> 116
   <211> 16
   <212> RNA
   <213> Artificial
<220>
   <223> Synthetic
<400> 116
   gcguggugug aucuag 16
<210> 117
   <211> 17
   <212> RNA
   <213> Artificial
<220>
   <223> Synthetic
<400> 117
   gcguggugug aucuaga 17
<210> 118
   <211> 18
   <212> RNA
   <213> Artificial
<220>
   <223> Synthetic
<400> 118
   gcguggugug aucuagau 18
<210> 119
   <211> 19
   <212> RNA
   <213> Artificial
<220>
   <223> Synthetic
<400> 119
   gcguggugug aucuagaug 19
<210> 120
   <211> 20
   <212> RNA
   <213> Artificial
<220>
   <223> Synthetic
<400> 120
   gcguggugug aucuagaugu 20
<210> 121
   <211> 21
   <212> RNA
   <213> Artificial
<220>
   <223> Synthetic
<400> 121
   gcguggugug aucuagaugu a 21
<210> 122
   <211> 22
   <212> RNA
   <213> Artificial
<220>
   <223> Synthetic
<400> 122
   gcguggugug aucuagaugu au 22
<210> 123
   <211> 23
   <212> RNA
   <213> Artificial
<220>
   <223> Synthetic
<400> 123
   gcguggugug aucuagaugu auu 23
<210> 124
   <211> 24
   <212> RNA
   <213> Artificial
<220>
   <223> Synthetic
<400> 124
   gcguggugug aucuagaugu auug 24
<210> 125
   <211> 25
   <212> RNA
   <213> Artificial
<220>
   <223> Synthetic
<400> 125
   gcguggugug aucuagaugu auugg 25
<210> 126
   <211> 26
   <212> RNA
   <213> Artificial
<220>
   <223> Synthetic
<400> 126
   gcguggugug aucuagaugu auuggc 26
<210> 127
   <211> 27
   <212> RNA
   <213> Artificial
<220>
   <223> Synthetic
<400> 127
   gcguggugug aucuagaugu auuggcu 27
<210> 128
   <211> 28
   <212> RNA
   <213> Artificial
<220>
   <223> Synthetic
<400> 128
   gcguggugug aucuagaugu auuggcug 28
<210> 129
   <211> 29
   <212> RNA
   <213> Artificial
<220>
   <223> Synthetic
<400> 129
   gcguggugug aucuagaugu auuggcuga 29
<210> 130
   <211> 30
   <212> RNA
   <213> Artificial
<220>
   <223> Synthetic
<400> 130
   gcguggugug aucuagaugu auuggcugau 30
<210> 131
   <211> 31
   <212> RNA
   <213> Artificial
<220>
   <223> Synthetic
<400> 131
   gcguggugug aucuagaugu auuggcugau c 31
<210> 132
   <211> 32
   <212> RNA
   <213> Artificial
<220>
   <223> Synthetic
<400> 132
   gcguggugug aucuagaugu auuggcugau cc 32
<210> 133
   <211> 33
   <212> RNA
   <213> Artificial
<220>
   <223> Synthetic
<400> 133
   gcguggugug aucuagaugu auuggcugau ccu 33
<210> 134
   <211> 34
   <212> RNA
   <213> Artificial
<220>
   <223> Synthetic
<400> 134
   gcguggugug aucuagaugu auuggcugau ccua 34
<210> 135
   <211> 35
   <212> RNA
   <213> Artificial
<220>
   <223> Synthetic
<400> 135
   gcguggugug aucuagaugu auuggcugau ccuag 35
<210> 136
   <211> 36
   <212> RNA
   <213> Artificial
<220>
   <223> Synthetic
<400> 136
   gcguggugug aucuagaugu auuggcugau ccuagu 36
<210> 137
   <211> 37
   <212> RNA
   <213> Artificial
<220>
   <223> Synthetic
<400> 137
   gcguggugug aucuagaugu auuggcugau ccuaguc 37
<210> 138
   <211> 38
   <212> RNA
   <213> Artificial
<220>
   <223> Synthetic
<400> 138
   gcguggugug aucuagaugu auuggcugau ccuaguca 38
<210> 139
   <211> 39
   <212> RNA
   <213> Artificial
<220>
   <223> Synthetic
<400> 139
   gcguggugug aucuagaugu auuggcugau ccuagucag 39
<210> 140
   <211> 40
   <212> RNA
   <213> Artificial
<220>
   <223> Synthetic
<400> 140
   gcguggugug aucuagaugu auuggcugau ccuagucagg 40
<210> 141
   <211> 41
   <212> RNA
   <213> Artificial
<220>
   <223> Synthetic
<400> 141
   gcguggugug aucuagaugu auuggcugau ccuagucagg u 41
<210> 142
   <211> 42
   <212> RNA
   <213> Artificial
<220>
   <223> Synthetic
<400> 142
   gcguggugug aucuagaugu auuggcugau ccuagucagg ua 42
<210> 143
   <211> 43
   <212> RNA
   <213> Artificial
<220>
   <223> Synthetic
<400> 193
   gcguggugug aucuagaugu auuggcugau ccuagucagg uac 43
<210> 144
   <211> 44
   <212> RNA
   <213> Artificial
<220>
   <223> Synthetic
<400> 144
   gcguggugug aucuagaugu auuggcugau ccuagucagg uacg 44
<210> 145
   <211> 45
   <212> RNA
   <213> Artificial
<220>
   <223> Synthetic
<400> 145
   gcguggugug aucuagaugu auuggcugau ccuagucagg uacgc 45
<210> 146
   <211> 40
   <212> RNA
   <213> Artificial
<220>
   <223> Synthetic
<400> 146
   gcacgucccc uaccggugca agugaagccg ugguccugcg 40

## Description

The present invention is related to methods for analyzing and/or determining the nucleotide sequence of nucleic acid molecules.

### Background of the invention

Nucleic acid molecules are used as diagnostic tools and/or as therapeutics, whereby the nucleic acid molecules can be identified by screening of partly or fully randomized nucleic acid molecule libraries, or predicted according to complementary sequences aided by computer algorithms such as done for antisense, siRNA and miRNA. Nucleic acid molecules can be single or double-stranded molecules, they can be structured or not, they can be conjugated to peptides, proteins, polysaccharides and other larger molecules, their sugar backbone can consists of ribose, deoxyribose and/or modified derivatives thereof.

The function of the nucleic acid molecules can be based on
a) sequence-specific hybridisation to and switch-off of mRNA in form of antisense nucleic acid molecules, catalytically nucleic acid molecules, siRNA molecules and micro RNA molecules (Couzin, 2004; Crooke, 2004; Hannon, 2002; Juliano et al, 2008; Scherer & Rossi, 2003; Schlosser et al, 2006; Usman & Blatt, 2000; Weigand et al, 2006; Zhang & Farwell, 2008);
b) or binding of nucleic acid molecules to a target molecule and/or blockage of the function of the target molecule by nucleic acid molecules, whereby the nucleic acid molecules comprise aptamers, Spiegelmers and decoy nucleic acids (Carothers & Szostak, 2006; Cload et al, 2006; Eulberg et al, 2006; Mann & Dzau, 2000; Nimjee et al, 2006; Realini et al, 2006);
c) or their stimulatory effect on the immune systems, e.g. in form of CpG-DNA (Weiner, 2000) and random oligonucleotides.

The production, development and use of such nucleic acid molecules as diagnostic tools and/ or as therapeutics necessitates a method for verification of the identity of the nucleic acid molecules, whereby there is a need for a sensitive, accurate and reproducible analysis. Verification of identity requires determination of the molecular mass and length of the nucleic acid molecules as well its base composition, sequence, and the identity of the sugar moieties and internucleotide linkages. The methods used should be specific, allowing identification of modified bases and modified sugar moieties, addition or deletion products, and depurination products. The determination of the nucleotide sequence must be complete, and it must be shown that any chemical or enzymatic manipulations do not adversely affect either the bases or the backbone. Characterisation of modified nucleic acid molecules is particular challenging if they are nuclease stable. Such nuclease-stable nucleic acid molecules are modified at the 2'-position of the sugar backbone or consist of mirror-image nucleotides. However, a number of techniques have been developed, including, inter alia, electrophoresis, enzymatic and chemical analysis, array technology and mass spectrometry, to determine the nucleotide sequence of nucleic acid molecules.

### Nucleotide sequence determination of nucleic acids by enzymatic and chemical analysis

In the 1970s three techniques of nucleic acid molecule sequencing were developed, that are common and relatively rapid procedures practiced in many laboratories.

*DNA sequencing method by Maxam and Gilbert.* The method described by Maxam and Gilbert describes a process whereby terminally labeled DNA molecules are chemically cleaved in a nucleobase-specific manner. Each base position in the nucleic acid molecule sequence is then determined from the molecular weights of the fragments produced by nucleobase-specific cleavage. Individual reactions were devised to cleave preferentially at guanine, at adenine, at cytosine and thymine, and at cytosine alone. When the products of these four reactions are resolved by molecular weight via the increasing negative charge of the increasing fragment size, using, for example, polyacrylamide gel electrophoresis, sequences of the DNA molecule can be read from the pattern of fragments on the resolved gel (Maxam & Gilbert, 1977).

*DNA sequencing method by Sanger.* The other method - developed by Sanger et al. - takes advantage of the chain terminating ability of dideoxynucleoside triphosphates (abbr. ddNTPs) and the ability of the DNA polymerase to incorporate ddNTPs with nearly equal fidelity as the natural substrate of the DNA polymerase, deoxynucleoside triphosphates (abbr. dNTPs). Briefly, a primer molecule, usually an oligonucleotide molecule, and a template DNA molecule are incubated in the presence of a useful concentration of all four dNTPs plus a limited amount of a single ddNTP. The DNA polymerase occasionally incorporates in the growing, amplified strand a dideoxynucleotide that terminates chain extension. Because the dideoxynucleotide has no 3'-hydroxyl, the initiation point for the polymerase enzyme is lost. Polymerization produces a mixture of nucleic acid molecule fragments of varied sizes, all having identical 5'-termini. Fractionation of the mixture by, for example, polyacrylamide gel electrophoresis, produces a pattern that indicates the presence and position of each nucleotide in the nucleic acid molecule. Reactions with each of the four ddNTPs permits the nucleic acid molecule sequence to be read from a resolved gel (Sanger et al, 1977) in a similar way as done using the technique developed by Maxam and Gilbert (Maxam & Gilbert, 1977).

*RNA sequencing method by Peattie.* Due to the different chemical properties of RNA molecules and greater lability of RNA molecules in comparison to DNA molecules the chemical method of Maxam and Gilbert is not applicable for RNA molecules. Peattie developed a chemical method of sequencing RNA molecules, whereby the RNA molecules are 3'-radiolabelled and chemically cleaved in a nucleobase-specific manner. Each nucleotide position in the nucleic acid sequence of the nucleic acid molecules is then determined from the molecular weights of the nucleic acid molecule fragments produced by nucleobase-specific cleavage. Individual reactions were devised to cleave preferentially at guanine, at adenine or guanine, at cytosine and uracil, and at uracil alone. When the products of these four reactions are resolved by molecular weight, using, for example, mobility differentiation via polyacrylamide gel electrophoresis, the sequence of the RNA molecule can be read from the pattern of fragments on the resolved gel (Peattie, 1979).

*RNA Sequencing method based on Sanger method.* The most common method for identification of the sequence of an RNA molecule is the method by Sanger as described supra. In the case of RNA molecules, the dideoxy chain termination reaction is catalyzed by reverse transcriptase that reads the RNA molecule template and inserts the complementary deoxynucleotide. As with the polymerases used in DNA sequencing, the reverse transcription reaction is inhibited by dideoxynucleotides (Zimmern & Kaesberg, 1978).

*RNA fingerprinting.* In the RNA fingerprinting approach the RNA molecule is digested separately with two or more endonucleases, whereby the endonucleases cleave specifically. The resulting fragments of the RNA molecules from each cleavage reaction are separated by charge (first dimension) and by length (second dimension). The separation by charge is done by the use of high-voltage electrophoresis on cellulose-acetate strips. Afterwards the RNA molecule fragments are transferred to DEAE cellulose paper for separation in the second dimension. The sequence is determined by overlapping the chromatographically resolved fragments from the separate enzymatic digestion reaction (Branch et al, 1989).

Based on and/or with the regard to the methods of Sanger, Maxam and Gilbert, and Peattie (Maxam & Gilbert, 1977; Peattie, 1979; Sanger et al, 1977), several improvements and/or modifications of the procesess were developed: Fluorescence-labeling instead of radioactive labeling, post-labeling techniques, enzymatic cleavage instead of chemical cleavage, step-wise wandering spot method, alternative cleavage reactions for RNA and DNA (Donis-Keller et al, 1977; Gupta et al, 1976; Gupta & Randerath, 1977; Lockard et al, 1978; Proudnikov & Mirzabekov, 1996; Stanley & Vassilenko, 1978; Tanaka et al, 1980; Waldmann et al, 1987; Wu et al, 1996).

However, each technique has inherent limitations. For example, Maxam and Gilbert (Maxam & Gilbert, 1977) and Peattie (Peattie, 1979) disclose a chemical degradation approach and Sanger et al. (Sanger et al, 1977) disclose a chain termination method using complementary strand primer extension. Each of these techniques utilizes four separate reaction mixtures to create a nested set of fragments differing by a single nucleotide in length, thus representing a complete nucleotide sequence. A resolution of the fragments based on their size and terminating nucleotide is carried out by polyacrylamide gel eletrophoresis to determine the order of the fragments and hence the nucleotide sequence of the nucleic acid molecule. The casting of gels and the electrophoretic separation of nucleic acid molecules are time-comsuming operations. The use of gel electrophoresis to determine the sequence of the nucleic acid molecule is a potential source of error due to band compression effects, where adjacent fragments of the nucleic acid molecules are unresolved, and the identification of each individual strand is based on the measurement of a relative value, i.e. migration time. A potential source of error is, for instance, the structure of the nucleic acid molecule and the fragments thereof. For instance, the RNA fingerprinting approach which uses Thin Layer Chromatography (abbr. TLC) is inappropriate for the characterisation of unknown (modified) structures (Limbach, 1996).

Hence, sequence determination of nucleic acid molecules by mass spectrometry was a promising approach to overcome these limitations (Limbach, 1996).

### Nucleotide sequence determination of nucleic acids by mass spectrometry

Mass spectrometry (abbr. MS) is a powerful tool for analyzing the molecular mass of compounds. With regard to nucleic acid molecule analysis, MS is applicable for nucleic acid molecule sequencing, nucleic acid molecule modification detection and determination of nucleic acid molecule fragments. Analysis of nucleic acids by MS is primarily limited by ionization efficiency and by the resolving power of several applicable detection methods.

Only charged molecules can be analyzed by a mass detector. Therefore, the molecules to be analyzed need to be efficiently ionized before they are introduced to a mass analyzer. For efficient ionization of nucleic acid molecules prior to mass analysis the following techniques are commonly used: electrospray ionization (abbr. ESI) (Fenn et al, 1989) and matrix-assisted laser desorption/ionization (abbr. MALDI) (Karas & Hillenkamp, 1988). ESI is the conversion of molecules or ions in solution into ions in the vapour phase, principally through the vaporization of charged droplets of the solution. ESI can produce a distribution of multiple charged ions having a mass-to-charge ratio within the linear range of commercially available mass analyzers. Although a mixture of compounds present in a solution can be directly analyzed by ESI-MS, this procedure can suffer for example, from complex spectra because of multiple charging of the different compounds, competition of excess charge and interference by salt adducts. Therefore, electrospray ionization is often directly coupled down-stream to a separation mechanism. This procedure promotes efficient ionization, when various critical parameters such as flow-rates, ionization mode, buffers and solvent additives are optimised.

Although ESI-MS is sensitive, requiring only femtomole quantities of sample, it relies on multiple charges to achieve efficient ionization and produces complex and difficult-to-interpret multiply-charged spectra for even simple nucleic acid molecules. Therefore, in practice, the application of ESI-MS relies on the availability of software packages enabling "deconvolution" of the data. Deconvolution involes the use of an algorithm-based calculation process to determine the uncharged (neutral) mass of the molecule from the multiple-charge mass-spectral data.

Matrix-assisted laser desorption ionization (abbr. MALDI) used e.g. in conjunction with a time-of-flight (abbr. TOF) mass analyzer has a great potential for sequencing nucleic acid molecules because of its relatively broad mass range and high sampling rate. For routine analysis of biomolecules of large mass like nucleic acid molecules, MALDI-MS is commonly preferred in comparison to ESI-MS because the biomolecules of large mass can be ionized and analyzed readily. In addition, MALDI-MS produces predominantly singly charged species, which greatly simplifies the interpretation of spectra, especially those containing mixtures of oligonucleotides.

However, in general, MALDI-MS analysis of nucleic acid molecules may suffer from a lack of resolution of high molecular weight nucleic acid molecule fragments, nucleic acid instability, and interference from sample preparation reagents. Longer nucleic acid molecules can give broader, less-intense signals, because MALDI imparts greater kinetic energies to ions of higher molecular weights. Although it may be used to analyze high molecular-weight nucleic acids, MALDI-MS can induce cleavage of the nucleic acid molecules' backbone, which further complicates the resulting spectrum. Although MALDI is less sensitive to ion suppression than ESI, ion suppression is still an issue for MALDI analysis, and necessitates the use of sample clean-up strategies, and/or chromatographic separation. However, MALDI is not readily amenable to direct coupling with solution-based techniques, and is typically operated in the off-line or in the at-line mode.

### Direct mass spectrometric methods for sequencing

Any mass spectrometric approach that does not depend upon an external reaction to generate sequence-specific ions is considered as direct method of sequencing. TAs mentioned supra, ESI and MALDI are the ionization methods of choice for nucleic acid molecules (Limbach, 1996). A detailed overview of the methods is given by Limbach and Nordhoff et al. (Limbach, 1996; Nordhoff et al, 1996).

*Desorption*/*Ionization-Induced Fragmentation.* Dissociation of nucleic acid molecules can occur as a result of the excess energy that is imparted to the nucleic acid molecules during desorption/ionization process. This dissociation occurs on relatively fast time scales, resulting in ions that are generally difficult to identify accurately. ESI mostly produces stable, intact molecular ions. Most dissociations that are desorption/ionization-induced are seen in MALDI, whereby in MALDI-TOF-MS exist four differing time-scales for desorption/ionization-induced dissociations: prompt, fast, fast metastable and metastable. In theory, dissociations occuring during any one of these time scales will generate nucleic acid molecule fragment ions that could be used to determine the sequence of the nucleic acid molecule. In practice, the analyst has little control over the extent of fragmentation. Most of these fragments result in a broadening of the molecular ion peak resulting in a loss of resolution and sensitivity (Limbach, 1996).

*Tandem mass spectrometry.* MS-MS (also called tandem mass spectrometry) involves the measurement of the mass-to-charge rations (*m*/*z*) of ions before and after a chemical reaction that occurs within a mass spectrometer whereby a change in *m*/*z* is involved (Baker et al, 1993; Boschenok & Sheil, 1996; Kawase et al, 1991; Limbach et al, 1995; Little et al, 1995; Marzilli et al, 1999; Ni et al, 1996; Wu et al, 1998b, W.M.A. Niessen, 2002). Before the chemical reaction, a m/z value is selected in the first stage of the mass spectrometer (this ion is called the precursor or parent ion). Then the chemical reaction takes place, which generally involves collision with neutral gas molecules (a process called collision-induced dissociation or CID). Mostly, Helium or Argon are used as collision gas. This reaction may take place in an intermediate zone (collision cell) between the two mass stages of the mass spectrometer. By this reaction, decomposition of the precursor ion may yield in various product ions (these are called daughter or product ions). The charged fragments can then be dectected by the second stage of the mass spectrometer. MS-MS can be done in two modes: Firstly, MS-MS "in space", i.e., the two mass analyzers can be separated in space, e.g. by a QTOF (quadrupole - time of flight) instrument. Second, MS-MS "in time", i.e., the different steps in the process can take place in the same space, but separated in time, e.g. in an ion-trap instrument. An accurate description of CID processes has been described by W.M.A. Niessen (2006).

The applicability of tandem mass spectrometry for sequence identification of nucleic acid molecules can be looked up in the review articles of Limbach and Nordhoff et al. (Limbach, 1996; Nordhoff et al, 1996). CID is the most widely applied method to induce fragmentation in MS-MS. Based on the dissociation of the multiply charged anionic nucleotides, the method utilizes the concept of "bidirectional" sequencing from both termini under the assumption that the backbone of the oligonucleotide is dissociated sequentially along the chain. The resulting fragments respresent, when applied successfully, a sequence specific fragmentation pattern. One of the first reports on the fragmentation of RNA has been given by Cerny et al. (1987). The "bidirectional" concept according to present knowledge utilizes c series ions which construct a sequence from 5' → 3' direction and y series ions constructing a sequence from the 3' → 5' direction (Schürch et. al, 2002). Nevertheless other daughter ions can be formed that may complicate, support or enable the sequencing process. Due to the fact that fragmentation can occur at the phosphate, the sugar and at the base site, the interpretation of the resulting spectra is complicated and the method is limited to nucleic acids with less than 25 nucleotides (Alazard et al, 2002). This limitation can be attributed to various factors such as neutral loss (daughter ions that are not ionized can not be detected), detection limit issues or limited resolution of the detector. The collision energy also plays a critical role. Low collision energies produce fewer sequence related ions while higher collision energies may result in other ion series which complicate the data interpretation. In contrast to the CID of DNA, which has been investigated thoroughly within the last few years, the aspects of CID with RNA are still not fully resolved.

Because of the limitations of the "direct methods" for sequencing of nucleic acid molecules by MS, the following indirect methods have been developed and utilized to determine the sequence of nucleic acid molecules by mass spectrometry.

### Indirect mass spectrometric methods for sequencing

"Indirect mass spectrometric methods" for sequencing as preferably used herein means that the preparation of the nucleic acid molecules, from which the sequence should be determined, is performed before gas-phase ions of the sample are generated.

The indirect mass spectrometric methods for mass measurement as a tool to confirm a predicted nucleic acid molecule composition are not discussed herein. Further information is provided in the review of Limbach (Limbach, 1996).

The utility of any mass spectrometric sequencing method that relies on consecutive backbone cleavage depends on the formation of a mass ladder. The sequence information is obtained by determining the mass difference between successive peaks in the mass spectrum. In the case of oligodeoxynucleotides, the expected mass difference between successive peaks will correspond to the loss of: dC = 289.05, dT = 304.05, dA = 313.06, and dG = 329.05 (Exact massbased values). With oligoribonucleotides, the mass difference will be: C = 305.04, U = 306.03, A = 329.05, and dG = 345.05 (Exact mass-based values). Because the nucleic acid sequence determination methods rely on the mass measurements of successive n-mers, DNA molecules are easier to characterize than RNA molecules due to the relatively large differences in mass among the four DNA molecule residues. Due to the small mass difference between the ribonucleotide U and C of only one Dalton unit, the required accuracy for measurement is much higher to correctly distinguish between U and C. Mass ladder methods have one distinct advantage for sequence determination: the difference in two mass measurements that results in the desired information gives the identy of the nucleotide residue (Limbach, 1996).

*Analysis of nucleic acid molecule ladders after nuclease digestion.* The DNA or RNA molecule fragments are generated by hydrolysis of the nucleotides using a 5'-->3' phophosdiesterase and/or a 3' --> 5' phosphodiesterase. Normally a combination of the two is used to identify all the nucleotides. The truncated and/or cleaved nucleic acid molecules are analyzed by MALDI-TOF-MS or ESI-MS. Enhanced resolution to up to 35 nucleotides was achieved (Alazard et al, 2002) by improved techniques such as delayed extraction, sample cleanup, optimisation of enzyme, buffer pH and matrices (Bentzley et al, 1998; Bentzley et al, 1996; Faulstich et al, 1997; Glover et al, 1995; Kirpekar et al, 1994; Owens et al, 1998; Pieles et al, 1993; Schuette et al, 1995; Smirnov et al, 1996; Wu & Aboleneen, 2001; Wu et al, 1998a).

However, enzymatic sequencing is restricted to nucleic acid molecules that comprise no modification of their sugar backbone. Moreover some nucleases are single-strand specific. Some nucleic acid molecules especially long oligonucleotides such as aptamers exhibit double-stranded sequence sections leading to intra- and/or intermolecular structures which are poorer substrates for nuclease digestions.

*Analysis of nucleic acid molecule ladders after chemical digestion.* Beside exonucleases, chemical agents can be used for the controlled degradation of the nucleic acid molecules before mass spectrometric measurement. Chemical agents are especially needed if the nucleic acid molecule is modified, whereby the modification is specifically chosen in order to increase the stability of the nucleic acid molecules towards enzymatic digestion. Comparable with enzymatic digestion methods, chemical cleavage reactions are classified by their specificity for DNA and RNA molecules and their specificity for the different nucleobases. Base specific reactions for RNA and DNA molecules, that can be used before MS analysis, are described by Peattie and Maxam-Gilbert (Maxam & Gilbert, 1977; Peattie, 1979). However, non-specific (random) cleavage of the phosphodiester backbone of DNA molecules is done by acid hydrolysis (Shapiro & Danzig, 1972); non-specific (random) cleavage of the phosphodiester backbone of RNA molecules is done by base hydrolysis, with acid (e.g. formic acid), (Farand & Beverly, 2008) and polyamines at physiological pH (Komiyama & Yoshinari, 1997)

The generation of a mass ladder of a nucleic acid molecule for sequence determination using non-specific (random) cleavage of the phosphodiester backbone of DNA or RNA molecules can be complicated because any linkage site can be potentially cleaved by the chemcial agent. The nucleobase specfic chemical cleavage can also randomly occur at every position in the nucleic acid molecule where the respective nucleobase is. If a single cleavage site is generated, then two specfic fragments occur: one from 5'-terminus and one from the 3'-terminus. If both fragments can be detected in the mass spectrum, more information is present than is needed for sequence determination of the nucleic acid molecule. These two ion series can be a source of confusion. The other source of confusion comes from the internal cleavages. As noted before, a single cleavage along the backbone of the nucleic acid molecule generates two fragments - one fragment originates from the 5'-terminus, and the other fragment originates from the 3'-terminus. One more cleavage reaction along the backbone of the nucleic acid molecule generates three fragments: the first fragment is the 5'-terminus, the second fragment is the 3'-terminus and the third fragment will not comprise either terminus. Because the 5'- or 3'- terminus is used as a reference point, the fragments comprising the 5'-or 3'-terminus can be used for the construction of the mass ladder. In contrast the internal fragment can not be used for the construction of the mass ladder. Additionally, in the case of mass identity of the internal fragment and one of the terminal fragments, an incorrect interpretation may result. Furthermore, the presence of these internal fragments can lead to ion suppression of the desired 5'- or 3'-terminus fragments. Therefore the reaction conditions for the chemical digestion have to be carefully adjusted to single cleavage conditions (Limbach, 1996) although with random cleavage reactions, the ability to control this is limited.

Nucleic acid sequencing can be done by chemical cleavage reactions followed by analysis of the cleavage reactions via mass spectrometry (Farand & Beverly, 2008). Farrand and Berverly used a highly modified nucleic acid molecule containing a mixture of 2'deoxyribonucleotides, 2'-fluororibonucleotides, 2'-O-methylribonucleotides, abasic ribonucleotides and ribonucleotides, whereby formic acid was used to degrade ribonucleotides; sodium hydroxide was used to degrade ribonucleotides, 2'-fluoro ribonucleotides, 2'-O-methyl ribonucleotides and abasic residues; piperidine was used for ribonucleotides, 2'-fluoro ribonucleotides and deoxy-guanosine. Base specific reactions (as reported by Peattie Maxam & Gilbert) were also used to obtain fragments. During accurate mass analysis, short fragments (1-3 nucleotides in length) containing the last nucleotides of the strand are poorly retained by LC-MS. Therefore, tandem mass spectrometry was needed to confirm the final two or three nucleotides containing the 3'-terminal hydroxyl (Farand & Beverly, 2008).

*Analysis of nucleic acid molecule ladders after after endouclease digestion and chemical digestion.* The small mass difference between U and C (one Da) in an RNA molecule makes unambiguous (as shown for DNA molecules) assignment difficult using partial exonuclease digestion followed by MALDI-TOF. Exonuclease digestion results in ambiguous sequence assignments where the pyrimidine bases C and U can not distinguished from each other. Therefore Tolson and Nicholson develeoped a method combining sequence specific endonucleases and chemical methods to resolve these sequence ambiguities of RNA molecules (Tolson & Nicholson, 1998). Because the specificity of the enzymatic reactions was not as expected, the authors used hydrazine/analine treatment of RNA resulting characteristic fragments formed by the scission at U's. (Tolson & Nicholson, 1998).

*Analysis of nucleic acid molecule ladders after Sanger dideoxy termination reactions.* The Sanger sequencing strategy allows assembling the sequence information by analysis of the nested fragments obtained by nucleobase-specific chain termination via their different molecular masses using mass spectrometry such as MALDI or ESI mass spectrometry. The method was improved by increasing amounts of termination groups using cycle sequencing, optimizing reaction conditions, purifying extension products, elimination salt adducts and utilizing delayed extraction technology for better resolution (Fu et al, 1998; Harksen et al, 1999; Kirpekar et al, 1998; Koster et al, 1996; Monforte & Becker, 1997; Mouradian et al, 1996; Roskey et al, 1996; Shaler et al, 1995; Taranenko et al, 1998; Taranenko et al, 1997). Using this MALDI-TOF sequencing method, sequences of DNA molecules consisting of more than 100 nucleotides could be analysed (Taranenko et al, 1998).

Alternatively, as shown in US 5,547,835 the one of the methods as described supra has been coupled with a solid-phase sequencing approach in which the template is labeled with biotin and bound to streptavidin-coated magnetic beads. Throughput can be increased by introducing mass modifications in the oligonucleotide primer, chain-terminating nucleoside triphosphates and/or in the chain- elongating nucleoside triphosphates, as well as using integrated tag sequences that allow multiplexing by hybridization of tag specific probes with mass differentiated molecular weights. However, all of these "Sanger-based" sequencing methods require either some prior knowledge of the target sequence or introduction of a known sequence to serve as the primer-binding site.

International patent application WO 2004/097369 discloses methods and systems, particularly mass spectrometric methods and systems, for the analysis and sequencing of biomolecules, particularly nucleic acids, by fragmentation.

Ohara R et al. (Ohara R et al., DNA Research 2, 123-128 (1995)) is related to a solid-phase DNA sequencing method which uses streptavidin-coated magnetic beads.

Isola N R et al., (Isola N R et al., Anal Chem. 1999, 71, 2266-2269) is related to chemical cleavage sequencing of DNA using matrix-assisted laser desorption/ionization time-of-flight mass spectrometry.

The problem underlying the present invention was thus to provide a method for determining the nucleotide sequence of a nucleic acid molecule, particularly in case nucleic acid molecule comprises or consists of L-nucleotides.

A further problem underlying the present inventoin was to provide a method for determining the nucleotide sequence of a nucleic acid molecule, particularly of a nucleic acid molecule comprising or consisting of L-nucleotides, whereby such method overcomes or avoids at least some of the disadvantages of the methods of the prior art.

This problem is solved by the subject matter of the independent claims. Preferred embodiments may be takne form the dependent claims.

More specifically, this problem underlying the present invention is solved in a first aspect, which is also the first embodiment of the first aspect by a method for determining the nucleotide sequence of a nucleic acid molecule comprising the following steps:
a) providing a plurality of molecules of the nucleic acid molecule having at least one modification;
b) cleaving at random the plurality of modified nucleic acid molecules thus providing modified nucleic acid molecule fragments and non-modified nucleic acid molecule fragments, wherein the cleaving provides for a mixture of fragments and wherein such mixture of fragments comprises all possible nucleotide sequence fragments of the nucleic acid molecule;
c) separating the modified nucleic acid molecule fragments from the non-modified nucleic acid molecule fragments;
d) separating or resolving the modified nucleic acid molecule fragments according to their length, mass and/or charge, whereby such separating or resolving generates a pattern of modified nucleic acid fragments; and
e) optionally visualizing the pattern of modified nucleic acid fragments;
wherein the modification is present at the 5' end of the nucleic acid molecule fragments and the smallest modified nucleic acid molecule fragment comprises the terminal 5' nucleotide of the full-length nucleic acid molecule or wherein the modification is present at the 3' end of the nucleic acid molecule fragments and the smallest modified nucleic acid molecule fragment comprises the terminal 3' nucleotide of the full-length nucleic acid molecule.

In a second embodiment of the first aspect which is also an embodiment of the first embodiment of the first aspect the method further comprises the step of
f) deducing from the pattern of modified nucleic acid fragments the nucleotide sequence of the nucleic acid molecule.

In a third embodiment of the first aspect which is also an embodiment of the first and second embodiment of the first aspect the the individual nucleic acid molecule of the plurality of molecules has at least one modification at the 5' end, at the 3' end or within the nucleotide sequence of the nucleic acid molecule the nucleotide sequence of which is to be determined.

In a fourth embodiment of the first aspect which is also an embodiment of the first, second and third embodiment of the first aspect the the cleaving is carried out by chemical cleaving, enzymatic cleaving, cleaving by heat and/or cleaving by use of a divalent cation.

In a fifth embodiment of the first aspect which is also an embodiment of the first, second, third and fourth embodiment of the first aspect the the cleaving is a chemical cleaving, preferably a nucleotide unspecific cleaving.

In a sixth embodiment of the first aspect which is also an embodiment of the first, second, third, fourth and fifth embodiment of the first aspect the cleaving is a limited cleaving.

In a seventh embodiment of the first aspect which is also an embodiment of the first, second, third, fourth, fifth and sixth embodiment of the first aspect cleaving is a limited random cleaving, preferably a limited chemical random cleaving.

In an eighth embodiment of the first aspect which is also an embodiment of the first, second, third, fourth, fifth, sixth and seventh embodiment of the first aspect the step of cleaving provides for a mixture of modified fragments, whereby such mixture of modified fragments comprises all possible nucleotide sequence fragments of the nucleic acid molecule.

In a ninth embodiment of the first aspect which is also an embodiment of the eighth embodiment of the first aspect the the mixture comprises a modified full length form of the nucleic acid molecule the nucleotide sequence of which is to be determined.

In a tenth embodiment of the first aspect which is also an embodiment of the first, second, third, fourth, fifth, sixth, seventh, eights and ninth embodiment of the first aspect the modified nucleic acid molecule fragments are separated from the non-modified nucleic acid molecule fragments through the interaction of the modification with an interaction partner, whereby such interaction partner is linked to a support.

In an eleventh embodiment of the first aspect which is also an embodiment of the tenth embodiment of the first aspect the support is a solid support.

In a 12^{th} embodiment of the first aspect which is also an embodiment of the tenth and eleventh embodiment of the first aspect the non-modified nucleic acid molecule fragments are removed from the modified nucleic acid molecule fragments interacting with the interaction partner, preferably by washing.

In a 13^{th} embodiment of the first aspect which is also an embodiment of the tenth, eleventh and 12^{th} embodiment of the first aspect the modified nucleic acid molecule fragments are released from the support, preferably by release of the modification from the interaction partner, by release from the interaction partner from the support or by cleaving the modification or a part or moiety thereof from the nucleic acid molecule fragments.

In a 14^{th} embodiment of the first aspect which is also an embodiment of the first, second, third, fourth, fifth, sixth, seventh, eighth and ninth embodiment of the first aspect the modified nucleic acid molecule fragments are separated from the non-modified nucleic acid molecule by separation due to mass discrimination, size discrimination, hydrophobicity discrimination, charge discrimination, ionic discrimination, hydrogen bonding discrimination and or liquid phase mediated extraction, whereby preferably the non-labeled nucleic acid molecule fragments are removed.

In a 15^{th} embodiment of the first aspect which is also an embodiment of any one of the first to the 14^{th} embodiment of the first aspect the pattern of modified nucleic acid fragments comprises a ladder of modified nucleic acid fragments.

In a 16^{th} embodiment of the first aspect which is also an embodiment of any one of the first to the 15^{th} embodiment of the first aspect the pattern of modified nucleic acid fragments is generated by mass spectrometry and preferably the nucleic sequence of the nucleic acid molecule is deduced.

In a 17^{th} embodiment of the first aspect which is also an embodiment of any one of the first to the 15^{th} embodiment of the first aspect the pattern of modified nucleic acid fragments is generated and the masses of the individual fragments are determined by mass spectrometry and preferably the nucleic sequence of the nucleic acid molecule is deduced.

In an 18^{th} embodiment of the first aspect which is also an embodiment of any one of the first to the 17^{th} embodiment of the first aspect the nucleotide sequence of the nucleic acid molecule is not known.

In a 19^{th} embodiment of the first aspect which is also an embodiment of any one of the first to the 18^{th} embodiment of the first aspect the step of deducing from the pattern of modified nucleic acid fragments the nucleotide sequence of the nucleic acid molecule comprises the following steps:
fa) determining the mass and/or nucleotide sequence of the smallest modified nucleic acid molecule fragment n + x, with x = 0;
fb) determining the mass of the modified nucleic acid molecule fragment n + x with x = 1 which differs from the mass of the smallest modified nucleic acid molecule fragment n + x with x = 0 by one nucleotide;
fc) determining the mass difference between the mass of the modified nucleic acid molecule fragment n + x with x = 1 and the mass of the smallest modified nucleic acid molecule fragment n + x with x = 0;
fd) attributing the mass difference to a distinct nucleotide species and generating the sequence of modified nucleic acid molecule fragment n + x with x = 1 by adding the distinct nucleotide species to the sequence of the smallest modified nucleic acid molecule fragment n + x with x = 0.

In a 20^{th} embodiment of the first aspect which is also an embodiment of the 19^{th} embodiment of the first aspect the steps fb) to fd) are repeated, whereby for each repetition x is increased by an addend of 1 and x is 2 for the first repetition and wherein in step fb) the mass of the modified nucleic acid molecule fragment n + x which differs from the mass of the modified nucleic acid molecule fragment n + (x - 1) by one nucleotide is determined, in step fc) the mass difference between the mass of the modified nucleic acid molecule fragment n + x and the mass of the modified nucleic acid molecule fragment n + (x - 1) is determined and in step fd) the mass difference is attributed to a distinct nucleotide species and the sequence of the modified nucleic acid molecule fragment n + x is generated by adding the distinct nucleotide species to the sequence of the modified nucleic acid molecule fragment n + (x - 1).

In a 21^{st} embodiment of the first aspect which is also an embodiment of any 20^{th} embodiment of the first aspect the m^{th} repetition of steps fb) to fd) x is as follows: x = m+1.

In a 22^{nd} embodiment of the first aspect which is also an embodiment of any one of the first to the 17^{th} embodiment of the first aspect the nucleotide sequence of the nucleic acid molecule is known and, preferably, the method is for confirming the nucleotide sequence of a nucleic acid molecule.

In a 23^{rd} embodiment of the first aspect which is also an embodiment of the 22^{rd} embodiment of the first aspect the step of deducing from the pattern of modified nucleic acid fragments the nucleotide sequence of the nucleic acid molecule comprises the following steps:
fa) determining the mass of the modified nucleic acid molecule fragment n + x with x = 1 which differs from the mass of the smallest modified nucleic acid molecule fragment n + x with x = 0 by one nucleotide;
fb) determining the mass difference between the mass of the modified nucleic acid molecule fragment n + x with x = 1 and the mass of the smallest modified nucleic acid molecule fragment n + x with x = 0;
fc) attributing the mass difference to a distinct nucleotide species and generating the sequence of the modified nucleic acid molecule fragment n + x with x = 1 by adding the distinct nucleotide species to the sequence of the smallest modified nucleic acid molecule fragment n + x with x = 0.

In a 24^{th} embodiment of the first aspect which is also an embodiment of the 23^{rd} embodiment of the first aspect the steps fa) to fc) are repeated, whereby for each repetition x is increased by an addend of 1 and x is 2 for the first repetition and wherein in step fa) the mass of the modified nucleic acid molecule fragment n + x which differs from the mass of the modified nucleic acid molecule fragment n + (x-1) by one nucleotide is determined, in step fb) the mass difference between the mass of the modified nucleic acid molecule fragment n + x and the mass of the modified nucleic acid molecule fragment n + (x-1) is determined and in step fc) the mass difference is attributed to a distinct nucleotide species and the sequence of the modified nucleic acid molecule fragment n + x is generated by adding the distinct nucleotide species to the sequence of the modified nucleic acid molecule fragment n + (x-1).

In a 25^{th} embodiment of the first aspect which is also an embodiment of the 24^{th} embodiment of the first aspect, for the m^{th} repetition of steps fa) to fc) x is as follows: x = m + 1.

In a 26^{th} embodiment of the first aspect which is also an embodiment of the 22^{nd}, 23^{rd}, 24^{th} and 25^{th} embodiment of the first aspect the mass and/or the nucleotide sequence of the smallest modified nucleic acid molecule fragment n + x with x = 0 is known.

In a 27^{th} embodiment of the first aspect which is also an embodiment of the 22^{nd} embodiment of the first aspect the step of deducing from the pattern of modified nucleic acid fragments the nucleotide sequence of the nucleic acid molecule comprises the following steps:
fa) determining the mass of the modified nucleic acid molecule fragment n + x with x = 1 which differs from the mass of the smallest modified nucleic acid molecule fragment n + x with x = 0 by one nucleotide;
fb) attributing the mass of the modified nucleic acid molecule fragment n + x with x = 1 to the calculated mass of the nucleic acid molecule fragment n + x with x = 1 of the nucleic acid molecule whose nucleotide sequence is known and generating the sequence of the modified nucleic acid molecule fragment n + x with x = 1 by adding the distinct nucleotide species to the sequence of the smallest modified nucleic acid molecule fragment n + x with x = 0.

In a 28^{th} embodiment of the first aspect which is also an embodiment of the 27^{th} embodiment of the first aspect steps fa) to fb) are repeated, whereby for each repetition x is increased by an addend of 1 and x is 2 for the first repetition and wherein in step fa) the mass of the modified nucleic acid molecule fragment n + x which differs from the mass of the modified nucleic acid molecule fragment n + (x-1) by one nucleotide is determined, and in step fb) the mass of the modified nucleic acid molecule fragment n + x with x = 1 is attributed to the calculated mass of the nucleic acid molecule fragment n + x with x = 1 of the nucleic acid molecule whose nucleotide sequence is known and the modified nucleic acid molecule sequence of fragment n + x is generated by adding the distinct nucleotide species to the sequence of the modified nucleic acid molecule fragment n + (x-1).

In a 29^{th} embodiment of the first aspect which is also an embodiment of the 28^{th} embodiment of the first aspect, for the m^{th} repetition of steps fa) to fc) x is as follows: x = m + 1.

In a 30^{th} embodiment of the first aspect which is also an embodiment of any one of the first to the 29^{th} embodiment of the first aspect the modification is a unipartite modification comprising one moiety.

In a 31^{st} embodiment of the first aspect which is also an embodiment of the 30^{th} embodiment of the first aspect the moiety is used in separating the modified nucleic acid molecule fragments from the non-modified nucleic acid molecules.

In a 32^{nd} embodiment of the first aspect which is also an embodiment of the 31^{st} embodiment of the first aspect the moiety is used in separating or resolving the modified nucleic acid molecule fragments in the generation of the pattern.

In a 33^{rd} embodiment of the first aspect which is also an embodiment of any one of the first to the 29^{th} embodiment of the first aspect the modification is a multipartite modification comprising at least a first moiety and a second moiety, whereby optionally the at least first and second moiety are linked through a linker.

In a 34^{th} embodiment of the first aspect which is also an embodiment of the 33^{rd} embodiment of the first aspect the first moiety is used in separating the modified nucleic acid molecule fragments from the non-modified nucleic acid molecules, and the second moiety is used in separating or resolving the modified nucleic acid molecule fragments in the generation of the pattern.

In a 35^{th} embodiment of the first aspect which is also an embodiment of any one of the 30^{th} to the 34^{th} embodiment of the first aspect the moiety which is used in separating the modified nucleic acid molecule fragments from the non-modified nucleic acid molecules comprises a ligand to an interaction partner, whereby such interaction partner is present on a support, preferably linked to such support, and the interaction between the ligand and the interaction partner mediates immobilization of the modified nucleic acid molecule fragments onto the support.

In a 36^{th} embodiment of the first aspect which is also an embodiment of 35^{th} embodiment of the first aspect the immobilization is selected from the group comprising chemical immobilization, affinity immobilization, magnetic immobilization.

In a 37^{th} embodiment of the first aspect which is also an embodiment of the 36^{th} embodiment of the first aspect the immobilization is affinity immobilization.

In a 38^{th} embodiment of the first aspect which is also an embodiment of the 37^{th} embodiment of the first aspect the interaction which mediates the immobilization of the nucleic acid molecule and the nucleic acid molecule fragments onto the support is selected from the group comprising biotin-avidin interaction, biotin-neutravidin interaction, biotin-streptavidin interaction, antigen-antibody interaction, interaction of two oligonucleotides, whereby the nucleic acid molecules consist of DNA, RNA, LNA, PNA or combinations thereof, interaction of calmodulin and calmodulin binding peptide, interaction of albumin and Cibracon Blue, interaction of a metal-chelator agent and metal-chelating support.

In a 39^{th} embodiment of the first aspect which is also an embodiment of any one of the 30^{th} to the 38^{th} embodiment of the first aspect the moiety which is used in separating the modified nucleic acid molecule fragments from the non-modified nucleic acid molecules is selected from the group comprising biotin, oligonucleotides, calmodulin binding peptides, albumins and metal-chelator agents.

In a 40^{th} embodiment of the first aspect which is also an embodiment of any one of the first to the 39^{th} embodiment of the first aspect the modified nucleic acid molecule fragments are separated form the non-modified nucleic acid molecules by a means selected from the group comprising filtration, dialysis, chromatography, magnetic fields, centrifugation and precipitation.

In a 41^{st} embodiment of the first aspect which is also an embodiment of the 40^{th} embodiment of the first aspect chromatography is size exclusion chromatography, wherein the modified nucleic acid fragments are separated from the non-modified nucleic acid molecules according to their size or due to the increased size of the modified fragments imparted to them by the modification.

In a 42^{nd} embodiment of the first aspect which is also an embodiment of any one of the 30^{th} to the 41^{st} embodiment of the first aspect the moiety which is used in separating or resolving the modified nucleic acid molecule fragments in the generation of the pattern is selected from mass tags or lipophilic tags.

In a 43^{th} embodiment of the first aspect which is also an embodiment of any one of the first to the 42^{nd} embodiment of the first aspect the modified nucleic acid molecule fragments are separated or resolved by a method for mass or size discrimination which is preferably selected from the group comprising filtration and dialysis and chromatography and mass spectrometry, preferably such method is MS, LCMS or ESI MS.

In a 44^{th} embodiment of the first aspect which is also an embodiment of any one of the first to the 43^{th} embodiment of the first aspect the modified nucleic acid molecule fragments are separated or resolved by a method based on hydrophobic interaction which is preferably RP-HPLC.

In a 45^{th} embodiment of the first aspect which is also an embodiment of any one of the 33^{rd} to the 44^{th} embodiment of the first aspect the linker is a hydrophobic linker.

In a 46^{th} embodiment of the first aspect which is also an embodiment of any one of the 33^{rd} to the 45^{th} embodiment of the first aspect the linker is a cleavable linker.

In a 47^{th} embodiment of the first aspect which is also an embodiment of the 46^{th} embodiment of the first aspect the linker is a selectively cleavable linker, more preferably the selectively cleavable linker is enzymatically cleavable, chemically cleavable, photocleavable or thermocleavable.

In a 48^{th} embodiment of the first aspect which is also an embodiment of any one of the first to the 47^{th} embodiment of the first aspect the nucleic acid molecule is selected from the group of RNA molecules, DNA molecules, nucleotide-modified RNA molecules and nucleotide-modified DNA molecules, PNA, LNA and combinations thereof, preferably RNA molecules, DNA molecules, nucleotide-modified RNA molecules, nucleotide-modified DNA molecules and nucleic acid molecules containing both deoxyribonucleotides and ribonucleotides.

In a 49^{th} embodiment of the first aspect which is also an embodiment of any one of the first to the 48^{th} embodiment of the first aspect the nucleic acid molecule is selected from the group consisting of aptamers, Spiegelmers, ribozymes, Spiegelzymes, antisense molecules, siRNA molecules and decoy molecules, preferably Spiegelmers.

In a 50^{th} embodiment of the first aspect which is also an embodiment of any one of the first to the 49^{th} embodiment of the first aspect the nucleic acid molecule is an RNA molecule and/or a nucleotide-modified RNA molecule.

In a 51^{st} embodiment of the first aspect which is also an embodiment of the 50^{th} embodiment of the first aspect the cleaving is a chemical cleaving of the RNA molecule and/or the nucleotide-modified RNA molecule which is done by alkaline hydrolysis, amines, or polyamines.

In a 52^{nd} embodiment of the first aspect which is also an embodiment of the 50^{th} embodiment of the first aspect the cleaving is an enzymatic cleaving of the RNA molecule and/or the nucleotide-modified RNA molecule which is done by use of nucleases, preferably ribonuclease, and/or nucleic-acid based enzymes, preferably nucleic acid based enzymes.

In a 53^{rd} embodiment of the first aspect which is also an embodiment of the 50^{th} embodiment of the first aspect the cleaving is a cleaving by heat of the RNA molecule and/or the nucleotide-modified RNA molecule.

In a 54^{th} embodiment of the first aspect which is also an embodiment of the 50^{th} embodiment of the first aspect the cleaving is a cleaving of the RNA molecule and/or the nucleotide-modified RNA molecule by use of divalent cations.

In a 55^{th} embodiment of the first aspect which is also an embodiment of any one of the first to the 49^{th} embodiment of the first aspect the nucleic acid is a DNA molecule and/or a nucleotide-modified DNA molecule.

In a 56^{th} embodiment of the first aspect which is also an embodiment of the 55^{th} embodiment of the first aspect the cleaving is a chemical cleaving of the DNA molecule and/or the nucleotide-modified DNA molecule which is done by use of acid hydrolysis.

In a 57^{th} embodiment of the first aspect which is also an embodiment of the 55^{th} embodiment of the first aspect the cleaving is an enzymatic cleaving of the DNA molecule and/or the nucleotide-modified DNA molecule which is done by use of nucleases, preferably deoxyribonuclease, and/or nucleic-acid based enzymes, preferably nucleic acid based enzymes.

In a 58^{th} embodiment of the first aspect which is also an embodiment of any one of the 16^{th} to the 57^{th} embodiment of the first aspect mass spectrometry is selected from the group comprising direct mass spectrometry, LC-MS and MS/MS.

In a 59^{th} embodiment of the first aspect which is also an embodiment of any one of the first to the 58^{th} embodiment of the first aspect a specific mass fingerprint of a nucleic acid molecule is determined.

In a 60^{th} embodiment of the first aspect which is also an embodiment the 59^{th} embodiment of the first aspect the specific mass fingerprint is used for identifying and/or quality control for a nucleic acid molecule.

In a 61^{st} embodiment of the first aspect which is also an embodiment of any one of the first to the 60^{th} embodiment of the first aspect the at least one modification of the nucleic acid molecule or of the plurality of molecules of the nucleic acid molecule is added to the 5' end or the 3' end of the nucleic acid molecule, prior to step a) or b).

In a 62^{nd} embodiment of the first aspect which is also an embodiment of any one of the first to the 61^{st} embodiment of the first aspect the nucleic acid molecule or the plurality of molecules of the nucleic acid molecule comprises(s) a non-nucleic acid moiety.

In a 63^{rd} embodiment of the first aspect which is also an embodiment of the 62^{nd} embodiment of the first aspect the non-nucleic acid moiety is removed from the nucleic acid molecule or the plurality of molecules of the nucleic acid molecule prior to step a) or b).

In a 64^{th} embodiment of the first aspect which is also an embodiment of the 63^{rd} embodiment of the first aspect, in a first step the non-nucleic acid moiety is removed from the nucleic acid molecule or the plurality of molecules of the nucleic acid molecule and in a second step the modification of the nucleic acid molecule or of the plurality of molecules of the nucleic acid molecule is added to the 5' end, the 3' end or a nucleotide within the nucleotide sequence of the nucleic acid molecule or of the plurality of molecules of the nucleic acid molecule prior to step a) or b).

Also disclosed is, as a second aspect, a method for determining the nucleotide sequence of a nucleic acid molecule comprising the following steps:
a) providing a plurality of molecules of the nucleic acid molecule having at least one modification;
b) cleaving at random the plurality of modified nucleic acid molecules thus providing modified nucleic acid molecule fragments;
c) separating or resolving the modified nucleic acid molecule fragments according to their length, mass and/or charge, wherein such separating or resolving generates a pattern of modified nucleic acid fragments; and
d) optionally visualizing the pattern of modified nucleic acid fragments.

In a 2^{nd} embodiment of the second aspect which is also an embodiment of the first embodiment of the second aspect a reaction mixture which is obtained after step b) or c), contains one or more nucleic acid molecules or fragments thereof not having said at least one modification.

In a 3^{rd} embodiment of the second aspect which is also an embodiment of the first and second embodiment of the second aspect the visualizing of the pattern of the modified nucleic acid fragments makes use of the at least one modification, preferably the modification allows to discriminate between a nucleic acid molecule having said modification and a nucleic acid molecule not having said modification.

In a 4^{th} embodiment of the second aspect which is also an embodiment of the first, second and third embodiment of the second aspect the modification is selected from the group comprising mass tags, moieties with significantly more UV absorbance at a given wavelength than the nucleic acid molecule lypophilic moieties, polymers with defined molecular mass, radiolabels and moieties imparting an altered ion mobility

In a 5^{th} embodiment of the second aspect which is also an embodiments of the fourth embodiment of the second aspect the moiety with significantly more UV absorbance at a given wavelength than the nucleic acid molecule is selected from the group comprising chromophores, dyes and fluorescence labels.

In a 6^{th} embodiment of the second aspect which is also an embodiment of any one of the first to the 5^{th} embodiment of the second aspect the method further comprises the step of
e) deducing from the pattern of modified nucleic acid fragments the nucleotide sequence of the nucleic acid molecule.

In a 7^{th} embodiment of the second aspect which is also an embodiment of any one of the first to the 6^{th} embodiment of the second aspect the individual nucleic acid molecule of the plurality of molecules has at least one modification at the 5' end, at the 3' end or within the nucleotide sequence of the nucleic acid molecule the nucleotide sequence of which is to be determined.

In an 8^{th} embodiment of the second aspect which is also an embodiment of any one of the first to the 7^{th} embodiment of the second aspect the cleaving is carried out by chemical cleaving, enzymatic cleaving, cleaving by heat and/or cleaving by use of a divalent cation.

In a 9^{th} embodiment of the second aspect which is also an embodiment of any one of the first to the 8^{th} embodiment of the second aspect the cleaving is a chemical cleaving, preferably a nucleotide unspecific cleaving.

In a 10^{th} embodiment of the second aspect which is also an embodiment of any one of the first to the 9^{th} embodiment of the second aspect the cleaving is a limited cleaving.

In an eleventh embodiment of the second aspect which is also an embodiment of any one of the first to the 10^{th} embodiment of the second aspect the cleaving is a limited random cleaving, preferably a limited chemical random cleaving.

In a 12^{th} embodiment of the second aspect which is also an embodiment of any one of the first to the 11^{th} embodiment of the second aspect the step of cleaving provides for a mixture of fragments, preferably modified fragments, whereby such mixture of fragments comprises all possible nucleotide sequence fragments of the nucleic acid molecule.

In a 13^{th} embodiment of the second aspect which is also an embodiment of the 12^{th} embodiment of the second aspect the mixture comprises a modified full length form of the nucleic acid molecule the nucleotide sequence of which is to be determined.

In a 14^{th} embodiment of the second aspect which is also an embodiment of any one of the first to the 13^{th} embodiment of the second aspect the pattern of modified nucleic acid fragments comprises a ladder of modified nucleic acid fragments.

In a 15^{th} embodiment of the second aspect which is also an embodiment of any one of the first to the 14^{th} embodiment of the second aspect the pattern of modified nucleic acid fragments is generated by mass spectrometry, preferably LC-MS, and preferably the nucleic sequence of the nucleic acid molecule is deduced.

In a 16^{th} embodiment of the second aspect which is also an embodiment of any one of the first to the 14^{th} embodiment of the second aspect the pattern of modified nucleic acid fragments is generated and the masses of the individual fragments are determined by mass spectrometry and preferably the nucleic sequence of the nucleic acid molecule is deduced.

In a 17^{th} embodiment of the second aspect which is also an embodiment of any one of the first to the 16^{th} embodiment of the second aspect the nucleotide sequence of the nucleic acid molecule is not known.

In an 18^{th} embodiment of the second aspect which is also an embodiment of any one of the first to the 17^{th} embodiment of the second aspect the step of deducing from the pattern of modified nucleic acid fragments the nucleotide sequence of the nucleic acid molecule comprises the following steps:
fa) determining the mass and/or nucleotide sequence of the smallest modified nucleic acid molecule fragment n + x, with x = 0;
fb) determining the mass of the modified nucleic acid molecule fragment n+x with x = 1 which differs from the mass of the smallest modified nucleic acid molecule fragment n+ x with x = 0 by one nucleotide;
fc) determining the mass difference between the mass of the modified nucleic acid molecule fragment n + x with x = 1 and the mass of the smallest modified nucleic acid molecule fragment n + x with x = 0;
fd) attributing the mass difference to a distinct nucleotide species and generating the sequence of the modified nucleic acid molecule fragment n + x with x = 1 by adding the distinct nucleotide species to the sequence of the smallest modified nucleic acid molecule fragment n + x with x = 0.

In a 19^{th} embodiment of the second aspect which is also an embodiment of the 18^{th} embodiment of the second aspect, steps fb) to fd) are repeated, whereby for each repetition x is increased by an addend of 1 and x is 2 for the first repetition and wherein in step fb) the mass of the modified nucleic acid molecule fragment n + x which differs from the mass of the modified nucleic acid molecule fragment n + (x-1) by one nucleotide is determined, in step fc) the mass difference between the mass of the modified nucleic acid molecule fragment n + x and the mass of the modified nucleic acid molecule fragment n + (x-1) is determined and in step fd) the mass difference is attributed to a distinct nucleotide species and the sequence of the modified nucleic acid molecule fragment n + x is generated by adding the distinct nucleotide species to the sequence of the modified nucleic acid molecule fragment n + (x-1).

In a 20^{th} embodiment of the second aspect which is also an embodiment of the 19^{th} embodiment of the second aspect, for the m^{th} repetition of steps fb) to fd) x is as follows: x = m+1.

In a 21^{st} embodiment of the second aspect which is also an embodiment of any one of the first to the 16^{th} embodiment of the second aspect the nucleotide sequence of the nucleic acid molecule is known and, preferably, the method is for confirming the nucleotide sequence of a nucleic acid molecule.

In a 22^{nd} embodiment of the second aspect which is also an embodiment of the 21^{st} embodiment of the second aspect the step of deducing from the pattern of modified nucleic acid fragments the nucleotide sequence of the nucleic acid molecule comprises the following steps:
fa) determining the mass of the modified nucleic acid molecule fragment n + x with x = 1 which differs from the mass of the smallest modified nucleic acid molecule fragment n + x with x = 0 by one nucleotide;
fb) determining the mass difference between the mass of the modified nucleic acid molecule fragment n + x with x = 1 and the mass of the smallest modified nucleic acid molecule fragment n + x with x = 0;
fc) attributing the mass difference to a distinct nucleotide species and generating the sequence of the modified nucleic acid molecule fragment n + x with x = 1 by adding the distinct nucleotide species to the sequence of the smallest modified nucleic acid molecule fragment n + x with x = 0.

In a 23^{rd} embodiment of the second aspect which is also an embodiment of the 22^{nd} embodiment of the second aspect, steps fa) to fc) are repeated, whereby for each repetition x is increased by an addend of 1 and x is 2 for the first repetition and wherein in step fa) the mass of the modified nucleic acid molecule fragment n+x which differs from the mass of the modified nucleic acid molecule fragment n + (x-1) by one nucleotide is determined, in step fb) the mass difference between the mass of the modified nucleic acid molecule fragment n + x and the mass of the modified nucleic acid molecule fragment n + (x-1) is determined and in step fc) the mass difference is attributed to a distinct nucleotide species and the sequence of fragment n + x is generated by adding the distinct nucleotide species to the sequence of the modified nucleic acid molecule fragment n + (x-1).

In a 24^{th} embodiment of the second aspect which is also an embodiment of the 23^{rd} embodiment of the second aspect, for the m^{th} repetition of steps fa) to fb) x is as follows: x = m+1.

In a 25^{th} embodiment of the second aspect which is also an embodiment of any one of the 21^{st} to the 24^{th} embodiment of the second aspect the mass and/or the nucleotide sequence of the smallest modified nucleic acid molecule fragment n + x with x = 0 is known.

In a 26^{th} embodiment of the second aspect-which is also an embodiment the 21^{st} embodiment of the second aspect the step of deducing from the pattern of modified nucleic acid fragments the nucleotide sequence of the nucleic acid molecule comprises the following steps:
fa) determining the mass of the modified nucleic acid molecule fragment n + x with x = 1 which differs from the mass of the smallest modified nucleic acid molecule fragment n + x with x = 0 by one nucleotide;
fb) attributing the mass of the modified nucleic acid molecule fragment n + x with x = 1 to the calculated mass of the nucleic acid molecule fragment n + x with x = I of the nucleic acid molecule whose nucleotide sequence is known and generating the sequence of the modified nucleic acid molecule fragment n + x with x = I by adding the distinct nucleotide species to the sequence of the smallest modified nucleic acid molecule fragment n + x with x = 0.

In a 27^{th} embodiment of the second aspect which is also an embodiment of the 26^{th} embodiment of the second aspect steps fa) to fb) are repeated, whereby for each repetition x is increased by an addend of 1 and x is 2 for the first repetition and wherein in step fa) the mass of the modified nucleic acid molecule fragment n + x which differs from the mass of the modified nucleic acid molecule fragment n + (x-1) by one nucleotide is determined, and in step fb) the mass of the modified nucleic acid molecule fragment n + x with x = 1 is attributed to the calculated mass of the nucleic acid molecule fragment n + x with x = 1 of the nucleic acid molecule whose nucleotide sequence is known and the modified nucleic acid molecule sequence of fragment n + x is generated by adding the distinct nucleotide species to the sequence of the modified nucleic acid molecule fragment n + (x-1).

In a 28^{th} embodiment of the second aspect which is also an embodiment of the 27^{th} embodiment of the second aspect, for the m^{th} repetition of steps fa) to fc) x is as follows: x = m + 1.

In a 29^{th} embodiment of the second aspect which is also an embodiment of any one of the 18^{th} to the 28^{th} embodiment of the second aspect the modification is present at the 5' end of the nucleic acid molecule fragments and the smallest modified nucleic acid molecule fragment comprises the terminal 5' nucleotide of the full-length nucleic acid molecule or wherein the modification is present at the 3' end of the nucleic acid molecule fragments and the smallest modified nucleic acid molecule fragment comprises the terminal 3' nucleotide of the full-length nucleic acid molecule.

In a 30^{th} embodiment of the second aspect which is also an embodiment of any one of the first to the 29^{th} embodiment of the second aspect the modification is used in separating or resolving the modified nucleic acid molecule fragments in the generation of the pattern.

In a 31^{st} embodiment of the second aspect which is also an embodiment of any one of the first to the 30^{th} embodiment of the second aspect the modification is a fluorescent label wose wavelength absorbance is different from the wavelength absorbance of the nucleobases of the nucleic acid molecules.

In a 32^{nd} embodiment of the second aspect which is also an embodiment of any one of the first to the 31^{st} embodiment of the second aspect the nucleic acid molecule is selected from the group of RNA molecules, DNA molecules, nucleotide-modified RNA molecules, nucleotide-modified DNA molecules, PNA, LNA and combinations thereof, preferably RNA molecules, DNA molecules, nucleotide-modified RNA molecules, nucleotide-modified DNA molecules and nucleic acid molecules containing both deoxyribonucleotides and ribonucleotides.

In a 33^{rd} embodiment of the second aspect which is also an embodiment of any one of the first to the 32^{nd} embodiment of the second aspect the nucleic acid molecule is selected from the group consisting of aptamers, Spiegelmers, ribozymes, Spiegelzymes, antisense molecules, siRNA molecules and decoy molecules, preferably Spiegelmers.

In a 34^{th} embodiment of the second aspect which is also an embodiment of any one of the first to the 33^{rd} embodiment of the second aspect the nucleic acid molecule is an RNA molecule and/or a nucleotide-modified RNA molecule.

In a 35^{th} embodiment of the second aspect which is also an embodiment of the 34^{th} embodiment of the second aspect the cleaving is a chemical cleaving of the RNA molecule and/or the nucleotide-modified RNA molecule and such cleaving is done by alkaline hydrolysis.

In a 36^{th} embodiment of the second aspect which is also an embodiment of the 34^{th} embodiment of the second aspect the cleaving is an enzymatic cleaving of the RNA molecule and/or the nucleotide-modified RNA molecule which is done by use of nucleases, preferably ribonuclease, and/or nucleic-acid based enzymes, preferably nucleic acid based enzymes.

In a 37^{th} embodiment of the second aspect which is also an embodiment of the 34^{th} is embodiment of the second aspect the cleaving is a cleaving by heat of the RNA molecule and/or the nucleotide-modified RNA molecule.

In a 38^{th} embodiment of the second aspect which is also an embodiment of the 34^{th} embodiment of the second aspect the cleaving is a cleaving of the RNA molecule and/or the modified RNA molecule by use of divalent cations, or a combination of cleaving by heat and a cleaving agent..

In a 39^{th} embodiment of the second aspect which is also an embodiment of any one of the first to the 33^{rd} embodiment of the second aspect the nucleic acid is a DNA molecule and/or a nucleotide-modified DNA molecule.

In a 40^{th} embodiment of the second aspect which is also an embodiment of the 39^{th} embodiment of the second aspect the cleaving is a chemical cleaving of the DNA molecule and/or the nucleotide-modified DNA molecule which is done by use of acid hydrolysis.

In a 41^{st} embodiment of the second aspect which is also an embodiment of the 39^{th} embodiment of the second aspect the cleaving is an enzymatic cleaving of the DNA molecule and/or the nucleotide-modified DNA molecule which is done by use of nucleases, preferably deoxyribonuclease, and/or nucleic-acid based enzymes, preferably nucleic acid based enzymes.

In a 42^{nd} embodiment of the second aspect which is also an embodiment of any one of the first to the 41^{st} embodiment of the second aspect, a specific mass fingerprint of a nucleic acid molecule is determined.

In a 43^{rd} embodiment of the second aspect which is also an embodiment of the 42^{th} embodiment of the second aspect the specific mass fingerprint is used for identifying and/or quality control for a nucleic acid molecule.

In a 44^{th} embodiment of the second aspect which is also an embodiment of any one of the first to the 43^{th} embodiment of the second aspect the at least one modification of the nucleic acid molecule or of the plurality of molecules of the nucleic acid molecule is added to the 5' end or the 3' end of the nucleic acid molecule, prior to step a) or b)..

In a 45^{th} embodiment of the second aspect which is also an embodiment of any one of the first to the 44^{th} embodiment of the second aspect the nucleic acid molecule or the plurality of molecules of the nucleic acid molecule comprises(s) a non-nucleic acid moiety.

In a 46^{th} embodiment of the second aspect which is also an embodiment of the 45^{th} embodiment of the second aspect the non-nucleic acid moiety is removed from the nucleic acid molecule or the plurality of molecules of the nucleic acid molecule prior to step a) or b). In a 47^{th} embodiment of the second aspect which is also an embodiment of the 46^{th} embodiment of the second aspect, in a first step the non-nucleic acid moiety is removed from the nucleic acid molecule or the plurality of molecules of the nucleic acid molecule and in a second step the modification of the nucleic acid molecule or of the plurality of molecules of the nucleic acid molecule is added to the 5' end, the 3' end or a nucleotide within the nucleotide sequence of the nucleic acid molecule or of the plurality of molecules of the nucleic acid molecule prior to step a) or b).

Also disclosed is, as a third aspect, a method for determining the nucleotide sequence of a nucleic acid molecule comprising the following steps:
a) providing a plurality of molecules of the nucleic acid molecule;
b) subjecting the plurality of molecules of the nucleic acid molecule to a nucleobase selective treatment, whereby one or several of the nucleobase species forming the nucleic acid molecule are selectively modified and whereby after such nucleobase selective treatment some of the selectively treatable nucleobases of the nucleic acid molecules are modified and some of the selectively treatable nucleotides or nucleobases of the nucleic acid molecules remain non-modified;
c) chemically cleaving the nucleic acid phosphate backbone selectively 3' to the modified nucleobases, whereby the nucleic acid phosphate backbone of not all of the modified nucleobases are cleaved;
d) analysing nucleic acid molecule fragments by LC-MS and/or LC-MS-MS; and
e) identifying nucleic acid molecule fragments in increasing order of size with an intact terminus and generating the sequence of the nucleic acid molecule therefrom, wherein, preferably, the nucleic acid molecule fragments have the same intact terminus, more preferably the same intact 3' terminus.

In a second embodiment of the third aspect which is also an embodiment of the first embodiment of the third aspect the nucleic acid molecule is selected from the group of RNA molecules, DNA molecules, nucleotide-modified RNA molecules and nucleotide-modified DNA molecules, PNA, LNA, nucleic acid molecules comprising both deoxyribonucleotides and ribonucleotides, and combinations thereof, preferably RNA molecules, DNA molecules, nucleotide-modified RNA molecules and nucleotide-modified DNA molecules

In a third embodiment of the third aspect which is also an embodiment of the first and second embodiment of the third aspect the nucleic acid molecule is selected from the group consisting of aptamers, Spiegelmers, ribozymes, Spiegelzymes, antisense molecules, siRNA molecules and decoy molecules, preferably Spiegelmers.

In a fourth embodiment of the third aspect which is also an embodiment of the first, second and third embodiment of the third aspect the nucleic acid molecule is an RNA molecule and/or a nucleotide-modified RNA molecule.

In a fifth embodiment of the third aspect which is also an embodiment of any one of the first to the fourth embodiment of the third aspect the selectively treatable nucleobase is selected from the group comprising guanosine, adenosine, cytidine, thymdine and uracil.

In a sixth embodiment of the third aspect which is also an embodiment of any one of the first to the fifth embodiment of the third aspect the nucleobase U is selectively treated with a combination of hydrazine, acetic acid and aniline leading to 5' phosphate appended 3' fragment and an aniline modified ribose 5' fragment.

In a seventh embodiment of the third aspect which is also an embodiment of the sixth embodiment of the third aspect the the 5' phosphate appended 3' fragment and the intact nucleic acid molecule are ionized more efficiently than aniline modified ribose 5' fragments in step d) of claim 113.

In an eighth embodiment of the third aspect which is also an embodiment the seventh embodiment of the third aspect the 5' phosphate appended 3' fragment is ionized more efficiently than aniline modified ribose 5' fragment in step d) of claim 113.

In a ninth embodiment of the third aspect which is also an embodiment of the eighth embodiment of the third aspect the 5' phosphate appended 3' fragments are used in step e) of claim 113.

In an embodiment of each and any embodiment of any of the first, second and third aspect of the instant invention the nucleic acid molecule comprises more than 25 nucleotides or nucleobases.

In an embodiment of each and any embodiment of any of the first, second and third aspect of the instant invention the nucleic acid molecule comprises more than 35 nucleotides or nucleobases.

In an embodiment of each and any embodiment of any of the first, second and third aspect of the instant invention the nucleic acid molecule comprises from 26 to 50 nucleobases, or from 36 to 50 nucleobases, preferably from 26 to 45 nucleobases or from 36 to 45 nucleobases. It will be acknowledged that the terms nucleobases and nucleotides may be used interchangeable in connection with the instant invention.

In an embodiment of each and any embodiment of any of the first, second and third aspect of the instant invention the aggregation of the nucleic acid molecules of the plurality of molecules of the nucleic acid molecule is reduced.

In an embodiment of each and any embodiment of any of the first, second and third aspect of the instant invention the aggregation is reduced by the addition of a chaotropic solution to any of steps a) to e), preferably any of steps a) and b).

The present inventors have surprisingly found that it is possible to deduce or determine the nucleotide sequence of a nucleic acid molecule by cleaving a plurality of said nucleic acid molecule at random in an incomplete manner and by resolving the mixture of thus generated fragments of said nucleic acid molecule into a pattern of fragments of nucleic acid molecules whereby from such pattern of fragments the nucleic acid sequence of said nucleic acid molecule can be deduced or determined. The mixture of the fragments typically also comprises modified fragments of the nucleic acid molecule, whereby said modified fragments of the nucleic acid molecule are also generated by said random and incomplete cleavage of the plurality of said nucleic acid molecule, typically generated from a or the plurality of said nucleic acid molecule, whereby said nucleic acid molecule comprises a modification. The pattern of fragments of the nucleic acid molecule as such is formed or displayed by the modified fragments of the nucleic acid molecule. In other words, the pattern based on which the nucleotide sequence is either directly or indirectly deduced, is a pattern of modified nucleic acid fragments. In connection with this method it is preferred that the incomplete and preferably random cleaving generates a representation of all possible fragments of said nucleic acid which differ from each other by a single nucleotide.

In connection with the instant application the terms fragments of the nucleic acid molecule and nucleic acid molecule fragments are used in an interchangeable manner if not explicitly indicated to the contrary.

Based on this principle, the instant invention encompasses three basic procedures. In a first procedure, as subject to the method of the invention according to the first aspect, the modified nucleic acid molecule fragments and the non-modified nucleic acid molecule fragments are separated. This separation provides for a mixture of modified nucleic acid molecule fragments which is subjected to the separating and/or resolving step which provides for the pattern of modified nucleic acid molecule fragments. In this first procedure the modification is, potentially among others, either directly or indirectly used for the separation of the modified nucleic acid molecule fragments from the non-modified nucleic acid molecules.

In a second procedure as subject to the method of the invention according to the second aspect, the modified nucleic acid molecule fragments and the non-modified nucleic acid molecule fragments are not separated after the cleavage step. Rather the mixture of modified nucleic acid molecule fragments and non-modified nucleic acid molecule fragments is subjected to the separating and/or resolving step which provides for the pattern of modified nucleic acid molecule fragments. In this second procedure the modification is, potentially among others, either directly or indirectly used in an addressing process. Such addressing process is a process which allows the targeting of the individual modified nucleic acid molecule fragments of the mixture. The targeting is typically such that after the separating step or the resolving step which provides for the pattern, only the modified nucleic acid molecule fragments are displayed, whereas the non-modified nucleic acid molecules are not displayed although they are still present in the mixture. Preferably such displaying is mediated by or caused by the at least one modification. Due to the targeting thus only the modified nucleic acid molecule fragments are factually subject to the further step(s) of the method according to the instant invention in the meaning.

In a third procedure as subject to the method of the invention according to the third aspect, the plurality of molecules of the nucleic acid molecule the nucleotide sequence of which is to be determined is subjected to a treatment. Basically, such treatment is modifying in a selective way one species of the nucleobases which form the nucleic acid molecule. For example, the treatment is such that only the Us of the nucleic acid molecule are - selectively - modified. However, it is essently that not all of the Us of a nucleic acid molecule are modified. However, if the plurality of molecules of the nucleic acid molecule is taken into consideration, statistically each of the selectively modified nucleobasis of such nucleic acid molecule is modified. Subsequently the thus modified nucleic acid molecules of the plurality of molecules of the nucleic acid molecule are cleaved, preferably chemically cleaved such that the backbone of the nucleic acid, preferbyl the nucleic acid phosphate backbone is cleaved in a selective matter in the 3' diretiocn of the individual modified nucleobase. By doing so, all possible fragments are generated which may be subject to an either direct or indirect analysis in terms of preferably their length. In a preferred embodiment this analysis is performed by means of LC-MS and/or LC-MS-MS.

It will be acknowledged that the potential features of the methods according to the present invention and more specifically in connecion with one the the three procedures and aspects of the present invention, respectively, which are outlined herein in connection with one of said three aspects may form part of any aspect and thus procedure of the invention and any method for determining the nucleotide sequence of a nucleic acid molecule of the invention as outlined herein.

As preferably used herein separation is the transformation, division or isolation of a mixture of substances into two or more distinct products. In certain embodiments, separation would involve transforming the mixture of 5', 3' and internal fragments into a mixture of just 5' or 3' fragments. In other embodiments it would involve dividing a mixture of 5' or 3' fragments into further divisions, such as individual components, as is done, for example, with LC where peaks represent individual fragments or small groups of fragments. In other embodiments it would involve isolating 5' or 3' fragments from a mixture of 5', 3' and internal fragments where by e.g. the LC would perform both the trasformation and division steps above. It will be acknowledged by a person skilled in the art that separation may not be absolute. Rather the separated product may still contain compounds which has also been contained in the starting material which has been subject ot the separation, although at a decreased level.

As preferable used herein resolution is the ability to distinguish, detect or display distinct products from a mixture of substances and/or one another. In certain embodiments, the resolution would distinguish/detect/display labeled fragments from non-labeled fragments. In other embodiments, it would be used to distinguish the labeled fragments from one another, e.g. mass spec, but also the LC to show fragment 1 at different retention time from fragment 2. Both embodiments can, in principle, be achieved simultaneously in the same step.

As preferably used herein "nucleic acid molecule" and "nucleic acid molecules" refer to polynucleotides or oligonucleotides such as deoxyribonucleic acid (abbr. DNA) and ribonucleic acid (abbr. RNA). Moreover, the term "a nucleic acid molecule" includes a plurality of nucleic acid molecules. The terms "nucleic acid molecule" and "nucleic acid molecules" should also be understood to include, as equivalents, variants and analogs of either RNA or DNA made from nucleotide analogs, single (sense or antisense) and double- stranded polynucleotides or oligonucleotides. Deoxyribonucleotides include deoxyadenosine, deoxycytidine, deoxyguanosine and deoxythymidine. Ribonucleotides include adenosine, cytidine, guanosine and uridine. Reference to a nucleic acid molecule as a "polynucleotide" is used in its broadest sense to mean two or more nucleotides or nucleotide analogs linked by a covalent bond, including single stranded or double stranded molecules. The term "oligonucleotide" also is used herein to mean two or more nucleotides or nucleotide analogs linked by a covalent bond, although as defined herein oligonucleotides comprise less one hundred nucleotides.

As used herein, the term nucleic acid molecule, in one embodiment, comprises both deoxyribonucleotides and ribonucleotides. This kind of nucleic acid molecule is also referred to as hybrid, hybrid nucleic acid molecule or chimeric nucleic acid molecule.

It will be acknowledged by a person skilled in the art that the sequencing of nucleic acid molecules in accordance with the methods of the invention as described herein can also be combined with other techniques to synthesise nucleic acid molecules that are hybrids that consist of RNA and any or all of the following: 2' functionalised RNA as 2'- O-methyl, 2'-amino, 2'-C-allyl, 2'-fluoro, 2'-O-allyl; DNA, LNA, combinations of D- and L- configured nucleic acid molecules, nucleotides with modifications at the phosphorous position. e.g. DNA+RNA: Alternating would get half the fragments with alkaline hydrolysis, but could use MS/MS techniques on the fragments that you do get to sequence the entire molecule. If it was a stretch of DNA then RNA, then the RNA could be sequenced, and MS/MS could be done on the DNA etc etc. It will also be acknowledged that, in the light of the instant invention many variations will immediately be evident to a person skilled in the art-

It will also be acknowledged by a person skilled in the art that the possibility remains to sequence nucleic acid molecules in accordance with the methods of the invention as described herein where the modification is not at the 3' or 5' terminus, but may be up to 25 nucleotides away from such terminus.This would be possible where the label was DNA-MOD-RNA for instance. Or if the modification was 5 nucleotides away from the end for instance, the smallest fragment that you could sequence would be an 11mer, with the rest of the 11mer being sequenced with MS/MS. Finally, it will also be acknowledged by a person skilled in the art that the modification can be on the nucleobase in this or any other embodiment.

Also, as preferanly used herein, the nucleic acid subject to the methods of the invention can comprises at least one LNA nucleotide. In an embodiment of the methods of the invention the nucleic acid consists of LNA nucleotides.

Also, as preferanly used herein, the nucleic acid subject to the methods of the invention can comprises at least one PNA nucleotide. In an embodiment of the methods of the invention the nucleic acid consists of PNA nucleotides.

The nucleic acid molecule is characterized in that all of the consecutive nucleotides forming the nucleic acid molecule are linked with or connected to each other by one or more than one covalent bond. More specifically, each of such nucleotides is linked with or connected to two other nucleotides, preferably through phosphodiester bonds or other bonds, forming a stretch of consecutive nucleotides. In such arrangement, however, the two terminal nucleotides, i.e. preferably the nucleotide at the 5' end and at the 3' end, are each linked to a single nucleotide only under the proviso that such arrangement is a linear and not a circular arrangement and thus a linear rather than a circular molecule.

In another embodiment of the present application the nucleic acid molecule comprises at least two groups of consecutive nucleotides, whereby within each group of consecutive nucleotides each nucleotide is linked with or connected to two other nucleotides, preferably through phosphodiester bonds or other bonds, forming a stretch of consecutive nucleotides. In such arrangement, however, the two terminal nucleotides, i.e. preferably the nucleotide at the 5' end and at the 3' end, are each linked to a single nucleotide only. In such embodiment, the two groups of consecutive nucleotides, however, are not linked with or connected to each other through a covalent bond which links one nucleotide of one group and one nucleotide of another or the other group through a covalent bond, preferably a covalent bond formed between a sugar moiety of one of said two nucleotides and a phosphor moiety of the other of said two nucleotides or nucleosides. In an alternative embodiment, the two groups of consecutive nucleotides, however, are linked with or connected to each other through a covalent bond which links one nucleotide of one group and one nucleotide of another or the other group through a covalent bond, preferably a covalent bond formed between a sugar moiety of one of said two nucleotides and a phosphor moiety of the other of said two nucleotides or nucleosides. Preferably, the at least two groups of consecutive nucleotides are not linked through any covalent bond. In another preferred embodiment, the at least two groups are linked through a covalent bond which is different from a phosphodiester bond.

The term nucleic acid molecule preferably also encompasses either D-nucleic acid molecules or L-nucleic acid molecules. Preferably, the nucleic acid molecules are L-nucleic acid molecules. In addition it is possible that one or several parts of the nucleic acid molecule is present as a D-nucleic acid molecules and at least one or several parts of the nucleic acid molecule is an L-nucleic acid molecule. The term "part" of the nucleic acid molecules shall mean as little as one nucleotide. Such nucleic acid molecules are generally referred to herein as D- and L-nucleic acid molecules, respectively. Therefore, in a preferred embodiment, the nucleic acid molecules according to the present invention consist of L-nucleotides and comprise at least one D-nucleotide. Such D-nucleotide is preferably attached to a part different from the stretches defining the nucleic acid molecule, preferably those parts thereof, where an interaction with other parts of the nucleic acid molecule is involved. Preferably, such D-nucleotide is attached at a terminus of any of the stretches and of any nucleic acid.

L-nucleic acid molecules as used herein are nucleic acid molecules consisting of L-nucleotides, preferably consisting completely of L-nucleotides.

D-nucleic acid molecule as used herein are nucleic acid molecules consisting of D-nucleotides, preferably consisting completely of D-nucleotides.

Also, if not indicated to the contrary, any nucleotide sequence is set forth herein in 5' → 3' direction.

Irrespective of whether the nucleic acid molecule consists of D-nucleotides, L-nucleotides or a combination of both with the combination being e.g. a random combination or a defined sequence of stretches consisting of at least one L-nucleotide and at least one D-nucleic acid, the nucleic acid molecule may consist of desoxyribonucleotide(s), ribonucleotide(s) or combinations thereof.

Regardless of whether the nucleic acid molecule is a D-nucleic acid, an L-nucleic acid, a mixture thereof, a DNA, or an RNA, or each and any combination thereof, the term nucleic acid molecule as preferably used herein shall also encompass single-stranded nucleic acid molecules and double-stranded nucleic acid molecules, whereby preferably the nucleic acid molecule as subjected to the method according to the present invention is a single-stranded nucleic acid. If the nucleic acid molecule the nucleotide sequence of which is to be determined is a double-stranded structure consisting of two separate strands, i.e. a first strand and a second strand, such strands are preferably separated and each separated strand is then subjected to the method according to the present invention. Alternatively such separation of a double-stranded nucleic acid is not necessary in case only a first strand of said two strands exhibits the modification which, according to the first procedure of the method according to the present invention is used for the separation of the modified nucleic acid molecule fragments from the non-modified nucleic acid molecule fragments, and which, according to the second procedure of the method according to the present invention is used in the addressing process. It will be understood that the nucleotide sequence of the second strand of such double-stranded nucleic acid molecule can be determined such that, preferably in a parallel approach, said second strand exhibits this kind of modification whereas the first strand does not. In a further alternative approach, the modification of the first strand and of the second strand is different.

The term nucleic acid molecule as preferably used herein, shall also encompass a nucleic acid molecule with an internal spacer. Preferably, the internal spacer is used for linkage of two nucleotide stretches of the nucleic acid molecule. Such internal spacer is preferably a hydrophilic spacer comprising at least one, preferably a multitude of ethylene glycol moieties. Various internal spacers, respectively, are known to the ones skilled in the art and can be selected using the following criteria as described, e. g., by Pils and Micura (Pils & Micura, 2000). The internal spacers should or do not interfere with the base pairs themselves. Spacer types that contain aromatic carbocycles stack on the terminal base pair and therefore are less suitable (Lewis et al, 1999). However, eythylene gylcol based or ethylene glycol derived spacers meet the requirement to not interfere with the base pairs as they have the advantage of good water solubility and high conformational flexibility (Durand et al, 1990; Ma et al, 1993; Thomson et al, 1993). Preferably, the spacer comprises or consists of one or several ethylene glycol moieties, whereby the oxygen is replaced or substituted by a CH₂, a phosphate or sulfur.

The term nucleic acid molecule as preferably used herein, shall also encompass a nucleotide-modified acid molecule. The nucleic acid molecules can be a nucleotide-modified RNA or a nucleotide-modified DNA molecule, whereby the RNA or DNA molecules are extensively modified at the individual nucleotides to enhance stability by modification with nuclease resistant groups, for example, 2'-amino, 2'-C-allyl, 2'-fluoro, 2'-O-methyl, 2'-H (for a review see Usman & Cedergren, 1992).

Chemically synthesizing nucleic acid molecules with modifications of the nucleotdide comprising base(s), the sugar backbone and/or the phosphate bond can prevent their degradation by serum ribonucleases, which can increase the *in vivo* potency of the nucleic acid molecules:

There are several examples in the art describing sugar, base and phosphate modifications that can be introduced into nucleic acid molecules with significant enhancement in their nuclease stability and efficacy. For example, nucleic acid molecules are modified to enhance stability and/or enhance biological activity by modification with nuclease resistant groups, for example, 2'-amino, 2'-C-allyl, 2'-fluoro, 2'-O-methyl, 2'-O-allyl, 2'-H, and nucleotide base modifications (for a review see Burgin et al, 1996; Usman & Cedergren, 1992). Sugar modification of nucleic acid molecules have been extensively described in the art (see international patent applications WO 91/03162, WO 92/07065, WO 93/15187, WO 97/26270; WO 98/13526; US patents US 5,334,711, US 5,716,824; US 5,627,053; (Beigelman et al, 1995; Pieken et al, 1991; Usman & Cedergren, 1992). Such publications describe general methods and strategies to determine the location of incorporation of sugar, base and/or phosphate modifications and the like into nucleic acid molecules without modulating catalysis. In view of such teachings, similar modifications can be used as described herein to modify the nucleic acid molecules of the instant invention so long as the ability of such nucleic acid molecules to bind their respective targets.

While chemical modification of oligonucleotide internucleotide linkages with phosphorothioate, phosphorodithioate, and/or 5'-methylphosphonate linkages improves stability, excessive modifications can cause some toxicity or decreased activity. Therefore, when designing nucleic acid molecules, the amount of these internucleotide linkages should be minimized. The reduction in the concentration of these linkages should lower toxicity,

The term nucleic acid molecule as preferably used herein, shall also encompass a fully closed nucleic acid molecule. A fully closed, i.e. circular structure for the nucleic acid molecule is realized if the nucleic acid molecule the nucleotide sequence of which is to be determined according to the present invention, is closed, preferably through a covalent linkage, whereby more preferably such covalent linkage is made between the 5' end and the 3' end of the nucleic acid molecules sequences as disclosed herein.

The term nucleic acid molecule as preferably used shall also encompass any nucleic acid molecule which comprises a non-nucleic acid molecule moiety. Such non-nucleic acid molecule moiety may be selected from a group comprising peptides, oligopeptides, polypeptides, proteins, carbohydrates, various groups as will be outlined in more detail in the following. The term nucleic acid molecule shall thus also encompass conjugates and/or complexes comprising at least one nucleic acid moiety and at least one further moiety that can be used to facilitate delivery of nucleic acid molecules into a biological system, such as a cell. The conjugates and complexes provided can impart therapeutic activity by transferring therapeutic compounds across cellular membranes, altering the pharmacokinetics, and/or modulating the localization of nucleic acid molecules of the invention. These kinds of conjugates and complexes are preferably suitable for the delivery of molecules, including, but not limited to, small molecules, lipids, phospholipids, nucleosides, nucleotides, nucleic acids, antibodies, toxins, negatively charged polymers and other polymers, for example proteins, peptides, hormones, carbohydrates, polyethylene glycols, or polyamines, across cellular membranes. In general, the transporters described are designed to be used either individually or as part of a multi-component system, with or without degradable linkers. These compounds are expected to improve delivery and/or localization of nucleic acid molecules into a number of cell types originating from different tissues, in the presence or absence of serum (see US patent US 5,854,038). Conjugates of the molecules described herein can be attached to biologically active molecules via linkers that are biodegradable, such as biodegradable nucleic acid linker molecules.

As will be detailed in the following in connection with the nucleic acid molecule the sequence of which is to be determined, the non-nucleic acid moiety may be a PEG moiety, i.e. a poly(ethylene glycol) moiety, or a HES moiety, i.e. a hydroxyethyl starch moiety.

The non-nucleic acid moiety and preferably the PEG and/or HES moiety can be attached to the nucleic acid molecule either directly or through a linker. It is also within the present invention that the nucleic acid molecule comprises one or more modifications, preferably one or more PEG and/or HES moiety. In an embodiment the individual linker molecule attaches more than one PEG moiety or HES moiety to a nucleic acid molecule. The linker used in connection with the present invention can itself be either linear or branched. These kind of linkers are known to the ones skilled in the art and are further described in the patent applications WO 2005/074993 and WO 2003/035665.

In a preferred embodiment the linker is a biodegradable linker. The biodegradable linker allows to modify the characteristics of the nucleic acid molecules in terms of, among other, residence time in the animal body, preferably in the human body, due to release of the modification from the nucleic acid molecules. Usage of a biodegradable linker may allow a better control of the residence time of the nucleic acid molecules. A preferred embodiment of such biodegradable linkers are biodegradable linkers such as those described in but not restricted to the international patent applications WO 2006/052790, WO 2008/034122, WO 2004/092191 and WO 2005/099768, whereby in the international patent applications WO 2004/092191 and WO 2005/099768, the linker is part of a polymeric oligonucleotide prodrug, that consists of one or two modifications as described herein, a nucleic acid molecule and the biodegradable linker in between.

As preferably used herein, "nucleotides" include, but are not limited to, the naturally occurring DNA nucleoside mono-, di-, and triphosphates: deoxyadenosine mono-, di- and triphosphate; deoxyguanosine mono-, di-and triphosphate; deoxythymidine mono-, di-and triphosphate; and deoxycytidine mono-, di-and triphosphate. (referred to herein as dA, dG, dT and dC or A, G, T and C, respectively). The term nucleotides also includes the naturally occurring RNA nucleoside mono-, di-, and triphosphates: adenosine mono-, di-and triphosphate; guanine mono-, di-and triphosphate; uridine mono-, di-and triphosphate; and cytidine mono-, di-and triphosphate (referred to herein as A, G, U and C, respectively) refers to a base-sugar-phosphate combination that is the monomeric unit of a nucleic acid molecule, i. e., a DNA molcule and an RNA molecule. However, in other words, the term "nucleotides" refers to any compound containing a cyclic furanoside-type sugar (p- D/L-ribose in RNA and P-D/L-2'-deoxyribose in DNA), which is phosphorylated at the 5' position and has either a purine or pyrimidine-type base attached at the C-l'sugar position via a -glycosol Cl'-N linkage. The nucleotides may be natural or synthetic, including a nucleotide that has been mass-modified including, inter alia, nucleotides having modified nucleosides with modified bases (e. g., 5-methyl cytosine) and modified sugar groups (e. g., 2'-O- methyl ribosyl, 2'-O-methoxyethyl ribosyl, 2'-fluoro ribosyl, 2'-amino ribosyl, and the like).

The term "nucleobase" covers the naturally occurring nucleobases adenine (A), guanine (G), cytosine (C), thymine (T) and uracil (U) as well as non-naturally occurring nucleobases such as xanthine, diaminopurine, 8-oxo-N6-methyladenine, 7-deazaxanthine, 7-deazaguanine, N4,N4-ethanocytosin, N6,N6-ethano-2,6-diaminopurine, 5-methylcytosine, 5∼(C3-C6)-alkynyl- cytosine, 5-fluorouracil, 5-bromouracil, pseudoisocytosine, 2-hydroxy-5-methyl-4-triazolopyridin, isocytosine, isoguanine, inosine and the "non-naturally occurring" nucleobases described in the US Patent US 5,432,272, in the publication of Freier & Altmann (Freier & Altmann, 1997). The term "nucleobase" thus includes not only the known purine and pyrimidine heterocycles, but also heterocyclic analogues and tautomers thereof.

In a first step, a plurality of the nucleic acid molecule the sequence of which is to be determined, is provided. The plurality of the nucleic acid molecule preferably comprises a number of individual molecules which allows, upon random cleavage of said plurality of nucleic acid molecules, the generation of a representation of all possible fragments or all relevant fragments of the nucleic acid molecule. The term fragments in the narrower sense as preferably used herein, refers to a nucleic acid molecule which comprises or consists of a nucleotide sequence which is, compared to the full length nucleic acid molecule, shorter in terms of the nucleotide sequence by one or more than one nucleotide of the full length nucleic acid molecule.

The nucleic acid molecule the sequence of which is to be determined is also referred to herein as the parent nucleic acid molecule.

The term 5' fragment as preferably used herein, refers to a fragment with an intact 5'-terminus, specifically, those fragments that include the 5'- terminal nucleotide of the parent nucleic acid molecule. Similarly, the term 3'- fragment as used herein, refers to a fragment with an intact 3'- terminus, i.e. those fragments that include the 3'- terminal nucleotide of the parent nucleic acid molecule. The term internal fragment, as used herein refers to those fragments that do not contain an intact terminus and are thus lacking both the 5'- and the 3'-terminal nucleotides.

The term intact as preferably used herein in connection with the 5' terminus and the 3' terminus, means, in case of the 5' terminus, that the 5'- terminal nucleotide, preferably of the nucleic acid molecule the nucleotide sequence of which is to be determined, is present in the nucleic acid molecule, more preferably in the plurality of molecules of the nuclei acid molecule, or fragment(s) thereof, and, in case of the 3' terminus, that the 3' terminal nucleotide, preferably of the nucleic acid molecule the nucleotide sequence of which is to be determined, is present in the nucleic acid molecule, more preferably in the plurality of molecules of the nuclei acid molecule, or fragment(s) thereof.

The term fragment, for easiness of describing the instant invention, shall preferably also encompass the full length nucleic acid molecule. A fragment of the nucleic acid molecule may thus be as short as one nucleotide and may be as long as the full length nucleic acid molecule.

It will be understood by a person skilled in the art that the plurality of fragments does not necessarily have to comprise all possible fragments of the nucleic acid molecule. Depending on the further purpose of the method described herein, it may suffice to have a limited number of fragments which allow to establish a fingerprint of the nucleic acid molecule whereby such fingerprint is sufficient for the identification of the nucleic acid molecule.

As preferably used herein, the term "plurality of molecules of the nucleic acid molecule" means a plurality of copies of the nucleic acid molecule and more preferably a plurality of copies of the parent nucleic acid molecule. Preferably, in connection therewith a plurality of copies means a number of copies which allows the practicing of the method of the invention. The precise number of the required copies depends on the particular embodiment of the methods of the invention and the steps and techniques used in connection with such steps and methods, respectively. The lower limit of the number of copies required of the individual fragment is preferably the one which still allows the generation of the pattern and the deducing of the nucleic acid sequence of said fragment. A common range for the copies is 1 x 10⁻¹⁸ to 1 x 10⁻³ moles.

As preferably used herein, a copy of a nucleic acid molecule is a nucleic acid molecule which has essentially the same nucleotide sequence. More preferably a copy of a nucleic acid molecule is identical in all of the physical and chemical characteristics of the nucleic acid molecule of which the copy is prepared.

The plurality of molecules of the nucleic acid molecules bears or has a modification. In connection with both the first and the second procedure according to the present invention, the plurality of said molecules bears or has modification to the extent that, as outlined above, upon random cleavage of said plurality of molecules, each possible fragment or each relevant fragment bears or has such modification. It will also be understood by a person skilled in the art that such fragment is a species of a nucleic acid molecule and such species is typically not only present as a single copy but again as a plurality of individual copies or molecules. It will also be understood that not each single copy of such fragments has to bear or have such modification. Again it is sufficient that a number of copies of the individual fragments is present which has or bears the modification. The minimum number of copies of the individual fragments depends on the methods used in the subsequent steps of the method according to the present invention, typically the methods used in the generation of the pattern. The lower limit of the number of copies required of the individual fragment is preferably the one which still allows the generation of the pattern and the deducing of the nucleic acid sequence of said fragment.

In order to be sequence as described by the methods herein, the nucleic acid molecules that pass through the sequencing methods as described herein either comprise a modification at the 5' or 3' end of their nucleotide sequence or are modified with a modification at the 5' or 3' end of their nucleotide sequence. Therefore un-modified nucleic molecules have to be modified in advance, before they can be sequenced by the methods as described herein.

The modification which the plurality of molecules of the nucleic acid molecule has, may be directly incorporated into the oligonucleotide during or prior to synthesis (e.g. US patents US 5,736,626 and US 5,141,813). Alternatively, e.g. a nucleophilic functionality such as a primary aliphatic amine, is introduced at a modification attachment site on a nucleic acid molecule, e.g. at the 5' terminus or 3'-terminus of nucleic acid molecule. After solid-support synthesis of nucleic acid molecule is complete, the nucleic acid molecule is cleaved from the support and all protecting groups are removed. Although, after the synthesis process, the nucleic molecule comprises a modification, the modification can, in another embodiment, be used to add another modification. The synthesized nucleophile-nucleic acid molecule is, e.g., reacted with an excess of a modification reagent containing an electrophilic moiety under homogeneous solution conditions. A modification reagent containing an electrophilic moiety is for example isothiocyanate or an activated ester such as N-hydroxysuccinimide (abr. NHS) (Hermanson, 1996).

The modification which the plurality of molecules of the nucleic acid molecule has, may further be incorporated into the oligonucleotide after the synthesis thereof and before the nucleic acid molecule is sequenced by the methods as described herien. Examples of methods employed to install modifications onto non-modified nucleic acid molecules include, but are not limited to, enzymatic and chemical manipulation. For instance it is possible to ligate a modification attached to nucleotides to the nucleic acid molecules using ligases. One such example is that of using T4 RNA ligase to ligate nucleotides carrying a modification or nucleotides containing an amino functionality onto the 5' end of a nucleic acid molecule (Kinoshita et al, 1997). The use of chemical ligation, for instance by using cyanogen bromide to attach oligonucleotides, is also an established technique (Dolinnaya et al, 1991; Elov et al, 1989). Other methods have been recently developed for the modifying of nucleic acid molecules without the use of this toxic chemical (Yoshimura et al, 2007).

An established technique for the introduction of modifications to the 3' end of RNA molecules is that of oxidising the terminal 2', 3' cis diol with sodium periodate to generate a dialdehyde, which is then subjected to a double reductive amination with either a diamine or a label-functionalised amine (Proudnikov & Mirzabekov, 1996). With the former, the modification is introduced using the resulting 3' amine as a reactive modification. Alternatively, the dialdehyde can be reacted with a modified carbazide derivative to install the modification without the need for subsequent reduction (Wu et al, 1996).

As to the length of the nucleic acid molecule the nucleotide sequence is to be determined, there are, basically, no limitations. Accordingly, the length of the nucleic acid molecule may be as short as two nucleotides and as long as several thousands nucleotides. Preferably, the length of the nucleic acid molecule is between 15 and 120 nucleotides. It will be acknowledged by the ones skilled in the art that any integer between 15 and 120 is a possible length for the nucleic acid molecule. More preferred ranges for the length of the nucleic acid molecule are lengths of about 20 to 100 nucleotides, about 20 to 80 nucleotides, about 20 to 60 nucleotides, about 20 to 50 nucleotides and about 30 to 50 nucleotides.

The modification can be any modification which is suitable to provide the effect which is required in connection with the present invention. More specifically, the modification needed in connection with the first procedure of the method of the present invention, allows the separation of the modified nucleic acid molecule fragments from the non-modified nucleic acid molecule fragments. In contrast thereto, the modification needed in connection with the second procedure of the method of the present invention, allows the practicing of the addressing process. It is to be acknowledged that in both the first procedure and the second procedure, the modification is involved in the separation or resolution of the modified nucleic acid molecule fragments. It is within the present invention that the modification may have a dual function or provides for two functions. In such case, the modification may be a uni-partite modification. Alternatively and particularly in those cases where a dual function is required, the modification may be a bi- or multipartite modification. A bi- or multipartite function comprises a first moiety and a second moiety which may be either connected directly to each other or through the use of a linker. In such case, either the first moiety or the second moiety is used for separating the modified nucleic acid molecule fragments from the non-modified nucleic acid molecule fragments, whereas the second or the first moiety is used in the separation or resolution of the modified nucleic acid molecule fragments.

In a further step the plurality of modified nucleic acid molecules are cleaved at random. Upon such cleavage modified nucleic acid molecule fragments and non-modified nucleic acid molecule fragments are generated and provided, respectively. Depending on the chemical nature of the nucleic acid molecule the nucleotide sequence of which is to be determined, various techniques are applicable, which are, as such, known in the art. The cleaving may be any of the following techniques or combinations thereof: physical fragmentation, chemical cleaving, enzymatic cleaving, cleaving by heat and/or cleaving by use of a divalent cation. These various techniques are applicable as long as they provide for a cleavage at a specific and predictable site in the nucleic acid molecules and is in accordance with the further requirements of the cleaving steps as outlined herein.

Cleavage of the nucleic acid molecules at a specific position in the nucleic acid molecule sequence is dependant from the structure of the nucleic acid molecules, the physicochemical nature of the covalent bond between the particular nucleotides of the nucleic acid molecule, the physicochemical nature of the sugar backbone of the nucleic acid molecule, the physicochemical nature of the bases of the nucleic acid molecule, the physicochemical nature of the covalent bond between the particluar base and the sugar backbone of the nucleic acid molecule, the particular atoms of the nucleic acid molecule; the specificity of the cleaving reagent towards a particular base and/or modified base of the nucleic acid molecule; or a combination thereof.

Physical fragmentation of a nucleic acid molecule can be achieved by the use of any physical force that can break a covalent bond, whereby preferably a specific and predictable fragmentation occurs. Such physical forces include but are not limited to heat, ionization radiation, such as X-rays, UV-rays, gamma-rays. The size of the nucleic acid molecule fragments can be adjusted by adjusting the intensity and duration of exposure to the radiation. The intensity and duration of exposure can also be adjusted to minimize undesirable effects of radiation on the nucleic acid molecule.

Heat, preferably approaching the boiling of water, can also produce fragments of nucleic acid molecules. Fragmentation of a nucleic acid molecule by heating a solution of a nucleic acid molecule is preferably done in a variety of standard buffers such as but not limited to primary alkyl amines such as TRIS (tris(hydroxymethyl)aminomethane), secondary amines such as Tricine (*N*-(Tri(hydroxymethyl)methyl)glycine), tertiary amines such as Triethylamine, Bis-Tris (Bis(2-hydroxyethyl)-imino-tris(hydroxymethyl)-methane) polyamines such as, spermidine, HEPES (4-(2-hydroxyethyl)-1-piperazineethanesulfonic acid) and PIPES (piperazine-N,N'-bis(2-ethanesulfonic acid), quaternary ions such as tetrabutylammonium and tetraethylammonium. Buffers containing aromatic amines such as imidazole are also known in the art. Such buffers can be used in conjunction with hydrochloric, hydrofluoric, hydrobromic, phosphoric, citric, phthalic, tartaric, boric acid and others known in the art. Other suitable buffers/solutions containing alkali metals are also known in the art. Examples of which are hydroxide, carbonate, hydrogen carbonate, phosphate, phthalate, tartrate, borate and acetate. The preferable pH range is pH -1 to pH15, more preferably pH 4 to pH 10. The preferable concentration is 0.01 to 100000 ODs/mL, more preferably 10 to 1000 ODs/mL. The reaction is run between 0.1 and 5000 mins, more preferably 5 to 100 mins.

Chemical cleavage of a nucleic acid molecule can be achieved by divalent cation catalyzed cleavage of the phosphodiester bond of the nucleic acid molecule, by alkylation and/or by hydrolysis reactions including base and acid hydrolysis.

Divalent cation catalyzed of the phosphodiester bond of RNA is preferably done in the presence of but not limited to Mg²⁺ Ca²⁺, Be²⁺, Ba²⁺, Fe²⁺, Zn²⁺, Cu²⁺, Mn²⁺, Cd²⁺, Sr²⁺, Ni²⁺, Co²⁺, Pb²⁺ between 0.000001-10 M, more preferably 0.00001 to 1 M. The temperature of the reaction is 0°C to 150 °C, more preferably 10 to 100°C. The reaction is run for 0.1 to 5000 min, more preferably 1 min. to 120 min.

Cleavage of the phosphodiester bond of RNA can also be achieved using solutions containing primary alkyl amines such as TRIS (tris(hydroxymethyl)aminomethane), secondary amines such as Tricine (*N*-(Tri(hydroxymethyl)methyl)glycine), tertiary amines such as Triethylamine, Bis-Tris (Bis(2-hydroxyethyl)-imino-tris(hydroxymethyl)-methane) polyamines such as, spermidine, HEPES (4-(2-hydroxyethyl)-1-piperazineethanesulfonic acid) and PIPES (piperazine-N,N'-bis(2-ethanesulfonic acid), quaternary ions such as tetrabutylammonium and tetraethylammonium. Buffers containing aromatic amines such as imidazole are also known in the art. between 0.000001-10 M, more preferably 0.00001 to 1 M. The temperature of the reaction is 0°C to 150 °C, more preferably 10 to 100°C. The reaction is run for 0.1 to 5000 min, more preferably 1 min. to 120 min.

Alkylation of a nucleic acid molecule as a method for fragmentation of a nucleic acid molecule was described by Browne and Gut & Beck (Browne, 2002; Gut & Beck, 1995).

Base hydrolysis can be used to cleave an RNA molecule because RNA is unstable under alkaline conditions (Nordhoff et al, 1993). Base hydrolysis of an RNA molecule is preferably done at a pH range of 7.5 to 15, more preferably at a pH range of 9 to 15. The temperature of the reaction is 0 to 150 °C, more preferably at 50 to 150 °C. The reaction is run for 0.1-5000 min., more preferably 1 to 100 min.

Acid hydrolysis can also be used to cleave a RNA molecule because RNA can be hydrolyzed in the presence of acids, preferably in the presence of strong acids such as mineral acids like HCl, and organic acids such as *para*-Tolene-sulfonic acid. Acid hydrolysis of an RNA molecule is preferably done at a pH range of -1 to 6.5, more preferably at a pH range of 1 to 4. The temperature of the reaction is preferably at 0°C to 150 °C, more preferably at 20 to 100 °C. The reaction is run for 0.1 to 5000 min, more preferably for 1 to 100 min.. Under rigorous conditions, hydrolysis can break both of the phosphate ester bonds and also the N-glycosidic bond between the ribose and the purines and pyrimidine bases.

Acid hydrolysis can be used to cleave a DNA molecule because DNA can be hydrolyzed in the presence of acids, preferably in the presence of strong acids such as mineral acids like HCl, and organic acids such as *para*-Tolene-sulfonic acid. Acid hydrolysis of an DNA molecule is preferably done at a pH range of 0 to 5.5, more preferably at a pH range of 1 to 2. The temperature of the reaction is at 0°C to 150 °C, more preferably at 20 to 100 °C. The reaction is run for 0.1 to 5000 min, more preferably for 1 to 100 min.. Depending on the conditions and length of reaction time, the nucleic acid molecule can be fragmented into various sizes including fragments of one nucleotide. In particular under rigorous conditions, hydrolysis can break both of the phosphate ester bonds and also the N-glycosidic bond between the deoxyribose and the purines and pyrimidine bases.

Protocols for producing fragments of a nucleic acid molecule based on acid and/or base hydrolysis were previously described (Maxam & Gilbert, 1977; Peattie, 1979; Sargent, 1988).

Enzymes are useful for fragmention of nucleic acid molecules and are often used in connection with sequencing of nucleic acids by MS (Alazard et al, 2002; Bentzley et al, 1998; Bentzley et al, 1996; Faulstich et al, 1997; Glover et al, 1995; Kirpekar et al, 1994; Owens et al, 1998; Pieles et al, 1993; Schuette et al, 1995; Smirnov et al, 1996; Wu & Aboleneen, 2001; Wu et al, 1998a). Such enzymes that cleave nucleic acid molecule are known in the art (Sambrook, 2001) and are commercially available. Depending on the enzyme used, the nucleic acid molecule are cut nonspecifically or at specific nucleotides sequences. Any enzyme capable of cleaving a nucleic acid molecule can be used including but not limited to endonucleases, exonucleases, ribozymes, and DNAzymes.

Endonucleases have the capability to cleave the bonds within a nucleic acid molecule strand, whereby the endonucleases can be specific for either a double-stranded or a single stranded nucleic acid molecule. The cleavage of the nucleic acid molecule can occur randomly within the nucleic acid molecule or can cleave at specific sequences of the nucleic acid molecule. Specific fragmentation of the nucleic acid molecule can be accomplished using one or more enzymes in sequential reactions or contemporaneously. Restriction endonucleases are a subclass of endonucleases which recognize specific sequences within a double-strand nucleic acid molecule and typically cleave both strands either within or close to the recognition sequence. Endonucleases can be specific for certain types of nucleic acid molecules, preferably specific for DNA or RNA molecules. Examples of RNA or DNA molecule specific endonucleases are ribonuclease H, ribonuclease A, ribonuclease T₁, ribonuclease U₂, ribonuclease P and ribonucleases as discussed in the international patent application WO2004/097369, page 43, line 5 to page 44, line 4.

In order to reduce ambiguities in sequence determination, additional limited alkaline hydrolysis can be performed. Since every phosphodiester bond is potentially cleaved under these conditions, information about omitted and/or specific cleavages can be obtained this way (Donis-Keller et al, 1977).

As alternative to endonucleases, for fragmentation of DNA molecules DNA glycosylases can be used. The DNA glycosylases specifically remove a certain type of nucleobase from a given DNA nucleic acid molecule. These enzymes can thereby produce abasic sites in the sequence of the nucleic acid molecule, whereby the abasic sites can be recognized either by another cleavage enzyme, cleaving the exposed phosphate backbone specifically at the abasic site and producing a set of nucleobase specific fragments indicative of the sequence, or by chemical means, such as alkaline solutions and or heat. The use of one combination of a DNA glycosylase and its targeted nucleotide would be sufficient to generate a base specific signature pattern of the nucleic acid molecule. Numerous DNA glycosylases are known and discusssed in the international patent application WO 2004/097369, page 44, line 13 to page 45, line 7.

However, the bases of DNA molecule can be modified with specific chemicals so that the modified bases are recognized by specific DNA glycosylases (see international patent application WO 2004/097369, page 45, line 8 to page 45, line 26). The fragments of the nucleic acid molecule are produced by glycosylase treatment and subsequent cleavage of the abasic site.

Fragmentation of a nucleic acid molecule herein can also be accomplished by dinucleotide-specific cleavage reagents are known to those of skill in the art (WO 94/21663;Cannistraro & Kennell, 1989).

Deoxyribonuclease (abbr. DNase) can also be used to generate DNA molecule fragments (Anderson, 1981). DNase I is an endonuclease that digests double-and single-stranded DNA into poly-and mono-nucleotides. Other DNAase are DNase II, DNase H, DNase IT, DNase IX etc. are discussed in the international patent application WO2004/097369, page 46, line 26 to page 47, line 6.

Exonucleases are enzymes that cleave nucleotides from the ends of single-strand or double nucleic acid molecules, for example a DNA molecule. There are 5'exonucleases (cleave the DNA molecule from its 5'-end) and 3'exonucleases (cleave the DNA from its 3'-end).

Beside the protein-based enzymes as described supra, DNAzymes and RNAzymes are known in the art and can be used to cleave nucleic acid molecules to produce nucleic acid molecule fragments (Santoro & Joyce, 1997; Schlosser et al, 2008a; Schlosser et al, 2008b); US patents US 6,326,174, US 6,194,180, US 6,265,167, US 6,096,715; US 5,646,020).

Ionization fragmentation of nucleic acid molecules is a further option so as to provide a cleaving at random and is, e.g., accomplished during mass spectrometric analysis by using high voltages in the ion source of the mass spectrometer to fragment by MS using collision-induced dissociation in the ion trap (Biemann, 1990). The base sequence is deduced from the molecular weight differences observed in the resulting MS fragmentation pattern of the nucleic acid molecule using the published masses associated with the individual nucleotide residues in the MS.

Fragments of a nucleic acid molecule can be formed using any combination of fragmentation methods as well as any combination of enzymes. It will thus be acknowledged by the person skilled in the art the that any combination of all these cleavage reactions such as by heat, basic pH, diamines, or even acidic pH which can degrade RNA, in particular at elevated temperatures, ionization and one or several enzymes as well as combinations of the above, is encompassed in the methods of the invention. Moreover, methods for producing specific fragments of a nucleic acid molecule can be combined with the methods for producing random fragments of a nucleic acid molecule. Additionally, one or more enzymes that cleave a nucleic acid molecule at a specific site can be used in combination with one or more enzymes that specifically cleave the nucleic acid molecule at a different site. In another example, enzymes that cleave specific kinds of a nucleic acid molecule can be used in combination. In another example, an enzyme that cleaves a nucleic acid molecule randomly can be used in combination with an enzyme that cleaves a nucleic acid molecule specifically. Used in combination means performing one or more methods after another or contemporaneously on a nucleic acid molecule.

In connection with the instant invention, the cleavage or fragmentation step comprises cleaving of the plurality of modified nucleic acid molecules at random. As preferably used herein the term at random is indicative that each nucleic acid molecule is cleaved at one or several sites within its nucleotide sequence, i.e. within its primary nucleic acid structure. Ion connection therewith it is essential that the cleavage occurs at a known site in a reproducible manner although it is statistical. For the practicing of the present invention it is irrelevant whether or not the individual molecule is cleaved once or several times as long as the overall cleaving provides for a representation of all possible fragments or all relevant fragments of the nucleic acid molecule. In connection with said cleaving it will be acknowledged that typically and if present in the respective reaction not only the modified nucleic acid molecule species will be cleaved, but also those species of the nucleic acid molecule which does not bear or have such modification.

Insofar, the cleaving is not only a random cleaving but also a limited cleaving as a non-limited cleaving or complete cleaving would result in the generation of single nucleotides or fragments which would not be suitable to provide such representation.

In a further step, the method according to the present invention comprises the step of separating the modified nucleic acid molecule fragments from the non-modified nucleic acid molecule fragments. As will be acknowledged by a person skilled in the art, this step is preferably only encompasses in the first procedure of the method according to the present invention.

This separation step is carried out based on the principle of discrimination of the modified nucleic acid molecule fragments, preferably in their entirety, from non-modified nucleic acid molecule fragments, again preferably in their entirety. Such discrimination may be based on mass, size or hydrophobic interaction which is inherent to or due to the modification conferred to the modified nucleic acid molecule fragments or is absent from the non-nucleic acid molecule fragment due to the modification. The techniques which allow such separation comprise among others filtration, dialysis and chromatography in its broadest sense, i.e. separation based on the interaction between a ligand and an interaction partner to said ligand. It will be acknowledged that, preferably, the representation of all possible fragments or all relevant fragments of the nucleic acid molecule is basically maintained in this separation step.

A particularly preferred principle for the separation of the modified nucleic acid molecule fragments from the non-modified nucleic acid molecule fragments is the use of a ligand as the modification, including its use as the first or second moiety of the bi- or multipartite modification.

In a preferred embodiment the modification of the modified nucleic molecules is a ligand that is directly or indirectly linked to the 5' or 3'-terminal nucleotide of the nucleic acid molecule. Indirectly linked means herein that between the ligand and the 5' or 3'-terminal nucleotide of the nucleic acid molecule a linker is installed. Ligand means something which binds. A ligand as used herein is moiety that is linked to a nucleic acid molecule, whereby the ligand interacts with a binding partner that allows the binding of the ligand to the binding partner, whereby as a result of the binding of the ligand and the binding partner the nucleic acid molecule that is linked to the ligand is immobilised.

In an embodiment the interaction partner is attached to a phase, whereby such phase is different from phase which comprises the modified nucleic acid molecule fragments and preferably also the non-modified nucleic acid molecule fragments. Preferably such phase is a solid phase. Such solid phase is formed, e.g., by a solid support. The solid support is preferably selected from the group comprising polymers, preferably plastics, glass, agarose, and metals.

Due to the interaction between the ligand and the interaction partner, the ligand and thus the modified nucleic acid molecule fragments is/are immobilized to the phase to which the interaction partner is attached. Depending on the kind of interaction generated by the ligand and the interaction partner, the immobilization may preferably be chemical immobilization, affinity immobilization, or magnetic immobilization.

A particularly preferred form of immobilization is chemical immobilization based on the following interactions whereby one of the elements providing such interaction is the ligand, whereas the other element providing such interaction is the interaction partner. Examples, the putting into practice of which is known by a person skilled in the art, include but are not limited to:
An amine and an activated carboxylic acid,
An amine plus an activated carbamate,
An amine and an isocyanate/ isothiocyanate,
An amine plus a halide,
An amine plus a maleimide moiety,
An amine plus an aldehyde/ketone
A hydroxylamine or a hydrazide plus a ketone/an aldehyde,
A hydrazine derivative and an activated carboxylic acid,
A hydrazine and an isocyanate/isothiocyanates,
A hydrazine plus a halide,
A hydrazine plus a maleimide moiety,
A hydrazine + an aldehyde/a ketone:
A hydrazine + an aldehyde/a ketone follwed by reductive amination
A thiol plus a halide,
A thiol plus a maleimide,
A thiol plus an activated thiol,
A thiol plus a vinyl sulfone and other Michael addition reactions
An azide plus an alkyne plus Cu salts and other "click chemistry" interaction partners (Kolb et. Al. 2001),
An azide plus an activated carboxylic acid via Staudinger reaction utilising alkyl or aryl P(III) moieties,
An azide plus a trivalent phosphine attached to an electrophilic trap (Staudinger ligation),
An azide plus a phosphinothiol ester - traceless Staudinger ligation,
An azide plus an aldehyde/a ketone + PPh₃ (Staudinger) to form an imine that can then be with optional reducuction to the corresponding amine,
An amine plus a carboxyl group -
A carboxylic acid functional group plus amino functionality such as amine, hydrazine,
A Cis-diol (e.g. as found on the 3' terminus of RNA molecules) oxidised to di-aldehyde that then forms cyclic amines for example, with either amines or hydrazine derivatives after e.g. borohydride mediated reduction.,
A thioester plus a cysteine - native ligation and derivatives,
A phosphothioate + an α-halocarbonyl containing conjugants,
A phosphate +an amine to phosphoramidate e.g. via phosphate activation
A phosphate + an alcohol to phosphodiester e.g. via activation,
An aldehyde to form secondary amines (after reduction with Borohydride), hydrazino groups to form hydrazones, semicarbazides to form semi-carbazones.
A Cysteine derivative + a thioester peptide
An epoxide plus amine
An alkene/an alkyne + a diene/diyne for Diels Alder reaction, and other Pericyclic reactions
Oxime formation through reacting aldehyde with a hydroxylamine
A hydroxy or amino + an epoxide

The above reactions or at least some therof are, among others described by Smith and March, 2007 and Hermanson, 2008.

It is also recognized that the chemical affixation of labels/tags or ligands can also be achieved based on, but not limited to the above listed functional group interactions whereby one of the elements providing such interaction is affixed to the nucleic acid molecule, whereas the other element providing such interaction is affixed to the label. the interaction partner.

A particularly preferred form of immobilization is affinity immobilization based on the following interactions whereby one of the elements providing such interaction is the ligand, whereas the other element providing such interaction is the interaction partner: biotin-avidin interaction, biotin-neutravidin interaction, biotin-streptavidin interaction, interaction of antibody and antigen or hapten, interaction of two oligonucleotides, whereby the nucleic acid molecules consist of DNA, RNA, LNA, PNA or combinations thereof, interaction of calmodulin and calmodulin binding peptide, interaction of albumin and Cibracon Blue, interaction of a metal-chelator agent and metal-chelating support.

Upon the immobilization of the modified nucleic acid molecule fragments, the non-modified nucleic acid molecule fragments are removed from the modified nucleic acid molecule fragments. Such removal is a standard procedure as known by a person skilled in the art. Preferably, the non-modified nucleic acid molecule fragments are removed by washing or by transferring the phase comprising the modified nucleic acid molecule fragments immobilized to the phase to which the interaction of the ligand is attached, from the reaction and reaction vessel, respectively, where the separation step has occurred, into a new reaction and reaction vessel, respectively.

The term washing as used to herein, refers to the application of liquid media in order to remove non-modified fragments or other chemical entities from the phase where the modified fragments are sequestered.

In a further sub-step, the immobilized modified nucleic acid molecule fragments are removed from the phase to which the interaction partner is attached thus releasing the modified nucleic acid molecule fragments. Such release can be affected by any means known to the persons skilled in the art. More specifically, such release can be affected by adding an excess of the interaction partner of the ligand which competes for the binding of the ligand to the interaction partner which is attached to the phase. An alternative to this procedure is to detach the interaction partner from the phase so that the released modified nucleic acid molecule fragments comprise also the interaction partner now released from the phase to which it was attached prior to such release. In a further embodiment, the interaction between the interaction partner and the ligand is formed by a covalent bond and the interaction partner is removed from the ligand, whereby the covalent bond is chemically and/or enzymatically cleaved or by light. In an alternative embodiment, the interaction between the interaction partner and the ligand is formed by a non-covalent bond and the interaction partner is removed from the ligand, whereby the non-covalent bound is cleaved by variation of pH, the temperature and/or the ion force, by denaturation of the ligand and/or the interaction partner, by elution with an competitor molecule, by use of organic solvents and chaotropic agents. In another embodiment, the modified nucleic acid molecule fragments are removed from the phase to which the interaction partner is attached by cleaving the linker which is used for the binding of the ligand to the (modified) nucleic acid molecule fragments. In such embodiment, it must be assured that the modified nucleic acid molecule fragments still comprise a modification which allows the separating or resolving of the modified nuclei acid molecule fragments.

In the next step of the method according to the present invention, which is applicable to both the first and the second procedure, the modified nucleic acid molecule fragments are separated or resolved according to their length, mass and/or charge, whereby such separating or resolving generates a pattern of modified nucleic acid fragments. Such separation occurs through the use of the or a modification which is part of the modified nucleic acid molecule fragments.

In such resolving step the modified nucleic acid molecule fragments which are present after the separation from the non-modified nucleic acid molecules fragments as a mixture, the individual fragments. i.e. the individual fragment species have to be rendered addressable. This process of rendering the individual fragment species addressable is based on the differences of said fragment species in terms of their length, mass and/or charge. Accordingly, a technique is applied to the mixture of the modified nucleic acid molecule fragments which resolves the mixture such that the individual species are separated from each other. Such separation may be a separation in time, space, mass, and/or mass to charge ratio. Methods for the performance of such separation are known to a person skilled in the art and also described herein, including the introduction the disclosure of which shall be incorporated into this part of the description to avoid any unnecessary repetition. As preferably used herein a separation in time is one where in a display one species of the fragments follows another one over time. At a given moment in time, only one or a limited number of such species is then present at the display, depending on the width of the time window and the time window such display encompasses. As preferably used herein a separation in space is one where in a display one species of the fragments is arranged or present at a location in the two or three dimensional space, whereby such location is different for the various fragments of the modified nucleic acid molecule. Depending on the space covered by the display either all of the species of the fragment or only a part thereof may be covered, i.e. displayed.

Insofar the term pattern as preferably used herein refers to the result of a resolving step and indicates either the sequence of modified nucleic acid molecule fragments over time preferably shown in a display, or the arrangement of the sequence of modified nucleic acid molecule fragments in a two or three dimensional space or the arrangement of the sequence of modified nucleic acid molecule fragments based on either mass or mass to charge ratio. Insofar, a pattern is preferably a ladder of modified nucleic acid molecule fragments arranged along a time axis, arranged in the two- or three-dimensional space or a combination thereof.

It is within the present invention that the step of deducing the nucleotide sequence of the nucleic acid molecule which makes use of the pattern of modified nucleic acid molecule fragments, is actually making use of modified nucleic acid molecule fragments which lack the modification that was used in the step of separating the modified nucleic acid molecule fragments from the non-modified nucleic acid molecule fragments. More specifically, after the separation of the modified nucleic acid molecule fragments from the non-modified nucleic acid molecule fragments, the modification is removed from the modified nucleic acid molecule fragments thus generating a pattern of modified nucleic acid molecule fragments which are lacking the modification which was used so as to separate the modified nucleic acid molecule fragments from the non-modified nucleic acid molecule fragments. Such modified nucleic acid molecule fragments which are lacking the modification may be generated by the use of a traceless linker which attaches the modification to the modified nucleic acid molecule fragments. Such traceless linker is one which, upon cleavage, leaves both the modification and the nucleic acid molecule fragments devoid of any atom(s), group(s) of atoms or moiety/moieties which once have been forming the traceless linker. Because of this, the modification and the nucleic acid molecule fragments do not show any change in length, mass and/or charge after the traceless linker has been cleaved and removed, respectively. An example of such traceless linker is schematically depicted in the following formula:

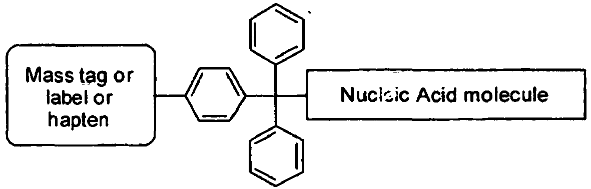

It is within the present invention that one modification or one moiety of such modification of the modified nucleic acid molecule fragments may be removed, preferably after separating the modified nucleic acid molecule fragments from the non-modified nucleic acid molecule fragments, from the modified nucleic acid molecule fragments whereby a further modification or moiety of said modification is still attached to the nucleic acid molecule fragments. In a further embodiment the removal of the modification leaves the linker which attached the modification to the nucleic acid molecule fragment, or part thereof attached to the nucleic acid molecule fragment, whereby such nucleic acid molecule fragment may still be regarded as a modified nucleic acid molecule fragment due to the presence of the linker or a part thereof, preferably under the proviso that such linker and part thereof, respectively, is suitable to confer to such nucleic acid molecule the characteristics of a modified nucleic acid molecule fragment.

It will be acknowledged by a person skilled in the art that in the first procedure of the method according to the present invention, the pattern essentially consists of modified nucleic acid molecule fragments only. However, it cannot be excluded that also some non-modified nucleic acid molecule fragments are contained in the reaction which is subjected to the resolving step, typically as side-products due to an incomplete separation of the modified nucleic acid molecule fragments from the non-modified nucleic acid molecule fragments.

It will also be acknowledged by a person skilled in the art that in connection with the second procedure of the method according to the present invention, a pattern is formed by both the modified nucleic acid molecule fragments and the non-modified nucleic acid molecule fragments. However, the pattern in the meaning of this step of resolving the modified nucleic acid molecule fragment is comprised only of the modified nucleic acid molecule fragments as only these modified nucleic acid molecule fragment comprise the modification which may be used in the addressing process. Accordingly, only the modified nucleic acid molecule fragments may be displayed in time, space, mass, and/or mass to charge ratio so as to generate the pattern of modified nucleic acid molecule fragments.

The modification which allows the resolving step, i.e. which is used in separating or resolving the modified nucleic acid molecule fragments, i.e. species, may be a uni-partite modification or part of a bi-or multi-partite modification as defined herein.

Such modification is preferably a label, a mass tag, a lipophilic tag or an affinity tag.

In a preferred embodiment the modification of the modified nucleic molecules is a label, that is directly or indirectly linked to the 5' or 3'-terminal nucleotide of the nucleic acid molecule. Indirectly linked means herein that between the label and the 5' or 3'-terminal nucleotide of the nucleic acid molecule a linker is installed. The term "label" as used herein refers to any atom, molecule and/or moiety which can be used to provide a detectable (preferably quantifiable) signal, and which can be attached to a nucleotide of a nucleic acid molecule. Labels may provide signals detectable by fluorescence, chemiluminescence, electrochemical luminescence, radioactivity, colorimetric, X- ray diffraction or absorption, magnetism, enzymatic activity, and the like. Detection labels include, but are not limited to fluorescent groups [groups which are able to absorb electromagnetic radiation, e.g. light or X-rays, of a certain wavelength, and which subsequently reemits the energy absorbed as radiation of longer wavelength; illustrative examples are DANSYL (5- dimethylamino)-1-naphthalenesulfonyl), DOXYL (N-oxyl-4,4-dimethyloxazolidine), PROXYL (N-oxyl-2,2,5,5-tetramethylpyrrolidine), TEMPO (N-oxyl-2,2,6,6-tetramethylpiperidine), dinitrophenyl, acridines, coumarins, Cy3 and Cy5, erythrosine, coumaric acid, umbelliferone, Texas red, rhodamine, tetramethyl rhodamine, Rox, 7-nitrobenzo-2-oxa-1-diazole (NBD), pyrene, fluorescein, Europium, Ruthenium, Samarium, and other rare earth metals], cyanine dyes (international patent application WO1997/45539, US patents US 5,366,860 and US 5,18.934) and chemiluminescent dyes (US patent US 4,931,223; Bronstein et al, 1994), radio isotopic labels, and chemiluminescence labels (labels that are detectable via the emission of light during a chemical reaction such as shown in US patent US 4,931,223; Bronstein et al, 1994).

Examples of fluorescein dyes include 6-carboxyfluorescein (6-FAM), 2', 4',1,4-tetrachlorofluorescein (TET), 2',4',5',7',1,4-hexachlorofluorescein (HEX), 2',7'-dimethoxy-4',5'-dichloro-6-carboxyrhodamin (JOE), 2'-chloro-5'-fluoro-7',8'-fused phenyl-1,4-dichloro-6-caroxyflurescein (NED), and 2'-chloro-7'-phenyl-1,4-dichloro-6-carboxyfluorescein (VIC).

It will be acknowledged by a person skilled in the art that this kind of modification, i.e. labels, are particularly useful in connection with the second procedure of the method according to the present invention. More preferably such labels exhibit an absorption or fluorescence characteristic which is different from the one of a nucleic acid molecule in general. Due to this kind of modification the modified nucleic acid molecule fragments can be discriminated from the non-modified nucleic acid molecules, and are preferably displayed in the display and subject to the addressing process.

In a preferred embodiment the modification of the modified nucleic molecules is a mass tag. In a preferred embodiment the modification of the modified nucleic molecules is a mass tag, that is directly or indirectly linked to the 5' or 3'-terminal nucleotide of the nucleic acid molecule. Indirectly linked means herein that between the mass tag and the 5' or 3'-terminal nucleotide of the nucleic acid molecule a linker is installed. Mass tags means something whose molecular weight is higher than the molecular weight of the nucleic acid molecule to be sequenced. Therefore a mass tag linked to a nucleic acid molecule, i.e. a modified nucleic molecule, whereby the modication is a mass tag, allows the separation of a modified nucleic molecule, from a un-modified nucleic acid molecule. The separation of mass-tag separation modified nucleic molecule from a un-modified nucleic acid molecule can be done by filtration, dialysis and/or chromatogrphic procedures.

Mass tags comprise moieties that are permanently attached to the nucleic acid molecule and tagged fragments thereof are of defined mass so as to enable the accurate determination of the sequence. Examples of such tags are defined hydrophilic polymers such as peptides, DNA, PNA. The mass tag can also be used merely to facilitate separation of tagged fragments from non-tagged fragments. Upon separation, these mass tags are removed to leave just the desired nucleic acid molecule fragments. Mass tags that are cleaved after separation from non-tagged fragments do not have to be of a defined mass. Therefore hydrophilic polymers such as but not limited to PEG, proteins, antibodies, polysaccharides can be used as well as defined polymers such as DNA, PNA and peptides.

A mass tag may also be considered to be a tag that is distinguishable by its mass. Such a distinction can be used to identify tagged fragments. The identification can be achieved using MS/MS fragmentation to liberate the unique mass of the tag and thus indicating that the parent molecule was tagged. Such a concept is known as the 'daughter ion' mass tag approach. In a further embodiment the mass tag may consist of a defined isotopic distribution so as to further establish the identity of the tag.

In a further preferred embodiment the modification is a lipophilic tag which is directly or indirectly linked to the 5' or 3'-terminal nucleotide of the nucleic acid molecule. Indirectly linked means herein that between the lipophilic tag and the 5' or 3'-terminal nucleotide of the nucleic acid molecule a linker is installed. Lipophilic tags means something that is more lipophilic than the nucleic acid molecule to be sequenced. Therefore a lipophilic tag linked to a nucleic acid molecule, i.e. a modified nucleic molecule, whereby the modication is a lipophilic tag, allows the separation of a modified nucleic molecule, from a un-modified nucleic acid molecule. The separation of lipophilic tag separation modified nucleic molecule from a un-modified nucleic acid molecule can be done by filtration, dialysis and/or chromatogrphic procedures.

Lipophilic tags comprise of but are not limited to aliphatic chains with 2 two 50 carbons, steroids, alkaloids, aromatic ring systems. The term "aliphatic", as used herein, includes both saturated and unsaturated, straight chain (i.e., unbranched), branched, acyclic, cyclic, or polycyclic aliphatic hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties. Thus, as used herein, the term "alkyl" includes straight, branched and cyclic alkyl groups. An analogous convention applies to other generic terms such as "alkenyl", "alkynyl", and the like. Furthermore, as used herein, the terms "alkyl", "alkenyl", "alkynyl", and the like encompass both substituted and unsubstituted groups. Illustrative aliphatic groups thus include, but are not limited to, for example, methyl, ethyl, n-propyl, isopropyl, cyclopropyl, -CH₂-cyclopropyl, vinyl, allyl. n-butyl, sec- butyl, isobutyl, tert-butyl, cyclobutyl, -CHb-cyclobutyl, n-pentyl, sec-pentyl, isopentyl, tert-pentyl, cyclopentyl, -CH₂-cyclopentyl, n-hexyl, sec-hexyl, cyclohexyl, -CH₂-cyclohexyl moieties and the like, which again, may bear one or more substituents. Alkenyl groups include, but are not limited to ethenyl, propenyl, butenyl, 1-methyl-2-buten-1-yl, and the like. Representative alkynyl groups include, but are not limited to, ethynyl, 2-propynyl (propargyl), 1-propynyl, and the like. In general, the terms "aryl" and "heteroaryl", as used herein, refer to stable mono-or polycyclic, heterocyclic, polycyclic, and polyheterocyclic unsaturated moieties. Substituents include, but are not limited to, any of the previously mentioned substituents, i.e., the substituents recited for aliphatic moieties, or for other moieties as disclosed herein, resulting in the formation of a stable compound. In certain embodiments of the present invention, "aryl" refers to a mono- or bicyclic carbocyclic ring system having one or two aromatic rings including, but not limited to, phenyl, naphthyl, tetrahydronaphthyl, indanyl, indenyl, and the like. In certain embodiments of the present invention, the term "heteroaryl", as used herein, refers to a cyclic aromatic radical having from five to ten ring atoms of which one ring atom is selected from S, O, and N; zero, one, or two ring atoms are additional heteroatoms independently selected from S, O, and N; and the remaining ring atoms are carbon, the radical being joined to the rest of the molecule via any of the ring atoms, such as, for example, pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, and the like. It will be appreciated that aryl and heteroaryl groups can be unsubstituted or substituted, wherein substitution includes replacement of one, two, three, or more of the hydrogen atoms thereon independently with any one or more of the following moieties including, but not limited to, aliphatic; heteroaliphatic; aryl; heteroaryl; arylalkyl; heteroarylalkyl; alkoxy; aryloxy; heteroalkoxy; heteroaryloxy; alkylthio; arylthio; heteroalkylthio; heteroarylthio; -F; - Cl; -Br; -I; -OH; -NO₂; -CN; - CF₃; -CH₂CF₃; -CHCl₂; -CH₂OH; -CH₂CH₂OH; -CH₂NH₂; - CH₂SO₂CH₃; -C(O)Rₓ; - CO₂(Rₓ); -CON(Rx)₂; -OC(O)Rₓ; -OCO₂Rₓ; -OCON(Rₓ)₂; -N(Rx)₂; -S(O)₂Rₓ; - NRₓ(CO)Rₓ, wherein for each occurrence of Rₓ Rₓ is, preferably, individually and independently selected from aliphatic, heteroaliphatic, aryl, heteroaryl, arylalkyl, or heteroarylalkyl, wherein any of the aliphatic, heteroaliphatic, arylalkyl, or heteroarylalkyl substituents described above and herein may be substituted or unsubstituted, branched or unbranched, cyclic or acyclic, and wherein any of the aryl or heteroaryl substituents described above and herein may be substituted or unsubstituted. The term "cycloalkyl", as used herein, refers specifically to groups having three to seven, preferably three to ten carbon atoms. Suitable cycloalkyls include, but are not limited to cyclopropyl, cyclobutyl, cyclopentyl, cyclohexyl, cycloheptyl and the like, which, as in the case of other aliphatic, heteroaliphatic, or hetercyclic moieties, may optionally be substituted with substituents including, but not limited to aliphatic; heteroaliphatic; aryl; heteroaryl; arylalkyl; heteroarylalkyl; alkoxy; aryloxy; heteroalkoxy; heteroaryloxy; alkylthio; arylthio; heteroalkylthio; heteroarylthio; -F; - CI; -Br; -I; -OH; -NO₂; -CN; -CF₃; -CH₂CF₃; -CHCl₂; -CH₂OH; - CH₂CH₂OH; -CH₂NH₂; - CH₂SO₂CH₃; -C(O)Rₓ; -CO₂(Rₓ); -CON(Rₓ)₂; -OC(O)Rₓ; - OCO₂Rx; -0C0N(Rₓ)₂; -N(Rₓ)₂; - S(O)₂Rₓ; -NRₓ(CO)Rₓ, wherein for each occurrence of Rₓ Rₓ is, preferably, individually and independently selected from aliphatic, heteroaliphatic, aryl, heteroaryl, arylalkyl, or heteroarylalkyl, wherein any of the aliphatic, heteroaliphatic, arylalkyl, or heteroarylalkyl substituents described above and herein may be substituted or unsubstituted, branched or unbranched, cyclic or acyclic, and wherein any of the aryl or heteroaryl substituents described above and herein may be substituted or unsubstituted. Additional examples of generally applicable substituents are illustrated by the specific embodiments shown in the Examples that are described herein. The term "heteroaliphatic", as used herein, refers to aliphatic moieties that contain one or more oxygen, sulfur, nitrogen, phosphorus, or silicon atoms, e.g., in place of carbon atoms. Heteroaliphatic moieties may be branched, unbranched, cyclic or acyclic and include saturated and unsaturated heterocycles such as morpholino, pyrrolidinyl, etc. In certain embodiments, heteroaliphatic moieties are substituted by independent replacement of one or more of the hydrogen atoms thereon with one or more moieties including, but not limited to aliphatic; heteroaliphatic; aryl; heteroaryl; arylalkyl; heteroarylalkyl; alkoxy; aryloxy; heteroalkoxy; heteroaryloxy; alkylthio; arylthio; heteroalkylthio; heteroarylthio; -F; -Cl; -Br; -I; -OH; -NO₂; -CN; - CF₃; -CH₂CF₃; -CHCl₂; - CH₂OH; -CH₂CH₂OH; -CH₂NH₂; -CH₂SO₂CH₃; -C(O)Rₓ; - CO₂(Rₓ); -CON(Rx)₂; -OC(O)Rₓ; -OCO₂Rₓ; -OCON(Rₓ)₂; -N(Rx)₂; -S(O)₂R_{X}; - NRₓ(CO)Rₓ, wherein for each occurrence of Rₓ Rₓ is, preferably, individually and selected from aliphatic, heteroaliphatic, aryl, heteroaryl, arylalkyl, or heteroarylalkyl, wherein any of the aliphatic, heteroaliphatic, arylalkyl, or heteroarylalkyl substituents described above and herein may be substituted or unsubstituted, branched or unbranched, cyclic or acyclic, and wherein any of the aryl or heteroaryl substituents described above and herein may be substituted or unsubstituted.

The uni-partite modification as defined herein which allows the resolving step, i.e. which is used in separating or resolving the modified nucleic acid molecule fragments, i.e. species, may be linked to the nucleic acid molecule by a linker.

The bi-or multi-partite modification as defined herein which allows the resolving step, i.e. which is used in separating or resolving the modified nucleic acid molecule fragments, i.e. species, may be linked to each other and to the nucleic acid molecule by a linker.

"Linker" refers to one or more atoms forming a linking moiety connecting the nucleic acid molecule to the modification or the moietiy/moieties of the or forming the modification to each other and to the nucleic acid molecule, respectively. The function of the linker is to connect said moieties or molecules in either a permanent or non-permanent manner. In an embodiment, the non-permanent linkage is a cleavable linkage. The linker may be acyclic, cyclic, aryl, heteroaryl in character, or a combination of these. It may comprise solely a carbon atom backbone or may be heteroaliphatic as defined above. It may contain other moieties such as but not limited to aliphatic; heteroaliphatic; aryl; heteroaryl; arylalkyl; heteroarylalkyl; alkoxy; aryloxy; heteroalkoxy; heteroaryloxy; alkylthio; arylthio; heteroalkylthio; heteroarylthio; -F; -Cl; -Br; -I; -OH; -NO₂; -CN; - CF₃; -CH₂CF₃; -CHCl₂; -CH₂OH; - CH₂CH₂OH; -CH₂NH₂; -CH₂SO₂CH₃; -C(O)Rₓ; - CO₂(Rₓ); -CON(Rx)₂; -OC(O)Rₓ; - OCO₂Rₓ; -OCON(Rₓ)₂; -N(Rₓ)₂; -S(O)₂Rₓ; - NRₓ(CO)Rₓ, wherein for each occurrence of Rₓ Rₓ is, preferably, individually and independently selected from aliphatic, heteroaliphatic, aryl, heteroaryl, arylalkyl, or heteroarylalkyl, wherein any of the aliphatic, heteroaliphatic, arylalkyl, or heteroarylalkyl substituents described above and herein may be substituted or unsubstituted, branched or unbranched, cyclic or acyclic, and wherein any of the aryl or heteroaryl substituents described above and herein may be substituted or unsubstituted. The linker may contain a cleavage site or a cleavable moiety, preferably in its back bone, that allows the separation of the molecules and moieties, respectively, connected or linked by means of said linker. For example, cleaving of the linker, in one embodiment, will separate the modification such as a label from the nucleic acid molecule and a nucleic acid molecule fragment, respectively. Such cleavable linkers may be cleaved under acid, alkali, or reducing conditions. They may also be cleaved enyzmatically or by light. In the latter case, they are photocleavable linkers.

In connection with the present invention the resolving step where the various species of the nucleic acid molecule fragments are resolved by means of the modification according to their length, mass or charge, any technique may be used which is suitable insofar. Such techniques comprise but are not limited to chromatography and mass spectrometry and may be used in connection with both the first and the second procedure of the method according to the present invention. Particularly preferred techniques are mass spectrometry techniques which may be combined with chromatography.

As used herein reference to mass spectrometry encompasses any suitable mass spectrometric format known to those skilled in the art. Mass spectrometry techniques that allow an accurate analysis of nucleic acid molecules are preferred. For example, due to the excessive fragmentation of the nucleic acid molecules which occurs, the so called "hard" ionisation techniques such as the methods of Electron and Fast Atom Bombardment (FAB) ionization are not suitable for the analysis of nucleic acid molecules. Various mass spectrometric formats (ionization principles in combination with different mass analyzers) are known to those skilled in the art which use soft ionisation techniques. Such formats include Electrospray, Atmospheric Pressure Photo Ionisation (abbr. APPI), Atmospheric Pressure Chemical lonisation (abbr. APCI), Matrix Assisted Laser Desorption Ionisation (abbr. MALDI), Matrix Assisted Laser Desorption Ionisation Time-of-flight (abbr. MALDI-TOF), infrared matrix-assisted laser desorption/ionization mass spectrometry (abbr. IR-MALDI), Orthogonal-TOF (abbr. O-TOF), Axial-Tof (abbr. A-TOF), Ion Cyclotron Resonance (abbr. ICR), Fourier Tranform Linear/Reflectron (abbr. RETOF), Laser Desorption Ionisation (abbr. LDI), Fast Atom Bombardment (abbr. FAB), Desorption ElectroSpray Ionisation (abbr. DESI), Desorption Ionisation On Silica (abbr. DIOS), Liquid Secondary Ions Mass Spectrometry (abbr. LSIMS).

Electrospray ionization (abbr. ESI) involves the spraying of a dilute solution of the analyte from the tip of a capillary to which a high potential is applied. The spray is then effected by electrostatic forces that cause charge separation at the liquid surface and thus deformation of the emerging drop (Taylor cone). This finally disintegrates to yield thousands of micrometer sized droplets that further evolve into charged molecules that are then analysed. Due to the mildness of this technique and its preference for polar and ionic compounds, it has found ready application in the field of biopolymer analysis.

Atmospheric Pressure Photo Ionisation (abbr. APPI) is often used for the ionisation of nonpolar entities such as steroids that are difficult to ionize but the technique is also applicable to polar entities. It is a LC/MS ionization technique whereby the LC eluent is vaporized using a heater at atmospheric pressure. The resulting gas is channelled through a beam of photons generated by a discharge lamp (e.g. UV lamp) which ionizes the gas molecules.

Atmospheric Pressure Chemical Ionisation (abbr. APCI) involves heating analyte containing solutions (typically the mobile phase from HPLC) to temperatures exceeding 400 °C spraying with high flow rates of nitrogen and subjecting the resulting aerosol cloud to a Corona discharge creating ions. It differs from ESI in that it is a gas phase ionisation process instead of a liquid phase one. Typically, APCI produces more fragmentation than ESI.

Fast atom bombardment (abbr. FAB) involves the use of a high-energy beam of netural atoms, typically Xe or Ar, that strike a solid matrix containing sample causing desorption and ionisation. It is used for large biological molecules that are difficult to get into the gas phase. The atomic beam is produced by accelerating ions from an ion source through a charge-exchange cell. The ions pick up an electron in collisions with neutral atoms to form a beam of high energy atoms. The FAB spectrum typically contains few fragments and a signal for the pseudo molecular ion, (e. g. [M+H]+, [M+Na]+, adducts) making FAB useful for molecular weight determination. However, the matrix contributes many low *m*/*z* signals whose lack of reproducibility complicates the interpretation of the spectra. Furthermore, the method is prone to suppression effects by small impurities.

Matrix Assisted Laser Desorption Ionisation (abbr. MALDI) is a laser mediated method of vaporizing and ionizing large biological molecules such as proteins or DNA fragments. The biological molecules are dispersed in a solid matrix such as 3-hydroxypicolinic acid (3-HPA). A UV laser pulse ablates the matrix which carries some of the large molecules into the gas phase in an ionized form so they can be extracted into a mass spectrometer. The large range of MALDI allows the determination of molecular weights up to 500 kDa, routinely of a molecular weight of 5 to 100 kDa (i.e. e.g. polymers, biomolecules, complexes, enzymes), depending on the analyzer. The MALDI techniques can e.g. be coupled with a time-of-flight analyzer or a Fourier-transform mass spectrometer. The former has low resolution and accuracy while the latter is very accurate but has a low dynamic range and is more complicated in its operation.

Laser Desorption Ionisation (abbr. LDI) is the irradiation of molecules with high-intensity laser pulses, forming ions that are then analysed. Limitations of this early technique are a sharp cut-off in mass at about 5 to 10 kDa, and the need to couple it to TOF mass analysers. Desorption electrospray ionisation (abbr. DESI), is an ionisation technique whereby an Electrospray source creates charged droplets that are directed at a solid sample within a few millimetres to a few centimetres away. The charged droplets acquire the sample through interaction with the surface and then form highly charged ions that can be extracted into a mass spectrometer.

Desorption ionisation on silica (abbr. DIOS), is laser desorption/ionization of a sample deposited on a porous silicon surface

Surface-enhanced laser desorption/ionization (abbr. SELDI) variand is similar to MALDI, but uses a biochemical affinity target.

Surface-enhanced neat desorption (abbr. SEND) is a variant of MALDI where the matrix is covalently linked to the target surface.

Surface-assisted laser desorption/ionization (abbr. SALDI) can be described as MALDI using a liquid plus particulate matrix

Secondary Ions Mass Spectrometry (abbr. SIMS), involves bombarding an analyte coated surface with high energy primary ions to generate sample (secondary) ions. Energy transfer causes sample molecules to be desorbed into the gas phase, where they undergo ion/molecule reactions to form secondary ions. Once formed, the sample ions can be accelerated out of the source by application of a high voltage to extraction and focusing lenses. In a common variation of this method, known as Liquid Secondary Ion Mass Spectrometry (abbr. LSIMS), the analyte is dissolved in an involatile liquid matrix before being placed on the probe tip. Bombarding this mixture with primary ions (usually Cs+ at 35 keV) results in the formation of matrix ions and leads to indirect sample ionisation. In this respect, LSIMS is very similar to an older technique known as Fast Atom Bombardment (abbr. FAB), which also uses a matrix. As its name indicates, FAB ionisation employs a beam of fast neutral atoms (e.g. Ar), rather than an ion beam, but the mechanism of ionisation in FAB and LSIMS is the same - indeed the two terms are often confused.

Such ion sources as described above may be provided with an eluent over a period of time, the eluent having been separated from a mixture by means of liquid chromatography or capillary electrophoresis.

Tandem mass spectrometry can also be used to enhance the method as described in order to act as an additional confirmation of the fragment's identity. Tandem mass spectrometry, also known as MS/MS, involves multiple steps of mass spectrometry selection, with some form of fragmentation occurring in between the stages. The applicability of tandem mass spectrometry for sequence identification of nucleic acid molecules can be looked up in several review articles (Limbach, 1996; Nordhoff et al, 1996; Wu & McLuckey, 2004) Gas-phase fragmentation by collision-induced dissociation (CID) can be done using tandem mass spectrometry using e.g. ion trap, Fourier transform ion cyclotron resonance and triple-quadrupole analyzers (Baker et al, 1993; Boschenok & Sheil, 1996; Kawase et al, 1991; Limbach et al, 1995; Little et al, 1995; Marzilli et al, 1999; Ni et al, 1996). Such fragments of unmodified and modified nucleic acid molecules can be generated via post source decay and prompt fragmentation following MALDI-MS (Juhasz et al, 1996; Stemmler et al, 1993; Talbo & Mann, 1996).

Liquid Chromatography - Mass Spectrometry (abbr. LC-MS) allows separation of complex mixtures of non-volatile compounds before introduction to the mass spectrometer. It is used extensively for compounds that have a high molecular weight or are too sensitive to heat to be analyzed by GC. The most common ionization methods that are interfaced to LC are ESI and Atmospheric Chemical Ionization (abbr. APCI) in positive and negative-ion modes. The LC is done in most cases by reverse-phase high-performance liquid chromatography (abbr. RP-HPLC).

The basis for Fourier Transform Mass Spectrometry (abbr. FTMS) which may also be used in the resolving of the various species of the modified nucleic acid molecule species is an ion trap (Penning cell) that allows ions formed by such techniques as electron impact ionization, chemical ionisation, MALDI, and ESI to be accumulated and stored for time periods as long as minutes. During this time, reactions of the ions with neutral molecules can be followed. The method has the highest resolving power in mass spectrometry, a high upper mass limit, high sensitivity, non-destructive detection, and high accuracy for mass measurement. Because it uses Fourier transform detection, signal averaging and simultaneous wide-mass detection are possible.

It is within the present invention that the mass spectrum refers to the presentation of data obtained from analyzing a nucleic acid molecule or fragment thereof by mass spectrometry (either graphically or encoded numerically). It is also within the present invention that the mass spectrum is an embodiment of the pattern generated in the separating or resolving step.

The fragmentation pattern of a nucleic acid molecule with reference to a mass spectrum refers to a characteristic distribution and number of signals (such as peaks or digital representations thereof). In general, a fragmentation pattern as used herein refers to a set of fragments that are generated by specific cleavage of the nucleic acid molecule. Such fragment pattern is an embodiment of the pattern generated in the separating or resolving step.

The utility of any mass spectrometric sequencing method that relies on consecutive backbone cleavage depends on the formation of a mass ladder. The sequence information is obtained by determining the mass difference between successive peaks in the mass spectrum. In the case of oligodeoxynucleotides, the expected mass difference between successive peaks will correspond to the loss of: dC = 289.05, dT = 304.05, dA = 313.06, and dG = 329.05 (Exact massbased values). With oligoribonucleotides, the mass difference will be: C = 305.04, U = 306.03, A = 329.05, and dG = 345.05 (Exact mass-based values).

As used herein, mass signal in the context of a mass spectrometry refers to the output data, which is the number or relative number of molecules having a particular mass. Signals include "peaks" and digital representations thereof. It is well known that mass spectrometers measure "mass to charge ratios" (*m*/*z*) instead of the actual "molecular mass" of the sample components. The calibration of the particular mass spectrometer used should be conducted before experimentation. For mass spectrometers that detect multiply charged molecules (e.g. when using Electrospray Ionization), the roughly estimated mass can e.g. be determined by multiplying the mass-to-charge- value obtained by the number of charges on the molecule. In practice, the calculation of the neutral molecular mass is perfomed by the application of software packages using a process called deconvolution. Accordingly, each of the methods known in the art for detecting, determining, and/or calculating mass can be used for obtaining the mass encompassed by the methods provided herein.

As used herein, the "deconvoluted mass" refers either to the average molecular mass or to the monoisotopic exact molecular mass. The use of the exact molecular mass is limited by the resolution of the mass analyzer. When molecules with a higher molecular mass are analyzed, it can become more difficult to elucidate the isotopic pattern of a compound. In such cases, the average mass can be used for identification of compounds exhibiting a higher molecular mass. However, when exact masses are used, the monoisotopic mass needs to be applied because this allows for a discrimination between C and U. However, it has to be verified that the monoisotopic mass is present in a suitable abundance in order to be identified.

As used herein, the term "peaks" refers to prominent upward projections from a baseline signal of a mass spectrometer spectrum ("mass spectrum") which corresponds to the mass and intensity of a fragment. Peaks can be extracted from a mass spectrum by a manual or automated "peak finding" procedure.

As used herein, the mass of a peak in a mass spectrum refers to the mass computed by the "peak finding" procedure.

As used herein, the intensity of a peak in a mass spectrum refers to the intensity computed by the "peak finding" procedure that is dependent on parameters including, but not limited to, the height of the peak in the mass spectrum and its signal-to-noise ratio.

The calculated mass as preferably used herein is defined as the theoretical mass of a molecule or fragment as determined by the summation of the mass contributions from the individual elements that the molecule comprises of as determined by its molecular formula. The mass calculated can either be the exact or the molecular mass depending on whether the exact masses or the average masses of the elements are used. For instance, the calculated mass of a molecule with a molecular formula of C3H6O2 would have a calculated monoisotopic exact mass of 74.037 Daltons, as derived from the equation: (3 x 12.000) + (6 x 1.0078) + (2 x 15.9949), whereas the average mass would be 74.079 Daltons, taking the naturally most abundant isotopes into account..

The observed mass as preferably used herein is the mass value that is experimentally found by the mass spectrometer.

The exact mass as preferably used herein is the exact molecular mass of the molecule, where atomic masses of each atom are based on the monoisotopic formst common isotope for each the element.

The exact mass observed as preferably used herein is the exact monoisotopic molecular mass of the molecule, as determined experimentally using a mass spectrometer.

The exact mass calculated as preferably used herein is the exact monoisotopic molecular mass of the molecule, as determined theoretically by the summation of mass contributions from the individual monoisotopic elements that the molecule is comprised of, as determined by its molecular formula.

The average molecular weight as preferably used herein is the average molecular mass of the structure, where atomic masses are based on the natural abundance of all isotopes of the element.

The average molecular weight observed as preferably used herein is the average molecular mass of a molecule, as determined experimentally using a mass spectrometer.

The average molecular weight calculated as preferably used herein is the average molecular mass of a molecule as determined theoretically by using the atomic weight of all elements the molecule is comprised of, as determined by its molecular formula.

It will be acknowledged that the methods according to the present invention may also be used in connection with specific nucleic acid molecules. Such specific nucleic acid molecules are, for example, aptamers, Spiegelmers, antisense molecules, ribozymes, decoy oligonucleotides and siRNA molecules. In preferred embodiment this kind of specific nucleic acid molecules are used in the therapeutic, diagnostic and/or cosmetic field.

It is within the present invention that the single-stranded nucleic acid molecules can form distinct and stable three-dimensional structures and specifically bind to a target molecules like antibodies. Such nucleic acid molecules composed of D-nucleotides are called aptamers. Aptamers can be identified against several target molecules, e.g. small molecules, proteins, nucleic acids, and even cells, tissues and organisms and can inhibit the *in vitro* and/or *in vivo* function of the specific target molecule. Aptamers are usually identified by a target-directed selection process, called *in vitro* selection or Systematic Evolution of Ligands by Exponential Enrichment (abbr. SELEX) (Bock et al, 1992; Ellington & Szostak, 1990; Tuerk & Gold, 1990). Non-modified aptamers are cleared rapidly from the bloodstream, with a half-life of minutes to hours, mainly due to nuclease degradation and clearance from the body by the kidneys, a result of the aptamer's inherently low molecular weight. Hence, in order to use aptamers therapeutically they have to be modified at the 2' position of the sugar (e.g. ribose) backbone (Cload et al, 2006).

The omnipresent nucleases which account for the instability of aptamers consist of chiral building blocks, i.e. L-amino acids. Consequently, the structure of nucleases is inherently chiral as well, resulting in stereospecific substrate recognition. Hence, these enzymes only accept substrate molecules in the adequate chiral configuration. Since aptamers and naturally occurring nucleic acid molecules are composed of D-nucleotides, an L-oligonucleotide should escape from enzymatic recognition and subsequent degradation. Due to the same principle, unfortunately in this case, nature developed no enzymatic activity to amplify such mirror-image nucleic acids. Accordingly, L-nucleic acid aptamers cannot be directly obtained employing the SELEX process. The principles of stereochemistry, though, reveal a detour which eventually leads to the desired functional L-nucleic acid aptamers.

If an in vitro selected (D-)aptamer binds its natural target, the structural mirror-image of this aptamer binds with the same characteristics the mirror-image of the natural target. Here, both interaction partners have the same (unnatural) chirality. Due to the homochirality of life and most biochemical compounds, such enantio-RNA ligands, of course, would be of limited practical use. If, on the other hand, the SELEX process is carried out against an (unnatural) mirror-image target, an aptamer recognizing this (unnatural) target will be obtained. The corresponding mirror-image configuration of said aptamer - the desired L-aptamer - in turn recognizes the natural target. This mirror-image selection process for the generation of biostable nucleic acid molecule was published first in 1996 (Klussmann et al, 1996; Nolte et al, 1996) and results in the generation of functional mirror-image nucleic acid molecule ligands that display not only high affinity and specificity for a given target molecule, but at the same time also biological stability. It is within the present invention that the single-stranded nucleic acid molecule is such a ligand-binding L-nucleic acid molecule that is referred as 'Spiegelmer' (from the German word 'Spiegel', mirror) (Eulberg et al, 2006).

It is within the present invention that the nucleic acid molecules disclosed herein, preferably a spiegelmer or aptamer, comprise a moiety which preferably is a high molecular weight moiety and/or which preferably allows to modify the characteristics of the nucleic acid molecules in terms of, among others, residence time in the animal body, preferably the human body. A particularly preferred embodiment of such modification is PEGylation and HESylation of the nucleic acid molecule as used herein PEG stands for poly(ethylene glycole) and HES for hydroxyethly starch. PEGylation as preferably used herein is the modification of a nucleic acid molecule whereby such modification consists of a PEG moiety which is attached to a nucleic acid molecule. HESylation as preferably used herein is the modification of a nucleic acid molecule, whereby such modification consists of a HES moiety which is attached to a nucleic acid molecule. These modifications as well as the process of modifying a nucleic acid molecule using such modifications, is described in European patent application EP 1 306 382.

Preferably, the molecular weight of a modification consisting of or comprising a high molecular weight moiety is about from 2,000 to 250,000 Da, preferably 20,000 to 200,000 Da. In the case of PEG being such high molecular weight moiety the molecular weight is preferably 20,000 to 120,000 Da, more preferably 40,000 to 80,000 Da. In the case of HES being such high molecular weight moiety the molecular weight is preferably 20,000 to 200,000 Da, more preferably 40,000 to 150,000 Da. The process of HES modification is, e.g., described in German patent application DE 1 2004 006 249.8.

It is within the present invention that either of PEG and HES may be used as either a linear or branched from as further described in the patent applications WO 2005/074993 and WO-2003/035665. Such modification can, in principle, be made to the nucleic acid molecules at any position thereof. Preferably such modification is made either to the 5' -terminal nucleotide, the 3'-terminal nucleotide and/or any nucleotide between the 5' nucleotide and the 3' nucleotide of the nucleic acid molecule.

The modification and preferably the PEG and/or HES moiety can be attached to the nucleic acid molecule of the present invention either directly or through a linker. It is also within the present invention that the nucleic acid molecule according to the present invention comprises one or more modifications, preferably one or more PEG and/or HES moiety. In an embodiment the individual linker molecule attaches more than one PEG moiety or HES moiety to a nucleic acid molecule according to the present invention. The linker used in connection with the present invention can itself be either linear or branched. This kind of linkers are known to the ones skilled in the art and are further described in the patent applications WO2005074993 and WO2003035665.

In a preferred embodiment the linker is a biodegradable linker. The biodegradable linker allows to modify the characteristics of the nucleic acid according to the present invention in terms of, among other, residence time in the animal body, preferably in the human body, due to release of the modification from the nucleic acid according to the present invention. Usage of a biodegradable linker may allow a better control of the residence time of the nucleic acid according to the present invention. A preferably embodiment of such biodegradable linker are biodegradable linker as described in but not limited to the international patent applications WO2006/052790, WO2008/034122, WO2004/092191 and WO2005/099768, whereby in the international patent applications WO2004/092191 and WO2005/099768, the linker is part of a polymeric oligonucleotide prodrug that consists of one or two modifications as described herein, a nucleic acid molecule and the biodegradable linker in between.

It is within the present invention that the modification of the nucleic acid molecule is a biodegradable modification, whereby the biodegradable modification can be attached to the nucleic acid molecule either directly or through a linker. The biodegradable modification allows to modify the characteristics of the nucleic acid molecule in terms of, among other, residence time in the animal body, preferably in the human body, due to release of the modification from the nucleic acid molecule. Usage of biodegradable modification may allow a better control of the residence time of the nucleic acid molecule. A preferably embodiment of such biodegradable modification are biodegradable as described in but not restricted to the international patent applications WO 2002/065963, WO 2003/070823, WO 2004/113394 and WO 2000/41647.

The term "biodegradable" as used herein, refers to degradation in a biological system, for example enzymatic degradation or chemical degradation.

Beside the modifications as described supra, other modifications can be used to modify the characteristics of the nucleic acids according to the present invention, whereby such modifications are selected from the group of proteins, lipids such as cholesterol and sugar chains such as amylase, dextran etc.

Antisense nucleic acid molecules are single-stranded nucleic acid molecules as well. Antisense nucleic acid molecules specifically binds to the mRNA strand, by what mRNA is blocked for the transcription of the mRNA into the gene product. Moreover the mRNA is degraded by RNAseH digestion (Scherer & Rossi, 2003). Antisense nucleic acid molecules are composed of D-nucleic acid molecules like RNA, modified RNA, DNA, modified DNA, PNA, LNA and combinations thereof.

Ribozymes are single-stranded D-nucleic acid molecules that catalyze a chemical reaction. Many natural ribozymes catalyze either their own cleavage or the cleavage of other RNAs, e.g. mRNAs. Ribozymes bind the mRNA strand and cleaves it specifically. By this cleavage or degradation of the target-specific mRNA molecule, the expression of the target molecule is avoided (Usman & Blatt, 2000).

As alterations in gene expression have become a better understood component of normal development and disease pathogenesis, transcription factors and other regulators of gene expression have become an increasingly attractive target for potential therapeutic intervention. Transcription factors are generally nuclear proteins that play a critical role in gene regulation and can exert either a positive or negative effect on gene expression. These regulatory proteins bind specific sequences found in the promoter regions of their target genes. These binding sequences are generally 6 to 10 base pairs in length and are occasionally found in multiple iterations. Because transcription factors can recognize their relatively short binding sequences even in the absence of surrounding genomic DNA, short radiolabeled oligodeoxynucleotides

(abbr. ODNs) bearing consensus binding sites can serve as probes in electrophoretic mobility shift assays, which identify and quantify transcription factor binding activity in nuclear extracts. More recently, ODNs bearing the consensus binding sequence of a specific transcription factor have been explored as tools for manipulating gene expression in living cells. This strategy involves the intracellular delivery of such "decoy" ODNs, which are then recognized and bound by the target factor. Occupation of the transcription factor's DNA-binding site by the decoy renders the protein incapable of subsequently binding to the promoter regions of target genes (Mann & Dzau, 2000). The use of decoy ODNs for the therapeutic manipulation of gene expression was firstly described by Morishita et al. in 1995 (Morishita et al, 1995). They reported the treatment of rat carotid arteries at the time of balloon injury with ODNs bearing the consensus binding site for the E2F family of transcription factors and found that a decoy specific to E2F-1 prevented this upregulation and blocked smooth muscle proliferation and neointimal hyperplasia in injured vessels (Morishita et al, 1995). In addition to this initial *in vivo* application, a transcription factor decoy was used to block a negative regulatory element in the promoter of the Renin gene in the mouse submandibular gland, demonstrating that decoys can be used to increase as well as to suppress gene activity in vivo (Tomita et al, 1999).

The basic design of siRNA molecules, miRNA molecules or RNAi molecules, which mostly differ in the size, is basically such that the nucleic acid molecule comprises a double-stranded structure. The double-stranded structure comprises a first strand and a second strand. More preferably, the first strand comprises a first stretch of contiguous nucleotides and the second stretch comprises a second stretch of contiguous nucleotides. At least the first stretch and the second stretch are essentially complementary to each other. Such complementarity is typically based on Watson-Crick base pairing or other base-pairing mechanism known to the one skilled in the art, including but not limited to Hoogsteen base-pairing and others. It will be acknowledged by the one skilled in the art that depending on the length of such double-stranded structure a perfect match in terms of base complementarity is not necessarily required. However, such perfect complementarity is preferred in some embodiments. A mismatch is also tolerable, mostly under the proviso that the double-stranded structure is still suitable to trigger the RNA interference mechanism, and that preferably such double-stranded structure is still stably forming under physiological conditions as prevailing in a cell, tissue and organism, respectively, containing or in principle containing such cell, tissue and organ. More preferably, the double-stranded structure is stable at 37 °C in a physiological buffer.

The first stretch, is typically at least partially complementary to a target nucleic acid and the second stretch is, particularly given the relationship between the first and second stretch, respectively, in terms of base complementarity, at least partially identical to the target nucleic acid. The target nucleic acid is preferably an mRNA, although other forms of RNA such as hnRNAs are also suitable for such purpose. Such siRNA molecule, miRNA molecule and RNAi molecule respectively, is suitable to trigger the RNA interference response resulting in the knock-down of the mRNA for the target molecule. Insofar, this kind of nucleic acid molecule is suitable to decrease the expression of a target molecule by decreasing the expression at the level of mRNA.

Although RNA interference can be observed upon using long nucleic acid molecules comprising several dozens and sometimes even several hundreds of nucleotides and nucleotide pairs, respectively, shorter siRNA molecules, miRNA molecules and RNAi molecules are generally preferred. A more preferred range for the length of the first stretch and/or second stretch is from about 15 to 29 consecutive nucleotides, preferably 19 to 25 consecutive nucleotides and more preferably 19 to 23 consecutive nucleotides. More preferably, both the first stretch and the second stretch have the same length. In a further embodiment, the double-stranded structure comprises preferably between 15 and 29, preferably 18 to 25, more preferably 19 to 23 and most preferably 19 to 21 base pairs.

It will be acknowledged by the ones skilled in the art that the particular design of the siRNA molecules, miRNA molecules, the RNAi molecules and other nucleic acids mediating RNAi, respectively, can vary in accordance with the current and future design principles. For the time being some design principles of the siRNA molecules, miRNA molecules and the RNAi molecules and other nucleic acids mediating RNAi, respectively, exist. The design principles of the siRNA molecules, miRNA molecules and the RNAi molecules and other nucleic acids mediating RNA are described in the international patent application WO/2008/052774.

Irrespective of these various designs of siRNA, it will be acknowledged by the ones skilled in the art that according to their origin or function, three types of naturally occurring small RNA have been described: short interfering RNAs (abbr. siRNAs), repeat-associated short interfering RNAs (abbr. rasiRNAs) and microRNAs (abbr. miRNAs). In nature, dsRNA can be produced by RNA-templated RNA polymerization (for example, from viruses) or by hybridization of overlapping transcripts (for example, from repetitive sequences such as transgene arrays or transposons). Such dsRNAs give rise to siRNAs or rasiRNAs, which generally guide mRNA degradation and/or chromatin modification. In addition, endogenous transcripts that contain complementary or near-complementary 20 to 50 base-pair inverted repeats fold back on themselves to form dsRNA hairpins. These dsRNAs are processed into miRNAs that mediate translational repression, although they may also guide mRNA degradation. Finally, artificial introduction of long dsRNAs or siRNAs has been adopted as a tool to inactivate gene expression, both in cultured cells and inliving organisms (Meister & Tuschl, 2004).

As preferably used the term mass discrimination means that the separation of the modified nucleic acid molecule fragments from the unmodified or non-modified nucleic acid molecule fragments is based and performed on differences in mass between both the modified nucleic acid molecule fragments and the unmodified nucleic acid fragments.

As preferably used the term size discrimination means that the separation of the modified nucleic acid molecule fragments from the unmodified or non-modified nucleic acid molecule fragments is based and performed on differences in size between both the modified nucleic acid molecule fragments and the unmodified nucleic acid fragments.

As preferably used the term hydrophobicity discrimination means that the separation of the modified nucleic acid molecule fragments from the unmodified or non-modified nucleic acid molecule fragments is based and performed on differences in hydrophobicity between both the modified nucleic acid molecule fragments and the unmodified nucleic acid fragments.

As preferably used the term charge discrimination means that the separation of the modified nucleic acid molecule fragments from the unmodified or non-modified nucleic acid molecule fragments is based and performed on differences in charge between both the modified nucleic acid molecule fragments and the unmodified nucleic acid fragments.

As preferably used the term ionic discrimination means that the separation of the modified nucleic acid molecule fragments from the unmodified or non-modified nucleic acid molecule fragments is based and performed on differences in ionic strength between both the modified nucleic acid molecule fragments and the unmodified nucleic acid fragments.

As preferably used the term hydrogen bonding discrimination means that the separation of the modified nucleic acid molecule fragments from the unmodified or non-modified nucleic acid molecule fragments is based and performed on differences in hydrogen bonding, preferably the extent of such hydrogen bonding between both the modified nucleic acid molecule fragments and the unmodified nucleic acid fragments.

As preferably used the term mass discrimination means that the separation of the modified nucleic acid molecule fragments from the unmodified or non-modified nucleic acid molecule fragments is based and performed on differences in mass between both the modified nucleic acid molecule fragments and the unmodified nucleic acid fragments.

With regard to the fact that this kind of specific nucleic acid molecules are used in the therapeutic, diagnostic and/or cosmetic field, the method according to the present invention may be used not only for determining the nucleotide sequence of the nucleic acid molecule, but also in quality control of preparations containing one or several of this kind of specific nucleic acid molecules. Insofar, the present invention is also related to a method of quality control which comprises the steps of determining the nucleotide sequence of a nucleic acid molecule according to the instant invention, whereby such nucleic acid molecule is contained in the preparation or a sample, whereby the preparation and sample, respectively, has been provided in a preceding step.

It is within the present invention that the nucleic acid molecule the nucleotide sequence of which is to be determined is not necessarily the full length nucleic acid molecule. Rather, it might be sufficient that only one or several parts of such full length nucleic acid molecule is used as the nucleic acid molecule the nucleotide sequence of which is to be determined by the method according to the present invention.

It is also within the present invention that the method of the invention is a method for determining the fingerprint of a nucleic acid molecule. A fingerprint of a nucleic acid molecule, as preferably used herein, is a characteristic pattern of fragments of the nucleic acid molecule. In other words, for the identification of a nucleic acid molecule or a fingerprint thereof, it is sometimes not necessary to know the exact nucleotide sequence but such characteristic pattern. Such characteristic pattern is, in a preferred embodiment, the pattern obtained in the step of the method according to the present invention where the modified nucleic acid molecule fragments are resolved and separated, respectively. It is to be acknowledged that such method for the identification or determination of a fingerprint of a nucleic acid molecule otherwise comprises the same step as the method for determining the nucleotide sequence according to the present invention.

The various SEQ.ID.Nos., the chemical nature of the nucleic acid molecules as used herein, the actual sequence thereof and the internal reference number is summarized in the following table.

**TABLE 1 (A) OLIGONUCLEOTIDE SEQUENCES REFERRED TO IN THIS APPLICATION**

| **Seq.-ID** | ***Type of RNA*** | **Sequence** | ***Internal Reference*** |
|---|---|---|---|
| 1 | L-RNA | **GCA CGU CCC UCA CCG GUG CAA GUG AAG CCG UGG CUC UGC G** | NOX-E36 |
| 2 | L-RNA | NH₂-(CH₂)₆-OP(O)(OH)-**GCA CGU CCC UCA CCG GUG CAA GUG AAG CCG UGG CUC UCC G** | NOX-E36 Intermediate |
| 3 | L-RNA | NH2-(CH2)6-OP(O)(OH)-GCA CGU CCC CUA CCG GUG CAA GUG AAG CCG UGG UCC UGC G | NOX-E36 mismatch control 01 |
| 4 | L-RNA | HOP(O)(OH)-**GCG** | |
| 5 | L-RNA | HOP(O)(OH)-**CUG CG** | |
| 6 | L-RNA | HOP(O)(OH)-**GGC UCU GCG** | |
| 7 | L-RNA | HOP(O)(OH)-**GAA GCC GUG GCU CUG CG** | |
| 8 | L-RNA | HOP(O)(OH)-**GCA AGU GAA GCC GUG GCU CUG CG** | |
| 9 | L-RNA | HOP(O)(OH)-**CAC CGG UGC AGU GAA GCC GUG GCU CU GCG** | |
| 10 | L-RNA | HOP(O)(OH)-**CCC UCA CCG GUG CAA GUG AAG CCG UGG CUC UGC G** | |
| 11 | L-RNA | HS-(CH₂)₂C(O)-NH-(CH₂)₆-OP(O)(OH)-**G_{cp}** | |
| 12 | L-RNA | HS-(CH₂)₂C(O)-NH-(CH₂)₆-OP(O)(OH)-**GC_{cp}** | |
| 13 | L-RNA | HS-(CH₂)₂C(O)-NH-(CH₂)₆-OP(O)(OH)-**GCA_{cp}** | |
| 14 | L-RNA | HS-(CH₂)₂C(O)-NH-(CH₂)₆-OP(O)(OH)-**GCA C_{cp}** | |
| 15 | L-RNA | HS-(CH₂)₂C(O)-NH-(CH₂)₆-OP(O)(OH)-**GCA CG_{cp}** | |
| 16 | L-RNA | HS-(CH₂)₂C(O)-NH-(CH₂)₆-OP(O)(OH)-**GCA CGU_{cp}** | |
| 17 | L-RNA | HS-(CH₂)₂C(O)-NH-(CH₂)₆-OP(O)(OH)-**GCA CGU C_{cp}** | |
| 18 | L-RNA | HS-(CH₂)₂C(O)-NH-(CH₂)₆-OP(O)(OH)-**GCA CGU CC_{cp}** | |
| 19 | L-RNA | HS-(CH₂)₂C(O)-NH-(CH₂)₆-OP(O)(OH)-**GCA CGU CCC_{cp}** | |
| 20 | L-RNA | HS-(CH₂)₂C(O)-NH-(CH₂)₆-OP(O)(OH)-**GCA CGU CCC U_{cp}** | |
| 21 | L-RNA | HS-(CH₂)₂C(O)-NH-(CH₂)₆-OP(O)(OH)-**GCA CGU CCC UC_{cp}** | |
| 22 | L-RNA | HS-(CH₂)₂C(O)-NH-(CH₂)₆-OP(O)(OH)-**GCA CGU CCC UCA_{cp}** | |
| 23 | L-RNA | HS-(CH₂)₂C(O)-NH-(CH₂)₆-OP(O)(OH)-**GCA CGU CCC UCA C_{cp}** | |
| 24 | L-RNA | HS-(CH₂)₂C(O)-NH-(CH₂)₆-OP(O)(OH)-**GCA CGU CCC UCA CC_{cp}** | |
| 25 | L-RNA | HS-(CH₂)₂C(O)-NH-(CH₂)₆-OP(O)(OH)-**GCA CGU CCC UCA CCG_{cp}** | |
| 26 | L-RNA | HS-(CH₂)₂C(O)-NH-(CH₂)₆-OP(O)(OH)-**GCA CGU CCC UCA CCG G_{cp}** | |
| 27 | L-RNA | HS-(CH₂)₂C(O)-NH-(CH₂)₆-OP(O)(OH)-**GCA CGU CCC UCA CCG GU_{cp}** | |
| 28 | L-RNA | HS-(CH₂)₂C(O)-NH-(CH₂)₆-OP(O)(OH)-**GCA CGU CCC UCA CCG GUG_{cp}** | |
| 29 | L-RNA | HS-(CH₂)₂C(O)-NH-(CH₂)₆-OP(O)(OH)-**GCA CGU CCC UCA CCG CUG C_{cp}** | |
| 30 | L-RNA | HS-(CH₂)₂C(O)-NH-(CH₂)₆-OP(O)(OH)-**GCA CGU CCC UCA CCG GUG CA_{cp}** | |
| 31 | L-RNA | HS-(CH₂)₂C(O)-NH-(CH₂)₆-OP(O)(OH)-**GCA CGU CCC UCA CCG GUG CAAₚ** | |
| 32 | L-RNA | HS-(CH₂)₂C(O)-NH-(CH₂)₆-OP(O)(OH)-**GCA CGU CCC UCA CCG GUG CAA G_{cp}** | |
| 33 | L-RNA | HS-(CH₂)₂C(O)-NH-(CH₂)₆-OP(O)(OH)-**GCA CGU CCC UCA CCG GUG CAA GU_{cp}** | |
| 34 | L-RNA | HS-(CH₂)₂C(O)-NH-(CH₂)₆-OP(O)(OH)-**GCA CGU CCC UCA CCG GUG CAA GUG_{cp}** | |
| 35 | L-RNA | HS-(CH₂)₂C(O)-NH-(CH₂)₆-OP(O)(OH)-**GCA CGU CCC UCA CCG GUG CAA GUG A_{cp}** | |
| 36 | L-RNA | HS-(CH₂)₂C(O)-NH-(CH₂)₆-OP(O)(OH)-**GCA CGU CCC UCA CCG GUG CAA GUG AA_{cp}** | |
| 37 | L-RNA | HS-(CH₂)₂C(O)-NH-(CH₂)₆-OP(O)(OH)-**GCA CGU CCC UCA CCG GUG CAA GUG AAG_{cp}** | |
| 38 | L-RNA | HS-(CH2)2C(O)-NH-(CH2)6-OP(O)(OH)-GCA CGU CCC UCA CCG GUG CAA GUG AAG Ccp | |
| 39 | L-RNA | HS-(CH2)2C(O)-NH-(CH2)6-OP(O)(OH)-GCA CGU CCC UCA CCG GUG CAA GUG AAG CCSD | |
| 40 | L-RNA | HS-(CH2)2C(O)-NH-(CH2)6-OP(O)(OH)-GCA CGU CCC UCA CCG GUG CAA GUG AAG CCGsp | |
| 41 | L-RNA | HS-(CH2)2C(O)-NH-(CH2)6-OP(O)(OH)-GCA CGU CCC UCA CCG GUG CAA GUG AAG CCG UcD | |
| 42 | L-RNA | HS-(CH2)2C(O)-NH-(CH2)6-OP(O)(OH)-GCA CGU CCC UCA CCG GUG CAA GUG AAG CCG UGcg | |
| 43 | L-RNA | HS-(CH2)2C(O)-NH-(CH2)6-OP(O)(OH)-GCA CGU CCC UCA CCG GUG CAA GUG AAG CCG UGG[?] | |
| 44 | L-RNA | HS-(CH2)2C(O)-NH-(CH2)6-OP(O)(OH)-GCA CGU CCC UCA CCG GUG CAA GUG AAG CCG UGG Ccn | |
| 45 | L-RNA | HS-(CH2)2C(O)-NH-(CH2)6-OP(O)(OH)-GCA CGU CCC UCA CCG GUG CAA GUG AAG CCG UGG CUsp | |
| 46 | L-RNA | HS-(CH2)2C(O)-NH-(CH2)6-OP(O)(OH)-GCA CGU CCC UCA CCG GUG CAA GUG AAG CCG UGG CUCcp | |
| 47 | L-RNA | HS-(CH2)2C(O)-NH-(CH2)6-OP(O)(OH)-GCA CGU CCC UCA CCG GUG CAA GUG AAG CCG UGG CUC Ucd | |
| 48 | L-RNA | HS-(CH2)2C(O)-NH-(CH2)6-OP(O)(OH)-GCA CGU CCC UCA CCG GUG CAA GUG AAG CCG UGG CUC UGcp | |
| 49 | L-RNA | HS-(CH2)2C(O)-NH-(CH2)6-OP(O)(OH)-GCA CGU CCC UCA CCG GUG CAA GUG AAG CCG UGG CUC UGCcp | |
| 50 | L-RNA | HS-(CH2)2C(O)-NH(CH2)6-OP(O)(OH)-GCA CGU CCC UCA CCG GUG CAA GUG AAG CCG UGG CUC UGC G | released acylated NOX-E36 Intermediate |
| 51 | L-RNA | FITC-NH-(CH₂)₆-OP(O)(OH)-**G_{cp}** | |
| 52 | L-RNA | FITC-NH-(CH₂)₆-OP(O)(OH)-**GC_{cp}** | |
| 53 | L-RNA | FITC-NH-(CH₂)₆-OP(O)(OH)-**GCA_{cp}** | |
| 54 | L-RNA | FITC-NH-(CH₂)₆-OP(O)(OH)-**GCA C_{cp}** | |
| 55 | L-RNA | FITC-NH-(CH₂)₆-OP(O)(OH)-**GCA CG_{cp}** | |
| 56 | L-RNA | NH₂-(CH₂)₆-OP(O)(OH)-**GCA CG**-OP(O)(OH)-OCH₂CH(OH)CH=C(OH)-CH=NPh | |
| 57 | L-RNA | HOP(O)(OH)-**CGC**-OP(O)(OH)-OCH₂CH(OH)CH=C(OH)-CH=NPh | |
| 58 | L-RNA | HOP(O)(OH)-**CAC CGG**-OP(O)(OH)-OCH₂CH(OH)CH=C(OH)-CH=NPh | |
| 59 | L-RNA | HOP(O)(OH)-**GCA AG**-OP(O)(OH)-OCH₂CH(OH)CH=C(OH)-CH=NPh | |
| 60 | L-RNA | HOP(O)(OH)-**GAA GCC G**-OP(O)(OH)-OCH₂CH(OH)CH=C(OH)-CH=NPh | |
| 61 | L-RNA | HOP(O)(OH)-**GGC**-OP(O)(OH)-OCH₂CH(OH)CH=C(OH)-CH=NPh | |
| 62 | L-RNA | HOP(O)(OH)-**GCC-**OP(O)(OH)-OCH₂CH(OH)CH=C(OH)-CH=NPh | |
| 63 | L-RNA | Biotinyl-NH(CH2)NH-C(O)-(CH2)2-SS-(CH2)2C(O)-NH(CH2)6-OP(O)(OH)-GCA CGU CCC UCA CCG GUG CAA GUG AAG CCG UGG CUC UGC G | biotin labeled NOX-E36 Intermediate |
| 64 | L-RNA | **GCG UGG UGU GAU CUA GAU GUA UUG GCU GAU CCU AGU CAG GUA CGC** | NOX-A12 |
| 65 | L-RNA | NH2-(CH2)6-OP(O)(OH)O-GCG UGG UGU GAU CUA GAU GUA UUG GCU GAU CCU AGU CAG GUA CGC | NOX-A12 Intermediate |
| 66 | L-RNA | FITC-NH-(CH₂)₆-OP(O)(OH)-**GCA CGU_{cp}** | |
| 67 | L-RNA | FITC-NH-(CH₂)₆-OP(O)(OH)-**GCA CGU C_{cp}** | |
| 68 | L-RNA | FITC-NH-(CH₂)₆-OP(O)(OH)-**GCA CGU CC_{cp}** | |
| 69 | L-RNA | FITC-NH-(CH₂)₆-OP(O)(OH)-**GCA CGU CCC_{cp}** | |
| 70 | L-RNA | FITC-NH-(CH₂)₆-OP(O)(OH)-**GCA CGU CCC U_{cp}** | |
| 71 | L-RNA | FITC-NH-(CH₂)₆-OP(O)(OH)-**GCA CGU CCC UC_{cp}** | |
| 72 | L-RNA | FITC-NH-(CH₂)₆-OP(O)(OH)-**GCA CGU CCC UCA_{cp}** | |
| 73 | L-RNA | FITC-NH-(CH₂)₆-OP(O)(OH)-**GCA CGU CCC UCA C_{cp}** | |
| 74 | L-RNA | FITC-NH-(CH₂)₆-OP(O)(OH)-**GCA CGU CCC UCA CC_{cp}** | |
| 75 | L-RNA | FITC-NH-(CH₂)₆-OP(O)(OH)-**GCA CGU CCC UCA CCG_{cp}** | |
| 76 | L-RNA | FITC-NH-(CH₂)₆-OP(O)(OH)-**GCA CGU CCC UCA CCG G_{cp}** | |
| 77 | L-RNA | FITC-NH-(CH₂)₆-OP(O)(OH)-**GCA CGU CCC UCA CCG GU_{cp}** | |
| 78 | L-RNA | FITC-NH-(CH₂)₆-OP(O)(OH)-**GCA CGU CCC UCA CCG GUG_{cp}** | |
| 79 | L-RNA | FITC-NH-(CH₂)₆-OP(O)(OH)-**GCA CGU CCC UCA CCG GUG C_{cp}** | |
| 80 | L-RNA | FITC-NH-(CH₂)₆-OP(O)(OH)-**GCA CGU CCC UCA CCG GUG CA_{cp}** | |
| 81 | L-RNA | FITC-NH-(CH₂)₆-OP(O)(OH)-**GCACGU CCC UCA CCG GUG CAAₚ** | |
| 82 | L-RNA | FITC-NH-(CH₂)₆-OP(O)(OH)-**GCA CGU CCC UCA CCG GUG CAA G_{cp}** | |
| 83 | L-RNA | FITC-NH-(CH₂)₆-OP(O)(OH)-**GCA CGU CCC UCA CCG GUG CAA GU_{cp}** | |
| 84 | L-RNA | FITC-NH-(CH₂)₆-OP(O)(OH)-**GCA CGU CCC UCA CCG GUG CAA GUG_{cp}** | |
| 85 | L-RNA | FITC-NH-(CH₂)₆-OP(O)(OH)-**GCA CGU CCC UCA CCG GUG CAA GUG A_{cp}** | |
| 86 | L-RNA | FITC-NH-(CH₂)₆-OP(O)(OH)-**GCA CGU CCC UCA CCG GUG CAA GUG AA_{cp}** | |
| 87 | L-RNA | FITC-NH-(CH₂)₆-OP(O)(OH)-**GCA CGU CCC UCA CCG GUG CAA GUG AAGcp** | |
| 88 | L-RNA | FITC-NH-(CH₂)₆-OP(O)(OH)-**GCA CGU CCC UCA CCG GUG CAA GUG AAG C_{cp}** | |
| 89 | L-RNA | FITC-NH-(CH₂)₆-OP(O)(OH)-**GCA CGU CCC UCA CCG GUG CAA GUG AAG CC_{cp}** | |
| 90 | L-RNA | FITC-NH-(CH₂)₆-OP(O)(OH)-**GCA CGU CCC UCA CCG GUG CAA GUG AAG CCG_{cp}** | |
| 91 | L-RNA | FITC-NH-(CH₂)₆-OP(O)(OH)-**GCA CGU CCC UCA CCG GUG CAA GUG AAG CCG U_{cp}** | |
| 92 | L-RNA | FITC-NH-(CH₂)₆-OP(O)(OH)-**GCA CGU CCC UCA CCG GUG CAA GUG AAG CCG UG_{cp}** | |
| 93 | L-RNA | FITC-NH-(CH2)6-OP(O)(OH)-GCA CGU CCC UCA CCG GUG CAA GUG AAG CCG UGGrn | |
| 94 | L-RNA | FITC-NH-(CH2)6-OP(O)(OH)-GCA CGU CCC UCA CCG GUG CAA GUG AAG CCG UGG C[?] | |
| 95 | L-RNA | FITC-NH-(CH2)6-OP(O)(OH)-GCA CGU CCC UCA CCG GUG CAA GUG AAG CCG UGG CUcp | |
| 96 | L-RNA | FITC-NH-(CH2)6-OP(O)(OH)-GCA CGU CCC UCA CCG GUG CAA GUG AAG CCG UGG CUCcp | |
| 97 | L-RNA | FITC-NH-(CH2)6-OP(O)(OH)-GCA CGU CCC UCA CCG GUG CAA GUG AAG CCG UGG CUC Ucp | |
| 98 | L-RNA | FITC-NH-(CH2)6-OP(O)(OH)-GCA CGU CCC UCA CCG GUG CAA GUG AAG CCG UGG CUC UGcp | |
| 99 | L-RNA | FITC-NH-(CH2)6-OP(O)(OH)-GCA CGU CCC UCA CCG GUG CAA GUG AAG CCG UGG CUC UGCcp | |
| 100 | L-RNA | FITC-NH-(CH2)6-OP(O)(OH)-GCA CGU CCC UCA CCG GUG CAA GUG AAG CCG UGG CUC UGC G | FITC labeled NOX-E36 Intermediate |
| 101 | L-RNA | HS-(CH₂)₂C(O)-NH(CH₂)₆-OP(O)(OH)-**G_{cp}** | |
| 102 | L-RNA | HS-(CH₂)₂C(O)-NH(CH₂)₆-OP(O)(OH)-**GC_{cp}** | |
| 103 | L-RNA | HS-(CH₂)₂C(O)-NH(CH₂)₆-OP(O)(OH)-**GCG_{cp}** | |
| 104 | L-RNA | HS-(CH₂)₂C(O)-NH(CH₂)₆-OP(O)(OH)-**GCG U_{cp}** | |
| 105 | L-RNA | HS-(CH₂)₂C(O)-NH(CH₂)₆-OP(O)(OH)-**GCG UG_{cp}** | |
| 106 | L-RNA | HS-(CH₂)₂C(O)-NH(CH₂)₆-OP(O)(OH)-**GCG UGG_{cp}** | |
| 107 | L-RNA | HS-(CH₂)₂C(O)-NH(CH₂)₆-OP(O)(OH)-**GCG UGG U_{cp}** | |
| 108 | L-RNA | HS-(CH₂)₂C(O)-NH(CH₂)₆-OP(O)(OH)-**GCG UGG UG_{cp}** | |
| 109 | L-RNA | HS-(CH₂)₂C(O)-NH(CH₂)₆-OP(O)(OH)-**GCG UGG UGU_{cp}** | |
| 110 | L-RNA | HS-(CH₂)₂C(O)-NH(CH₂)₆-OP(O)(OH)-**GCG UGG UGU G_{cp}** | |
| 111 | L-RNA | HS-(CH₂)₂C(O)-NH(CH₂)₆-OP(O)(OH)-**GCG UGG UGU GA_{cp}** | |
| 112 | L-RNA | HS-(CH₂)₂C(O)-NH(CH₂)₆-OP(O)(OH)-**GCG UGG UGU GAU_{cp}** | |
| 113 | L-RNA | HS-(CH₂)₂C(O)-NH(CH₂)₆-OP(O)(OH)-**GCG UGG UGU GAU C_{cp}** | |
| 114 | L-RNA | HS-(CH₂)₂C(O)-NH(CH₂)₆-OP(O)(OH)-**GCG UGG UGU GAU CU_{cp}** | |
| 115 | L-RNA | HS-(CH₂)₂C(O)-NH(CH₂)₆-OP(O)(OH)-**GCG UGG UGU GAU CUA_{cp}** | |
| 116 | L-RNA | HS-(CH₂)₂C(O)-NH(CH₂)₆-OP(O)(OH)-**GCG UGG UGU GAU CUA G**_{**c**p} | |
| 117 | L-RNA | HS-(CH₂)₂C(O)-NH(CH₂)₆-OP(O)(OH)-**GCG UGG UGU GAU CUA GA_{cp}** | |
| 118 | L-RNA | HS-(CH₂)₂C(O)-NH(CH₂)₆-OP(O)(OH)-**GCG UGG UGU GAU CUA GAU_{cp}** | |
| 119 | L-RNA | HS-(CH₂)₂C(O)-NH(CH₂)₆-OP(O)(OH)-**GCG UGG UGU GAU CUA GAU G_{cp}** | |
| 120 | L-RNA | HS-(CH₂)₂C(O)-NH(CH₂)₆-OP(O)(OH)-**GCG UGG UGU GAU CUA GAU GU_{cp}** | |
| 121 | L-RNA | HS-(CH₂)₂C(O)-NH(CH₂)₆-OP(O)(OH)-**GCG UGG UGU GAU CUA GAU GUA_{cp}** | |
| 122 | L-RNA | HS-(CH₂)₂C(O)-NN(CH₂)₆-OP(O)(OH)-**GCG UGG UGU GAU CUA GAU GUA U_{cp}** | |
| 123 | L-RNA | HS-(CH₂)₂C(O)-NH(CH₂)₆-OP(O)(OH)-**GCG UGG UGU GAU CUA GAU GUA UU_{cp}** | |
| 124 | L-RNA | HS-(CH₂)₂C(O)-NH(CH₂)₆-OP(O)(OH)-**GCG UGG UGU GAU CUA GAU GUA UUG_{cp}** | |
| 125 | L-RNA | HS-(CH₂)₂C(O)-NH(CH₂)₆-OP(O)(OH)-**GCG UGG UGU GAU CUA GAU GUA UUG G_{cp}** | |
| 126 | L-RNA | HS-(CH₂)₂C(O)-NH(CH₂)₆-OP(O)(OH)- **GCG UGG UGU GAU CUA GAU GUA UUG GC_{cp}** | |
| 127 | L-RNA | HS-(CH₂)₂C(O)-NH(CH₂)₆-OP(O)(OH)-**GCG UGG UGU GAU CUA GAU GUA UUG GCU_{cp}** | |
| 128 | L-RNA | HS-(CH2)2C(O)-NH(CH2)6-OP(O)(OH)-GCG UGG UGU GAU CUA GAU GUA UUG GCU Gcp | |
| 129 | L-RNA | HS-(CH2)2C(O)-NH(CH2)6-OP(O)(OH)-GCG UGG UGU GAU CUA GAU GUA UUG GCU GAcp | |
| 130 | L-RNA | HS-(CH2)2C(O)-NH(CH2)6-OP(O)(OH)-GCG UGG UGU GAU CUA GAU GUA UUG GCU GAUcp | |
| 131 | L-RNA | HS-(CH2)2C(O)-NH(CH2)6-OP(O)(OH)-GCG UGG UGU GAU CUA GAU GUA UUG GCU GAU Ccp | |
| 132 | L-RNA | HS-(CH2)2C(O)-NH(CH2)6-OP(O)(OH)-GCG UGG UGU GAU CUA GAU GUA UUG GCU GAU CCcp | |
| 133 | L-RNA | HS-(CH2)2C(O)-NH(CH2)6-OP(O)(OH)-GCG UGG UGU GAU CUA GAU GUA UUG GCU GAU CCUcp | |
| 134 | L-RNA | HS-(CH2)2C(O)-NH(CH2)6-OP(O)(OH)-GCG UGG UGU GAU CUA GAU GUA UUG GCU GAU CCU Acp | |
| 135 | L-RNA | HS-(CH2)2C(O)-NH(CH2)6-OP(O)(OH)-GCG UGG UGU GAU CUA GAU GUA UUG GCU GAU CCU AGcp | |
| 136 | L-RNA | HS-(CH2)2C(O)-NH(CH2)6-OP(O)(OH)-GCG UGG UGU GAU CUA GAU GUA UUG GCU GAU CCU AGUcD | |
| 137 | L-RNA | HS-(CH2)2C(O)-NH(CH2)6-OP(O)(OH)-GCG UGG UGU GAU CUA GAU GUA UUG GCU GAU CCU AGU Ccp | |
| 138 | L-RNA | HS-(CH2)2C(O)-NH(CH2)6-OP(O)(OH)-GCG UGG UGU GAU CUA GAU GUA UUG GCU GAU CCU AGU CAcp | |
| 139 | L-RNA | HS-(CH2)2C(O)-NH(CH2)6-OP(O)(OH)-GCG UGG UGU GAU CUA GAU GUA UUG GCU GAU CCU AGU CAGcp | |
| 140 | L-RNA | HS-(CH2)2C(O)-NH(CH2)6-OP(O)(OH)-GCG UGG UGU GAU CUA GAU GUA UUG GCU GAU CCU AGU CAG Gcp | |
| 141 | L-RNA | HS-(CH2)2C(O)-NH(CH2)6-OP(O)(OH)-GCG UGG UGU GAU CUA GAU GUA UUG GCU GAU CCU AGU CAG GU[?] | |
| 142 | L-RNA | HS-(CH2)2C(O)-NH(CH2)6-OP(O)(OH)-GCG UGG UGU GAU CUA GAU GUA UUG GCU GAU CCU AGU CAG GUAcp | |
| 143 | L-RNA | HS-(CH2)2C(O)-NH(CH2)6-OP(O)(OH)-GCG UGG UGU GAU CUA GAU GUA UUG GCU GAU CCU AGU CAG GUA Ccp | |
| 144 | L-RNA | HS-(CH2)2C(O)-NH(CH2)6-OP(O)(OH)-GCG UGG UGU GAU CUA GAU GUA UUG GCU GAU CCU AGU CAG GUA CGcn | |
| 145 | L-RNA | HS-(CH2)2C(O)-NH(CH2)6-OP(O)(OH)-GCG UGG UGU GAU CUA GAU GUA UUG GCU GAU CCU AGU CAG GUA CGC | released acylated NOX-A12 Intermediate |
| 146 | L-RNA | HS-(CH2)2C(O)-NH(CH2)6-OP(O)(OH)-GCA CGU CCC CUA CCG GUG CAA GUG AAG CCG UGG UCC UGC G | |

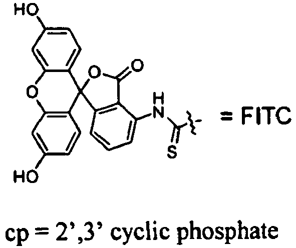

It will be understood that the above is a representation of the molecules as they were used in connection with the instant invention. The attached sequence listing does only reflect the mere nucleotide sequence thereof and not any further feature of said molecules as indicated in the above table.

The present invention is further illustrated by the figures, examples and the sequence listing from which further features, embodiments and advantages may be taken, wherein
- Fig. 1: This Figure shows a representation of the cleavage products that result upon consecutive hydrazine then acetic acid/aniline treatment of an RNA molecule: Uridine moieties are susceptible to modification resulting in phosphate backbone cleavage producing a 5' -phosphate appended 3' fragment and a 5' fragment with an aniline derived Schiff's base at the Uridine position ("modified Uridine", abbr. Umod) as proposed by Ehresmann et al. (Ehresmann et al, 1987).

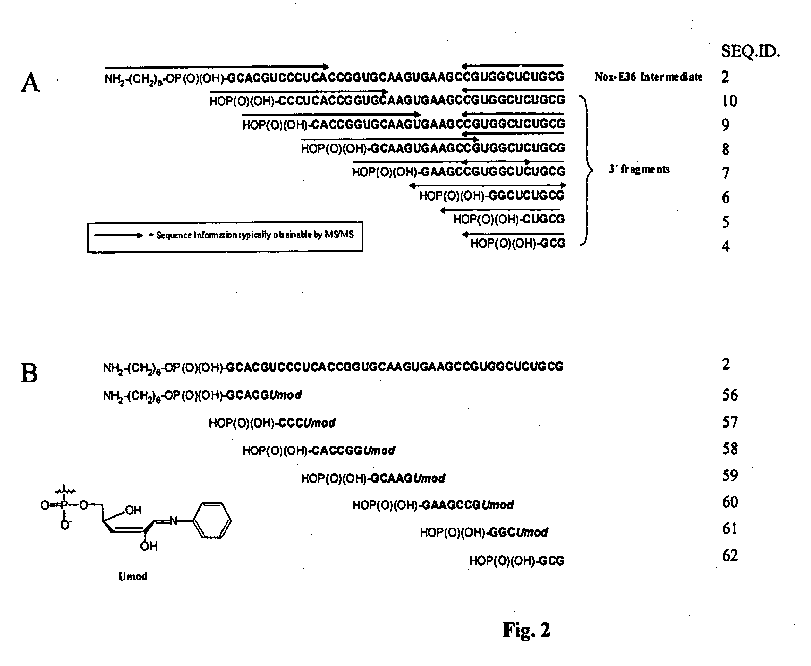

- Fig. 2A-B: A: Shows all possible 3' terminal fragments (SEQ ID 4-10) that can be generated from consecutive hydrazine then acetic acid/aniline treatment of NOX-E36 Intermediate (SEQ ID 2). The arrows depict the sequence information that can typically be achieved with standard MS/MS sequencing techniques (10-15 nucleobases) As the fragments get shorter, the ability to sequence the entire fragment increases. B. When the hydrazine treatment is not carefully controlled, complete cleavage of the parent molecule occurs. The fragments cannot be used for sequencing as the relationship between them is destroyed.
- Fig. 3A: This Figure shows a Total Ion Chromatogram (abbr. TIC) of the intact nucleic acid molecule Spiegelmer NOX-E36 Intermediate (SEQ.ID. 2);
- Fig. 3B: This Figure shows a deconvoluted mass of the intact nucleic acid molecule Spiegelmer NOX-E36 Intermediate (SEQ.ID. 2) derived from the mass spectrum of the main peak at 4.2 min in Fig 3A. The mass is in accordance with that of SEQ. ID. 2.
- Fig. 4: This Figure shows a clearly defined fragments of Spiegelmer NOX-E36 Intermediate discernable by Reversed Phase-HPLC column chromatography;
- Fig. 5: This Figure shows a deconvoluted mass spectra of the individual peaks as demonstrated for fragments 5 (observable exact mass = 7494.03 Da), 6 (observable average molecular mass = 9738.83 Da) and the intact nucleic acid molecule NOX-E36 Intermediate (SEQ.ID 2, observable average molecular mass = 12995.84 Da);
- Fig. 6: shows a table representing the fragments of nucleic acid molecule NOX-E36 Intermediate (SEQ.ID 2) as generated by the hydrazine-aniline/acetic acid treat (Example 2) including sequence, calculated mass and observed masses from TIC (Fig 4 and 5) for identification of the fragments;

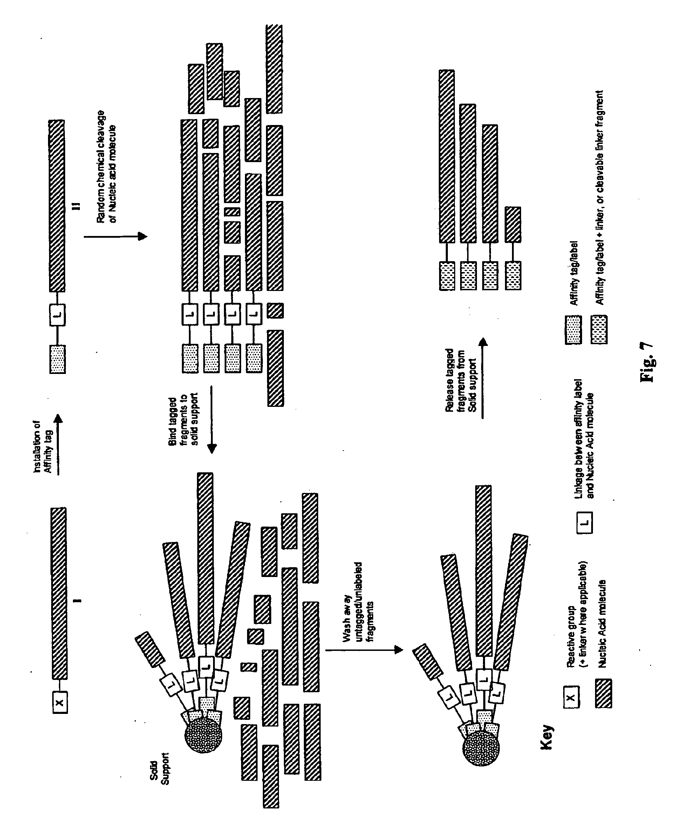

- Fig. 7: shows sequencing of a nucleic acid molecule with immobilization of the nucleic acid molecule and selected fragments thereof; whereby the nucleic acid molecule and the selected fragmentspossess an affinity label or tag: The nucleic acid molecule either possesses a selectively reactive functional group (I) that is used to append the affinity label or tag, or already possesses such affinity label as depicted in the generic labeled/tagged nucleic acid structure (II). The labeled nucleic acid molecule II undergoes limited random cleavage by chemical cleavage to create a mix of fragments representing random strand scission plus uncleaved full length material; the labeled fragments are then immobilized, in this example through interaction with an interaction partner on solid support, and the non-labeled fragments are washed away. The labeled fragments are then released from the solid support and can be analysed through LCMS or other appropriate techniques.

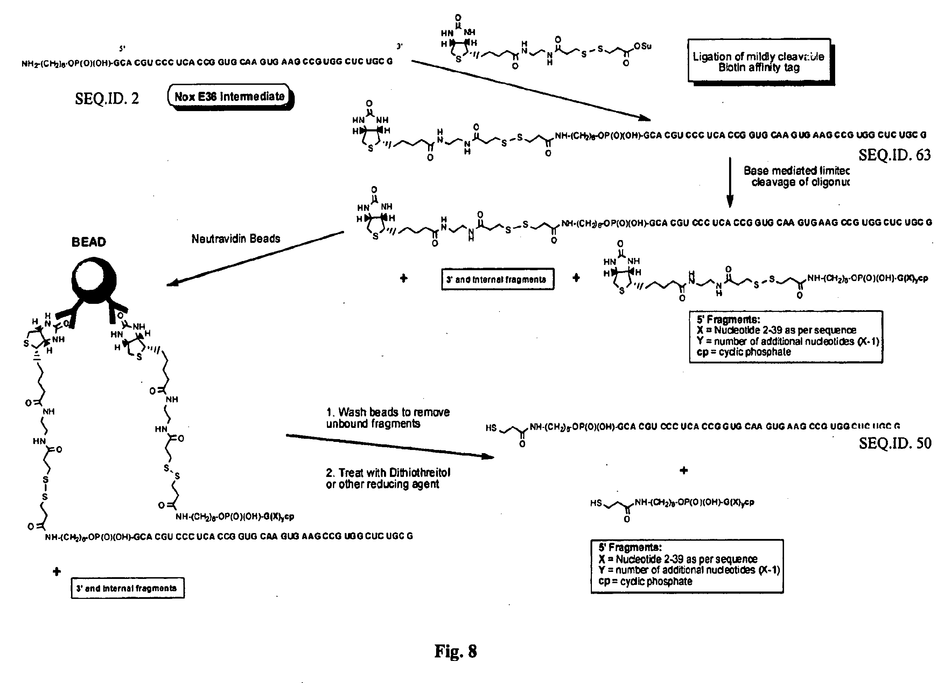

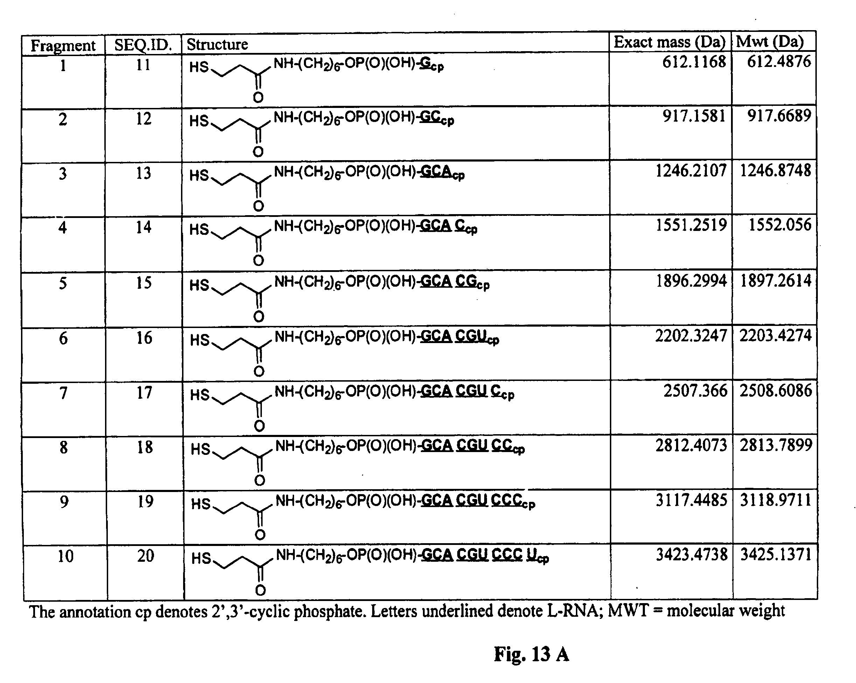

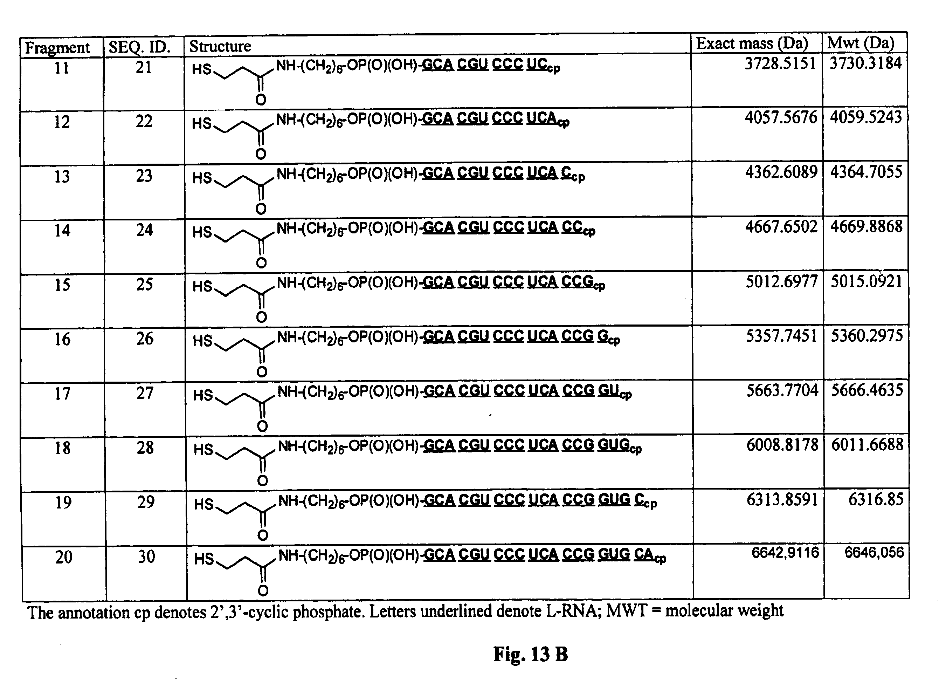

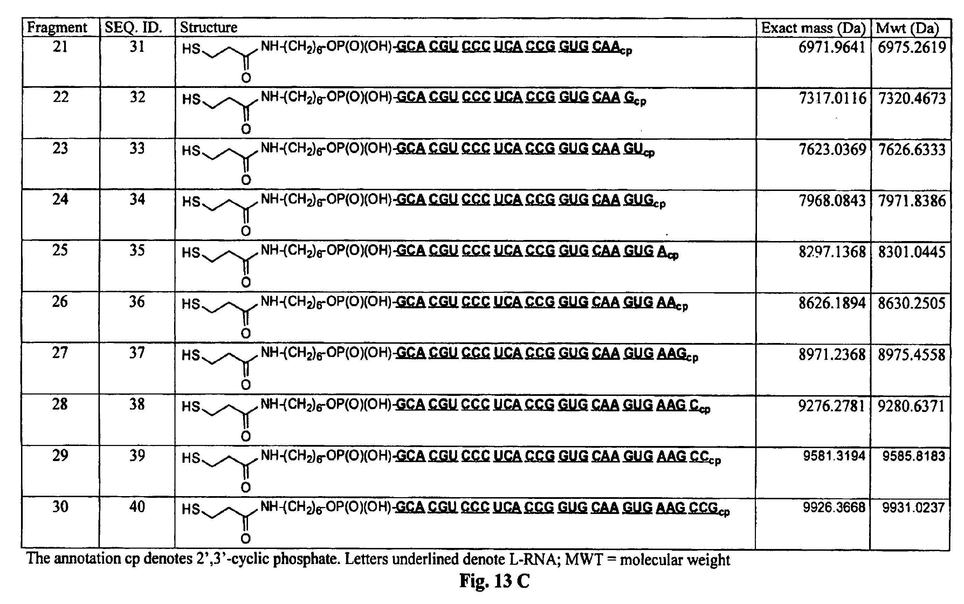

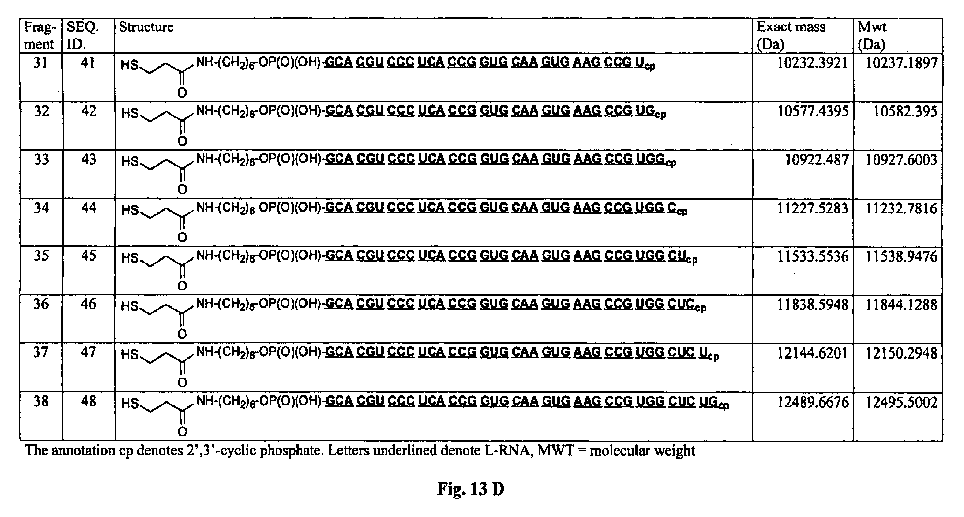

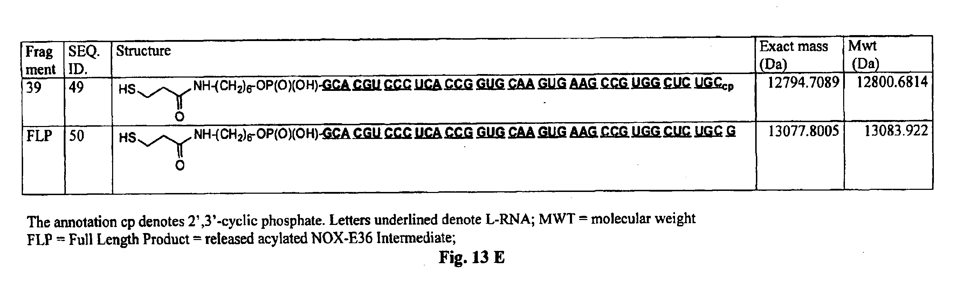

- Fig. 8: This Figure shows a scheme for the example of the sequencing of a nucleic acid molecule with immobilization of the nucleic acid molecule, whereby the nucleic acid molecule is the nucleic molecule Spiegelmer NOX-E36 Intermediate (SEQ.ID. 2), a 5'-amino-modified derivative of Spiegelmer NOX-E36 (SEQ.ID. 1); after modifying the 5'-amino moiety of NOX-E36 Intermediate (SEQ.ID. 2) with a biotin affinity tag, the biotinylated NOX-E36 Intermediate (SEQ. ID. 63) was chemically cleaved in a random fashion using a basic solution, whereby the cleavage was carefully controlled so as not to drive the cleavage to completion; from the random fragmentation that occurs which produces 5'- fragments, 3'- fragments and random internal fragments of biotinylated NOX-E36 Intermediate (SEQ.ID. 64), all biotinylated 5' fragments of NOX-E36 Intermediate and remaining biotinylated NOX-E36 Intermediate (SEQ. ID. 63) (i.e. full-length product [abbr. FLP]) were selectively pulled out from the mix via the affinity tag (in this case biotin) using tag-specific solid support for immobilisation (in this case Neutravidin beads); the unbound fragments, i.e. 3'- fragments and random internal fragments that do not possess the affinity tag, can be washed away and the bound 5' fragments are then liberated from the beads by reductively cleaving the disulfide bond within the linker connecting the biotin moiety and nucleic acid. These fragments correspond to strand scission between every ribonucleoside position. 5' fragments in this schematic have been encompassed using the formula "R"-NH-(CH₂)₆-OP(O)(OH)-**G(X)_{y}cp** where "R" is either the strucurally drawn cleavable biotin affinity tag or cleaved fragment, letters in bold and underligned, respectively, represent the nucleic acid sequence, X indicates the identity of the particular nucleotide (A, C, G, U) read from the sequence 5'- to 3'- and y represents how many additional nucleotides are in the fragment over the first fragment. E.g. fragment 2: y = 1 (one extra nucleotide). The extra nucleotide(s) X over the first fragment is one (C) therefore the fragment, fragment 2 is "R"-NH-(CH₂)₆-OP(O)(OH)-**GCcp**. Similarly fragment 15: y = 14 therefore extra nucleotides X over the first fragment are fourteen **(CACGUCCCUCACCG**), therefore the identity of fragment 15 is "R"-NH-(CH₂)₆-OP(O)(OH)-**GCACGUCCCUCACCGcp**. Further representation of the released 5' fragments is found in Figure 13.;
- Fig. 9: This Figure shows a an anion exchange HPLC chromatogram of the crude NOX-E36 Intermediate (SEQ.ID. 2) which was used for the biotinylation reaction;
- Fig. 10: This Figure shows a an anion exchange HPLC chromatogram of the crude biotinylation reaction after 60 mins reaction time;
- Fig. 11: This Figure shows a Total Ion Chromatogram (abbr. TIC) obtained from the LCMS experiment after biotin labeled NOX-E36 Intermediate (SEQ. ID. 63) has been subjected to steps 3.3.2-3.3.5 of the protocol as shown in Example 3;
- Fig. 12: This Figure shows an example of a deconvoluted molecular weight of a fragment (mass peak value = 10237.1897 Da), in this case Fragment 31 (Fig. 13D, [SEQ. ID. 41]), whereby low abundance of the deconvoluted molecular weight of the Fragment 29 (Fig. 13C, SEQ. ID. 39) whose 2',3'-cyclic phosphate has been hydrolysed (mass peak value = 9603.77) can also be detected;
- Fig. 13 A-E: This Figure shows all expected 5' fragments of released acylated NOX-E36 Intermediate SEQ ID 50 (Seq. ID. 11-50). This table can be used for comparing to observed mass values for the sequence confirmation of a known molecule;
- Fig. 14 A+B: This Figure shows a Sequence Confirmation Table that lists either the deconvoluted observable masses obtained from the TIC, and the retention time that these masses were observed, whereby the exact mass or molecular weight of each expected fragment as depicted in Fig 13 A-E is included and the fragments identified;
- Fig. 15: This Figure shows an annotated version of Fig. 11 whereby the peaks of the identified cyclic phosphate fragments and the released acylated NOX-E36 Intermediate are shown. For each fragment, the corresponding 2',3'-cyclic phosphate predominates over the corresponding 2'(3') phosphate derivative thus greatly simplifying the chromatogram enabling easier identification and sequencing;
- Fig. 16: shows a flow chart for Sequence Determination/Validation. This flow chart can be used for the sequence identification without prior knowledge of the sequence.
- Fig. 17: This Figure shows a Total Ion Chromatogram (abbr. TIC) from the LCMS after NOX-E36 mismatch control 01 (SEQ.ID. 3) has been subjected to steps 3.3.1-3.3.5 of the protocol as shown in Example 3;
- Fig. 18A-C: This Figure shows a Sequence Determination Table: The flow chart as depicted in Fig. 16 is applied to the observed masses obtained from the TIC from Fig. 17.. The switched C/U pairs, in comparison to the parent sequence, NOX-E36 are highlighted
- Fig. 19A-C: This Figure shows a LCMS of FITC labelled NOX-E36 Intermediate after base mediated limited random cleavage whereby the label has a selective wavelength absorbance at 495 nm; in Fig. 19A the UV chromatogram extracted at 495 nm is shown; in Fig. 19B the UV chromatogram extracted at 260 nm is shown; Fig, 19C the Total Ion Chromatogram (abbr. TIC) is shown;.
- Fig. 20A: This Figure shows a Zoom-in of Fig 19B;
- Fig. 20B: This Figure shows a Zoom-in of Fig 19A:
- Fig. 21 A+B: This Figure shows a deconvoluted exact masses of A: fragment 1 (SEQ.ID 51, Fig. 23A), and B: fragment 2 (SEQ.ID 52, Fig 23A) found at 6.31 and 7.13 mins respectively;
- Fig. 22A-C: This Figure shows a aeconvoluted exact masses of non-labelled fragments found at 5.54 (4106.55 Da), 6.53 (4451.60 Da), 7.77 (4780.64 Da) mins respectively;
- Fig. 25A-C: This Figure shows a Zoom of Fig. 19A-C (16.6-18.5 min) illustrating FITC-labelled and non-FITC-labelled fragments co-eluting. A: Extracted wavelength chromatogram at 495 nm indicating FITC labelled nucleic acid fragments. B: Extracted wavelength chromatogram at 260 nm indicating all nucleic acid fragments (labelled and non-labelled). C: TIC showing all ions in sample material. It is clear to see that in the marked area for the labelled fragment as determined by 25A (see broken lines) there are other species present (Fig. 25B) that produce ions (Fig. 25C).
- Fig. 26: This Figure shows raw mass spectrum for the area between the broken lines in Fig. 25.
- Fig. 27: This Figure shows a deconvoluted average mass spectrum of the corresponding raw mass spectrum (Fig. 26). The arrowed peak is the labelled fragment (4666.21 Da, fragment 13, Fig 23, SEQ. ID. 73). Other masses are significantly higher in value (7693.26, 9335.48, 9664.90 Da) than those anticipated according to the incremental buildup of the sequencing ladder, and therefore can be disregarded.

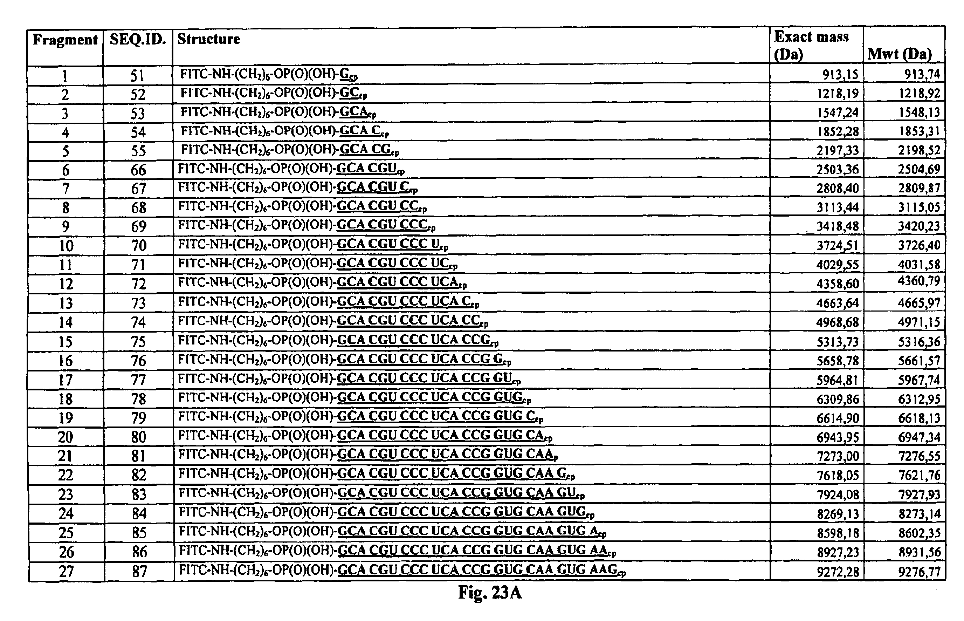

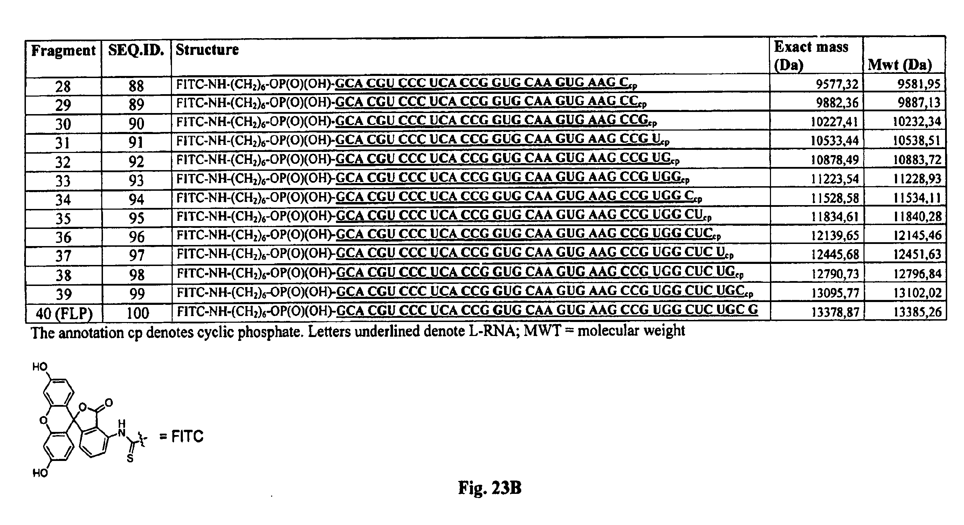

- Fig. 23A+B: This Figure shows an exemplary table for the sequence confirmation of the FITC-labelled fragments (SEQ. ID. 51-55, 66-100) (analogous to that of Fig 13);

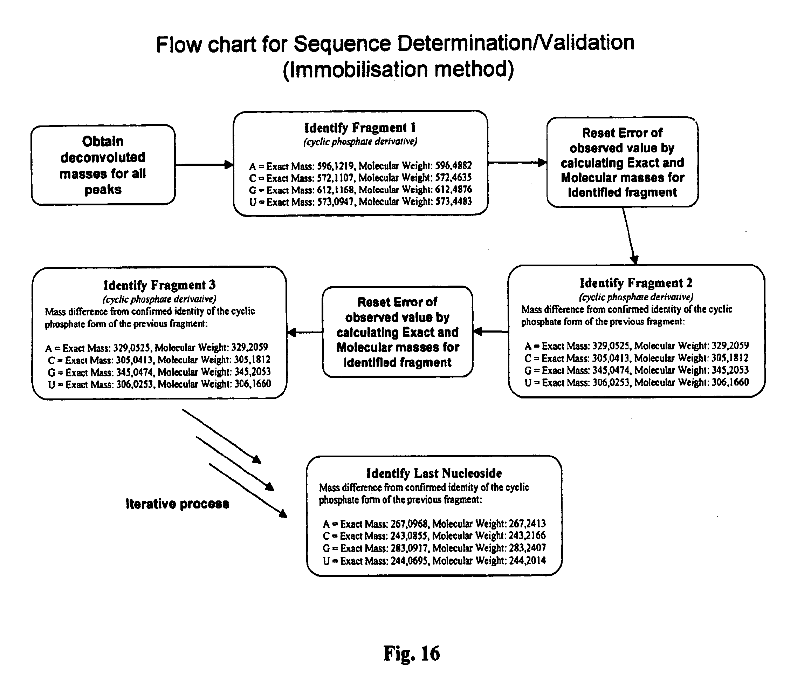

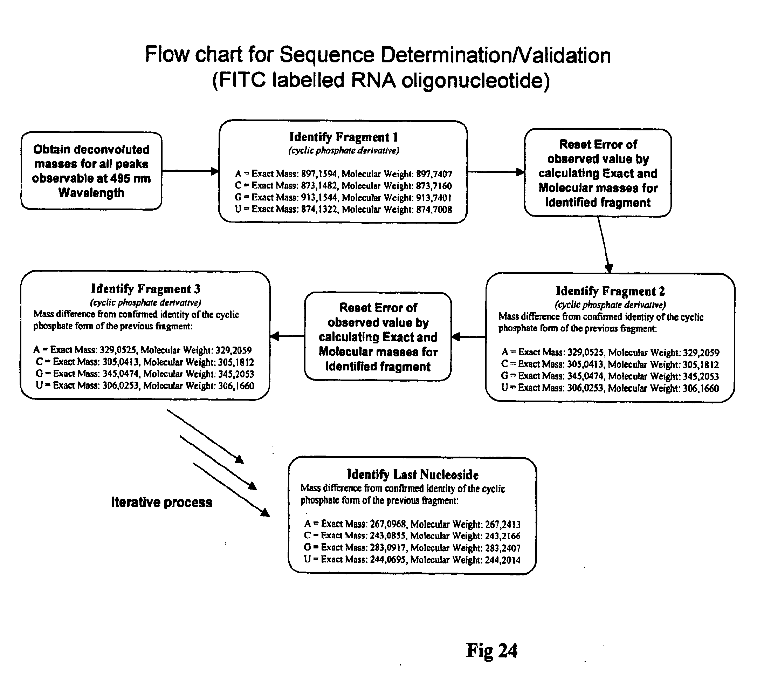

- Fig. 24: This Figure shows an exemplary flow chart for the sequence determination of FITC labelled RNA molecules (analogous to that of Fig 16).

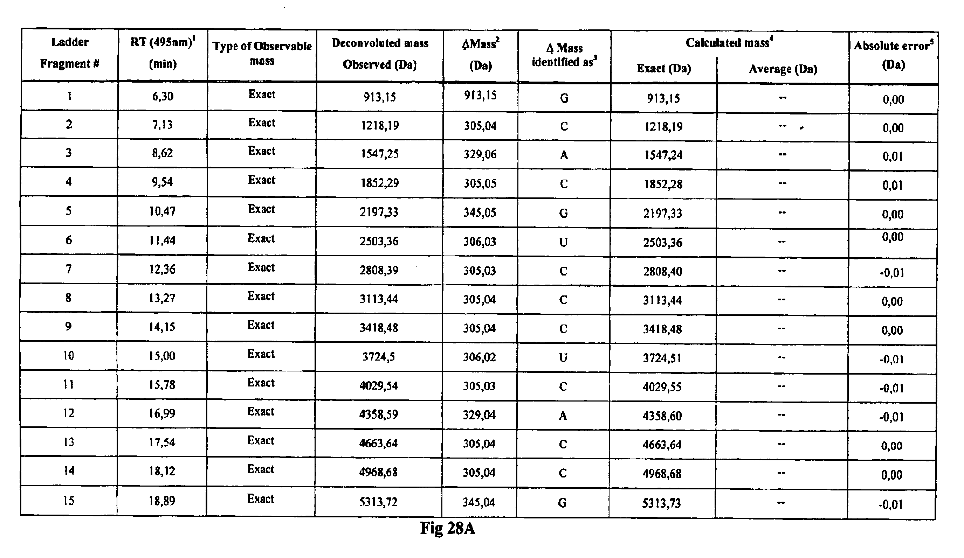

- Fig 28A-C: Shows a Sequence Determination Table that lists either the deconvoluted exact mass or molecular weight masses obtained from the TIC and the retention time (obtained from the 495 nm Extracted Wave Chromatogram) that these masses were observed; using the flow chart as depicted in Fig. 24, the observed masses are used to determine the sequence

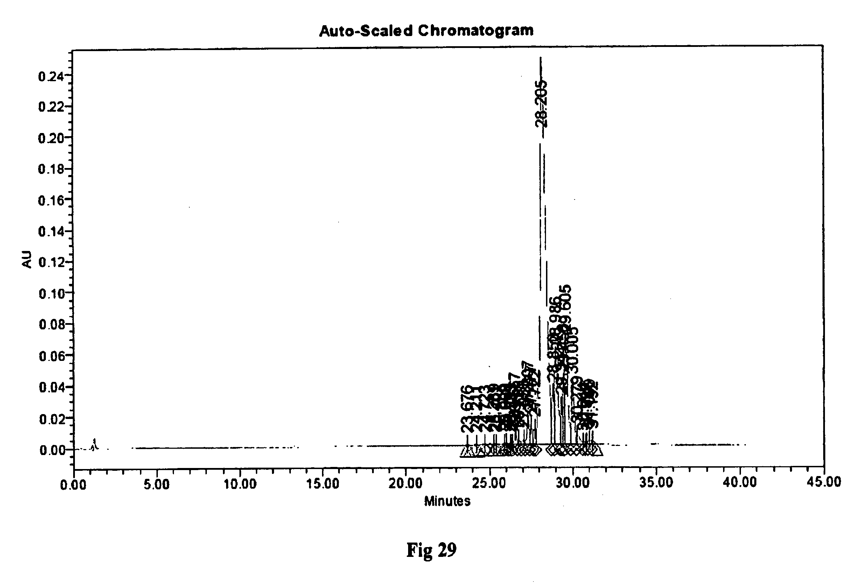

- Fig. 29: This Figure shows an anion exchange HPLC chromatogram of the crude NOX-A12 Intermediate (SEQ.ID. 65) which was used for the biotinylation reaction. The presence of the shortmers does not affect the ability to carry out steps 5.3.1-5.3.5 and to sequence the NOX-A12 Intermediate (SEQ.ID. 65).

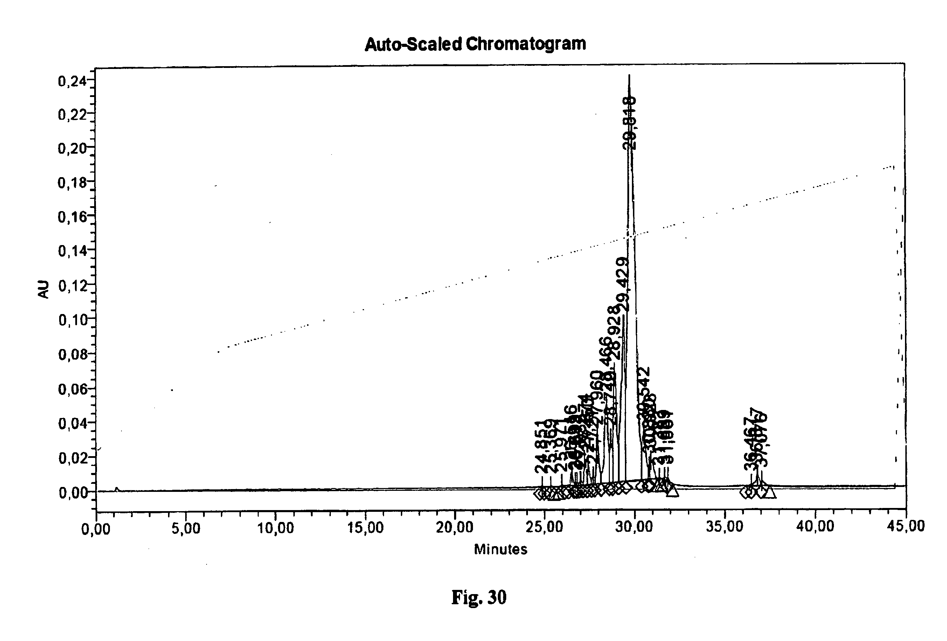

- Fig 30: This Figure shows an anion exchange HPLC chromatogram of the biotinylation reaction after 60 mins reaction and desalting.

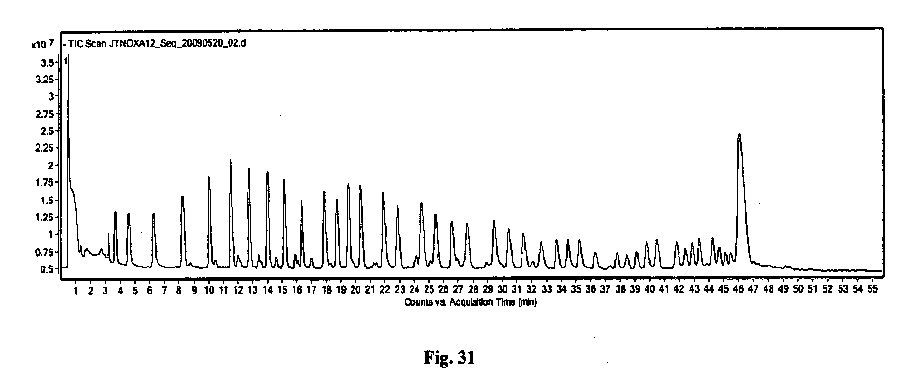

- Fig. 31: This Figure shows a Total Ion Chromatogram (abbr. TIC) from the LCMS after the biotin labeled NOX-A12 Intermediate has been subjected to steps 5.3.2-5.3.5.

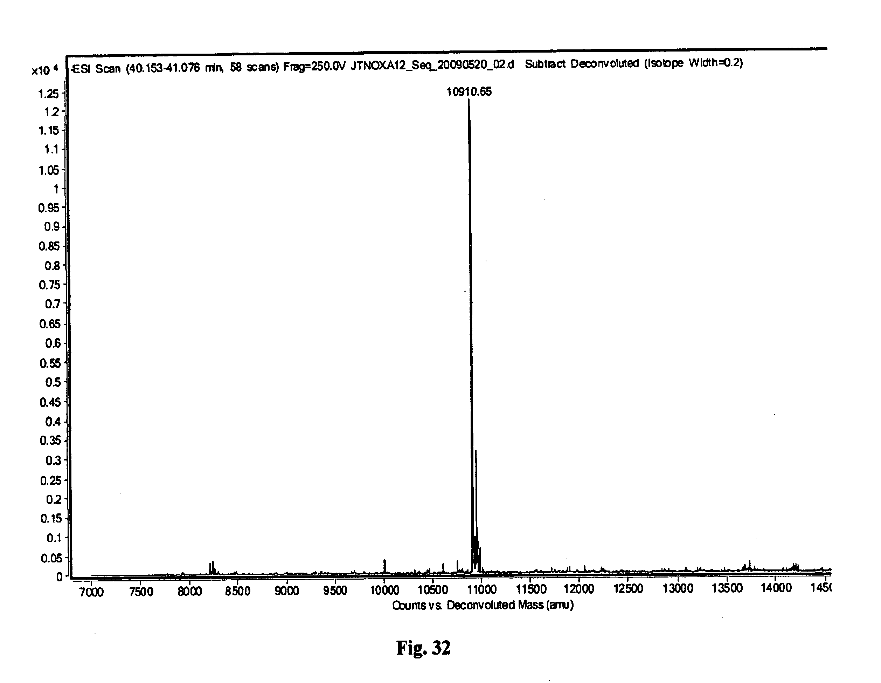

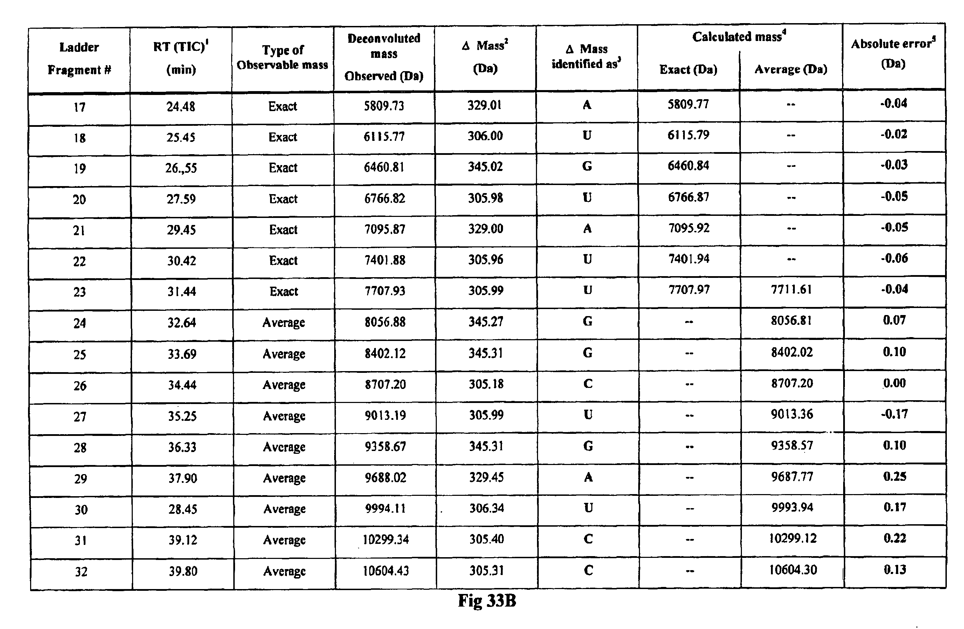

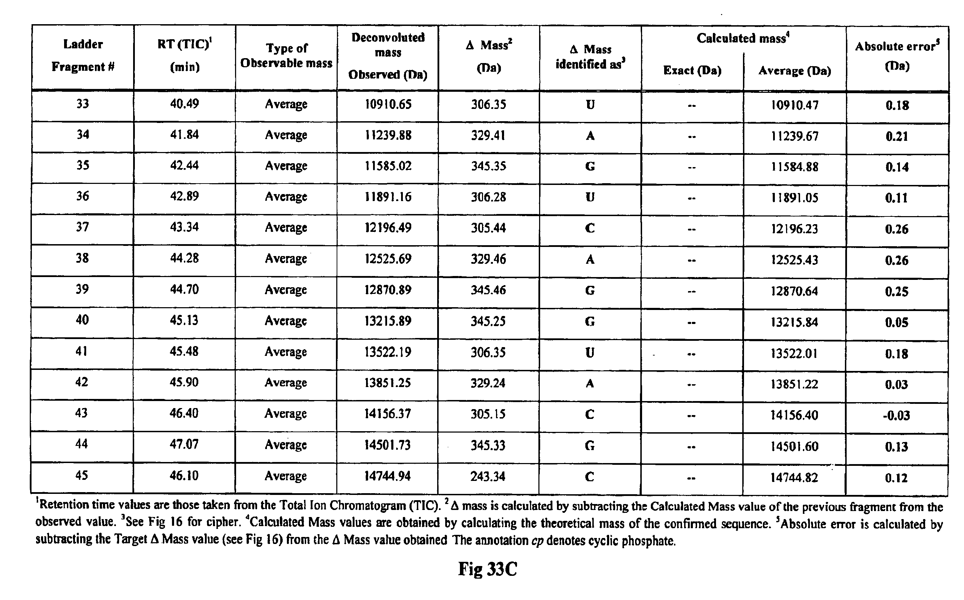

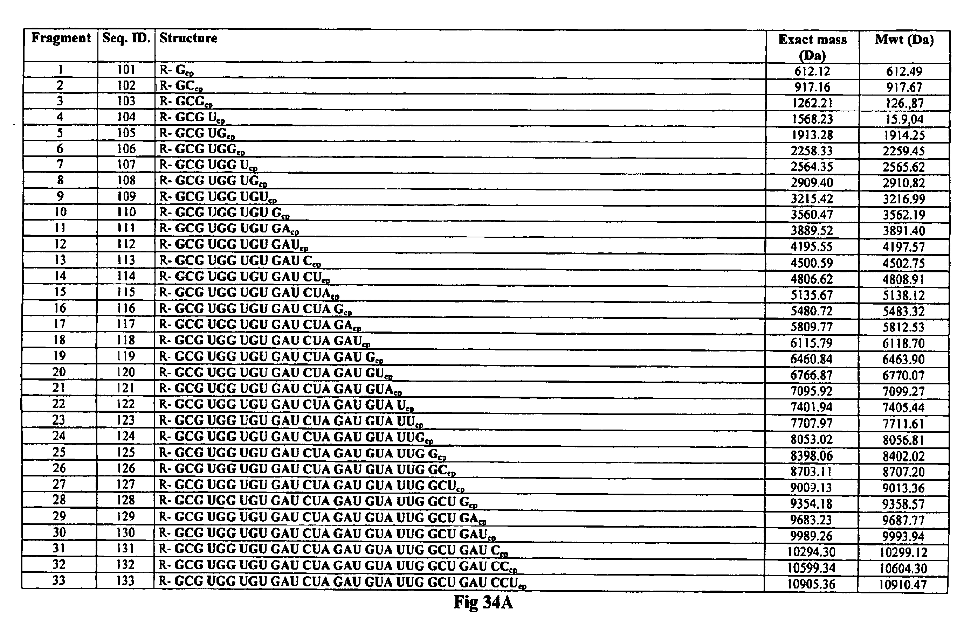

- Fig. 32: This Figure shows a example of a deconvoluted molecular weight of a fragment (mass peak value = 10910.65 Da), in this case Fragment 33 (Fig. 33C and 34A, [SEQ. ID. 133])

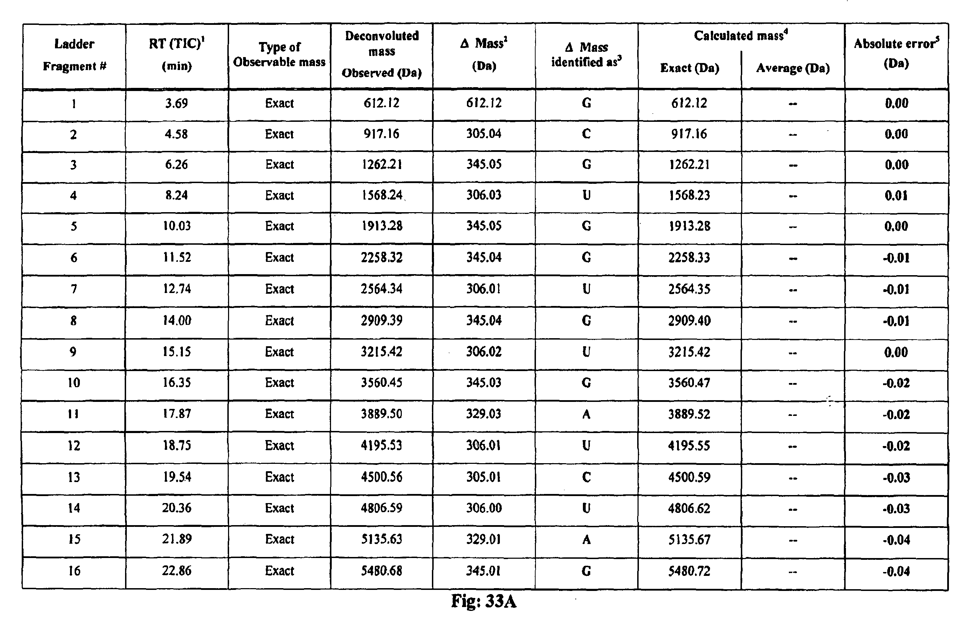

- Fig. 33A-C: This Figure shows a Sequence Determination Table that lists either the deconvoluted exact mass or molecular weight masses obtained from the TIC and the retention time that these masses were observed. Using the flow chart as depicted in Fig. 16, the observed masses are used to determine the sequence. An absolute error is included that notes the error in relation to the expected mass of the proposed fragment identity.

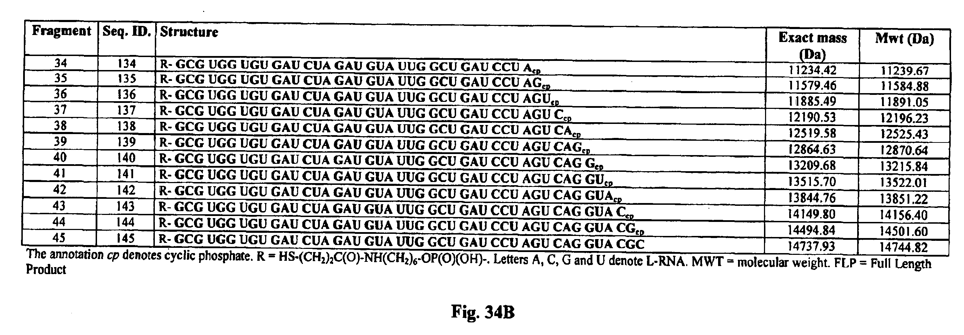

- Fig. 34A+B: This Figure shows a Sequence confirmation table NOXA12: Listed are all expected 5' fragments of released acylated NOX-A12 Intermediate SEQ ID 145 (Seq. ID. 101-145). This table can be used for comparing to observed mass values for the sequence confirmation of a nucleic acid molecule whose sequence is known.

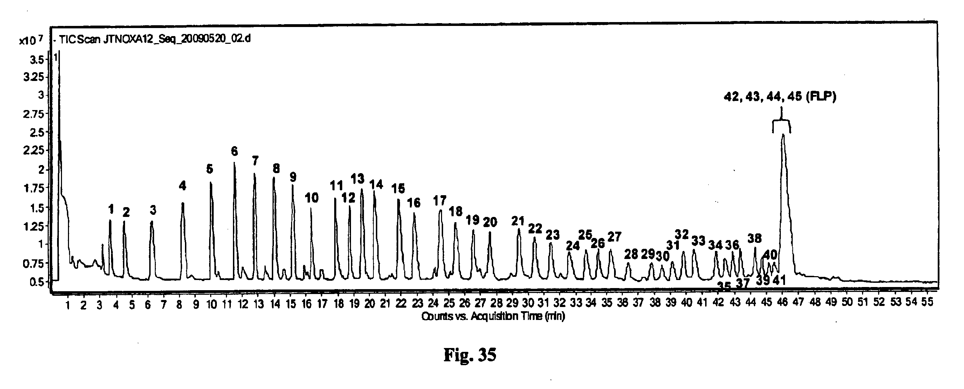

- Fig. 35: This Figure shows an annotated version of Fig. 31 whereby the peaks of the identified cyclic phosphate fragments and the released acylated NOX-A12 Intermediate (SEQ. ID. 145) are assigned their corresponding fragment numbers.

Referring to the Figures, two particularly preferred embodiments that are described in more detail in examples 3, 4 and 5 are described in the following section.

As outlined in more detail in the instant specification and example 3 (Spiegelmer NOX-E36) and example 5 (NOX-A12), whereby in the following it is only referred to example 3, a method for sequencing of a nucleic acid molecule by mass spectrometry is provided, whereby the nucleic acid and selected fragments are immobilised with the process of sequence determination. According to the present invention, in a first step the nucleic acid molecule is endowed with a modification (Fig. 7, I) such as an affinity label or tag that can be used for the immobilisation of the nucleic acid molecule and fragments thereof. The second step is the limited random cleavage of the nucleic acid molecule by chemical cleavage to create a mix of fragments representing random strand scission (as shown in principle in Fig. 7) plus uncleaved full length material. From this random mix, those fragments and molecules of the uncleaved full length material that contain the label are pulled out of the mix using the affinity label or tag as a handle, binding to a solid support, be that in a column, on a chip, or in bead format. The other fragments that do not contain the label are washed away. In the third step the immobilised fragments are released by cleavage or elution from the solid phase and furnishes the fragments to be analysed by LCMS, direct infusion MS or MALDI and other MS methods as described herein. The result is a mass ladder representing all possible fragments representing cleavages 3' to every nucleotide of the nucleic acid molecule. If the modification is appended to the 5' terminus, the resulting mass ladder would consist solely of 5' fragments, similarly if the modification is appended to the 3' terminus, the resulting mass ladder would consist solely of 3' fragments. Said mass ladder is actually formed or arising from a row of 5' or 3' fragments.

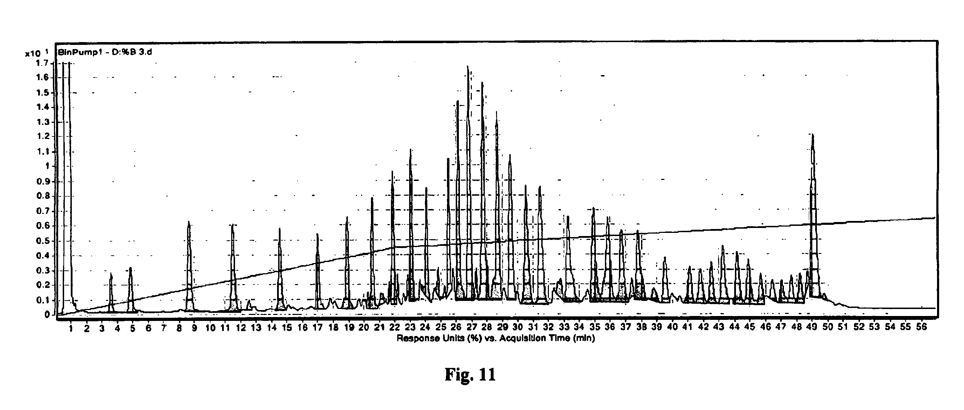

To test this method, the Spiegelmer NOX-E36 Intermediate (SEQ.ID. 2), an RNA molecule with a length of 40 nucleotides was used (synthesised according to example 1). NOX-E36 Intermediate (SEQ.ID. 2) is a 5'-amino-modified derivative of NOX-E36 (SEQ.ID. 1). After modifying the 5'-amino moiety of NOX-E36 Intermediate (SEQ.ID. 2) with a biotin affinity tag, the biotinylated NOX-E36 Intermediate (SEQ. ID. 63) was chemically cleaved in a random fashion using a basic solution (reaction scheme as shown in Fig. 8). The cleavage was carefully controlled so as not to drive the cleavage to completion. From the random fragmentation that occurs 5'- fragments, 3'- fragments and random internal fragments of biotinylated NOX-E36 Intermediate (SEQ.ID. 63) are produced. All biotinylated 5' fragments (Fig 8, series 1) and remaining biotinylated NOX-E36 Intermediate (SEQ. ID. 63) were selectively pulled out from the mix via the affinity tag (in this case biotin) using tag-specific solid support for immobilisation. The unbound fragments, i.e. 3'- fragments and random internal fragments that do not possess the affinity tag, are washed away. The bound 5' fragments and the full-length molecule were then liberated from the beads by reductively cleaving the disulfide bond within the linker connecting the biotin moiety and NOX-E36 Intermediate. These released fragments correspond to strand scission between every ribonucleoside position (see Figs 8 and 13). The strand scission results first in the formation of 2',3'-cyclic phosphate containing fragments whereby the cyclic phosphate slowly hydrolyses to the 2'(3') phosphate. The liberated fragments were then analysed by LC-(ESI)MS, and the Total Ion Chromatogram (abbr. TIC) was analysed. Sample chromatograms display discrete peaks that correspond to all 5'-fragments generated and the intact released acylated NOX-E36 Intermediate (SEQ. ID. 50), as shown in Fig 11. The mass(es) contained in the discrete peaks were then obtained through deconvolution of the derived mass spectra pertaining to each discrete peak. This mass information can then be used to determine the sequence of the parentnucleic acid sequence which is sometimes also referred to as the parent oligonucleotide.

In general, the masses seen are those of the 2',3'-cyclic phosphates, although in some cases, the low abundance of fragments containing the hydrolysed 2' (3') phosphate can also be detected, which serve to further confirm the identity of the fragments generated. Typically these hydrolysed fragments elute later than the parent 2',3'-cyclic phosphate using the analysis parameters as described (Example 3).

The masses of the fragments generated can in the first instance be compared to the expected masses of the calculated 5' fragments of released NOX-E36 Intermediate (SEQ.ID. 50) to confirm the sequence. Alternatively, the sequence can be derived without prior knowledge of the sequence due to the differences between the fragments generated. In this scenario, the first fragment of the nucleic acid molecule can be easily predicted since the modification (HS-(CH₂)₂C(O)-NH(CH₂)₆-OP(O)(OH)-) is known, and therefore there are only limited discrete mass values possible for this fragment (e.g. 4 for A, C, G, U for unmodified D- or L- RNA). The incremental differences of the subsequent fragments can then be used to determine the sequence of the nucleic acid molecule, as demonstrated in the 'Flow chart for Sequence Determination/Validation' in Fig. 16 and in Example 3. Once Fragment 1 has been identified, the calculated exact mass and molecular weight are used to identify the next fragment, Fragment 2. The identity and therefore sequence of the next fragment, Fragment 2, is derived from the mass difference between Fragment 2 and the calculated exact mass or molecular weight of Fragment 1. The mass difference is unique for each nucleoside A, C, G, U (as shown in Fig 16). Once Fragment 2 has been identified, the calculated exact mass and molecular weight are used to identify the next fragment, Fragment 3. In an identical procedure to that used to identify Fragment 2, the identity of Fragment 3, is derived from the mass difference between Fragment 3 and the calculated exact mass or molecular weight of Fragment 2. This iterative process is used to identify all the 5' fragments. The need to use the calculated mass values for the previous fragment arises from the potential accumulative errors that can occur if only the observed values are used. For example, a 0.3 Da error would still enable the unambiguous identification of a fragment, however, without resetting this error by using the calculated values of the identified fragment, further 0.3 Da errors could accumulate so that unambiguous identification may not be possible due to the small mass difference of 1 Da between C and U nucleosides.

For the identification of the last nucleoside, the same process is used whereby the mass difference between the intact released acylated NOX-E36 Intermediate (Seq. ID. 50) and the calculated mass of the final cyclic phosphate containing fragment is used to confirm the identity of the last nucleotide. As the released acylated NOX-E36 Intermediate possesses no 2',3' cyclic phosphate, the mass difference is not the same as for those fragments calculated previously. The mass difference corresponds to the mass of the last nucleoside.

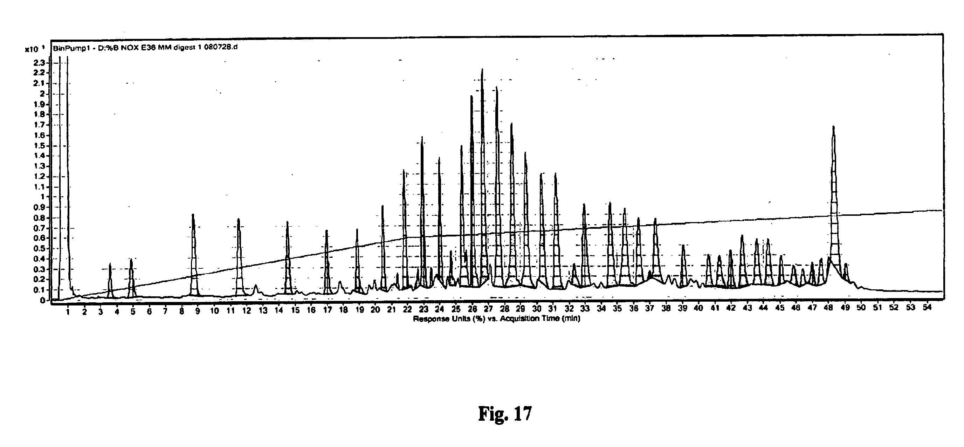

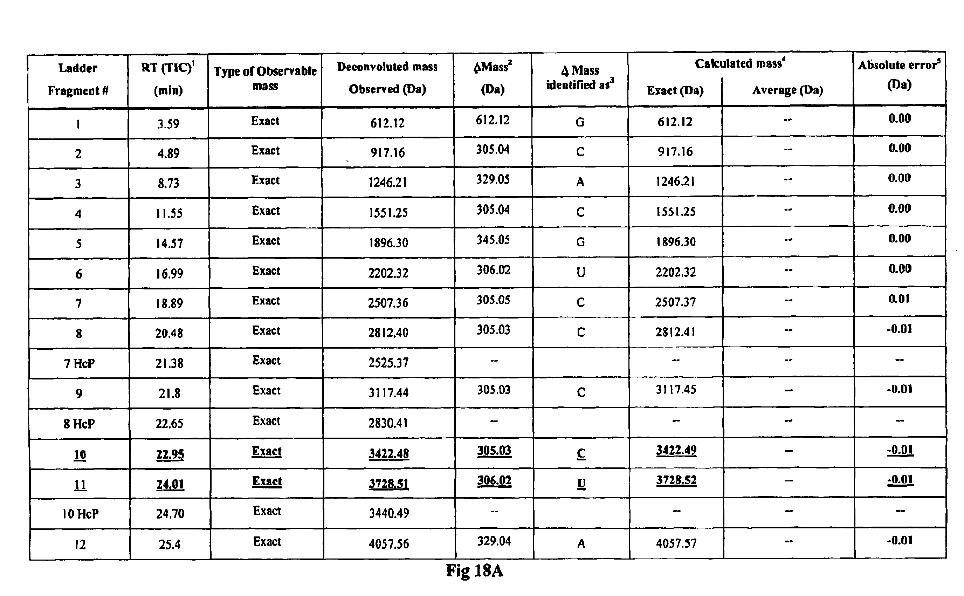

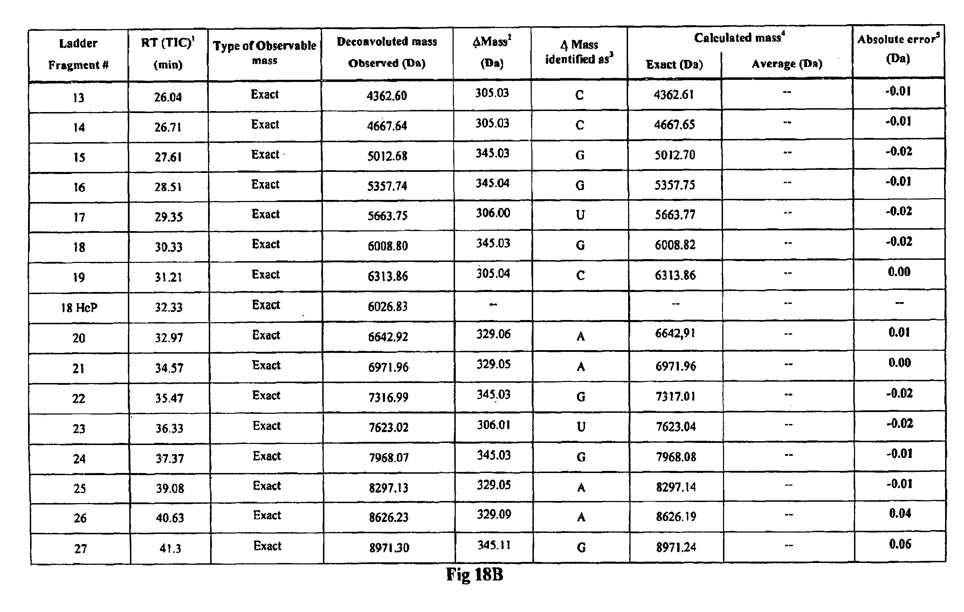

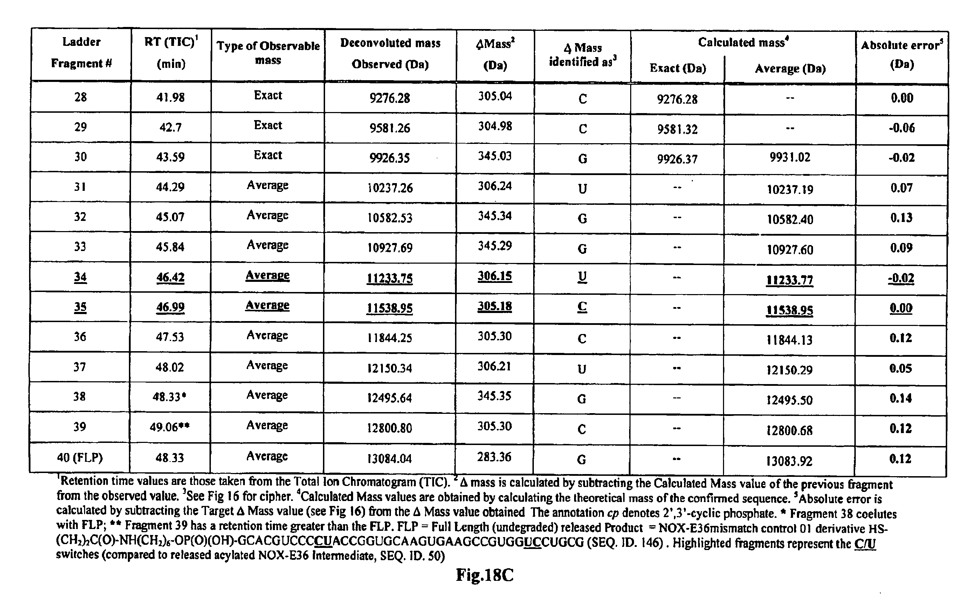

As a test to demonstrate the power of the method, NOX-E36 mismatch control 01 (SEQ.ID. 3), which is identical in sequence to NOX-E36 Intermediate (SEQ.ID. 2) except for two instances of a cytosine and a uridine switched around, was processed using the protocol described in the example 3 and the sequence identified using the 'Flow chart for Sequence Determination/Validation' as shown Fig. 16. The cytosine/uridine switch is the most challenging to detect and was therefore chosen. The method as described was able to easily identify the two mutations to the parent sequence (see Example 3, Figs 17 and 18).

As outlined in more detail in the claims and example 4, an alternative method for sequencing of a nucleic acid molecule by mass spectrometry is provided herein, whereby the nucleic acid is not immobilised. According to the present invention, in a first step the nucleic acid molecule is endowed with a modification, in this example a label possessing a selective wavelength absorbance that nucleobases do not absorb at. The next step is the limited random cleavage of the nucleic acid molecule by chemical cleavage to create a mix of fragments representing random strand scission, and intact full length material. Subsequently, the crude reaction mixture is analysed by LC-MS. At the selective wavelength absorbance of the label, there is no UV absorbance attributable to the nucleic acid component of the molecule or fragments thereof. Therefore at this wavelength a mass ladder depicting all possible fragments representing cleavages 3' to every nucleotide of the nucleic acid molecule are selectively observed. By identifying the retention time of these 5' fragments, and deriving and deconvoluting the mass spectra at the retention times of these 5' fragments, it is possible to either confirm the sequence of the nucleic acid molecule or determine it without prior knowledge of the sequence. As there is no isolation of the labeled fragments, the TIC is complicated due to the presence of the non-labelled fragments: Whereas smaller fragments are well resolved on the column and the absolute separation of labelled fragments from non-labelled fragments is possible, larger labelled fragments co-elute with non-labelled fragments, which also generate mass signals, and can therefore interfere with the identification of the desired 5' fragments. However, due to the lypophilicity of the label, labelled fragments of a certain mass value typically elute later than non-labelled fragments of a similar mass value. As such, it is possible through reason to eliminate spurious masses obtained from co-eluting non-labeled fragments and identify the intended labelled fragments.

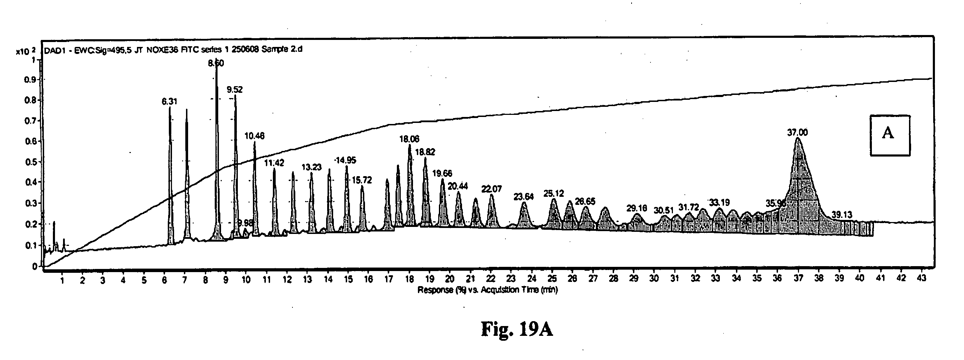

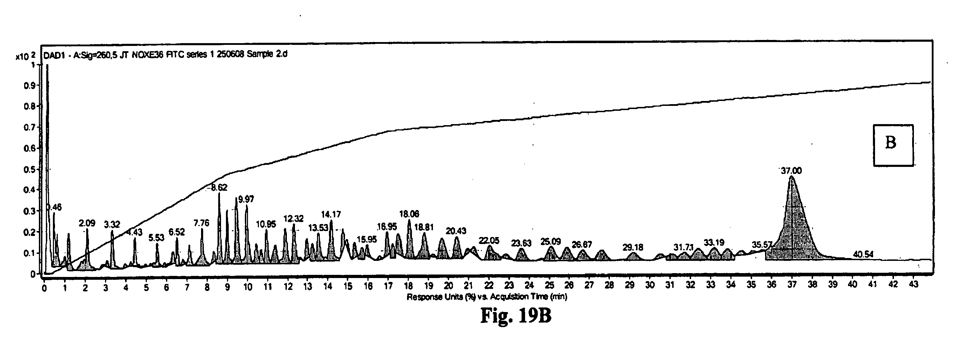

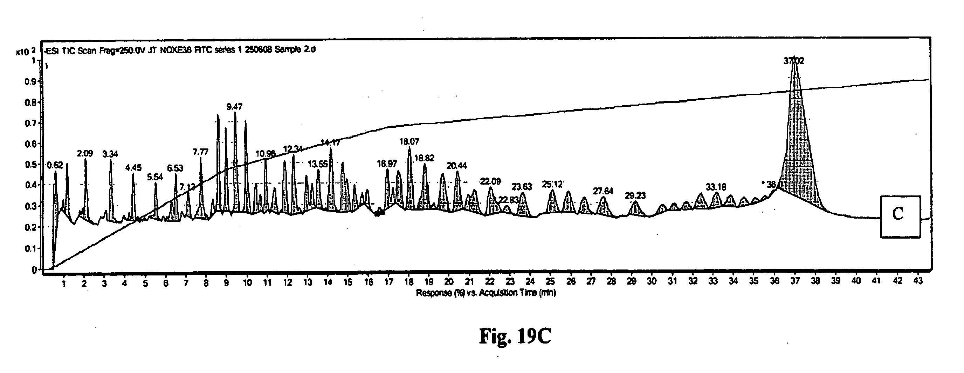

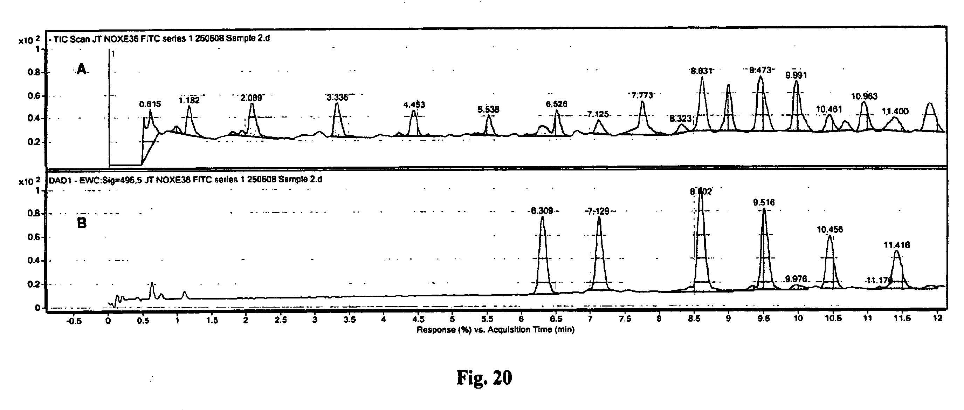

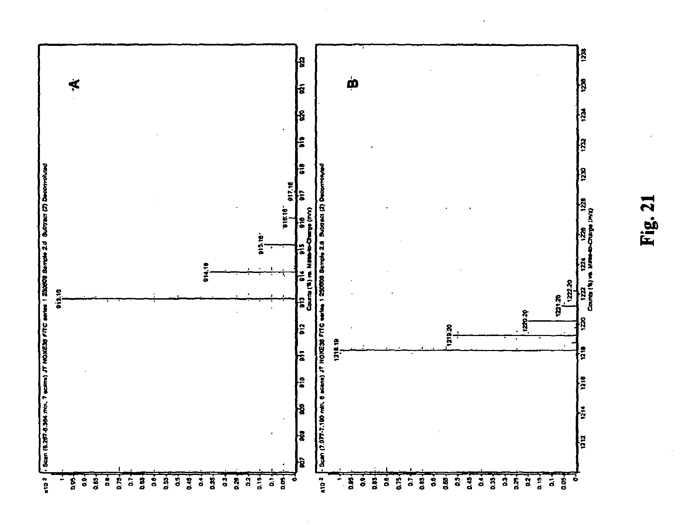

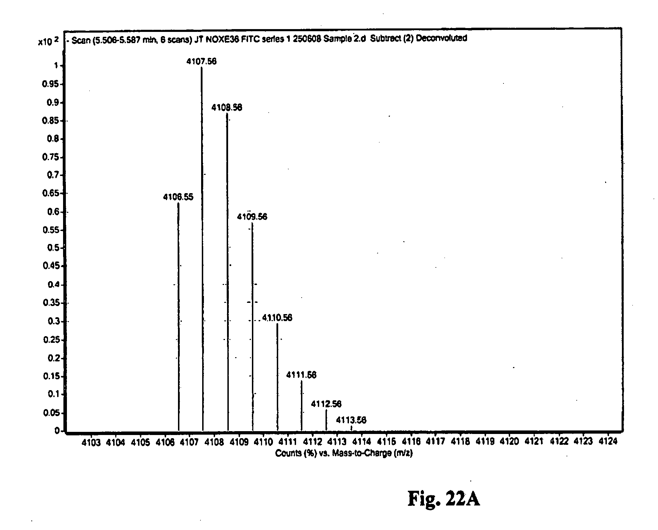

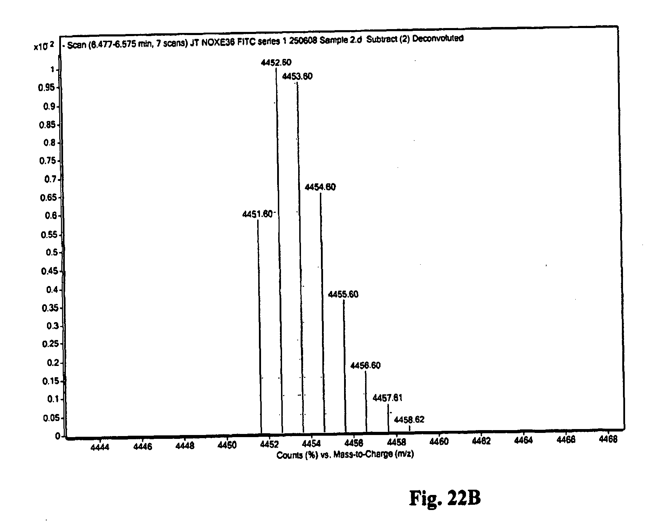

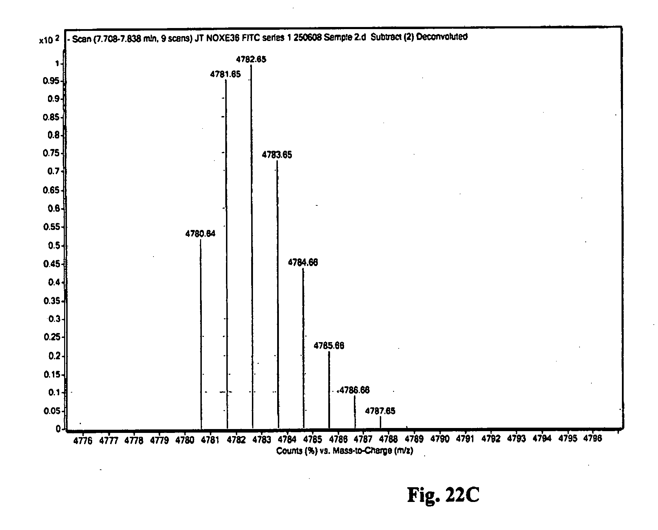

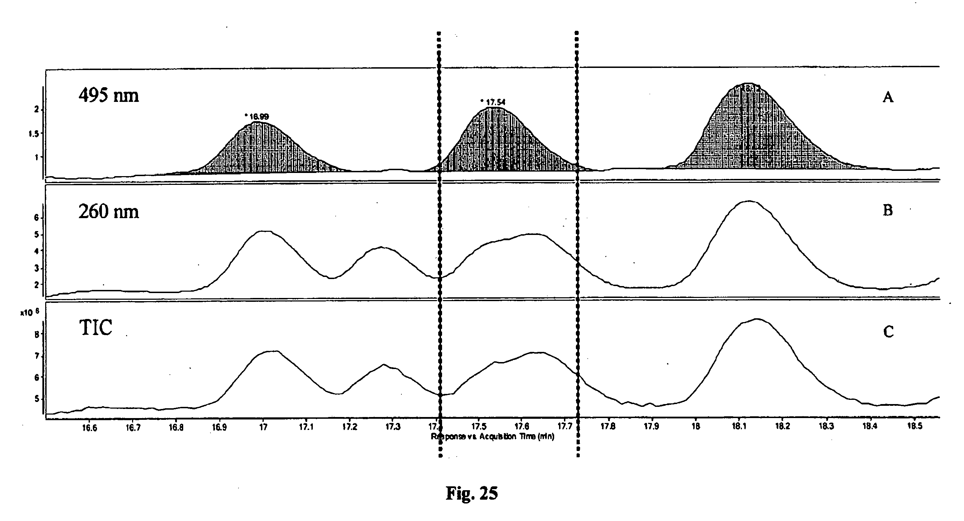

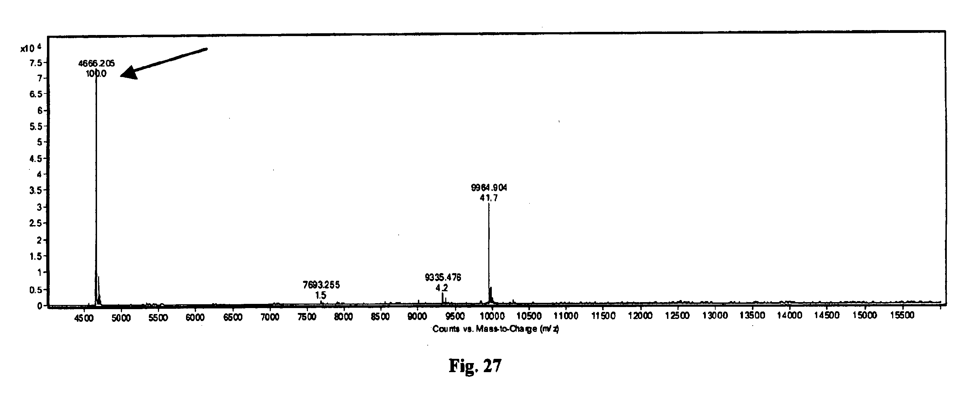

To demonstrate the feasibility of this method, a Fluorescein-5-isothiocyanate (FITC Isomer I) label was attached to NOX-E36 Intermediate (SEQ.ID. 2) to give FITC-NOX-E36 (SEQ. ID. 100). NOX-E36 Intermediate (SEQ.ID. 2) is a 5'-amino-modified derivative of NOX-E36 (SEQ.ID. 1). The labelled NOX-E36 Intermediate was then subjected to base mediated limited random cleavage and the sample analysed by LCMS. The label has a selective wavelength absorbance whose maximum is at approx 495 nm, therefore, only nucleic acid molecules containing an intact 5' end will be observed at this wavelength absorbance. Comparing the UV chromatogram extracted at 495 nm (Fig 19A) with the corresponding UV chromatogram extracted at 260 nm (Fig. 19B), at which wavelength all nucleic acids are detected, it can be clearly seen that on the latter UV chromatogram many fragments that are not 5' fragments of the nucleic acid molecule are present in the sample. It can be seen by comparing Fig 19B with Fig 19C (corresponding TIC of Figs 19A and 19B) that all fragments of the nucleic acid molecule either with or without label generate mass data. As determined through the chromatogram extracted at 495 nm, the first 5' fragment of the nucleic acid molecule, can be readily identified to be that eluting at 6.31 minutes (Fig 19A and enlargement Fig 20B, deconvoluted exact mass Fig. 21A). It can also be clearly seen by comparing Fig 19A with 19B (and more easily with zoom-in Figs 20A and 20B) that there are many non-labelled fragments that elute earlier than this peak. However, these represent fragments of between 8 and 14 nucleotides in length as estimated according to their observed masses (For an examples of 1 such peaks, see Fig 22A). Therefore due to the lypophilicity afforded to the labelled fragments, any non-labelled fragments that co-elute with the labelled fragments can be eliminated due to the significant difference in mass (c.a . 2000-6000 Da) to that expected for a particular fragment size (see Figs 25-27, example 4). This ability to discount spurious masses, or in other words, determine the mass of the labelled fragment in the cases that they co-elute with non-labelled fragments, allows for both the sequence confirmation (Fig. 24) and the sequence determination of nucleic acid molecules with this method (Fig 28A-C).

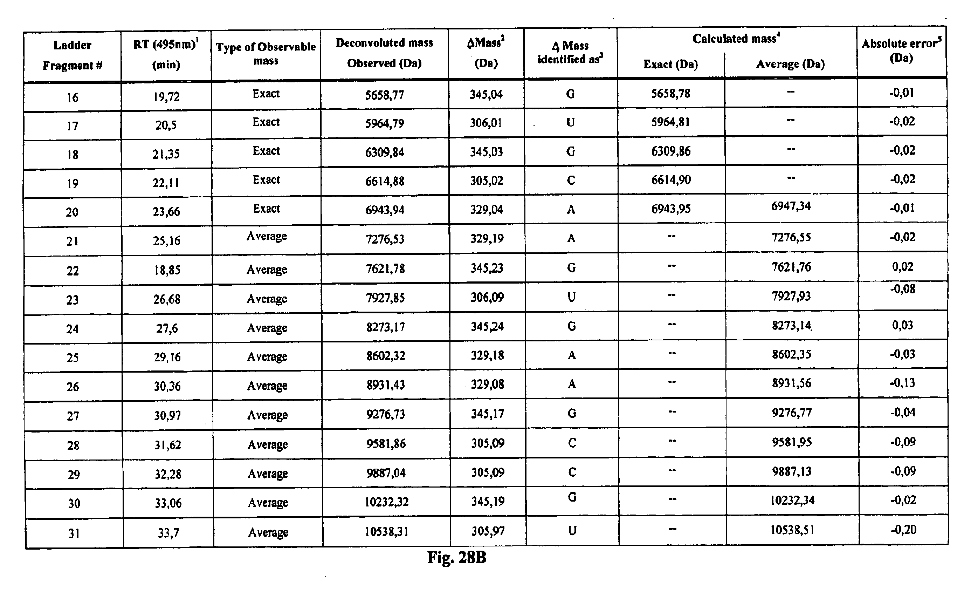

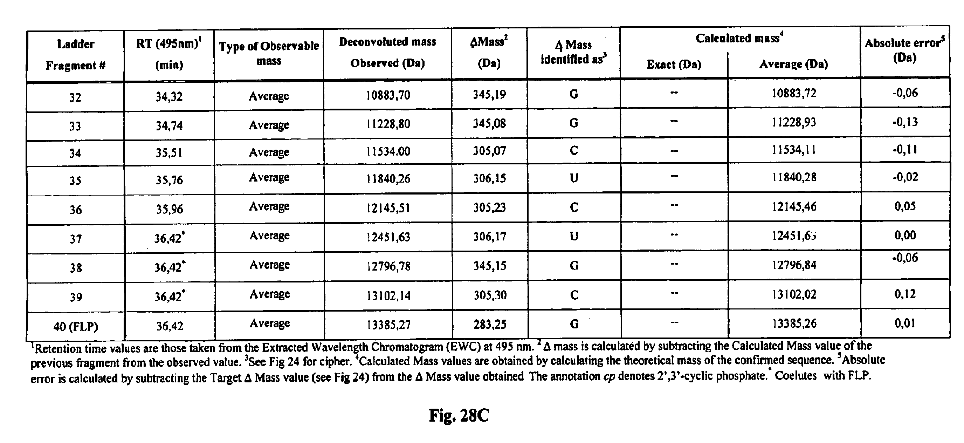

Therefore, in a similar manner to Example 3 analogous sequence confirmation tables or analogous flow charts can be generated (see Figs 23 and 24) for the sequence confirmation or determination without prior knowledge of the sequence (Fig 28) as the principle of determining the sequence through the discrete mass differences between the fragments is analogous to that applied in example 3.

Example 5 is analogous to example 3, except that instead of the Spiegelmer NOX-E36 which comprises 40 nucleotides the sequencing of Spiegelmer NOX-A12 comprising 45 nucleotides is described.

### Example 1: Synthesis and derivatization of Spiegelmers

### 1.1 Small scale synthesis

Spiegelmers were produced by solid-phase synthesis with an ABI 394 synthesizer (Applied Biosystems, Foster City, CA, USA) using 2'TBDMS RNA phosphoramidite chemistry (Damha and Ogilvie, 1993). rA(N-Bz)-, rC(Ac)-, rG(N-ibu)-, and rU- phosphoramidites in the L-configuration were purchased from ChemGenes, Wilmington, MA. Spiegelmers were purified by gel electrophoresis.

### 1.2 Large scale synthesis plus modification

The Spiegelmers were produced by solid-phase synthesis with an AktaPilot100 synthesizer (Amersham Biosciences; General Electric Healthcare, Freiburg) using 2'TBDMS RNA phosphoramidite chemistry (Damha & Ogilvie, 1993). L-rA(N-Bz)-, L-rC(Ac)-, L-rG(N-ibu)-, and L-rU- phosphoramidites were purchased from ChemGenes (Wilmington, MA, USA). The 5'-amino-modifier was purchased from American International Chemicals Inc. (Framingham, MA, USA). Synthesis of the Spiegelmers was started on L-riboG; L-riboC, L-riboA, L-riboU respectively modified CPG pore size 1000 Å (Link Technology, Glasgow, UK). For coupling (15 min per cycle), 0.3 M benzylthiotetrazole (American International Chemicals Inc., Framingham, MA, USA) in acetonitrile, and 3.5 equivalents of the respective 0.2 M phosphoramidite solution in acetonitrile was used. An oxidation-capping cycle was used. Further standard solvents and reagents for oligonucleotide synthesis were purchased from Biosolve (Valkenswaard, NL). The Spiegelmers were synthesized DMT-ON; after deprotection, it was purified via preparative RP-HPLC (Reverse-Phase High-Performance Liquid-Chromatography) (Wincott et al, 1995) using Source15RPC medium (Amersham, Freiburg, Germany). The 5'DMT-group was removed with 80% acetic acid (90 min at RT). Subsequently, aqueous 2 M NaOAc solution was added and the Spiegelmer was desalted by tangential-flow filtration using a 5 K regenerated cellulose membrane (Millipore, Bedford, MA).

### Example 2: Sequencing of a nucleic acid molecule without immobilization of the nucleicacid molecule using nucleobase specific cleavage reactions

### 2.1 Principle of the method

For the sequencing of a nucleic acid molecule without immobilization of the nucleic acid molecule the following steps are done:
1) Base selective treatment of the nucleic acid molecule, to modify a specifically chosen nucleobase (i.e. A, C, G, T or U), followed by a second step where the nucleic acid phosphate backbone is selectively chemically cleaved 3' to the modified nucleobase. Thereby it is necessary to develop conditions where the fragmentation reaction is not driven to completion so that there are still fragments of the nucleic acid molecule present that contain non-modified nucleobases (that was not point of modification and/or chemical cleavage).
2) Analysis of the fragments of the nucleic acid molecule, generated by LCMS and LC/MS/MS.

The result of fragmentation is a set of fragments of the nucleic acid molecule representing cleavages 3' to every occurrence of the modified nucleobase. By identifying a set of expected fragments generated (e.g. 3'- or 5' fragments of the nucleic acid molecule), it is possible to perform tandem mass spectrometry experiments on these fragments and by virtue of the overlapping nature of these fragments, and their relation to the intact nucleic acid molecule (i.e. the full-length molecule), it is possible to the confirm of the nucleic acid molecule's sequence. Thereby it is necessary to control the extent of chemical cleavage so that this relationship is preserved, and to identify a specific set of fragments, preferably with an intact 5' or 3' terminus on which MS/MS experiments can be performed.

### 2.2 Sequencing of Spiegelmer NOX-E36

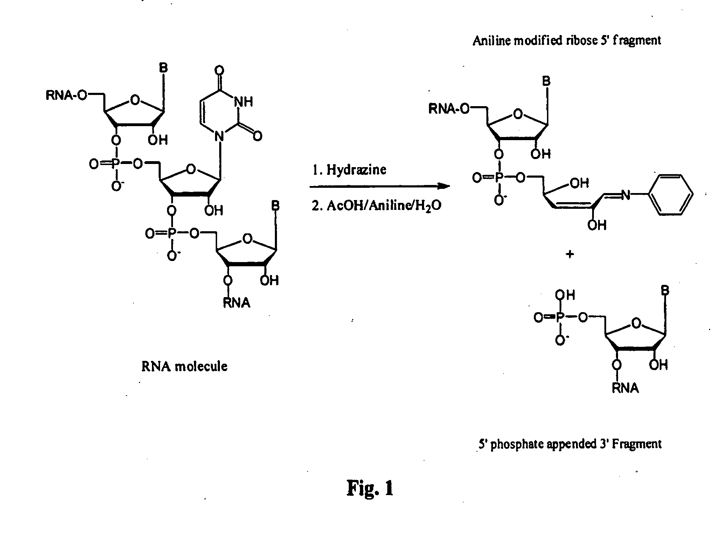

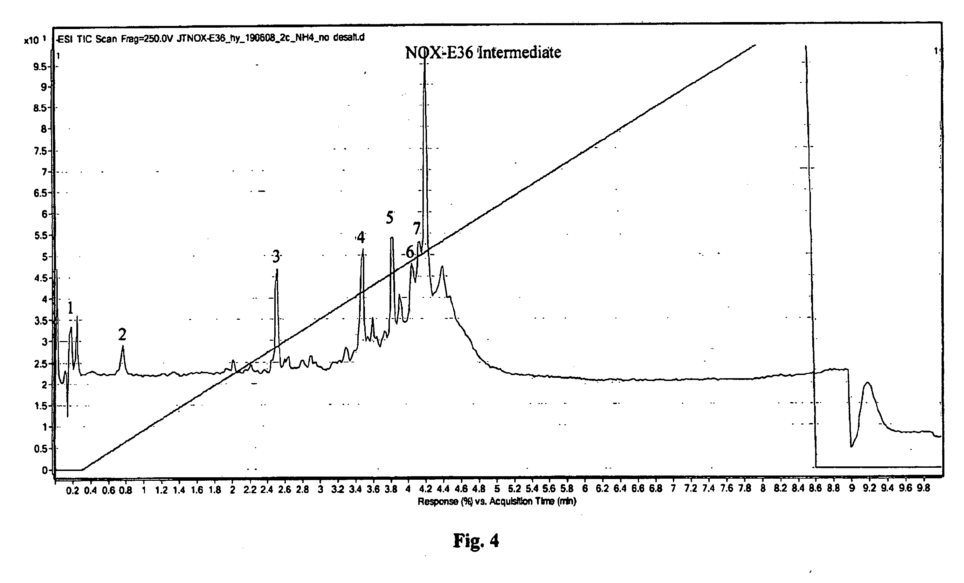

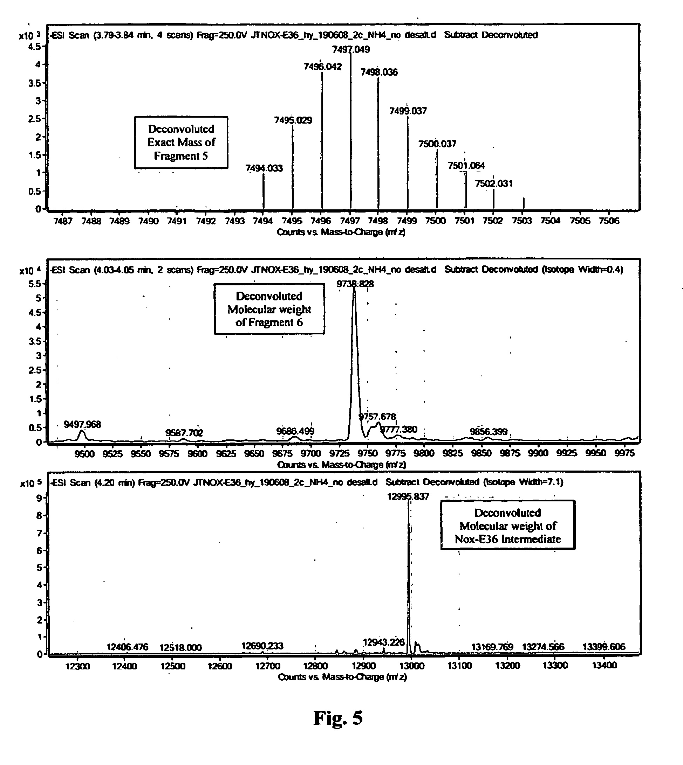

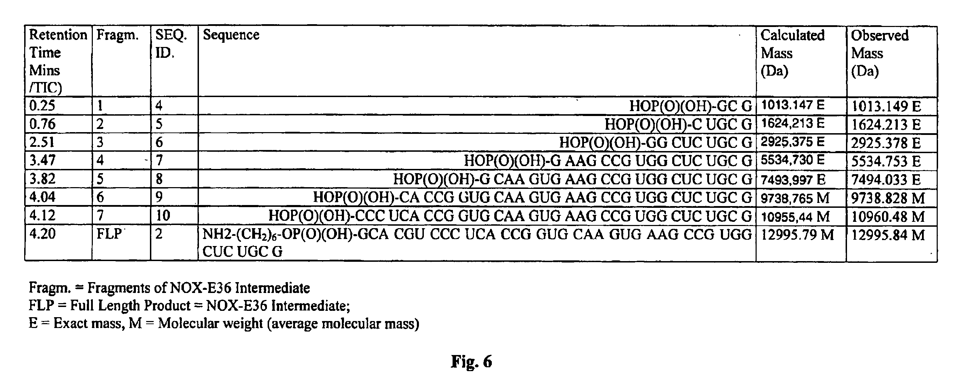

To proof the method described, the RNA-molecule NOX-E36 Intermediate (SEQ.ID. 2) was used. NOX-E36 Intermediate is a Spiegelmer, whereby NOX-E36 Intermediate is a 5'-amino-modified derivative of Spiegelmer NOX-E36 (SEQ.ID. 1). The uridine nucleobase was chosen to be selectively modified. The modification is effected by the use of a two-step hydrazine-acetic acid/aniline treatment that leads to the chemical cleavage of an RNA molecule after uridine moieties providing fragments of the RNA molecule. In general, the reaction products after hydrazine-acetic acid/aniline treatment are those of a 5'-phosphate appended 3'-fragment of an RNA molecule and an aniline modified ribose 5' fragment of an RNA molecule carrying a modified ribose moiety [abbr. Umod to highlight nucleobase cleavage site] as shown in Fig. 1 and 2B. Such structures have been proposed by Ehresmann et al. (Ehresmann et al, 1987) (see Fig. 1). Subjection of Spiegelmer NOX-E36 Intermediate (SEQ.ID. 2) to hydrazine-acetic acid/aniline treatment and analysis of the fragmentation products revealed that surprisingly, 3'- fragments and the intact nucleic acid molecule (Fig. 2A) are ionized more efficiently than 5'- and internal fragments, thus greatly simplifying the interpretation of the data generated and identifying fragments of interest (Fig. 4-6).. As a result, overlapping 3'- fragments containing a 5'-phosphate that represent cleavages after each occurrence of a uridine nucleotide and the intact starting molecule NOX-E36 Intermediate (SEQ.ID. 2) can be readily identified (Fig. 6) through deconvolution of the derived mass data pertaining to each peak (For examples, see Fig. 5). Deconvolution is a common technique well known to those skilled in the art whereby an algorithm is applied to a mass spectrum to identify multiply charged ions of a single species and reconstitute them into the mass of this species. This technique is highly valuable in combination with ESI and other ionisation techniques which observe large molecules as a distribution of multiply charged ions. Depending on the algorithm applied either the isotopic resolved masses (to obtain the exact mass) or the molecular weight is obtained. Typically for oligonucleotides a mass spectrometer calibrated at 5 ppm is able to produce resolved isotope spectra up to approximately 6-10 kDa depending on the ionisation efficiency. Above this mass, typically an algorithm, such as the Maxent algorithm, is used for deconvolution to the molecular weight (average molecular mass) of the species.

By utilizing MS/MS techniques familiar to those skilled in the art (see description), the sequence confirmation of the smallest fragment can be achieved. Then, using the data generated as a reference, an 'overlapping principle' can be employed for the following fragment of the nucleic acid molecule so that only the additional sequence information, the unknown section of the following fragment, is required for sequence confirmation of this fragment. In such a way, this overlapping principle can be employed to confirm the sequence of the entire molecule as the gap between any one specific nucleobase (A, C, G, U in the RNA series) is typically no more than 10-15 nucleobases (Fig. 2A). Furthermore, this overlapping principle renders the fragments of the nucleic acid molecule or the intact molecule needing only to be sequenced from their 5'- extremities (as opposed to both the 5'- and 3'-extremities), thus making the MS/MS analysis more straightforward. In order for this overlapping principle to be employed, it is necessary to control the extent of chemical cleavage so that this overlapping relationship of the fragments is not destroyed. When the reaction is driven to completion (Fig. 2B) the relationship between the fragments cannot be elucidated i.e. the position of the fragments cannot be confirmed and therefore the sequence cannot be confirmed. Hence, the protocol described herein represents a controlled fragmentation of Spiegelmer NOX-E36 Intermediate (SEQ.ID. 2). MS/MS of these fragments can be achieved either through LC/MS/MS or by isolating the individual 3'- fragments via standard Liquid Chromatographyand then directly infusing them into the mass spectrometer for MS/MS experiments.

### 2.3 Protocol

8 µl (0.85 OD/µl water) Spiegelmer NOX-E36 Intermediate (SEQ.ID. 2), i.e. a derivative of Spiegelmer NOX-E36 (SEQ.ID. 1) with 5' amino linker, was placed in a 500 µl microfuge tube and chilled on ice whereupon 24 µl hydrazine hydrate 50-60% (22,581-9, Sigma Aldrich, Taufkirchen, Germany) was added. After 45 mins, 4 µl 10 M ammonium acetate p.a. (Sigma Aldrich, Taufkirchen, Germany) was added, the solution briefly vortexed and chilled ethanol (300µl) was added. The solution was re-vortexed and allowed to chill in a freezer at -18 °C for 2 h whereupon it was centrifuged (12,000 g) for 15 minutes at 4 °C and the supernatant decanted. The pellet was washed with 300 µl chilled ethanol by vortexing and centrifuged (12,000 g) for 5 min. The supernatant was removed and the pellet dried in a Concentrator 5301 (Eppendorf AG, Hamburg, Germany) and then treated with a solution of 170 µl water, 18 µl Aniline (≥99.5 %, 242284 Sigma Aldrich, Taufkirchen, Germany), 11 µl Acetic Acid (≥99% A6283, Sigma Aldrich, Taufkirchen, Germany) at 65°C for 40 min excluding light from the reaction. The solution was then dried in a Concentrator 5301 (Eppendorf AG, Hamburg, Germany) and redissolved in sterile water (70 µl) and subjected to LCMS analysis.

LCMS analysis: The LCMS analysis of the fragments generated from the protocol above were analysed using a 6520 Accurate Mass Q-TOF LCMS system (Agilent Technologies, Waldbronn, Germany) with Rapid Resolution Pump and an Acquity BEH C18 Column (1.7 µm, 130 A pore size, 2.1 x 30 mm, Waters, Eschenbronn, Germany). Gradient 0-70% B in 7.7 min. Buffer A: 10 mM Triethylamine, 100 mM Hexafluoroisopropanol, 10 µM EDTA (NH₄⁺ form), 1% Methanol in Water, Buffer B: 10 mM Triethylamine, 100 mM Hexafluoroisopropanol, 10 µM EDTA (NH₄⁺ form), 50% Methanol in Water. Column temperature 65°C, Flow rate 1.2 ml/min.

### 2.4 Results

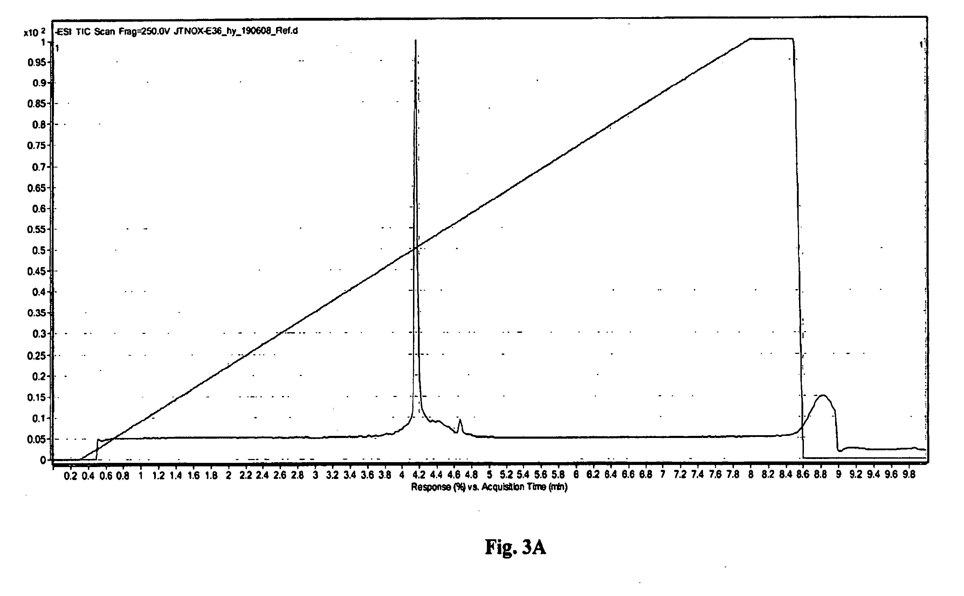

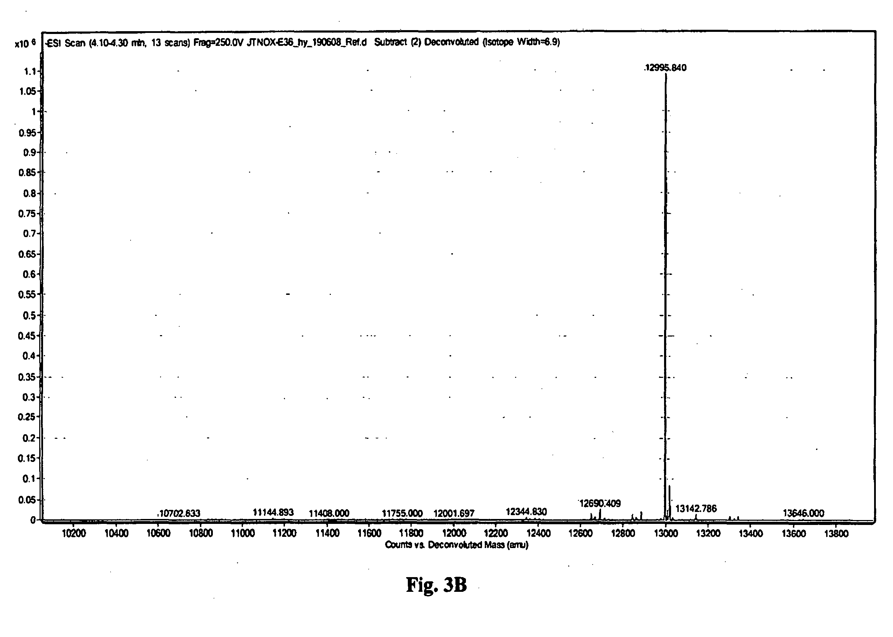

Figure 3A shows the Total Ion Chromatogram (abbr. TIC) of the intact nucleic acid molecule Spiegelmer NOX-E36 Intermediate (SEQ.ID. 2) displaying one major peak, which has a deconvoluted observable mass of 12995.84 Da (Fig 3B).. Treatment of Spiegelmer NOX-E36 Intermediate (SEQ.ID. 2) as described in the protocol above (see Section 2.3) and analysis using LCMS led to clearly defined fragments that were discernable in the first instance by Reversed Phase-HPLC column chromatography in combination with mass spectrometry, as evidenced in the TIC (Fig. 4) and subsequently identified through the deconvoluted mass spectra of the individual peaks as demonstrated for fragments 5, 6 and the intact nucleic acid molecule NOX-E36 Intermediate (SEQ.ID 2) (Fig. 5). Surprisingly, it was found that despite the various products that are possible (5'-, 3' and internal fragments), it was observed that the fragments derived from the 3' end were the major products and clearly distinguishable despite the presence of other fragments such as 5' fragments and internal fragments. Consequently, all the expected 3'-fragments, i.e. those resulting from strand scission 3' of Uridine moieties were readily identified by comparing the mass values to those calculated from the predicted fragments (Fig. 6). As such it would be possible to perform tandem MS/MS experiments on the 3' fragments as previously described, to obtain a confirmation of the sequence as follows:

State-of-the-art mass spectrometry machines such as ESI-MS machines typically allow for the sequence confirmation of the first 10-15 nucleotides from each end of a nucleic acid molecule using established MS/MS techniques. Therefore by performing MS/MS on the smallest fragment (Fragment 1, SEQ.ID. 4), the sequence of this fragment can be readily confirmed. Next, the sequence identity of Fragment 2 (SEQ.ID. 5) can also be confirmed. With Fragment 2 however, it is only necessary to obtain information for the additional nucleotides on the 5' extremity of the 3' fragments as they overlap on their 3' extremities (Fig. 2A). The sequence of Fragment 3 can be confirmed in an analogous way. This iterative process can be used to confirm the sequence of the entire Spiegelmer NOX-E36 Intermediate (SEQ.ID 2).

In summary, using chemical reactions that induce nucleobase specific strand scission, it is shown that it is possible to fragment a nucleic acid molecule in a carefully controlled fashion such that fragments are produced with a clear relationship to each other, which can be used to sequence a nucleic acid molecule following the 'overlapping principle' as described above. The ability to do this is greatly facilitated by virtue of the surprising discovery that only one set of fragments (3' fragments) predominates in the TIC when the crude mixture is analysed by LCMS, despite the presence of significant amounts of 5' and internal fragments.

### Example 3: Sequencing of a nucleic acid molecule with immobilization of the nucleic acid molecule and selected fragments thereof

### 3.1 Principle

For the sequencing of a nucleic acid molecule with immobilization of the nucleic acid molecule and selected fragments thereof, the following steps are done: Labeling the nucleic acid molecule with an affinity label or tag (where one is not already affixed), limited random cleavage of the nucleic acid molecule by chemical cleavage to create a mix of fragments representing random strand scission according to the scheme shown in Fig. 7 plus uncleaved full length material. From this random mix, those fragments of the nucleic acid molecule that contain the label are pulled out of the mix using the affinity label as a handle, binding to a solid support, be that in a column, on a chip, or in bead format, and the other fragments are washed away. Release by cleavage or elution of the desired fragments of the nucleic acid molecule from the solid phase furnishes the fragments to be analyzed by mass spectrometry. This example demonstrates the use of LCMS as a mass spectrometry technique suitable for sequencing nucleic acid molecules. The result obtained is a ladder representing all possible fragments of the nucleic acid molecule, more precisely representing cleavages 3' to every nucleotide of the nucleic acid molecule. The analysis methods used in this example enables separation of these fragments firstly by Liquid Chromatography, i.e. the LC part of the LCMS, and then by mass spectrometry. This two-dimensional approach facilitates the identification of the individual fragments which, enables a two-dimensional identification of the fragments that are readily separable.

### 3.2 Sequencing of Spiegelmer NOX-E36

To test this method, the nucleic acid molecule Spiegelmer NOX-E36 Intermediate (SEQ.ID. 2) was used. NOX-E36 Intermediate (SEQ.ID. 2) is a 5'-amino-modified derivative of Spiegelmer NOX-E36 (SEQ.ID. 1). As shown in Fig. 8, after modifying the 5'-amino moiety of NOX-E36 Intermediate (SEQ.ID. 2) with a biotin affinity tag, the biotinylated NOX-E36 Intermediate (SEQ. ID. 63) was chemically cleaved in a random fashion using a basic solution. The cleavage was carefully controlled so as not to drive the cleavage to completion. From the random fragmentation that occurs which produces 5'- fragments, 3'- fragments and random internal fragments of NOX-E36 Intermediate (SEQ.ID. 2), all biotinylated 5' fragments of NOX-E36 Intermediate (Fig. 8, series 1) and remaining biotinylated NOX-E36 Intermediate (i.e. full-length product [abbr. FLP]) were selectively pulled out from the mix via the affinity tag (in this case biotin) using tag-specific solid support for immobilisation (in this case Neutravidin beads). The unbound fragments, i.e. 3'- fragments and random internal fragments that do not possess the affinity tag, can be washed away. The bound 5' fragments of Spiegelmer NOX-E36 and the FLP (SEQ. ID. 63) are then liberated from the beads by reductively cleaving the disulfide bond within the linker connecting the biotin moiety and NOX-E36 Intermediate (SEQ.ID. 2). These released fragments correspond to strand scission between every ribonucleoside position (see Figs 8 and 13). The strand scission results first in the formation of 2',3'-cyclic phosphate containing fragments whereby the cyclic phosphate slowly hydrolyses to the 2'(3') phosphate. The liberated fragments were then analysed by LC-(ESI)MS, and the Total Ion Chromatogram (abbr. TIC) was analysed. Sample chromatograms display discrete peaks that correspond to all 5'-fragments generated and the intact released acylated NOX-E36 Intermediate (SEQ. ID. 50), as shown in Fig 11. The mass(es) contained in the discrete peaks were then obtained through deconvolution of the derived mass spectra pertaining to each discrete peak.

Deconvolution is a common technique well known to those skilled in the art whereby an algorithm is applied to a mass spectrum to identify multiply charged ions of a single species and reconstitute them into the mass of this species. This technique is highly valuable in combination with ESI and other ionisation techniques which observe large molecules as a distribution of multiply charged ions. Depending on the algorithm applied either the monoisotopic resolved masses (to obtain the exact mass) or the molecular weight is obtained. Typically for oligonucleotides a mass spectrometer calibrated at 5 ppm is able to produce resolved isotope spectra up to approximately 6-10 kDa. Above this mass, typically an algorithm, such as the Maxent algorithm, is used that deconvolutes to the molecular weight of the species.

In general, the masses seen are those of the 2',3'-cyclic phosphates, although in some cases, the low abundance of fragments containing the hydrolysed 2' (3') phosphate can also be detected, which serve to further confirm the identity of the fragments generated. Typically these hydrolyzed fragments elute later than the parent 2',3'-cyclic phosphate.

The masses of the fragments generated can in the first instance be compared to the calculated masses of the predicted 5' fragments of released NOX-E36 Intermediate (SEQ.ID. 50) to confirm the sequence (Fig. 13A-E). Alternatively, the sequence can be derived without prior knowledge of the sequence due to the differences between the fragments generated. In this scenario, the first fragment of the nucleic acid molecule can be easily predicted and the incremental differences of the subsequent fragments can be used to determine the sequence of the nucleic acid molecule, as demonstrated in the 'Flow chart for Sequence Determination/Validation' (Fig. 16). This flow chart describes a step by step process whereby the smallest fragment (denoted Fragment 1) is first identified. The first fragment represents the first 5' nucleotide with both a 5'-affixed acylated aminohexyl linker and 2',3'-cyclic phosphate such as depicted in Fig. 8 (series 2, y = 0) and Fig. 13A (SEQ. ID. 11). Consequently it is straightforward to calculate all possible RNA permutations (A, C, G or U) for the first fragment (Fig. 16). The identification of this first fragment is facilitated by the knowledge that Fragment 1 will be the earliest eluting 5' fragment using Ion-Pair Reversed Phase HPLC (abbr. IP RP-HPLC) as is known by those familiar with the art of IP RP-HPLC (Azarani et al. 2001 and references cited therein). Once Fragment 1 has been identified, the calculated exact mass and molecular weight are used to identify the next fragment, Fragment 2. The identity and therefore sequence of the next fragment, Fragment 2, is derived from the mass difference between Fragment 2 and the calculated exact mass or molecular weight of Fragment 1. The mass difference is unique for each nucleoside A, C, G, U (Fig 16). Once Fragment 2 has been identified, the calculated exact mass and molecular weight are used to identify the next fragment, Fragment 3. In an identical procedure to that used to identify Fragment 2, the identity of Fragment 3, is derived from the mass difference between Fragment 3 and the calculated exact mass or molecular weight of Fragment 2. This iterative process is used to identify all the 5' fragments. The need to use the calculated mass values for the previous fragment arises from the potential accumulative errors that can occur if only the observed values are used. For example, a 0.3 Da error would still enable the unambiguous identification of a fragment, however, without resetting this error by using the calculated values of the identified fragment, further 0.3 Da errors could accumulate so that unambiguous identification may not be possible due to the small mass difference of one Da between C and U nucleosides.

For the identification of the last nucleoside, the same process is used whereby the mass difference between the intact released acylated NOX-E36 Intermediate (Fig. 8) and the calculated mass of the final cyclic phosphate containing fragment (in the case of an oligonucleotide of 40 nucleotides in length X = 39, Series 2, Fig. 8) is used to confirm the identity of the last nucleotide. As the released acylated NOX-E36 Intermediate possesses no 2',3' cyclic phosphate, the mass difference is not the same as for those fragments calculated previously. The mass difference corresponds to the mass of the last nucleoside (Fig. 16).

As a test to demonstrate the power of the method, NOX-E36 mismatch control 01 (SEQ. ID. 3), which is identical in sequence to NOX-E36 Intermediate (SEQ.ID. 2) except for two instances of a cytosine and a uridine switched around, was processed using the protocol described in this example. and the sequence identified using the 'Flow chart for Sequence Determination/Validation' (Fig. 16). The cytosine/uridine switch is the most challenging to detect and was therefore chosen. The method as described was able to easily identify the two mutations to the parent sequence (see Fig. 17 for chromatogram and Fig. 18 A-C for sequence determination).

### 3..3 Protocol

### 3.3.1 Biotinylation of Spiegelmer NOX-E36 Intermediate

10mg (250 ODs) of crude Spiegelmer NOX-E36 Intermediate (SEQ.ID. 2), i.e. a derivative of Spiegelmer NOX-E36 (SEQ.ID. 1) with 5' amino linker, were placed in a reaction tube and dissolved in 260µl Theorell and Stenhagen's Universal buffer pH8.5 (33 mM Sodium Citrate, 33 nM Sodium Phosphate, 57 mM Sodium Borate, pH 8,5). To this were added 200µl *N,N-*dimethylformamide (abbr. DMF). The solution was vortexed and spun down, whereupon 2,2 mg Biotin disulfide N-hydroxy-succinimide ester (Sigma B4531, Taufkirchen, Germany) pre-dissolved in 50µl DMF was added. The solution was incubated at room temperature for 60 minutes, whereupon an aliquot was taken and analysed by Anion-Exchange HPLC (Dionex DNA-Pac 200 column, Buffer A: 100mM Tris;; 10%ACN in H₂O Buffer B: 1M NaCl, 100mM Tris; 25 mM NaClO₄;; 10%ACN in H₂O. Gradient 10-30 %B in 6 min then 30-70 %B in 35 min, temperature of column 80°C) which determined that the reaction was complete. The crude reaction mixture was desalted using a NAP25 column (Amersham Biosciences, Freibug, Germany) and lyophilised.

### 3.3.2Basic hydrolysis of the biotin labeled Spiegelmer NOX-E36 Intermediate

To 20 µl biotinylated Spiegelmer NOX-E36 Intermediate (SEQ.ID. 2) (at 0.54 OD/µl) was added 30 µl sterilised water and 2.5 µl 0.5 M K₂CO₃ at room temperature. The solution was vortexed and then incubated on a Eppendorf Thermomixer Comfort machine (Eppendorf, Hamburg, Germany) at 70°C at 1350 rpm for 12.5 mins. whereupon it was frozen in liquid nitrogen and allowed to thaw out. Then 4 µl 1M AcOH was added (approx. pH 7) to quench the reaction and the solution vortexed and spun down.

### 3.3.3 Binding of biotinylated fragments to Neutravidin beads

Neutravidin Agarose beads were treated as follows: 150 µl of Neutravidin bead slurry (Pierce, Milwaukee, MI, USA) was put in 500 µl reaction tube. The beads were spun down and the supernatant carefully removed. Whereupon 300 µl 1M Tris HCl pH 8.0 (Ambion; Huntindon, UK) was added. The slurry vortexed, spun down and the supernatant carefully removed. The beads were then washed 2 x 300 µl in the same manner with sterile H₂O. The quenched hydrolysis mix as prepared above was then added to the beads and the resulting slurry mixed vigorously (1350 rpm) at 10°C for 2h. The beads were then isolated through filtration using a spin microfuge tube (Ultrafree-MC GV, 0,22 µm, Millipore, Schwalbach, Germany) and washed with 2 x 300 µl sterile H₂O.

### 3.3.4 Cleavage of the biotinylated fragments from the Neutravidin beads

The disulfide linker of the biotin labeled fragments of NOX-E36 Intermediate (SEQ.ID. 2) was cleaved using a 0.05 M Na phosphate buffer (pH 8.5), 100 µl with 5 µl 1M DTT solution. This was vigorously mixed at 25 deg C for 2h on a Eppendorf Thermomixer Comfort machine. The slurry was filtered using a spin microfuge tube (Ultrafree-MC GV, 0,22 µm, Millipore, Schwalbach, Germany), and the beads washed with a further 50 µl sterile water. A UV measurement was taken to determine the Optical Density Units at 260 nm, and of that 0.25 ODs was analysed by LCMS.

### 3.3.5 LCMS analysis of the fragments

The LCMS analysis of the 5'-fragments of NOX-E36 Intermediate (SEQ.ID. 2) generated from the protocol above were analysed using a 6520 Accurate Mass Q-TOF LCMS system (Agilent Technologies, Waldbronn, Germany) with Rapid Resolution Pump and an Acuity BEH C18 Column (1.7 µm, 130 Å pore size, 2.1 x 30 mm, Waters, Eschborn, Germany). Gradient 0-20% B in 22 min, 20-30% B in 40 min. Buffer A: 10 mM Triethylamine, 100 mM Hexafluoroisopropanol, 10 µM EDTA (NH₄⁺ form), 1% Methanol in Water, Buffer B: 10 mM Triethylamine, 100 mM Hexafluoroisopropanol, 10 µM EDTA (NH₄⁺ form), 50% Methanol in Water. Column temperature 65°C, Flow rate 0.2 ml/min: Mass spectra from the TIC were derived for each peak and then deconvoluted according to standard techniques known to those of average skill in the art.

### 3.4 Results

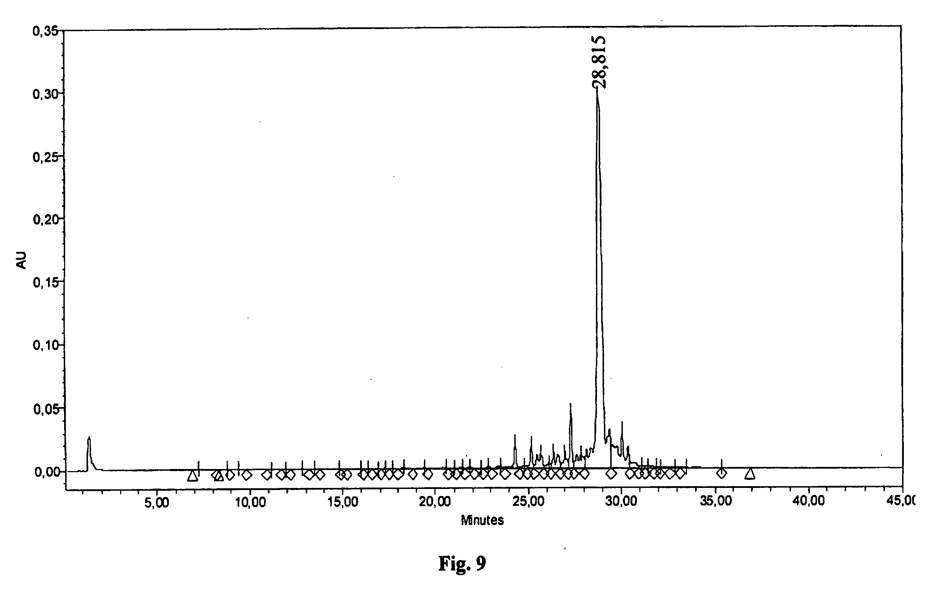

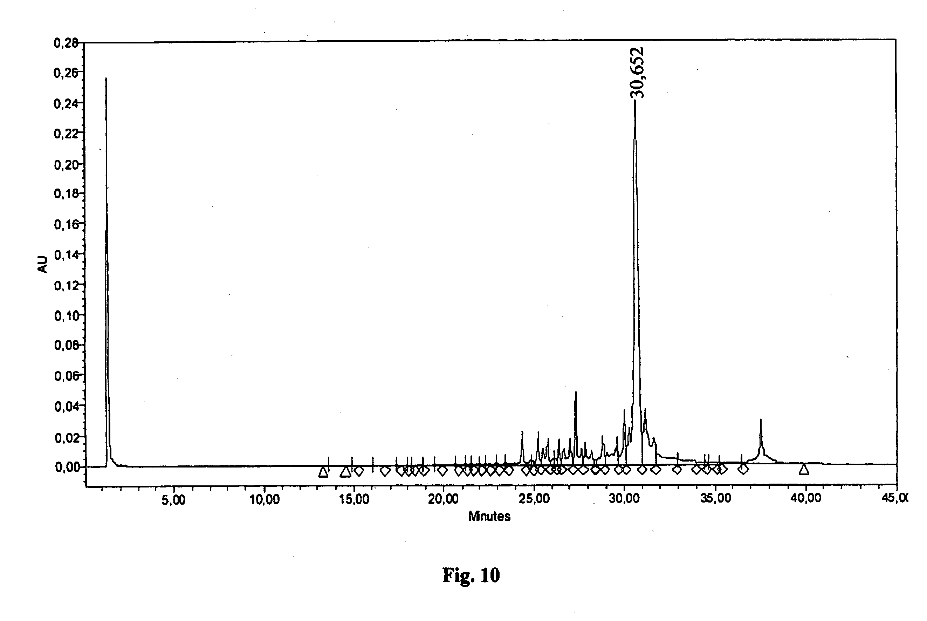

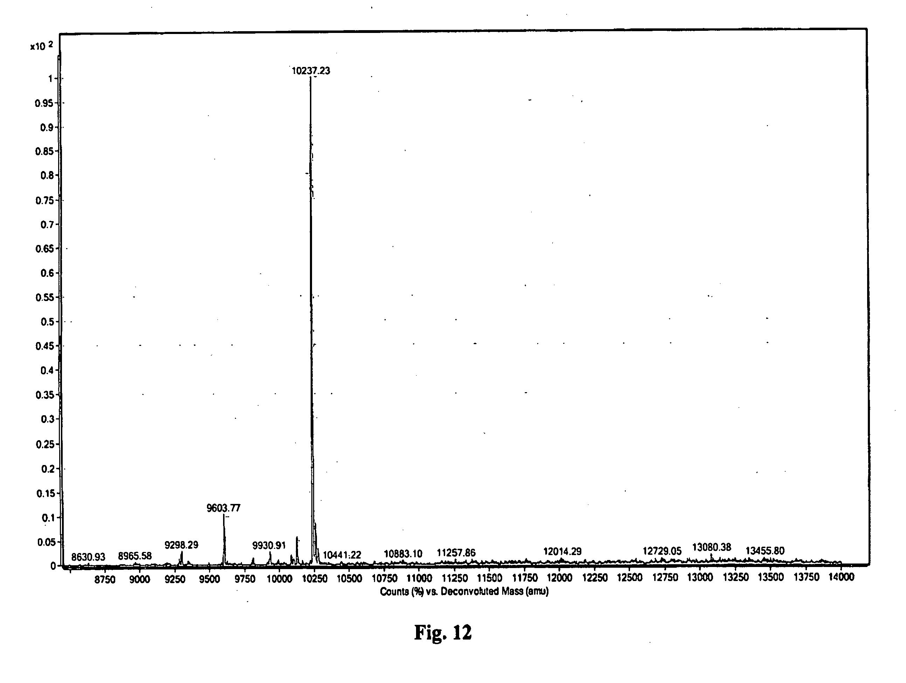

Crude NOX-E36 Intermediate (SEQ.ID. 2) (Fig 9) was efficiently labeled with the cleavable biotin moiety as described in the experimental section (Fig. 10), as determined by the appearance of a later eluting peak (30.65 min c.f. 28.82 min elution time for starting material, Fig 9) utilizing anion-exchange chromatography. The presence of failure sequences from the solid phase synthesis of NOX-E36 Intermediate [SEQ.ID. 2], and other impurties does not affect the ability to carry out steps 3.3.1-3.3.5 and to sequence the nucleic acid molecule. The crude labeling mixture was not purified, save for a rudimentary desalting step using a size exclusion purification column (NAP25, see experimental). This crude material was then fragmented, the labeled fragments immobilized, washed, and then released from solid support as described (sections 3.3.2-3.3.4). The reaction mixture obtained was then analyzed using LCMS (section 3.3.5). The Total Ion Chromatogram (abbr. TIC, Fig 11) shows a peak pattern that represents each possible 5' fragment (Seq. ID. 11-50, Fig 13A-E). Raw mass data, and the subsequent the corresponding deconvoluted masses were obtained for each of the discrete peaks observed in the TIC. Fig. 12 shows an example of a deconvoluted molecular weight of a fragment (mass peak value = 10237.1897 Da), in this case Fragment 31 (Fig. 13D, [SEQ. ID. 41]). Low abundance of the deconvoluted molecular weight of the Fragment 29 (Fig. 13C, SEQ. ID. 39) whose 2',3'-cyclic phosphate has been hydrolysed (mass peak value = 9603.77) can also be detected.

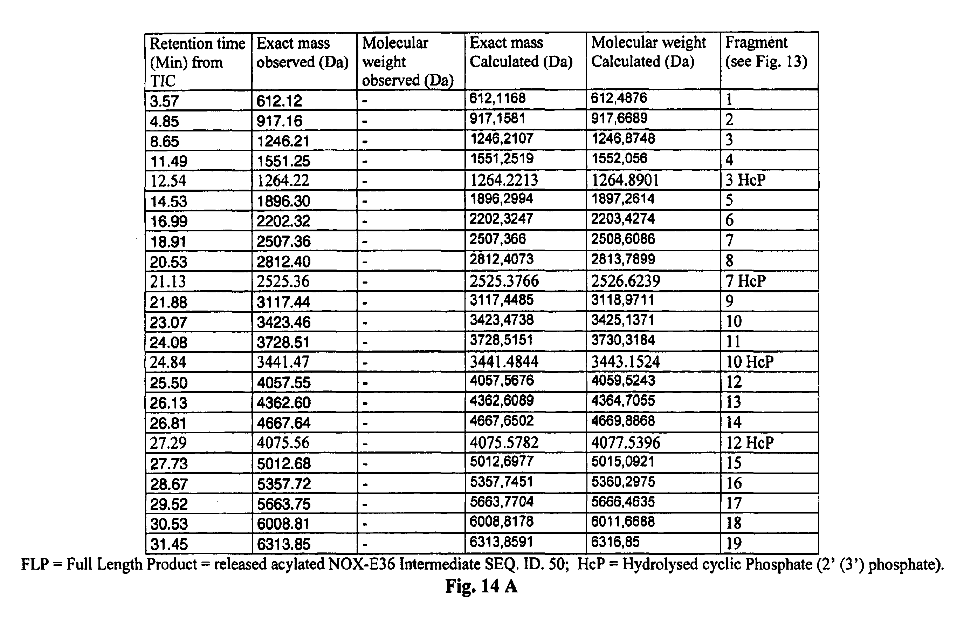

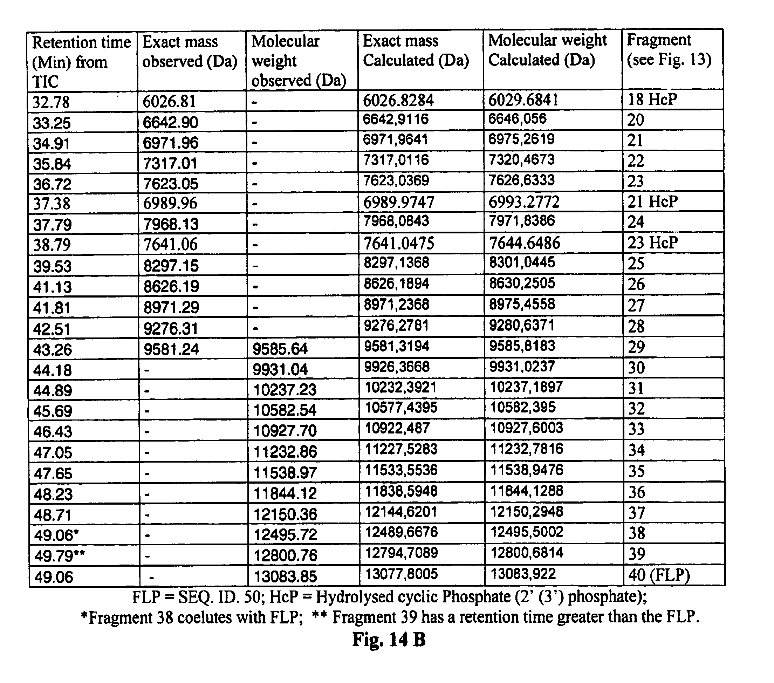

By comparing the masses obtained/observed to the calculated masses as depicted in Fig 13 A-E, the sequence of NOX-E36 was confirmed (Fig 14).

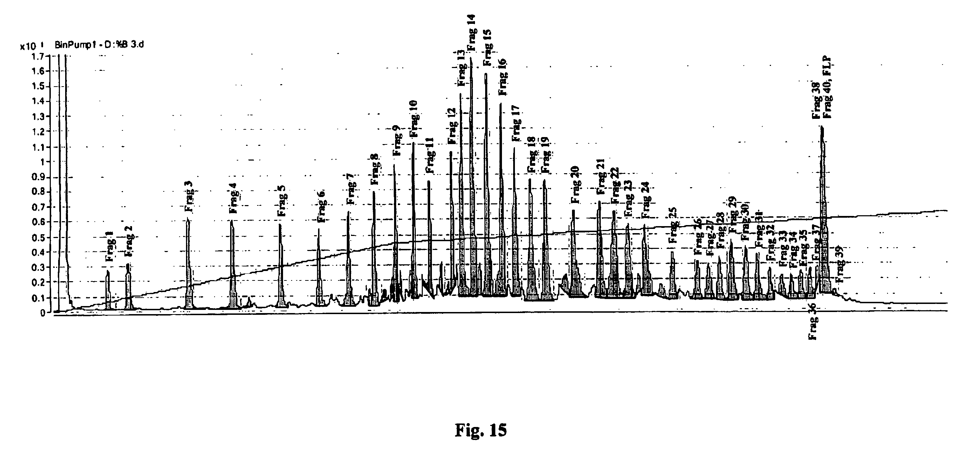

Figure 15 shows the power of the 2 dimensional (LC+MS) approach employed by assigning fragments to the peaks in the TIC of Fig. 11. The assignments are limited to the the released acylated NOX-E36 Intermediate and corresponding identified cyclic phosphate fragments. For each fragment, the corresponding 2',3'-cyclic phosphate predominates over the corresponding 2'(3') phosphate derivative thus greatly simplifying the chromatogram enabling easier identification and sequencing. As can be seen, there is a clear trend of increasing fragment size with increasing retention time. Such a trend facilitates the sequencing of unknown molecules by enabling a visual estimation of the size of fragment prior to obtaining the actual mass from TIC processing.

To test the power of the sequencing method as described in this example, the NOX-E36 mismatch control 01 (SEQ.ID. 3) which differs from the parent NOX-E36 sequence by 2 specific C/U switches, was subjected to the sequencing protocol as described for NOX-E36 Intermediate (SEQ. ID. 2) in the experimental section. Steps 3.3.1-3.3.5 were carried out exactly analogously as described, to furnish the corresponding Total Ion Chromatogram (Fig. 17). The mass spectra of the fragments were obtained and deconvoluted as before, however, this time the compound was treated as an unknown. By following the flow chart as described in Figure 16, the observed masses were used to unambiguously determine the sequence and reveal the two C/U switches in the sequence (highlighted, Fig. 18A-C) compared to the parent NOX-E36 sequence, as exemplified in the sequence determination table depicted in Figure 18A-C. An absolute error is included that notes the error in relation to the expected mass of the proposed fragment identity.

In summary, by applying the principle of immobilization as described above, the sequence of NOX-E36 Intermediate (SEQ. ID. 2) was readily confirmed and that of NOX-E36 mismatch control 01 (SEQ.ID. 3) was readily determined with errors well within acceptable limits for unambiguous determination.

### Example 4: Sequencing of a nucleic acid molecule without immobilization of the nucleic acid molecule or fragments thereof using selective wavelength absorbance labels

### 4.1 Principle

For the sequencing of a nucleic acid molecule without immobilization of the nucleic acid molecule the following steps are done: Labeling the nucleic acid with a label possessing a selective wavelength absorbance that nucleobases do not absorb at, limited random cleavage of the nucleic acid molecule by chemical cleavage to create a mix of fragments representing random strand scission (similar to that as depicted in Fig. 7) and intact full length material. The crude reaction mixture is analyzed by LCMS. At the selective wavelength absorbance of the label, there is no UV absorbance attributable to the nucleic acid molecule or fragments thereof. Therefore at this wavelength a ladder depicting all possible fragments representing cleavages 3' to every nucleotide of the nucleic acid molecule can be selectively observed by virtue of the selective wavelength absorbance of the label attached. By identifying the retention time of these 5' fragments, and deriving and deconvoluting the mass spectra at the retention times of these 5' fragments, it is possible to either confirm the sequence of the nucleic acid molecule or determine it without prior knowledge of the sequence. As there is no isolation of the labelled fragments the mass spectra is complicated by the presence of the non-labelled fragments: Whereas smaller fragments are well resolved on the column and the absolute separation of labelled fragments from non-labelled fragments (Fig 19, A-C) is probable, larger labelled fragments co-elute with non-labelled fragments, which also generate mass signals, and can potentially interfere with the identification of the desired 5' fragment (Fig 25-27). However, due to the lypophilicity of the label, labelled fragments of a certain mass value typically elute later than non-labelled fragments of a similar mass value (Fig 21, 22, 26, 27). As such, it is possible through reason to eliminate certain masses that co-elute and identify the intended labelled fragment.

### 4.2 Sequencing of Spiegelmer NOX-E36

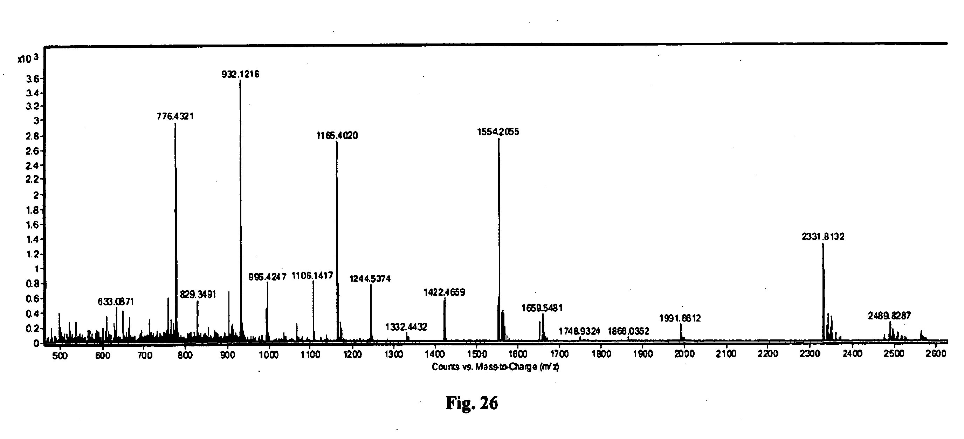

To demonstrate the feasibility of this method, a Fluorescein-5-isothiocyanate (FITC Isomer I) label was attached to NOX-E36 Intermediate (SEQ.ID. 2). The labelled NOX-E36 Intermediate was then subjected to base mediated limited random cleavage and the sample analysed by LCMS. The label has a selective wavelength absorbance at 495 nm (data as provided by the supplier). Therefore at 495 nm, only nucleic acid molecules containing an intact 5' end (5' fragments of the nucleic acid molecule and the full-length product [abbr. FLP]) will be observed. As can be seen from Fig. 19A, the chromatogram looks very similar to that observed from example 3 (Fig. 11). However, comparing the UV chromatogram extracted at 495 nm (Fig. 19 A) with the corresponding UV chromatogram extracted at 260 nm (Fig. 19, B) it can be clearly seen that there are many fragments that are not 5' fragments of the nucleic acid molecule. A comparison of the UV chromatogram extracted at 260 nm with the Total Ion Chromatogram (abbr. TIC) (Fig 19, C) shows that the two chromatograms B and C are very similar. This shows that all fragments either with or without label generate mass data. As determined through the chromatogram extracted at 495 nm, the first 5' fragment, (representing in this example FITC-NH-(CH₂)₆-OP(O)(OH)-Gcp, Fig. 23A, Fragment 1, SEQ. ID. 51) can be readily identified to be that eluting at 6.31 minutes (Fig. 19A and enlargement Fig. 20B). It can also be clearly seen by comparing Figs. 19A with 19B (and more easily with zoom-in Figs. 20A and 20B) that there are many non-labelled fragments that elute earlier than this peak, however, these represent fragments of between 8 and 14 nucleotides in length as estimated according to their observed masses. Therefore due to the lypophilicity afforded to the labelled fragments, any non-labelled fragments that co-elute with the labelled fragments can be eliminated due to the significant difference in mass (c.a. 2-6000 Da greater) to that expected for a particular fragment size (see Figs 25-27). An illustration of this is depicted in Figure 25, where Fig. 25A due to the extracted wavelength of 495 nm represents the FITC labelled nucleic acid fragments, Fig. 25B due to the extracted wavelength of 260 nm represents all nucleic acid fragments, as does the TIC (Fig. 25C). By obtaining a mass spectra for the ions within the range marked by the 2 dotted lines (Fig. 26), which would represent a typical ion window for obtaining the mass spectra of the labeled fragment, and deconvoluting this spectrum, it is possible to observe more than one peak. However, the peaks vary greatly in mass value, thus rendering only one, in this case the average molecular mass deconvoluted value of 4666.205 Da as possible for the labeled fragment. This ability to discount spurious masses, or in other words, determine the mass of the labelled fragment in the cases that they co-elute with non-labelled fragments, allows for both the sequence confirmation (Fig. 24) and the sequence determination of nucleic acid molecules with this method (Fig. 28A-C). Therefore, in a similar manner to Example 3 analogous sequence confirmation tables or analogous flow charts can be generated (see Fig 23 and Fig 24) for the sequence confirmation or determination without prior knowledge of the sequence (see Fig. 28A-C) as the principle of determining the sequence through the discrete mass differences between the fragments is analogous to that applied in example 3.

### 4.3 Protocol

### 4.3.1 Fluorescein-5-isothiocyanate labeling of Spiegelmer NOX-E36 Intermediate

848 ODs of crude Spiegelmer NOX-E36 Intermediate (SEQ.ID. 2) i.e. a derivative of Spiegelmer NOX-E36 (SEQ.ID. 1) with 5' amino linker, were placed in a reaction tube and dissolved in 250µl H₂O. To this were added 3mg Fluorescein-5-isothiocyanate (FITC Isomer I) (Sigma, Taufkirchen, Germany) pre-dissolved in 250µl *N,N*-dimethylformamide (abbr. DMF). The solution was vortexed and spun down, whereupon 6 mg Sodium Bicarbonate (Merck, Darmstadt, Germany) was added. The solution was incubated at room temperature for 6 h, whereupon the crude mixture was desalted by size-exclusion chromatography using a NAP25 column (Amersham Biosciences, Freiburg, Germany) and lyophilized. The lyophilisate was redissolved in water and purified via preparative RP-HPLC (Reverse-Phase High-Performance Liquid-Chromatography) (Wincott et al, 1995) using Source15RPC medium (Amersham, Freiburg, Germany) and was desalted using size exclusion chromatography using NAP25 columns.

### 4.3.2 Limited random Basic hydrolysis of the Fluorescein-5-isothiocyanate labelled Spiegelmer NOX-E36 Intermediate

To 13.5 µl (1 OD) FITC labelled Spiegelmer NOX-E36 Intermediate (SEQ.ID. 2) was added 1.5 µl 0.5 M K₂CO₃ at room temperature. The solution was vortexed and then incubated on a Eppendorf Thermomixer Comfort machine (Eppendorf, Hamburg, Germany) at 70°C at 1350 rpm for 5 mins whereupon it was frozen in liquid nitrogen and allowed to thaw out. Then 2.5 µl 1M AcOH was added (final pH ≈ 7) to quench the reaction and the solution vortexed, spun down and then analysed by LCMS.

### 4.3.3 LCMS analysis of the fragments

The LCMS analysis of the 5'-fragments of NOX-E36 Intermediate (SEQ.ID. 2) generated from the protocol above were analysed using a 6520 Accurate Mass Q-TOF LCMS system (Agilent Technologies, Waldbronn, Germany) with Rapid Resolution Pump and an Acuity BEH C18 Column (1.7 µm, 130 Å pore size, 2.1 x 30 mm, Waters, Eschborn, Germany). Gradient 0-20% B in 22 min, 20-30% B in 40 min. Buffer A: 10 mM Triethylamine, 100 mM Hexafluoroisopropanol, 10 µM EDTA (NH₄⁺ form), 1% Methanol in Water, Buffer B: 10 mM Triethylamine, 100 mM Hexafluoroisopropanol, 10 µM EDTA (NH₄⁺ form), 50% Methanol in Water. Column temperature 65 °C, Flow rate 1.2 ml/min. Mass spectra from the TIC were derived for each peak from the UV chromatogram extracted at 495 nm, and then deconvoluted.

### 4.4 Results

Having subjected NOX-E36 Intermediate (SEQ. ID. 2) to the sequencing protocol as laid out in steps 4.3.1-4.3.3, the following data are obtained as depicted in Fig 19A-C:
A) UV chromatogram extracted at 495 nm.
B) UV chromatogram extracted at 260 nm.
C) Total Ion Chromatogram (TIC).

The locations/retention time of the labeled (5'-) fragments are revealed at 495 nm (Fig 19, A) and the location of the first 5' fragment at 6.31 min is clearly visible. The corresponding UV chromatogram extracted at 260 nm (Fig 19, B) shows all fragments: 5'-(labeled), 3'- and internal fragments. It can be seen that there are many fragments that elute earlier than the first labelled fragment. These represent either 3' or internal fragments. The TIC (Fig 19, C) reveals that all fragments observed in the 260 nm UV chromatogram give a signal in the TIC, or in other words all nucleic acid fragments give mass data.

As determined through the chromatogram extracted at 495 nm, the first 5' fragment can be readily identified to be that eluting at 6.31 minutes (Fig. 19A, Fig. 20B). Confirmation was obtained by deconvoluting the mass spectrum of the corresponding peak in the TIC (Fig. 21A) and comparing its mass value either to the expected mass value of first fragment in the sequence confirmation table (Fragment 1, Fig. 23A) or by following the sequencing flow chart as depicted in Fig. 24 (Ladder Fragment 1, Fig. 28A). The next 5' fragment as observed at 7.13 minutes in the chromatogram extracted at 495 nm was treated in an iterative way to identify the second fragment (Fig 21B; Fragment 2 Fig. 23A and Ladder fragment 2 Fig 28A). This process was repeated for all other 5'- fragments whereby the sequence was determined using the flow chart as described (Fig. 24) and a sequence determination table constructed (Fig. 28A-C), which successfully determined the sequence. A sequence confirmation table could also have been used (as illustrated in Fig. 24) as the sequence was known. As can be seen from Figure 28A-C, the errors associated with the sequence determination were perfectly within range for unambiguous determination of the sequence. As has been discussed previously in this example, in the event of co-eluting non-labeled fragments, as depicted in Figure 25, such non-labeled fragments have mass values significantly higher than the expected range of mass value for the labelled fragments, irrespective of whether the sequence is known or not. As can be seen by comparing Figures 21, 22, 26, 27, this mass difference is typically in the 3-6kDa range. In summary, the sequencing of nucleic acid molecules using a selective modification, in this particular example, a modification endowed with a selective wavelength absorbance has been successfully demonstrated by sequencing NOX-E36 Intermediate (SEQ. ID. 2) via its FITC derivative (SEQ. ID. 100) in a de-novo fashion with experimental well within acceptable limits for unambiguous determination.

### Example 5: Sequencing of a nucleic acid molecule with immobilization of the nucleic acid molecule and selected fragments thereof. NOX A12

### 5.1 Principle

For the sequencing of a nucleic acid molecule with immobilization of the nucleic acid molecule and selected fragments thereof, the principle has been described previously in Example 3. This additional example uses a different nucleic acid sequence, that of NOX-A12 (SEQ. ID. 64). This additional example also has a modified washing step to ensure the complete removal of non-labeled fragments that may be co-immobilised with the labeled fragments (see section 5.3.3) due to the aggregation properties of the oligonucleotide. To effect this, a chaotropic solution, in this example, 8M Urea is used.

### 5.2 Sequencing of Spiegelmer NOX-A12

To test this method, the nucleic molecule Spiegelmer NOX-A12 Intermediate (SEQ.ID. 65) was used. NOX-A12 Intermediate (SEQ.ID. 65) is a 5'-amino-modified derivative of Spiegelmer NOX-A12 (SEQ.ID. 64). As shown in Fig. 8 for NOX-E36, after modifying the 5'-amino moiety with a biotin affinity tag, the biotinylated Spiegelmer is chemically cleaved in a random fashion using a basic solution. The cleavage was carefully controlled so as not to drive the cleavage to completion. From the random fragmentation that occurs which produces 5'- fragments, 3'- fragments and random internal fragments, all biotinylated 5' fragments of NOX-A12 Intermediate and remaining biotinylated NOX-A12 Intermediate (i.e. full-length product [abbr. FLP]) are selectively pulled out from the mix via the affinity tag (in this case biotin) using tag-specific solid support for immobilisation (in this case Neutravidin beads). The unbound fragments, i.e. 3'- fragments and random internal fragments that do not possess the affinity tag, can be washed away. With some sequences, particularly those that tend to self aggregate, it is possible that non-labeled fragments are co-immobilised as they bind to the immobilised labelled fragments. To ensure their removal, the bound fragments are washed with a chaotropic agent. The bound 5' fragments of Spiegelmer NOX-A12 and the FLP are then liberated from the beads by reductively cleaving the disulfide bond within the linker connecting the biotin moiety and NOX-A12 Intermediate (SEQ.ID. 65), to furnish fragments (101-145). These fragments correspond to strand scission between every ribonucleoside position (see Fig. 8 for NOX-E36 example). The strand scission results first in the formation of 2',3'-cyclic phosphate containing 5' fragments whereby the cyclic phosphate slowly hydrolyses to the 2'(3') phosphate. The liberated fragments are then analysed by LC-(ESI)MS, and the Total Ion Chromatogram (abbr. TIC) is analysed. What is found are discrete peaks which correspond to all 5' fragments generated and the intact released acylated NOX-A12 Intermediate (seq. ID. 101-145) (see Fig. 31 for sample chromatogram). The mass(es) contained in the discrete peaks are then obtained through deconvolution of the derived mass spectra pertaining to each discrete peak (for example see Fig 32). Deconvolution is a common technique well known to those skilled in the art whereby an algorithm is applied to a mass spectrum to identify multiply charged ions of a single species and reconstitute them into the mass of this species. This technique is highly valuable in combination with ESI and other ionisation techniques which observe large molecules as a distribution of multiply charged ions. Depending on the algorithm applied either the isotopic resolved masses (to obtain the exact mass) or the molecular weight is obtained. Typically for oligonucleotides a mass spectrometer calibrated at 5 ppm is able to produce resolved isotope spectra up to approximately 6-10 kDa. Above this mass, typically an algorithm, such as the Maxent algorithm, is used that deconvolutes to the molecular weight of the species.

In general, the masses seen are those of the 2',3'-cyclic phosphates, although in some cases, the low abundance of fragments containing the hydrolysed 2' (3') phosphate can also be detected, which serve to further confirm the identity of the fragments generated. Typically these hydrolyzed fragments elute later than the parent 2',3'-cyclic phosphate.

The masses of the fragments generated can in the first instance be compared to the calculated masses of the 5' fragments of NOX-A12 Intermediate (Fig. 34A+B) to confirm the sequence (Analogous to the NOX-E36 example Fig. 13A-E). Alternatively, the sequence can be derived without prior knowledge of the sequence due to the differences between the fragments generated. In this scenario, the first fragment of the nucleic acid molecule can be easily predicted and the incremental differences of the subsequent fragments can be used to determine the sequence of the nucleic acid molecule, as demonstrated in the 'Flow chart for Sequence Determination/Validation' (Fig. 16). This flow chart describes a step by step process whereby the smallest fragment (denoted Fragment 1) is first identified. The first fragment represents the first 5' nucleotide with both a 5'-affixed acylated aminohexyl linker and 2',3'-cyclic phosphate such as depicted in Fig. 34A (SEQ. ID. 101). Consequently it is straightforward to calculate all possible RNA permutations (A, C, G or U) for the first fragment (Fig. 16). The identification of this first fragment is facilitated by the knowledge that Fragment 1 will be the earliest eluting 5' fragment using Ion-Pair Reversed Phase HPLC (abbr. IP RP-HPLC) as is known by those familiar with the art of IP RP-HPLC. Once Fragment 1 has been identified, the calculated exact mass and molecular weight are used to identify the next fragment, Fragment 2. The identity and therefore sequence of the next fragment, Fragment 2, is derived from the mass difference between Fragment 2 and the calculated exact mass or molecular weight of Fragment 1. The mass difference is unique for each nucleoside A, C, G, U (Fig 16). Once Fragment 2 has been identified, the calculated exact mass and molecular weight are used to identify the next fragment, Fragment 3. In an identical procedure to that used to identify Fragment 2, the identity of Fragment 3, is derived from the mass difference between Fragment 3 and the calculated exact mass or molecular weight of Fragment 2. This iterative process is used to identify all the 5' fragments. The need to use the calculated mass values for the previous fragment arises from the potential accumulative errors that can occur if only the observed values are used. For example, a 0.3 Da error would still enable the unambiguous identification of a fragment, however, without resetting this error by using the calculated values of the identified fragment, further 0.3 Da errors could accumulate so that unambiguous identification may not be possible due to the small mass difference of one Da between C and U nucleosides.

For the identification of the last nucleoside, the same process is used whereby the mass difference between the intact released acylated NOX-A12 Intermediate (SEQ. ID. 145) and the calculated mass of the final cyclic phosphate containing fragment is used to confirm the identity of the last nucleotide. As the released acylated NOX-A12 Intermediate possesses no 2',3' cyclic phosphate, the mass difference is not the same as for those fragments calculated previously. The mass difference corresponds to the mass of the last nucleoside (Fig. 16).

NOX-A12, being a longer Spiegelmer than NOX-E36 was used as a further test to evaluate this sequencing method. NOX-A12 was processed using the protocol described in this example. The sequence was identified using the 'Flow chart for Sequence Determination/Validation' (Fig. 16), and compiling the results of this in a sequence determination table (Fig. 33A-C).

### 5.3 Protocol

### 5.3.1 Biotinylation of Spiegel mer NOX-A12 Intermediate

10mg (250 ODs) of crude Spiegelmer NOX-A12 Intermediate (SEQ.ID. 65), i.e. a derivative of Spiegelmer NOX-A12 (SEQ.ID. 64) with 5' amino linker, were placed in a reaction tube and dissolved in 260µl Theorell and Stenhagen's Universal buffer pH8.5 (33 mM Sodium Citrate, 33 nM Sodium Phosphate, 57 mM Sodium Borate, pH 8,5). To this were added 200µl N,N-dimethylformamide (abbr. DMF). The solution was vortexed and spun down, whereupon 2,2 mg Biotin disulfide N-hydroxy-succinimide ester (Sigma B4531, Taufkirchen, Germany) pre-dissolved in 50µl DMF was added. The solution was incubated at room temperature for 60 minutes, whereupon an aliquot was taken and analysed by Anion-Exchange HPLC (Dionex DNA-Pac 200 column, Buffer A: 100mM Tris; 10%ACN in H₂O. Buffer B: 1M NaCl, 100mM Tris; 25 mM NaClO₄; 10%ACN in H₂O. Gradient 10-30 %B in 6 min then 30-70 %B in 35 min, column temperature 80°C) which determined that the reaction was complete. The crude reaction mixture was desalted using a NAP25 column (Amersham Biosciences, Freibug, Germany) and lyophilised.

### 5.3.2Basic hydrolysis of the biotin labeled Spiegelmer NOX-E36 Intermediate

To 20 µl biotinylated Spiegelmer NOX-A12 Intermediate (SEQ.ID. 65) (at 0.5 OD/µl) was added 30 µl sterilised water and 2.5 µl 0.5 M K₂CO₃ at room temperature. The solution was vortexed and then incubated on a Eppendorf Thermomixer Comfort machine (Eppendorf, Hamburg, Germany) at 70°C at 1350 rpm for 20 mins. Whereupon it was frozen in liquid nitrogen and allowed to thaw out. Then 4 µl 1M AcOH was added (approx. pH 7) to quench the reaction and the solution vortexed and spun down.

### 5.3.3 Binding of biotinylated fragments to Neutravidin beads

Neutravidin Agarose beads were treated as follows: 150 µl of Neutravidin bead slurry (Pierce, Milwaukee, MI, USA) was put in 500 µl reaction tube. The beads were spun down and the supernatant carefully removed. Whereupon 300 µl 1M Tris HCl pH 8.0 (Ambion; Huntindon, UK) was added. The slurry vortexed, spun down and the supernatant carefully removed. The beads were then washed 2 x 300 µl in the same manner with sterile H₂O. The quenched hydrolysis mix as prepared above was then added to the beads and the resulting slurry mixed vigorously (1350 rpm) at 10°C for 2h. The beads were then spun down and the supernatant removed. 1 x 300 µl 8M Urea was added and the mixture vortexed and spun down. The supernatant was carefully removed and the beads washed a further 4 times with sterilized water.

### 5.3.4 Cleavage of the biotinylaled fragments from the Neutravidin beads

The disulfide linker of the biotin labeled fragments of NOX-A12 Intermediate (SEQ.ID. 65) was cleaved using a 0.05 M Na phosphate buffer (pH 8.5), 100 µl with 5 µl 1M DTT solution. This was vigorously mixed at 25 deg C for 2h on a Eppendorf Thermomixer Comfort machine. The slurry was filtered using a spin microfuge tube (Ultrafree-MC GV, 0,22 µm, Millipore, Schwalbach, Germany), and the beads washed with a further 50 µl sterile water. A UV measurement was taken to determine the Optical Density Units at 260 nm, and of that 0.25 ODs was analysed by LCMS.

### 5.3.5 LCMS analysis of the fragments

The LCMS analysis of the 5'-fragments of NOX-A12 Intermediate (SEQ.ID. 65) generated from the protocol above were analysed using a 6520 Accurate Mass Q-TOF LCMS system (Agilent Technologies, Waldbronn, Germany) with Rapid Resolution Pump and an Acuity BEH C18 Column (1.7 µm, 130 Å pore size, 2.1 x 30 mm, Waters, Eschborn, Germany). Gradient 0-20% B in 22 min, 20-30% B in 40 min. Buffer A: 10 mM Triethylamine, 100 mM Hexafluoroisopropanol, 10 µM EDTA (NH₄⁺ form), 1% Methanol in Water, Buffer B: 10 mM Triethylamine, 100 mM Hexafluoroisopropanol, 10 µM EDTA (NH₄⁺ form), 50% Methanol in Water. Column temperature 65 °C, Flow rate 0.2 ml/min: Mass spectra from the TIC were derived for each peak and then deconvoluted.

### 5.4 Results

Crude NOX-A12 Intermediate (SEQ.ID. 65) was efficiently labeled with the cleavable biotin moiety as described in the experimental section, as determined utilizing anion-exchange chromatography by the appearance of a later eluting peak in the crude reaction mixture (Fig. 30, 29.82 mins) compared to the starting material (Fig. 29, 28.21 mins). The presence of failure sequences from the solid phase synthesis of NOX-A12 Intermediate [SEQ.ID. 65], and other impurties does not affect the ability to carry out the labeling or subsequent steps 5.3.2-5.3.5 and to sequence the nucleic acid molecule. The crude labeling mixture was not purified, save for a rudimentary desalting step using a size exclusion purification column (NAP25, see experimental). This crude material was then fragmented, the labeled fragments immobilized, washed, and then released from solid support as described (sections 5.3.2-5.3.4). The reaction mixture obtained was then analyzed using LCMS (section 5.3.5) . The resulting Total Ion Chromatogram (abbr. TIC, Fig 31) shows a peak pattern that represents each possible 5' fragment (Seq. ID. 101-145, Fig 34). Raw mass data, and the subsequent corresponding deconvoluted masses were obtained for each of the discrete peaks observed in the TIC. Fig. 32 shows an example of a deconvoluted molecular weight of a fragment (mass peak value = 10910.65 Da), in this case Fragment 33 (Fig.34A, [SEQ. ID. 133]). By following the flow chart as described in Figure 16, the observed masses were used to unambiguously determine the sequence of NOX-A12 (Fig. 33A-C). Figures 34A+B show the corresponding sequence confirmation table, and Figure 35 displays an annotated TIC whereby the peaks are assigned the corresponding fragment numbers in the sequence determination (Fig. 33A-C) and sequence confirmation (Fig. 34A+B) tables.

In summary, by applying the principle of immobilization as described above, the sequence of NOX-A12 Intermediate (SEQ. ID. 65) was readily readily determined with errors well within acceptable limits for unambiguous determination.

### References

The complete bibliographic data of the documents recited herein are, if not indicated to the contrary, as follows.
Alazard D, Filipowsky M, Raeside J, Clarke M, Majlessi M, Russell J, Weisburg W (2002) Sequencing of production-scale synthetic oligonucleotides by enriching for coupling failures using matrix-assisted laser desorption/ionization time-of-flight mass spectrometry. Anal Biochem 301(1): 57-64
Anderson S (1981) Shotgun DNA sequencing using cloned DNase I-generated fragments. Nucleic Acids Res 9(13): 3015-3027
Azarani A, Hecker K.H (2001) RNA analysis by ion-pair reversed-phase high performance liquid chromatography, Nucleic Acids Res. 29(2): e7.
Baker TR, Keough T, Dobson RL, Riley TA, Hasselfield JA, Hesselberth PE (1993) Antisense DNA oligonucleotides. I: The use of ionspray tandem mass spectrometry for the sequence verification of methylphosphonate oligodeoxyribonucleotides. Rapid Commun Mass Spectrom 7(3): 190-194
Beigelman L, McSwiggen JA, Draper KG, Gonzalez C, Jensen K, Karpeisky AM, Modak AS, Matulic-Adamic J, DiRenzo AB, Haeberli P, et al. (1995) Chemical modification of hammerhead ribozymes. Catalytic activity and nuclease resistance. J Biol Chem 270(43): 25702-25708
Bentzley CM, Johnston MV, Larsen BS (1998) Base specificity of oligonucleotide digestion by calf spleen phosphodiesterase with matrix-assisted laser desorption ionization analysis. Anal Biochem 258(1): 31-37
Bentzley CM, Johnston MV, Larsen BS, Gutteridge S (1996) Oligonucleotide sequence and composition determined by matrix-assisted laser desorption/ionization. Anal Chem 68(13): 2141-2146
Biemann K (1990) Sequencing of peptides by tandem mass spectrometry and high-energy collision-induced dissociation. Methods Enzymol 193: 455-479
Bock LC, Griffin LC, Latham JA, Vermaas EH, Toole JJ (1992) Selection of single-stranded DNA molecules that bind and inhibit human thrombin. Nature 355(6360): 564-566
Boschenok J, Sheil MM (1996) Electrospray tandem mass spectrometry of nucleotides. Rapid Commun Mass Spectrom 10(1): 144-149
Branch AD, Benefeld BJ, Robertson HD (1989) RNA fingerprinting. Methods Enzynology 180: 130-154
Bronstein I, Fortin J, Stanley PE, Stewart GS, Kricka LJ (1994) Chemiluminescent and bioluminescent reporter gene assays. Anal Biochem 219(2): 169-181
Browne KA (2002) Metal Ion-Catalyzed Nucleic Acid Alkylation and Fragmentation J Am Chem Soc 127(27): 7950-7962
Burgin AB, Jr., Gonzalez C, Matulic-Adamic J, Karpeisky AM, Usman N, McSwiggen JA, Beigelman L (1996) Chemically modified hammerhead ribozymes with improved catalytic rates. Biochemistry 35(45): 14090-14097
Cannistraro VJ, Kennell D (1989) Purification and characterization of ribonuclease M and mRNA degradation in Escherichia coli. Eur J Biochem 181(2): 363-370
Carothers JM, Szostak JW (2006) In vitro Selection of Functional Oligonucleotides and the Origins of Biochemical Activity. In The aptamer Handbook, Klussmann S (ed), 1, pp 3-28. Weinheim: WILEY-VCH Verlag GmbH & Co. KgaA
Cerny RL, Tomer KB, Gross ML, Grotjahn L Fast Atom Bombardment Combined with Tandem Mass Spectrometry for Determining Structures of Small Oligonucleotides (1987) Analytical Biochemistry 165, pp 175-182
Cload ST, McCauley TG, Keefe AD, Healy JM, Wilson C (2006) Properties of Therapeutic Aptamers. In The Aptamer Handbook, Klussmann S (ed), 17, pp 363-416. Weinheim: WILEY-VCH Verlag GmbH & Co. KGaA
Couzin J (2004) Molecular biology. RNAi shows cracks in its armor. Science 306(5699): 1124-1125
Crooke ST (2004) Progress in antisense technology. Annu Rev Med 55: 61-95
Damha MJ, Ogilvie KK (1993) Oligoribonucleotide synthesis. The silyl-phosphoramidite method. Methods Mol Biol 20: 81-114
Dolinnaya NG, Sokolova NI, Ashirbekova DT, Shabarova ZA (1991) The use of BrCN for assembling modified DNA duplexes and DNA-RNA hybrids; comparison with water-soluble carbodiimide. Nucleic Acids Res 19(11): 3067-3072
Donis-Keller H, Maxam AM, Gilbert W (1977) Mapping adenines, guanines, and pyrimidines in RNA. Nucleic Acids Res 4(8): 2527-2538
Durand M, Chevrie K, Chassignol M, Thuong NT, Maurizot JC (1990) Circular dichroism studies of an oligodeoxyribonucleotide containing a hairpin loop made of a hexaethylene glycol chain: conformation and stability. Nucleic Acids Res 18(21): 6353-6359
Ehresmann C, Baudin F, Mougel M, Romby P, Ebel JP, Ehresmann B (1987) Probing the structure of RNAs in solution. Nucleic Acids Res 15(22): 9109-9128
Ellington AD, Szostak JW (1990) In vitro selection of RNA molecules that bind specific ligands. Nature 346(6287): 818-822
Elov AA, Volkov EM, Reintamm TG, Oretskaia TS, Shabarova ZA (1989) [RNA synthesis using T7 phage RNA polymerase: transcription of synthetic DNA templates in solution and on polymer support]. Bioorg Khim 15(2): 159-165
Eulberg D, Jarosch F, Vonhoff S, Klussmann S (2006) Spiegelmers for Therapeutic Applications - Use of Chiral Principles in Evolutionary Selection Techniques. In The Aptamer Handbook, Klussmann S (ed), 18, pp 417-442. Weinheim: WILEY-VCH Verlag GmbH & Co. KGaA
Farand J, Beverly M (2008) Sequence Confirmation of Modified Oligonucleotides Using Chemical Degradation, Electrospray Ionization, Time-of-Flight, and Tandem Mass Spectrometry. Anal Chem
Faulstich K, Worner K, Brill H, Engels JW (1997) A sequencing method for RNA oligonucleotides based on mass spectrometry. Anal Chem 69(21): 4349-4353
Fenn JB, Mann M, Meng CK, Wong SF, Whitehouse CM (1989) Electrospray ionization for mass spectrometry of large biomolecules. Science 246(4926): 64-71
Freier SM, Altmann KH (1997) The ups and downs of nucleic acid duplex stability: structure-stability studies on chemically-modified DNA:RNA duplexes. Nucleic Acids Res 25(22): 4429-4443
Fu DJ, Tang K, Braun A, Reuter D, Darnhofer-Demar B, Little DP, O'Donnell MJ, Cantor CR, Koster H (1998) Sequencing exons 5 to 8 of the p53 gene by MALDI-TOF mass spectrometry. Nat Biotechnol 16(4):.381-384
Glover RP, Sweetman GM, Farmer PB, Roberts GC (1995) Sequencing of oligonucleotides using high performance liquid chromatography and electrospray mass spectrometry. Rapid Commun Mass Spectrom 9(10): 897-901
Gupta RC, Randerath E, Randerath K (1976) A double-labeling procedure for sequence analysis of picomole amounts of nonradioactive RNA fragments. Nucleic Acids Res 3(11): 2895-2914
Gupta RC, Randerath K (1977) Use of specific endonuclease cleavage in RNA sequencing. Nucleic Acids Res 4(6): 1957-1978
Gut IG, Beck S (1995) A procedure for selective DNA alkylation and detection by mass spectrometry. Nucleic Acids Res 23(8): 1367-1373
Hannon GJ (2002) RNA interference. Nature 418(6894): 244-251
Harksen A, Ueland PM, Refsum H, Meyer K (1999) Four common mutations of the cystathionine beta-synthase gene detected by multiplex PCR and matrix-assisted laser desorption/ionization time-of-flight mass spectrometry. Clin Chem 45(8 Pt 1): 1157-1161 1
Hermanson GT (2008) Bioconjugate Techniques, 2nd Edition, San Diego: Academic Press.
Juhasz P, Roskey MT, Smirnov IP, Haff LA, Vestal ML, Martin SA (1996) Applications of delayed extraction matrix-assisted laser desorption ionization time-of-flight mass spectrometry to oligonucleotide analysis. Anal Chem 68(6): 941-946
Juliano R, Alam MR, Dixit V, Kang H (2008) Mechanisms and strategies for effective delivery of antisense and siRNA oligonucleotides. Nucleic Acids Res 36(12): 4158-4171
Karas M, Hillenkamp F (1988) Laser desorption ionization of proteins with molecular masses exceeding 10,000 daltons. Anal Chem 60(20): 2299-2301
Kawase Y, Umeda Y, Kato I (1991) Analysis of nucleic acids by ion-spray mass spectrometry. Nucleic Acids Symp Ser(25): 127-128
Kinoshita Y, Nishigaki K, Husimi Y (1997) Fluorescence-, isotope- or biotin-labeling of the 5 '-end of single-stranded DNA/RNA using T4 RNA ligase. Nucleic Acids Res 25(18): 3747-3748
Kirpekar F, Nordhoff E, Kristiansen K, Roepstorff P, Lezius A, Hahner S, Karas M, Hillenkamp F (1994) Matrix assisted laser desorption/ionization mass spectrometry of enzymatically synthesized RNA up to 150 kDa. Nucleic Acids Res 22(19): 3866-3870
Kirpekar F, Nordhoff E, Larsen LK, Kristiansen K, Roepstorff P, Hillenkamp F (1998) DNA sequence analysis by MALDI mass spectrometry. Nucleic Acids Res 26(11): 2554-2559
Klussmann S, Nolte A, Bald R, Erdmann VA, Furste JP (1996) Mirror-image RNA that binds D-adenosine. Nat Biotechnol 14(9): 1112-1115
Kolb HC, Finn MG, Sharpless KB (2001). Click Chemistry: Diverse Chemical Function from a Few Good Reactions". Angewandte Chemie International Edition 40 (11): 2004-2021
Komiyama M, Yoshinari K (1997) Kinetic Analysis of Diamine-Catalyzed RNA Hydrolysis. J Org Chem 62(7): 2155-2160
Koster H, Tang K, Fu DJ, Braun A, van den Boom D, Smith CL, Cotter RJ, Cantor CR (1996) A strategy for rapid and efficient DNA sequencing by mass spectrometry. Nat Biotechnol 14(9): 1123-1128
Lewis FD, Liu X, Wu Y, Miller SE, Wasielewski MR, Letsinger RL, Sanishvili R, Joachimiak A, Tereshko V, Egli M (1999) Structure and Photoinduced Electron Transfer in Exceptionally Stable Synthetic DNA Hairpins with Stilbenediether Linkers. JAmChemSoc 121(41): 9905-9906
Limbach PA (1996) Indirect Mass Spectrometric Methods for Characterizing and Sequencing Oligonucleotides. Mass Spectrometry Reviews 15: 297-336
Limbach PA, Crain PF, McCloskey JA (1995) Characterization of oligonucleotides and nucleic acids by mass spectrometry. Curr Opin Biotechnol 6(1): 96-102
Little DP, Thannhauser TW, McLafferty FW (1995) Verification of 50- to 100-mer DNA and RNA sequences with high-resolution mass spectrometry. Proc Natl Acad Sci U S A 92(6): 2318-2322
Lockard RE, Alzner-Deweerd B, Heckman JE, MacGee J, Tabor MW, RajBhandary UL (1978) Sequence analysis of 5'[32P] labeled mRNA and tRNA using polyacrylamide gel electrophoresis. Nucleic Acids Res 5(1): 37-56
Ma MY, Reid LS, Climie SC, Lin WC, Kuperman R, Sumner-Smith M, Barnett RW (1993) Design and synthesis of RNA miniduplexes via a synthetic linker approach. Biochemistry 32(7): 1751-1758
Mann MJ, Dzau VJ (2000) Therapeutic applications of transcription factor decoy oligonucleotides. J Clin Invest 106(9): 1071-1075
Marzilli LA, Barry JP, Sells T, Law SJ, Vouros P, Harsch A (1999) Oligonucleotide sequencing using guanine-specific methylation and electrospray ionization ion trap mass spectrometry. J Mass Spectrom 34(4): 276-280
Maxam AM, Gilbert W (1977) A new method for sequencing DNA. Proc Natl Acad Sci U S A 74(2): 560-564
Meister G, Tuschl T (2004) Mechanisms of gene silencing by double-stranded RNA. Nature 431(7006): 343-349
Monforte JA, Becker CH (1997) High-throughput DNA analysis by time-of-flight mass spectrometry. Nat Med 3(3): 360-362
Morishita R, Gibbons GH, Horiuchi M, Ellison KE, Nakama M, Zhang L, Kaneda Y, Ogihara T, Dzau VJ (1995) A gene therapy strategy using a transcription factor decoy of the E2F binding site inhibits smooth muscle proliferation in vivo. Proc Natl Acad Sci U S A 92(13): 5855-5859
Mouradian S, Rank DR, Smith LM (1996) Analyzing sequencing reactions from bacteriophage M13 by matrix-assisted laser desorption/ionization mass spectrometry. Rapid Commun Mass Spectrom 10(12): 1475-1478
Ni J, Pomerantz C, Rozenski J, Zhang Y, McCloskey JA (1996) Interpretation of oligonucleotide mass spectra for determination of sequence using electrospray ionization and tandem mass spectrometry. Anal Chem 68(13): 1989-1999
W.M.A. Niessen WMA The encyclopedia of Mass Spectrometry (2002), Volume 8, Elsevier, Amsterdam
Nimjee SM, Rusconi CP, Sullenger BA (2006) Aptamers to Proteins. In The Aptamer Handbook, Klussmann S (ed), 1, pp 131-166. Weinheim: WILEY-VCH Verlag GmbH & Co. KGaA
Nolte A, Klussmann S, Bald R, Erdmann VA, Furste JP (1996) Mirror-design of L-oligonucleotide ligands binding to L-arginine. Nat Biotechnol 14(9): 1116-1119
Nordhoff E, Cramer R, Karas M, Hillenkamp F, Kirpekar F, Kristiansen K, Roepstorff P (1993) Ion stability of nucleic acids in infrared matrix-assisted laser desorption/ionization mass spectrometry. Nucleic Acids Res 21(15): 3347-3357
Nordhoff E, Kirpekar F, Roepstorff P (1996) Mass spectrometry of nucleic acids. Mass Spectrometry Reviews 15: 67-138
Owens DR, Bothner B, Phung Q, Harris K, Siuzdak G (1998) Aspects of oligonucleotide and peptide sequencing with MALDI and electrospray mass spectrometry. Bioorg Med Chem 6(9): 1547-1554
Peattie DA (1979) Direct chemical method for sequencing RNA. Proc Nall Acad Sci U S A 76(4): 1760-1764
Pieken WA, Olsen DB, Benseler F, Aurup H, Eckstein F (1991) Kinetic characterization of ribonuclease-resistant 2'-modified hammerhead ribozymes. Science 253(5017): 314-317
Pieles U, Zurcher W, Schar M, Moser HE (1993) Matrix-assisted laser desorption ionization time-of-flight mass spectrometry: a powerful tool for the mass and sequence analysis of natural and modified oligonucleotides. Nucleic Acids Res 21(14): 3191-3196
Pils W, Micura R (2000) Flexible non-nucleotide linkers as loop replacements in short double helical RNAs. Nucleic Acids Res 28(9): 1859-1863
Proudnikov D, Mirzabekov A (1996) Chemical methods of DNA and RNA fluorescent labeling. Nucleic Acids Res 24(22): 4535-4542
Realini T, Ng EWM, Adamis AP (2006) Applications in the Clinic: The Anti-VEGF Aptamer. In The Apatmer Handbook, Klussmann S (ed), pp 443-460. Weinheim: WILEY-VCH Verlag GmbH & Co. KGaA
Roskey MT, Juhasz P, Smirnov IP, Takach EJ, Martin SA, Haff LA (1996) DNA sequencing by delayed extraction-matrix-assisted laser desorption/ionization time of flight mass spectrometry. Proc Natl Acad Sci U S A 93(10): 4724-4729
Sarnbrook J, Russell, D. W., (2001) Molecular Cloning A Laboratory Manual, the third edition,, Cold Spring Harbor, New York, : Cold Spring Harbor Laboratory Press.
Sanger F, Nicklen S, Coulson AR (1977) DNA sequencing with chain-terminating inhibitors. Proc Natl Acad Sci U S A 74(12): 5463-5467
Santoro SW, Joyce GF (1997) A general purpose RNA-cleaving DNA enzyme. Proc Natl Acad Sci U S A 94(9): 4262-4266
Sargent TD (1988) Isolation of differentially expressed genes Methods Enzymol 152: 423-432
Scherer LJ, Rossi JJ (2003) Approaches for the sequence-specific knockdown of mRNA. Nat Biotechnol 21(12): 1457-1465
Schlosser K, Gu J, Lam JC, Li Y (2008a) In vitro selection of small RNA-cleaving deoxyribozymes that cleave pyrimidine-pyrimidine junctions. Nucleic Acids Res 36(14): 4768-4777
Schlosser K, Gu J, Sule L, Li Y (2008b) Sequence-function relationships provide new insight into the cleavage site selectivity of the 8-17 RNA-cleaving deoxyribozyme. Nucleic Acids Res 36(5): 1472-1481
Schlosser K, McManus SA, Y. L (2006) Deoxyribozymes: Catalytically Active DNA Molecules. In The Aptamer Handbook, Klussmann S (ed), pp 228-264. Weinheim: WILEY-VCH Verlag GmbH & Co. KGaA
Schuette JM, Pieles U, Maleknia SD, Srivatsa GS, Cole DL, Moser HE, Afeyan NB (1995) Sequence analysis of phosphorothioate oligonucleotides via matrix-assisted laser desorption ionization time-of-flight mass spectrometry. J Pharm Biomed Anal 13(10): 1195-1203
Schürch S, Bernal-Méndez E, Leumann C.J, (2002) Electrospray Tandem Mass Spectrometry of Mixed-Sequence RNA/DNA Oligonucleotides, Journal of the American Society for Mass Spectrometry, 13 (8): pp936-945
Shaler TA, Tan Y, Wickham JN, Wu KJ, Becker CH (1995) Analysis of enzymatic DNA sequencing reactions by matrix-assisted laser desorption/ionization time-of-flight mass spectrometry. Rapid Commun Mass Spectrom 9(10): 942-947
Shapiro R, Danzig M (1972) Acidic hydrolysis of deoxycytidine and deoxyuridine derivatives. The general mechanism of deoxyribonucleoside hydrolysis. Biochemistry 11(1): 23-29
Smirnov IP, Roskey MT, Juhasz P, Takach EJ, Martin SA, Haff LA (1996) Sequencing oligonucleotides by exonuclease digestion and delayed extraction matrix-assisted laser desorption ionization time-of-flight mass spectrometry. Anal Biochem 238(1): 19-25
Smith MB and March J (2007) March's Advanced Organic Chemistry: Reactions, Mechanisms and Structure 6th Edition. Wiley Interscience - John Wiley & Sons, Inc. Hoboken, New Jersey, USA.
Stanley J, Vassilenko S (1978) A different approach to RNA sequencing. Nature 274(5666): 87-89
Stemmler EA, Hettich RL, Hurst GB, Buchanan MV (1993) Matrix-assisted laser desorption/ionization Fourier-transform mass spectrometry of oligodeoxyribonucleotides. Rapid Commun Mass Spectrom 7(9): 828-836
Talbo G, Mann M (1996) Aspects of the sequencing of carbohydrates and oligonucleotides by matrix-assisted laser desorption/ionization post-source decay. Rapid Commun Mass Spectrom 10(1): 100-103
Tanaka Y, Dyer TA, Brownlee GG (1980) An improved direct RNA sequence method; its application to Vicia faba 5.8S ribosomal RNA. Nucleic Acids Res 8(6): 1259-1272
Taranenko NI, Allman SL, Golovlev VV, Taranenko NV, Isola NR, Chen CH (1998) Sequencing DNA using mass spectrometry for ladder detection. Nucleic Acids Res 26(10): 2488-2490
Taranenko NI, Chung CN, Zhu YF, Allman SL, Golovlev VV, Isola NR, Martin SA, Haff LA, Chen CH (1997) Matrix-assisted laser desorption/ionization for sequencing single-stranded and double-stranded DNA. Rapid Commun Mass Spectrom 11(4): 386-392
Thomson JB, Tuschl T, Eckstein F (1993) Activity of hammerhead ribozymes containing non-nucleotidic linkers. Nucleic Acids Res 21(24): 5600-5603
Tolson DA, Nicholson NH (1998) Sequencing RNA by a combination of exonuclease digestion and uridine specific chemical cleavage using MALDI-TOF. Nucleic Acids Res 26(2): 446-451
Tomita S, Tomita N, Yamada T, Zhang L, Kaneda Y, Morishita R, Ogihara T, Dzau VJ, Horiuchi M (1999) Transcription factor decoy to study the molecular mechanism of negative regulation of renin gene expression in the liver in vivo. Circ Res 84(9): 1059-1066
Tuerk C, Gold L (1990) Systematic evolution of ligands by exponential enrichment: RNA ligands to bacteriophage T4 DNA polymerase. Science 249(4968): 505-510
Usman N, Blatt LM (2000) Nuclease-resistant synthetic ribozymes: developing a new class of therapeutics. J Clin Invest 106(10): 1197-1202
Usman N, Cedergren R (1992) Exploiting the chemical synthesis of RNA. Trends Biochem Sci 17(9): 334-339
Waldmann R, Gross HJ, Krupp G (1987) Protocol for rapid chemical RNA sequencing. Nucleic Acids Res 15(17): 7209
Weigand BS, Zerressen A, Schlatterer JC, Helm M, Jaeschke A (2006) Catalytically Active RNA Molecules: Tools in Organic Chemistry. In The Aptamer Handbook, Klussmann S (ed), 9, pp 211-225. Weinheim: WILEY-VCH Verlag GmbH & Co. KGaA
Weiner GJ (2000) The immunobiology and clinical potential of immunostimulatory CpG oligodeoxynucleotides. J Leukoc Biol 68(4): 455-463
Wincott F, DiRenzo A, Shaffer C, Grimm S, Tracz D, Workman C, Sweedler D, Gonzalez C, Scaringe S, Usman N (1995) Synthesis, deprotection, analysis and purification of RNA and ribozymes. Nucleic Acids Res 23(14): 2677-2684
Wu H, Aboleneen H (2001) Improved oligonucleotide sequencing by alkaline phosphatase and exonuclease digestions with mass spectrometry. Anal Biochem 290(2): 347-352
Wu H, Chan C, Aboleneen H (1998a) Sequencing regular and labeled oligonucleotides using enzymatic digestion and ionspray mass spectrometry. Anal Biochem 263(2): 129-138
Wu H, Morgan RL, Aboleneen H (1998b) Characterization of labeled oligonucleotides using enzymatic digestion and tandem mass spectrometry. JAm Soc Mass Spectrom 9(7): 660-667
Wu J, McLuckey SA (2004) Gas-phase fragmentation of oligonucleotide ions International Journal of Mass Spectrometry 237(2-3): 197-241
Wu TP, Ruan KC, Liu WY (1996) A fluorescence-labeling method for sequencing small RNA on polyacrylamide gel. Nucleic Acids Res 24(17): 3472-3473
Yoshimura Y, Noguchi Y, Fujimoto K (2007) Highly sequence specific RNA terminal labeling by DNA photoligation. Organic & Biomolecular Chemistry 5: 139-142
Zhang B, Farwell MA (2008) microRNAs: a new emerging class of players for disease diagnostics and gene therapy. J Cell Mol Med 12(1): 3-21
Zimmern D, Kaesberg P (1978) 3'-terminal nucleotide sequence of encephalomyocarditis virus RNA determined by reverse transcriptase and chain-terminating inhibitors. Proc Natl Acad Sci USA 75(9): 4257-4261

### Described Embodiments

**Embodiment 1:** A method for determining the nucleotide sequence of a nucleic acid molecule comprising the following steps:
   a) providing a plurality of molecules of the nucleic acid molecule having at least one modification;
   b) cleaving at random the plurality of modified nucleic acid molecules thus providing modified nucleic acid molecule fragments and non-modified nucleic acid molecule fragments;
   c) separating the modified nucleic acid molecule fragments from the non-modified nucleic acid molecule fragments;
   d) separating or resolving the modified nucleic acid molecule fragments according to their length, mass and/or charge, whereby such separating or resolving generates a pattern of modified nucleic acid fragments; and
   e) optionally visualizing the pattern of modified nucleic acid fragments.
**Embodiment 2:** The method according to embodiment 1, wherein the method further comprises the step of
   f) deducing from the pattern of modified nucleic acid fragments the nucleotide sequence of the nucleic acid molecule.
**Embodiment 3:** The method according to any of embodiments 1 to 2, wherein the individual nucleic acid molecule of the plurality of molecules has at least one modification at the 5' end, at the 3' end or within the nucleotide sequence of the nucleic acid molecule the nucleotide sequence of which is to be determined.
**Embodiment 4:** The method according to any of embodiments 1 to 3, wherein the cleaving is carried out by chemical cleaving, enzymatic cleaving, cleaving by heat and/or cleaving by use of a divalent cation.
**Embodiment 5:** The method according to any of embodiments 1 to 4, wherein the cleaving is a chemical cleaving, preferably a nucleotide unspecific cleaving.
**Embodiment 6:** The method according to any of embodiments 1 to 5, wherein the cleaving is a limited cleaving.
**Embodiment 7:** The method according to any of embodiments 1 to 6, wherein the cleaving is a limited random cleaving, preferably a limited chemical random cleaving.
**Embodiment 8:** The method according to any of embodiments 1 to 7, wherein the step of cleaving provides for a mixture of fragments, preferably modified fragments, whereby such mixture of fragments comprises all possible nucleotide sequence fragments of the nucleic acid molecule.
**Embodiment 9:** The method according to embodiment 8, wherein the mixture comprises a modified full length form of the nucleic acid molecule the nucleotide sequence of which is to be determined.
**Embodiment 10:** The method according to any of embodiments 1 to 9, wherein the modified nucleic acid molecule fragments are separated from the non-modified nucleic acid molecule fragments through the interaction of the modification with an interaction partner, whereby such interaction partner is linked to a support.
**Embodiment 11:** The method according to embodiment 10, whereby the support is a solid support.
**Embodiment 12:** The method according to any of embodiments 10 to 11, wherein the non-modified nucleic acid molecule fragments are removed from the modified nucleic acid molecule fragments interacting with the interaction partner, preferably by washing.
**Embodiment 13:** The method according to any of embodiments 10 to 12, wherein the modified nucleic acid molecule fragments are released from the support, preferably by release of the modification from the interaction partner, by release from the interaction partner from the support or by cleaving the modification or a part or moiety thereof from the nucleic acid molecule fragments.
**Embodiment 14:** The method according to any of embodiments 1 to 9, wherein the modified nucleic acid molecule fragments are separated from the non-modified nucleic acid molecule by separation due to mass discrimination, size discrimination, and/or hydrophobicity discrimination, charge discrimination, ionic discrimination, hydrogen bonding discrimination or liquid phase mediated extraction, whereby preferably the non-labeled nucleic acid molecule fragments are removed.
**Embodiment 15:** The method according to any of embodiments 1 to 14, wherein the pattern of modified nucleic acid fragments comprises a ladder of modified nucleic acid fragments.
**Embodiment 16:** The method according to any of embodiments 1 to 15, wherein the pattern of modified nucleic acid fragments is generated by mass spectrometry and preferably the nucleic sequence of the nucleic acid molecule is deduced.
**Embodiment 17:** The method according to any of embodiments 1 to 15, wherein the pattern of modified nucleic acid fragments is generated and the masses of the individual fragments are determined by mass spectrometry and preferably the nucleic sequence of the nucleic acid molecule is deduced.
**Embodiment 18:** The method according to any of embodiments 1 to 17, wherein the nucleotide sequence of the nucleic acid molecule is not known.
**Embodiment 19:** The method according to any of embodiment 1 to 18, wherein the step of deducing from the pattern of modified nucleic acid fragments the nucleotide sequence of the nucleic acid molecule comprises the following steps:
   fa) determining the mass and/or nucleotide sequence of the smallest modified nucleic acid molecule fragment n + x, with x = 0;
   fb) determining the mass of the modified nucleic acid molecule fragment n + x with x = 1 which differs from the mass of the smallest modified nucleic acid molecule fragment n + x with x = 0 by one nucleotide;
   fc) determining the mass difference between the mass of the modified nucleic acid molecule fragment n + x with x = 1 and the mass of the smallest modified nucleic acid molecule fragment n + x with x = 0;
   fd) attributing the mass difference to a distinct nucleotide species and generating the sequence of modified nucleic acid molecule fragment n + x with x = 1 by adding the distinct nucleotide species to the sequence of the smallest modified nucleic acid molecule fragment n + x with x = 0.
**Embodiment 20:** The method according to embodiment 19, wherein steps fb) to fd) are repeated, whereby for each repetition x is increased by an addend of 1 and x is 2 for the first repetition and wherein in step fb) the mass of the modified nucleic acid molecule fragment n + x which differs from the mass of the modified nucleic acid molecule fragment n + (x - 1) by one nucleotide is determined, in step fc) the mass difference between the mass of the modified nucleic acid molecule fragment n + x and the mass of the modified nucleic acid molecule fragment n + (x - 1) is determined and in step fd) the mass difference is attributed to a distinct nucleotide species and the sequence of the modified nucleic acid molecule fragment n + x is generated by adding the distinct nucleotide species to the sequence of the modified nucleic acid molecule fragment n + (x - 1).
**Embodiment 21:** The method according to embodiment 20, wherein for the m^{th} repetition of steps fb) to fd) x is as follows: x = m+1.
**Embodiment 22:** The method according to any of embodiments 1 to 17, wherein the nucleotide sequence of the nucleic acid molecule is known and, preferably, the method is for confirming the nucleotide sequence of a nucleic acid molecule.
**Embodiment 23:** The method according to embodiment 22, wherein the step of deducing from the pattern of modified nucleic acid fragments the nucleotide sequence of the nucleic acid molecule comprises the following steps:
   fa) determining the mass of the modified nucleic acid molecule fragment n + x with x = 1 which differs from the mass of the smallest modified nucleic acid molecule fragment n + x with x = 0 by one nucleotide;
   fb) determining the mass difference between the mass of the modified nucleic acid molecule fragment n + x with x = 1 and the mass of the smallest modified nucleic acid molecule fragment n + x with x = 0;
   fc) attributing the mass difference to a distinct nucleotide species and generating the sequence of the modified nucleic acid molecule fragment n + x with x = 1 by adding the distinct nucleotide species to the sequence of the smallest modified nucleic acid molecule fragment n + x with x = 0.
**Embodiment 24:** The method according to embodiment 23, wherein steps fa) to fc) are repeated, whereby for each repetition x is increased by an addend of 1 and x is 2 for the first repetition and wherein in step fa) the mass of the modified nucleic acid molecule fragment n + x which differs from the mass of the modified nucleic acid molecule fragment n + (x-1) by one nucleotide is determined, in step fb) the mass difference between the mass of the modified nucleic acid molecule fragment n + x and the mass of the modified nucleic acid molecule fragment n + (x-1) is determined and in step fc) the mass difference is attributed to a distinct nucleotide species and the sequence of the modified nucleic acid molecule fragment n + x is generated by adding the distinct nucleotide species to the sequence of the modified nucleic acid molecule fragment n + (x-1).
**Embodiment 25:** The method according to embodiment 24, wherein for the m^{th} repetition of steps fa) to fc) x is as follows: x = m + 1.
**Embodiment 26:** The method according to any of embodiments 22 to 25, wherein the mass and/or the nucleotide sequence of the smallest modified nucleic acid molecule fragment n + x with x = 0 is known.
**Embodiment 27:** The method according to embodiment 22, wherein the step of deducing from the pattern of modified nucleic acid fragments the nucleotide sequence of the nucleic acid molecule comprises the following steps:
   fa) determining the mass of the modified nucleic acid molecule fragment n + x with x = 1 which differs from the mass of the smallest modified nucleic acid molecule fragment n + x with x = 0 by one nucleotide;
   fb) attributing the mass of the modified nucleic acid molecule fragment n + x with x = 1 to the calculated mass of the nucleic acid molecule fragment n + x with x = 1 of the nucleic acid molecule whose nucleotide sequence is known and generating the sequence of the modified nucleic acid molecule fragment n + x with x = 1 by adding the distinct nucleotide species to the sequence of the smallest modified nucleic acid molecule fragment n + x with x = 0.
**Embodiment 28:** The method according to embodiment 27, wherein steps fa) to fb) are repeated, whereby for each repetition x is increased by an addend of 1 and x is 2 for the first repetition and wherein in step fa) the mass of the modified nucleic acid molecule fragment n + x which differs from the mass of the modified nucleic acid molecule fragment n + (x-1) by one nucleotide is determined, and in step fb) the mass of the modified nucleic acid molecule fragment n + x with x = 1 is attributed to the calculated mass of the nucleic acid molecule fragment n + x with x = 1 of the nucleic acid molecule whose nucleotide sequence is known and the modified nucleic acid molecule sequence of fragment n + x is generated by adding the distinct nucleotide species to the sequence of the modified nucleic acid molecule fragment n + (x-1).
**Embodiment 29:** The method according to embodiment 28, wherein for the m^{th} repetition of steps fa) to fc) x is as follows: x = m + 1.
**Embodiment 30:** The method according to any of embodiments 19 to 29, wherein the modification is present at the 5' end of the nucleic acid molecule fragments and the smallest modified nucleic acid molecule fragment comprises the terminal 5' nucleotide of the full-length nucleic acid molecule or wherein the modification is present at the 3' end of the nucleic acid molecule fragments and the smallest modified nucleic acid molecule fragment comprises the terminal 3' nucleotide of the full-length nucleic acid molecule.
**Embodiment 31:** The method according to any of embodiments 1 to 30, wherein the modification is a unipartite modification comprising one moiety.
**Embodiment 32:** The method according to embodiment 31, wherein the moiety is used in separating the modified nucleic acid molecule fragments from the non-modified nucleic acid molecules.
**Embodiment 33:** The method according to embodiment 32, wherein the moiety is used in separating or resolving the modified nucleic acid molecule fragments in the generation of the pattern.
**Embodiment 34:** The method according to any of embodiments 1 to 30, wherein the modification is a multipartite modification comprising at least a first moiety and a second moiety, whereby optionally the at least first and second moiety are linked through a linker.
**Embodiment 35:** The method according to embodiment 34, wherein the first moiety is used in separating the modified nucleic acid molecule fragments from the non-modified nucleic acid molecules, and the second moiety is used in separating or resolving the modified nucleic acid molecule fragments in the generation of the pattern.
**Embodiment 36:** The method according to any of embodiments 31 to 35, wherein the moiety which is used in separating the modified nucleic acid molecule fragments from the non-modified nucleic acid molecules comprises a ligand to an interaction partner, whereby such interaction partner is present on a support, preferably linked to such support, and the interaction between the ligand and the interaction partner mediates immobilization of the modified nucleic acid molecule fragments onto the support.
**Embodiment 37:** The method according to the embodiment 36, wherein the immobilization is selected from the group comprising chemical immobilization, affinity immobilization, magnetic immobilization.
**Embodiment 38:** The method according to embodiment 37, wherein the immobilization is affinity immobilization.
**Embodiment 39:** The method according to embodiment 38, wherein the interaction which mediates the immobilization of the nucleic acid molecule and the nucleic acid molecule fragments onto the support is selected from the group comprising biotin-avidin interaction, biotin-neutravidin interaction, biotin-streptavidin interaction, antigen-antibody interaction, interaction of two oligonucleotides, whereby the nucleic acid molecules consist of DNA, RNA, LNA, PNA or combinations thereof, interaction of calmodulin and calmodulin binding peptide, interaction of albumin and Cibracon Blue, interaction of a metal-chelator agent and metal-chelating support.
**Embodiment 40:** The method according to any of embodiments 31 to 39, wherein the moiety which is used in separating the modified nucleic acid molecule fragments from the non-modified nucleic acid molecules is selected from the group comprising biotin, oligonucleotides, calmodulin binding peptides, albumins and metal-chelator agents. **Embodiment 41:** The method according to any of embodiments 1 to 40, wherein the modified nucleic acid molecule fragments are separated form the non-modified nucleic acid molecules by a means selected from the group comprising filtration, dialysis, chromatography, magnetic fields, centrifugation and precipitation.
**Embodiment 42:** The method according to embodiment 41, wherein chromatography is size exclusion chromatography, wherein the modified nucleic acid fragments are separated from the non-modified nucleic acid molecules according to their size or due to the increased size of the modified fragments imparted to them by the modification.
**Embodiment 43:** The method according to any of embodiments 31 to 42, wherein the moiety which is used in separating or resolving the modified nucleic acid molecule fragments in the generation of the pattern is selected from mass tags or lipophilic tags.
**Embodiment 44:** The method according to any of embodiments 1 to 43, wherein the modified nucleic acid molecule fragments are separated or resolved by a method for mass or size discrimination which is preferably selected from the group comprising filtration and dialysis and chromatography and mass spectrometry, preferably such method is MS, LCMS or ESI MS.
**Embodiment 45:** The method according to any of embodiments 1 to 44, wherein the modified nucleic acid molecule fragments are separated or resolved by a method based on hydrophobic interaction which is preferably RP-HPLC.
**Embodiment 46:** The method according to any of embodiments 34 to 45, wherein the linker is a hydrophobic linker.
**Embodiment 47:** The method according to any of embodiments 34 to 46, wherein the linker is a cleavable linker.
**Embodiment 48:** The method according to embodiment 47, wherein the linker is a selectively cleavable linker, more preferably the selectively cleavable linker is enzymatically cleavable, chemically cleavable, photocleavable or thermocleavable.
**Embodiment 49:** The method according to any of embodiment 1 to 48, whereby the nucleic acid molecule is selected from the group of RNA molecules, DNA molecules, nucleotide-modified RNA molecules and nucleotide-modified DNA molecules, PNA, LNA and combinations thereof, preferably RNA molecules, DNA molecules, nucleotide-modified RNA molecules, nucleotide-modified DNA molecules and nucleic acid molecules containing both deoxyribonucleotides and ribonucleotides.
**Embodiment 50:** The method according to any of embodiments 1 to 49, whereby the nucleic acid molecule is selected from the group consisting of aptamers, Spiegelmers, ribozymes, Spiegelzymes, antisense molecules, siRNA molecules and decoy molecules, preferably Spiegelmers.
**Embodiment 51:** The method according to any of embodiments 1 to 50, whereby the nucleic acid molecule is an RNA molecule and/or a nucleotide-modified RNA molecule.
**Embodiment 52:** The method according to embodiment 51, whereby the cleaving is a chemical cleaving of the RNA molecule and/or the nucleotide-modified RNA molecule which is done by alkaline hydrolysis, amines, or polyamines.
**Embodiment 53:** The method according to embodiment 51, whereby the cleaving is an enzymatic cleaving of the RNA molecule and/or the nucleotide-modified RNA molecule which is done by use of nucleases, preferably ribonuclease, and/or nucleic-acid based enzymes, preferably nucleic acid based enzymes.
**Embodiment 54:** The method according to embodiment 51, whereby the cleaving is a cleaving by heat of the RNA molecule and/or the nucleotide-modified RNA molecule.
**Embodiment 55:** The method according to embodiment 51, whereby the cleaving is a cleaving of the RNA molecule and/or the nucleotide-modified RNA molecule by use of divalent cations.
**Embodiment 56:** The method according to any of embodiments 1 to 50, whereby the nucleic acid is a DNA molecule and/or a nucleotide-modified DNA molecule.
**Embodiment 57:** The method according to embodiment 56, whereby the cleaving is a chemical cleaving of the DNA molecule and/or the nucleotide-modified DNA molecule which is done by use of acid hydrolysis.
**Embodiment 58:** The method according to embodiment 56, whereby the cleaving is an enzymatic cleaving of the DNA molecule and/or the nucleotide-modified DNA molecule which is done by use of nucleases, preferably deoxyribonuclease, and/or nucleic-acid based enzymes, preferably nucleic acid based enzymes.
**Embodiment 59:** The method according to any of embodiments 16 to 58, whereby mass spectrometry is selected from the group comprising direct mass spectrometry, LC-MS and MS/MS.
**Embodiment 60:** The method according to any of embodiments 1 to 59, whereby a specific mass fingerprint of a nucleic acid molecule is determined.
**Embodiment 61:** The method according to embodiment 60, whereby the specific mass fingerprint is used for identifying and/or quality control for a nucleic acid molecule.
**Embodiment 62:** The method according to any of embodiments 1 to 61, wherein the at least one modification of the nucleic acid molecule or of the plurality of molecules of the nucleic acid molecule is added to the 5' end or the 3' end of the nucleic acid molecule, prior to step a) or b).
**Embodiment 63:** The method according to any of embodiments 1 to 62, whereby the nucleic acid molecule or the plurality of molecules of the nucleic acid molecule comprises(s) a non-nucleic acid moiety.
**Embodiment 64:** The method according to embodiment 63, wherein the non-nucleic acid moiety is removed from the nucleic acid molecule or the plurality of molecules of the nucleic acid molecule prior to step a) or b).
**Embodiment 65:** The method according to embodiment 64, wherein in a first step the non-nucleic acid moiety is removed from the nucleic acid molecule or the plurality of molecules of the nucleic acid molecule and in a second step the modification of the nucleic acid molecule or of the plurality of molecules of the nucleic acid molecule is added to the 5' end, the 3' end or a nucleotide within the nucleotide sequence of the nucleic acid molecule or of the plurality of molecules of the nucleic acid molecule prior to step a) or b).
**Embodiment 66:** A method for determining the nucleotide sequence of a nucleic acid molecule comprising the following steps:
   a) providing a plurality of molecules of the nucleic acid molecule having at least one modification;
   b) cleaving at random the plurality of modified nucleic acid molecules thus providing modified nucleic acid molecule fragments;
   c) separating or resolving the modified nucleic acid molecule fragments according to their length, mass and/or charge, wherein such separating or resolving generates a pattern of modified nucleic acid fragments; and
   d) optionally visualizing the pattern of modified nucleic acid fragments.
**Embodiment 67:** The method according to embodiment 66, wherein a reaction mixture which is obtained after step b) or c), contains one or more nucleic acid molecules or fragments thereof not having said at least one modification.
**Embodiment 68:** The method according to one of embodiments 66 and 67, wherein the visualizing of the pattern of the modified nucleic acid fragments makes use of the at least one modification, preferably the modification allows to discriminate between a nucleic acid molecule having said modification and a nucleic acid molecule not having said modification.
**Embodiment 69:** The method according to any of embodiments 66 to 68, wherein the modification is selected from the group comprising mass tags, moieties with significantly more UV absorbance at a given wavelength than the nucleic acid molecule lypophilic moieties, polymers with defined molecular mass, radiolabels and moieties imparting an altered ion mobility
**Embodiment 70:** The method according to embodiment 69, wherein the moiety with significantly more UV absorbance at a given wavelength than the nucleic acid molecule is selected from the group comprising chromophores, dyes and fluorescence labels.
**Embodiment 71:** The method according to any of embodiments 66 to 70, wherein the method further comprises the step of
   e) deducing from the pattern of modified nucleic acid fragments the nucleotide sequence of the nucleic acid molecule.
**Embodiment 72:** The method according to any of embodiments 66 to 71, wherein the individual nucleic acid molecule of the plurality of molecules has at least one modification at the 5' end, at the 3' end or within the nucleotide sequence of the nucleic acid molecule the nucleotide sequence of which is to be determined.
**Embodiment 73:** The method according to any of embodiments 66 to 72, wherein the cleaving is carried out by chemical cleaving, enzymatic cleaving, cleaving by heat and/or cleaving by use of a divalent cation.
**Embodiment 74:** The method according to any of embodiments 66 to 73, wherein the cleaving is a chemical cleaving, preferably a nucleotide unspecific cleaving.
**Embodiment 75:** The method according to any of embodiments 66 to 74, wherein the cleaving is a limited cleaving.
**Embodiment 76:** The method according to any of embodiments 66 to 75, wherein the cleaving is a limited random cleaving, preferably a limited chemical random cleaving.
**Embodiment 77:** The method according to any of embodiments 66 to 76, wherein the step of cleaving provides for a mixture of fragments, preferably modified fragments, whereby such mixture of fragments comprises all possible nucleotide sequence fragments of the nucleic acid molecule.
**Embodiment 78:** The method according to embodiment 77, wherein the mixture comprises a modified full length form of the nucleic acid molecule the nucleotide sequence of which is to be determined.
**Embodiment 79:** The method according to any of embodiments 66 to 78, wherein the pattern of modified nucleic acid fragments comprises a ladder of modified nucleic acid fragments.
**Embodiment 80:** The method according to any of embodiments 66 to 79, wherein the pattern of modified nucleic acid fragments is generated by mass spectrometry, preferably LC-MS, and preferably the nucleic sequence of the nucleic acid molecule is deduced.
**Embodiment 81:** The method according to any of embodiments 66 to 79, wherein the pattern of modified nucleic acid fragments is generated and the masses of the individual fragments are determined by mass spectrometry and preferably the nucleic sequence of the nucleic acid molecule is deduced.
**Embodiment 82:** The method according to any of embodiments 66 to 81, wherein the nucleotide sequence of the nucleic acid molecule is not known.
**Embodiment 83:** The method according to any of embodiments 66 to 82, wherein the step of deducing from the pattern of modified nucleic acid fragments the nucleotide sequence of the nucleic acid molecule comprises the following steps:
   fa) determining the mass and/or nucleotide sequence of the smallest modified nucleic acid molecule fragment n + x, with x = 0;
   fb) determining the mass of the modified nucleic acid molecule fragment n+x with x = 1 which differs from the mass of the smallest modified nucleic acid molecule fragment n+ x with x = 0 by one nucleotide;
   fc) determining the mass difference between the mass of the modified nucleic acid molecule fragment n + x with x = 1 and the mass of the smallest modified nucleic acid molecule fragment n + x with x = 0;
   fd) attributing the mass difference to a distinct nucleotide species and generating the sequence of the modified nucleic acid molecule fragment n + x with x = 1 by adding the distinct nucleotide species to the sequence of the smallest modified nucleic acid molecule fragment n + x with x = 0.
**Embodiment 84:** The method according to embodiment 83, wherein steps fb) to fd) are repeated, whereby for each repetition x is increased by an addend of 1 and x is 2 for the first repetition and wherein in step fb) the mass of the modified nucleic acid molecule fragment n + x which differs from the mass of the modified nucleic acid molecule fragment n + (x-1) by one nucleotide is determined, in step fc) the mass difference between the mass of the modified nucleic acid molecule fragment n + x and the mass of the modified nucleic acid molecule fragment n + (x-1) is determined and in step fd) the mass difference is attributed to a distinct nucleotide species and the sequence of the modified nucleic acid molecule fragment n + x is generated by adding the distinct nucleotide species to the sequence of the modified nucleic acid molecule fragment n + (x-1).
**Embodiment 85:** The method according to embodiment 84, wherein for the m^{th} repetition of steps fb) to fd) x is as follows: x = m+1.
**Embodiment 86:** The method according to any of embodiments 66 to 81, wherein the nucleotide sequence of the nucleic acid molecule is known and, preferably, the method is for confirming the nucleotide sequence of a nucleic acid molecule.
**Embodiment 87:** The method according to embodiment 86, wherein the step of deducing from the pattern of modified nucleic acid fragments the nucleotide sequence of the nucleic acid molecule comprises the following steps:
   fa) determining the mass of the modified nucleic acid molecule fragment n + x with x = 1 which differs from the mass of the smallest modified nucleic acid molecule fragment n + x with x = 0 by one nucleotide;
   fb) determining the mass difference between the mass of the modified nucleic acid molecule fragment n + x with x = 1 and the mass of the smallest modified nucleic acid molecule fragment n + x with x = 0;
   fc) attributing the mass difference to a distinct nucleotide species and generating the sequence of the modified nucleic acid molecule fragment n + x with x = 1 by adding the distinct nucleotide species to the sequence of the smallest modified nucleic acid molecule fragment n + x with x = 0.
**Embodiment 88:** The method according to embodiment 87, wherein steps fa) to fc) are repeated, whereby for each repetition x is increased by an addend of I and x is 2 for the first repetition and wherein in step fa) the mass of the modified nucleic acid molecule fragment n+x which differs from the mass of the modified nucleic acid molecule fragment n + (x-1) by one nucleotide is determined, in step fb) the mass difference between the mass of the modified nucleic acid molecule fragment n + x and the mass of the modified nucleic acid molecule fragment n + (x-1) is determined and in step fc) the mass difference is attributed to a distinct nucleotide species and the sequence of fragment n + x is generated by adding the distinct nucleotide species to the sequence of the modified nucleic acid molecule fragment n + (x-1).
**Embodiment 89:** The method according to embodiment 88, wherein for the m^{th} repetition of steps fa) to fb) x is as follows: x = m + 1.
**Embodiment 90:** The method according to any of embodiments 86 to 89, wherein the mass and/or the nucleotide sequence of the smallest modified nucleic acid molecule fragment n + x with x = 0 is known.
**Embodiment 91:** The method according to embodiment 86, wherein the step of deducing from the pattern of modified nucleic acid fragments the nucleotide sequence of the nucleic acid molecule comprises the following steps:
   fa) determining the mass of the modified nucleic acid molecule fragment n + x with x = 1 which differs from the mass of the smallest modified nucleic acid molecule fragment n + x with x = 0 by one nucleotide;
   fb) attributing the mass of the modified nucleic acid molecule fragment n + x with x = 1 to the calculated mass of the nucleic acid molecule fragment n + x with x = 1 of the nucleic acid molecule whose nucleotide sequence is known and generating the sequence of the modified nucleic acid molecule fragment n + x with x = 1 by adding the distinct nucleotide species to the sequence of the smallest modified nucleic acid molecule fragment n + x with x = 0.
**Embodiment 92:** The method according to embodiment 91, wherein steps fa) to fb) are repeated, whereby for each repetition x is increased by an addend of 1 and x is 2 for the first repetition and wherein in step fa) the mass of the modified nucleic acid molecule fragment n + x which differs from the mass of the modified nucleic acid molecule fragment n + (x-1) by one nucleotide is determined, and in step fb) the mass of the modified nucleic acid molecule fragment n + x with x = 1 is attributed to the calculated mass of the nucleic acid molecule fragment n + x with x = 1 of the nucleic acid molecule whose nucleotide sequence is known and the modified nucleic acid molecule sequence of fragment n + x is generated by adding the distinct nucleotide species to the sequence of the modified nucleic acid molecule fragment n + (x-1).
**Embodiment 93:** The method according to embodiment 92, wherein for the m^{th} repetition of steps fa) to fc) x is as follows: x = m + 1.
**Embodiment 94:** The method according to any of embodiments 83 to 93, wherein the modification is present at the 5' end of the nucleic acid molecule fragments and the smallest modified nucleic acid molecule fragment comprises the terminal 5' nucleotide of the full-length nucleic acid molecule or wherein the modification is present at the 3' end of the nucleic acid molecule fragments and the smallest modified nucleic acid molecule fragment comprises the terminal 3' nucleotide of the full-length nucleic acid molecule.
**Embodiment 95:** The method according to any of embodiments 66 to 94, wherein the modification is used in separating or resolving the modified nucleic acid molecule fragments in the generation of the pattern.
**Embodiment 96:** The method according to any of embodiments 66 to 95, wherein the modification is a fluorescent label wose wavelength absorbance is different from the wavelength absorbance of the nucleobases of the nucleic acid molecules.
**Embodiment 97:** The method according to any of embodiments 66 to 96, whereby the nucleic acid molecule is selected from the group of RNA molecules, DNA molecules, nucleotide-modified RNA molecules, nucleotide-modified DNA molecules, PNA, LNA and combinations thereof, preferably RNA molecules, DNA molecules, nucleotide-modified RNA molecules, nucleotide-modified DNA molecules and nucleic acid molecules containing both deoxyribonucleotides and ribonucleotides .
**Embodiment 98:** The method according to any of embodiments 66 to 97, whereby the nucleic acid molecule is selected from the group consisting of aptamers, Spiegelmers, ribozymes, Spiegelzymes, antisense molecules, siRNA molecules and decoy molecules, preferably Spiegelmers.
**Embodiment 99:** The method according to any of embodiments 66 to 98, whereby the nucleic acid molecule is an RNA molecule and/or a nucleotide-modified RNA molecule.
**Embodiment 100:** The method according to embodiment 99, whereby the cleaving is a chemical cleaving of the RNA molecule and/or the nucleotide-modified RNA molecule and such cleaving is done by alkaline hydrolysis.
**Embodiment 101:** The method according to embodiment 99, whereby the cleaving is an enzymatic cleaving of the RNA molecule and/or the nucleotide-modified RNA molecule which is done by use of nucleases, preferably ribonuclease, and/or nucleic-acid based enzymes, preferably nucleic acid based enzymes.
**Embodiment 102:** The method according to embodiment 99, whereby the cleaving is a cleaving by heat of the RNA molecule and/or the nucleotide-modified RNA molecule.
**Embodiment 103:** The method according to embodiment 99, whereby the cleaving is a cleaving of the RNA molecule and/or the modified RNA molecule by use of divalent cations, or a combination of cleaving by heat and a cleaving agent..
**Embodiment 104:** The method according to any of embodiments 66 to 98, whereby the nucleic acid is a DNA molecule and/or a nucleotide-modified DNA molecule.
**Embodiment 105:** The method according to embodiment 104, whereby the cleaving is a chemical cleaving of the DNA molecule and/or the nucleotide-modified DNA molecule which is done by use of acid hydrolysis.
**Embodiment 106:** The method according to embodiment 104, whereby the cleaving is an enzymatic cleaving of the DNA molecule and/or the nucleotide-modified DNA molecule which is done by use of nucleases, preferably deoxyribonuclease, and/or nucleic-acid based enzymes, preferably nucleic acid based enzymes.
**Embodiment 107:** The method according to any of embodiments 66 to 106, whereby a specific mass fingerprint of a nucleic acid molecule is determined.
**Embodiment 108:** The method according to embodiment 107, whereby the specific mass fingerprint is used for identifying and/or quality control for a nucleic acid molecule.
**Embodiment 109:** The method according to any of embodiments 66 to 108,wherein the at least one modification of the nucleic acid molecule or of the plurality of molecules of the nucleic acid molecule is added to the 5' end or the 3' end of the nucleic acid molecule, prior to step a) or b).
**Embodiment 110:** The method according to any of embodiments 66 to 109, whereby the nucleic acid molecule or the plurality of molecules of the nucleic acid molecule comprises(s) a non-nucleic acid moiety.
**Embodiment 111:** The method according to embodiment 110, wherein the non-nucleic acid moiety is removed from the nucleic acid molecule or the plurality of molecules of the nucleic acid molecule prior to step a) or b).
**Embodiment 112** The method according to embodiment 111, wherein in a first step the non-nucleic acid moiety is removed from the nucleic acid molecule or the plurality of molecules of the nucleic acid molecule and in a second step the modification of the nucleic acid molecule or of the plurality of molecules of the nucleic acid molecule is added to the 5' end, the 3' end or a nucleotide within the nucleotide sequence of the nucleic acid molecule or of the plurality of molecules of the nucleic acid molecule prior to step a) or b).
**Embodiment 113:** A method for determining the nucleotide sequence of a nucleic acid molecule comprising the following steps:
   a) providing a plurality of molecules of the nucleic acid molecule;
   b) subjecting the plurality of molecules of the nucleic acid molecule to a nucleobase selective treatment, whereby one or several of the nucleobase species forming the nucleic acid molecule are selectively modified and whereby after such nucleobase selective treatment some of the selectively treatable nucleobases of the nucleic acid molecules are modified and some of the selectively treatable nucleotides or nucleobases of the nucleic acid molecules remain non-modified;
   c) chemically cleaving the nucleic acid phosphate backbone selectively 3' to the modified nucleobases, whereby the nucleic acid phosphate backbone of not all of the modified nucleobases are cleaved;
   d) analysing nucleic acid molecule fragments by LC-MS and/or LC-MS-MS; and
   e) identifying nucleic acid molecule fragments in increasing order of size with an intact terminus and generating the sequence of the nucleic acid molecule therefrom, wherein, preferably, the nucleic acid molecule fragments have the same intact terminus, more preferably the same intact 3' terminus.
**Embodiment 114:** The method according to embodiment 113, wherein the nucleic acid molecule is selected from the group of RNA molecules, DNA molecules, nucleotide-modified RNA molecules and nucleotide-modified DNA molecules, PNA, LNA, nucleic acid molecules comprising both deoxyribonucleotides and ribonucleotides, and combinations thereof, preferably RNA molecules, DNA molecules, nucleotide-modified RNA molecules and nucleotide-modified DNA molecules.
**Embodiment 115:** The method according to any of embodiments 113 to 114, wherein the nucleic acid molecule is selected from the group consisting of aptamers, Spiegelmers, ribozymes, Spiegelzymes, antisense molecules, siRNA molecules and decoy molecules, preferably Spiegelmers.
**Embodiment 116:** The method according to any of embodiments 113 to 115, wherein the nucleic acid molecule is an RNA molecule and/or a nucleotide-modified RNA molecule.
**Embodiment 117:** The method according to any of embodiments 113 to 116, wherein the selectively treatable nucleobase is selected from the group comprising guanosine, adenosine, cytidine, thymdine and uracil.
**Embodiment 118:** The method according to any of embodiments 113 to 117, wherein the nucleobase U is selectively treated with a combination of hydrazine, acetic acid and aniline leading to 5' phosphate appended 3' fragment and an aniline modified ribose 5' fragment.
**Embodiment 119:** The method according to embodiment 118, wherein the 5' phosphate appended 3' fragment and the intact nucleic acid molecule are ionized more efficiently than aniline modified ribose 5' fragments in step d) of embodiment 113.
**Embodiment 120:** The method according to embodiment 119, wherein the 5' phosphate appended 3' fragment is ionized more efficiently than aniline modified ribose 5' fragment in step d) of embodiment 113.
**Embodiment 121:** The method according to embodiment 120, wherein the 5' phosphate appended 3' fragments are used in step e) of embodiment 113.
**Embodiment 122:** The method according to any of embodiments 1 to 121, wherein the nucleic acid molecule comprises more than 25 nucleotides or nucleobases.
**Embodiment 123:** The method according to any of embodiments 1 to 122, wherein the nucleic acid molecule comprises more than 35 nucleotides or nucleobases.
**Embodiment 124:** The method according to any of embodiments 1 to 123, wherein the nucleic acid molecule comprises from 26 to 50 nucleobases, or from 36 to 50 nucleobases, preferably from 26 to 45 nucleobases or from 36 to 45 nucleobases.
**Embodiment 125:** The method according to any of embodiments 1 to 124, wherein the aggregation of the nucleic acid molecules of the plurality of molecules of the nucleic acid molecule is reduced.
**Embodiment 126:** The method according to embodiment 125, wherein the aggregation is reduced by the addition of a chaotropic solution to any of steps a) to e), preferably any of steps a) and b).

## Claims

1. A method for determining the nucleotide sequence of a nucleic acid molecule comprising the following steps:
a) providing a plurality of molecules of the nucleic acid molecule having at least one modification;
b) cleaving at random the plurality of modified nucleic acid molecules thus providing modified nucleic acid molecule fragments and non-modified nucleic acid molecule fragments, wherein the cleaving provides for a mixture of fragments and wherein such mixture of fragments comprises all possible nucleotide sequence fragments of the nucleic acid molecule;
c) separating the modified nucleic acid molecule fragments from the non-modified nucleic acid molecule fragments;
d) separating or resolving the modified nucleic acid molecule fragments according to their length, mass and/or charge, whereby such separating or resolving generates a pattern of modified nucleic acid fragments; and
e) optionally visualizing the pattern of modified nucleic acid fragments;
wherein the modification is present at the 5' end of the nucleic acid molecule fragments and the smallest modified nucleic acid molecule fragment comprises the terminal 5' nucleotide of the full-length nucleic acid molecule or wherein the modification is present at the 3' end of the nucleic acid molecule fragments and the smallest modified nucleic acid molecule fragment comprises the terminal 3' nucleotide of the full-length nucleic acid molecule.

2. The method according to claim 1, wherein the method further comprises the step of
f) deducing from the pattern of modified nucleic acid fragments the nucleotide sequence of the nucleic acid molecule.

3. The method according to claim 2, wherein the step of deducing from the pattern of modified nucleic acid fragments the nucleotide sequence of the nucleic acid molecule comprises the following steps:
fa) determining the mass and/or nucleotide sequence of the smallest modified nucleic acid molecule fragment n + x, with x = 0;
fb) determining the mass of the modified nucleic acid molecule fragment n + x with x = 1 which differs from the mass of the smallest modified nucleic acid molecule fragment n + x with x = 0 by one nucleotide;
fc) determining the mass difference between the mass of the modified nucleic acid molecule fragment n + x with x = 1 and the mass of the smallest modified nucleic acid molecule fragment n + x with x = 0;
fd) attributing the mass difference to a distinct nucleotide species and generating the sequence of modified nucleic acid molecule fragment n + x with x = 1 by adding the distinct nucleotide species to the sequence of the smallest modified nucleic acid molecule fragment n + x with x = 0.

4. The method according to any of claims 1 to 3, wherein the step of cleaving provides for a mixture of modified fragments, whereby such mixture of modified fragments comprises all possible nucleotide sequence fragments of the nucleic acid molecule.

5. The method according to claim 4, wherein the mixture comprises a modified full length form of the nucleic acid molecule the nucleotide sequence of which is to be determined.

6. The method according to any of claims 1 to 5, wherein the modified nucleic acid molecule fragments are separated from the non-modified nucleic acid molecule fragments through the interaction of the modification with an interaction partner, whereby such interaction partner is linked to a support.

7. The method according to any of claims 1 to 5, wherein the modified nucleic acid molecule fragments are separated from the non-modified nucleic acid molecule by separation due to mass discrimination, size discrimination, and/or hydrophobicity discrimination, charge discrimination, ionic discrimination, hydrogen bonding discrimination or liquid phase mediated extraction, whereby preferably the non-labeled nucleic acid molecule fragments are removed.

8. The method according to any of claims 1 to 7, wherein the pattern of modified nucleic acid fragments comprises a ladder of modified nucleic acid fragments.

9. The method according to any of claims 1 to 8, wherein the nucleotide sequence of the nucleic acid molecule is not known.

10. The method according to any of claims 1 to 8, wherein the nucleotide sequence of the nucleic nucleic acid molecule is known and, preferably, the method is for confirming the nucleotide sequence of a nucleic acid molecule.

11. The method according to any of claims 1 to 10, wherein the modified nucleic acid molecule fragments are separated or resolved by a method for mass or size discrimination which is preferably selected from the group comprising filtration and dialysis and chromatography and mass spectrometry, preferably such method is MS, LCMS or ESI MS.

12. The method according to any of claims 1 to 11, whereby the nucleic acid molecule is selected from the group of RNA molecules, DNA molecules, nucleotide-modified RNA molecules and nucleotide-modified DNA molecules, PNA, LNA and combinations thereof; preferably RNA molecules, DNA molecules, nucleotide-modified RNA molecules, nucleotide-modified DNA molecules and nucleic acid molecules containing both deoxyribonucleotides and ribonucleotides.

13. The method according to any of claims 1 to 12, whereby the nucleic acid molecule is selected from the group consisting of aptamers, Spiegelmers, ribozymes, Spiegelzymes, antisense molecules, siRNA molecules and decoy molecules, preferably Spiegelmers.

14. The method according to any of claims 1 to 13, whereby a specific mass fingerprint of a nucleic acid molecule is determined.

15. The method according to any of claims 1 to 14, wherein the nucleic acid molecule comprises more than 25 nuclootides or nucleobases.

## Patentansprüche

1. Verfahren zum Bestimmen der Nukleotidsequenz eines Nukleinsäuremoleküls, umfassend die folgenden Schritte:
a) Bereitstellen einer Vielzahl von Molekülen des Nukleinsäuremoleküls mit wenigstens einer Modifikation;
b) zufälliges Spalten der Vielzahl von modifizierten Nukleinsäuremolekülen, wodurch modifizierte Nukleinsäuremolekülfragmente und nicht-modifizierte Nukleinsäuremolekülfragmente bereitgestellt werden, wobei die Spaltung eine Mischung aus Fragmenten bereitstellt und wobei diese Mischung von Fragmenten alle möglichen Nukleotidsequenzfragmente des Nukleinsäuremoleküls umfasst;
c) Trennen der modifizierten Nukleinsäuremolekülfragmente von den nicht-modifizierten Nukleinsäuremolekülfragmenten;
d) Trennen oder Auflösen der modifizierten Nukleinsäuremolekülfragmente gemäß ihrer Länge, Masse und / oder Ladung, wobei dieses Trennen oder Auflösen ein Muster aus modifizierten Nukleinsäurefragmenten erzeugt; und
e) optional Visualisieren des Musters aus modifizierten Nukleinsäurefragmenten;
wobei die Modifikation an dem 5'-Ende der Nukleinsäuremolekülfragmente vorhanden ist und das kleinste modifizierte Nukleinsäuremolekülfragment das terminale 5'-Nukleotid des Volllängennukleinsäuremoleküls umfasst oder wobei die Modifikation an dem 3'-Ende der Nukleinsäuremolekülfragmente vorhanden ist und das kleinste modifizierte Nukleinsäuremolekülfragment das terminale 3'-Nukleotid des Volllängennukleinsäuremoleküls umfasst.

2. Verfahren nach Anspruch 1, wobei das Verfahren weiter den Schritt umfasst
f) Ableiten der Nukleotidsequenz des Nukleinsäuremoleküls von dem Muster aus modifizierten Nukleinsäurefragmenten.

3. Verfahren nach Anspruch 2, wobei der Schritt des Ableitens der Nukleotidsequenz des Nukleinsäuremoleküls von dem Muster aus modifizierten Nukleinsäurefragmenten die folgenden Schritte umfasst:
fa) Bestimmen der Masse und / oder Nukleotidsequenz des kleinsten modifizierten Nukleinsäuremolekülfragmentes n + x, mit x = 0;
fb) Bestimmen der Masse des modifizierten Nukleinsäuremolekülfragmentes n + x, mit x = 1, das sich von der Masse des kleinsten modifizierten Nukleinsäuremolekülfragmentes n + x, mit x = 0, durch ein Nukleotid unterscheidet;
fc) Bestimmen des Massenunterschiedes zwischen der Masse des modifizierten Nukleinsäuremolekülfragmentes n + x mit x = 1 und der Masse des kleinsten modifizierten Nukleinsäuremolekülfragmentes n + x mit x = 0;
fd) Zuweisen des Massenunterschiedes zu einer bestimmten Nukleotidspezies und Erzeugen der Sequenz von modifiziertem Nukleinsäuremolekülfragment n + x mit x = 1 durch Hinzufügen der bestimmten Nukleotidspezies an die Sequenz des kleinsten modifizierten Nukleinsäuremolekülfragmentes n + x mit x = 0.

4. Verfahren nach einem der Ansprüche 1 bis 3, wobei der Schritt des Spaltens eine Mischung aus modifizierten Fragmenten bereitstellt, wobei diese Mischung aus modifizierten Fragmenten alle möglichen Nukleotidsequenzfragmente des Nukleinsäuremoleküls umfasst.

5. Verfahren nach Anspruch 4, wobei die Mischung eine modifizierte Volllängenform des Nukleinsäuremoleküls umfasst, dessen Nukleotidsequenz bestimmt werden soll.

6. Verfahren nach einem der Ansprüche 1 bis 5, wobei die modifizierten Nukleinsäuremolekülfragmente von den nicht-modifizierten Nukleinsäuremolekülfragmenten getrennt werden durch die Wechselwirkung der Modifikation mit einem Wechselwirkungspartner, wobei dieser Wechselwirkungspartner mit einem Träger verbunden ist.

7. Verfahren nach einem der Ansprüche 1 bis 5, wobei die modifizierten Nukleinsäuremolekülfragmente von dem nicht-modifizierten Nukleinsäuremolekül getrennt werden durch Trennen basierend auf Massendiskriminierung, Größendiskriminierung und / oder Hydrophobizitätsdiskriminierung, Ladungsdiskriminierung, ionischer Diskriminierung, Wasserbrückenbindungsdiskriminierung oder Flüssigphasen-vermittelter Extraktion, wobei bevorzugterweise die nicht-markierten Nukleinsäuremolekülfragmente entfernt werden.

8. Verfahren nach einem der Ansprüche 1 bis 7, wobei das Muster aus modifizierten Nuleinsäurefragmenten eine Leiter aus modifizierten Nukleinsäurefragmenten umfasst.

9. Verfahren nach einem der Ansprüche 1 bis 8, wobei die Nukleotidsequenz des Nukleinsäuremoleküls nicht bekannt ist.

10. Verfahren nach einem der Ansprüche 1 bis 8, wobei die Nukleotidsequenz des Nukleinsäuremoleküls bekannt ist und, bevorzugterweise, das Verfahren ein solches zum Bestätigen der Nukleotidsequenz eines Nukleinsäuremoleküls ist.

11. Verfahren nach einem der Ansprüche 1 bis 10, wobei die modifizierten Nukleinsäuremolekülfragmente getrennt oder aufgelöst werden durch ein Verfahren für Massen- oder Größendiskriminierung, das bevorzugterweise ausgewählt aus der Gruppe umfassend Filtration und Dialyse und Chromatographie und Massenspektrometrie, bevorzugterweise ist dieses Verfahren MS, LCMS oder ESI MS.

12. Verfahren nach einem der Ansprüche 1 bis 11, wobei das Nukleinsäuremolekül ausgewählt ist aus der Gruppe von RNA-Molekülen, DNA-Molekülen, Nukleotid-modifizierten RNA-Molekülen und Nukleotid-modifizierten DNA-Molekülen, PNA, LNA und Kombinationen davon, bevorzugterweise RNA-Molekülen, DNA-Molekülen, Nukleotid-modifizierten RNA-Molekülen, Nukleotid-modifizierten DNA-Molekülen, und Nukleinsäuremoleküle, die sowohl Desoxyribonukleotide als auch Ribonukleotide enthalten.

13. Verfahren nach einem der Ansprüche 1 bis 12, wobei das Nukleinsäuremolekül ausgewählt ist aus der Gruppe bestehend aus Aptameren, Spiegelmeren, Ribozymen, Spiegelzymen, Antisense-Molekülen, siRNA-Molekülen und Decoy-Molekülen, bevorzugterweise Spiegelmeren.

14. Verfahren nach einem der Ansprüche 1 bis 13, wobei ein spezifischer Massenfingerabdruck eines Nukleinsäuremoleküls bestimmt wird.

15. Verfahren nach einem der Ansprüche 1 bis 14, wobei das Nukleinsäuremolekül mehr als 25 Nukleotide oder Nukleobasen umfasst.

## Revendications

1. Procédé pour déterminer la séquence nucléotidique d'une molécule d'acide nucléique comprenant les étapes suivantes :
a) fourniture d'une pluralité de molécules de la molécule d'acide nucléique ayant au moins une modification ;
b) clivage aléatoire de la pluralité de molécules d'acide nucléique modifiées de manière à produire des fragments de molécule d'acide nucléique modifiée et des fragments de molécule d'acide nucléique non modifiée, dans lequel le clivage produit un mélange de fragments et dans lequel un tel mélange de fragments comprend tous les fragments de séquence nucléotidique possibles de la molécule d'acide nucléique ;
c) séparation des fragments de molécule d'acide nucléique modifiée des fragments de molécule d'acide nucléique non modifiée ;
d) séparation ou résolution des fragments de molécule d'acide nucléique modifiée en fonction de leur longueur, masse et/ou charge, de telle manière qu'une telle séparation ou résolution génère un profil de fragments d'acide nucléique modifié ; et
e) visualisation facultative du profil de fragments d'acide nucléique modifié ;
**caractérisé en ce que** la modification est présente à l'extrémité 5' des fragments de molécule d'acide nucléique et le plus petit fragment de molécule d'acide nucléique modifiée comprend le nucléotide 5'-terminal de la molécule d'acide nucléique de pleine longueur ou dans lequel la modification est présente à l'extrémité 3' des fragments de molécule d'acide nucléique et le plus petit fragment de molécule d'acide nucléique modifiée comprend le nucléotide 3'-terminal de la molécule d'acide nucléique de pleine longueur.

2. Procédé selon la revendication 1, **caractérisé en ce que** le procédé comprend en outre l'étape de
f) déduction à partir du profil de fragments d'acide nucléique modifié de la séquence nucléotidique de la molécule d'acide nucléique.

3. Procédé selon la revendication 2, **caractérisé en ce que** l'étape de déduction à partir du profil de fragments d'acide nucléique modifié de la séquence nucléotidique de la molécule d'acide nucléique comprend les étapes suivantes :
fa) détermination de la masse et/ou séquence nucléotidique du plus petit fragment de molécule d'acide nucléique modifiée n + x, avec x=0;
fb) détermination de la masse du fragment de molécule d'acide nucléique modifiée n + x avec x = 1 qui diffère de la masse du plus petit fragment de molécule d'acide nucléique modifiée n + x avec x = 0 d'un nucléotide ;
fc) détermination de la différence de masse entre la masse du fragment de molécule d'acide nucléique modifiée n + x avec x = 1 et la masse du plus petit fragment de molécule d'acide nucléique modifiée n + x avec x = 0 ;
fd) attribution de la différence de masse à une espèce nucléotidique distincte et génération de la séquence de fragment de molécule d'acide nucléique modifiée n + x avec x = 1 par ajout de l'espèce nucléotidique distincte à la séquence du plus petit fragment de molécule d'acide nucléique modifiée n + x avec x = 0.

4. Procédé selon l'une quelconque des revendications 1 à 3, **caractérisé en ce que** l'étape de clivage produit un mélange de fragments modifiés, de telle manière qu'un tel mélange de fragments modifiés comprenne tous les fragments de séquence nucléotidique possibles de la molécule d'acide nucléique.

5. Procédé selon la revendication 4, **caractérisé en ce que** le mélange comprend une forme de pleine longueur de la molécule d'acide nucléique modifiée dont la séquence nucléotidique doit être déterminée.

6. Procédé selon l'une quelconque des revendications 1 à 5, **caractérisé en ce que** les fragments de molécule d'acide nucléique modifiée sont séparés des fragments de molécule d'acide nucléique non modifiée par l'interaction de la modification avec un partenaire d'interaction, de telle manière qu'un tel partenaire d'interaction soit lié à un support.

7. Procédé selon l'une quelconque des revendications 1 à 5, **caractérisé en ce que** les fragments de molécule d'acide nucléique modifiée sont séparés de la molécule d'acide nucléique non modifiée par séparation due à la discrimination de masse, la discrimination de taille, et/ou la discrimination d'hydrophobicité, la discrimination de charge, la discrimination ionique, la discrimination de liaison hydrogène ou l'extraction en phase liquide, de sorte que, de préférence, les fragments de molécule d'acide nucléique non marqués soient éliminés.

8. Procédé selon l'une quelconque des revendications 1 à 7, **caractérisé en ce que** le profil de fragments d'acide nucléique modifié comprend une échelle de fragments d'acide nucléique modifié.

9. Procédé selon l'une quelconque des revendications 1 à 8, **caractérisé en ce que** la séquence nucléotidique de la molécule d'acide nucléique n'est pas connue.

10. Procédé selon l'une quelconque des revendications 1 à 8, **caractérisé en ce que** la séquence nucléotidique de la molécule d'acide nucléique est connue et, de préférence, le procédé est destiné à confirmer la séquence nucléotidique d'une molécule d'acide nucléique.

11. Procédé selon l'une quelconque des revendications 1 à 10, **caractérisé en ce que** lequel les fragments de molécule d'acide nucléique modifiée sont séparés ou résolus par un procédé pour la discrimination de masse ou de taille qui est de préférence choisi dans le groupe comprenant la filtration et la dialyse et la chromatographie et la spectrométrie de masse, de préférence un tel procédé est MS, LCMS ou ESI-MS.

12. Procédé selon l'une quelconque des revendications 1 à 11, **caractérisé en ce que** la molécule d'acide nucléique est choisie dans le groupe des molécules d'ARN, molécules d'ADN, molécules d'ARN modifiées par nucléotide et molécules d'ADN modifiées par nucléotide, ANP, LNA et des combinaisons de ceux-ci, de préférence des molécules d'ARN, des molécules d'ADN, des molécules d'ARN modifiées par nucléotide, des molécules d'ADN modifiées par nucléotide et des molécules d'acide nucléique contenant à la fois des désoxyribonucléotides et des ribonucléotides.

13. Procédé selon l'une quelconque des revendications 1 à 12, **caractérisé en ce que** la molécule d'acide nucléique est choisie dans le groupe constitué d'aptamères, spiegelmères, ribozymes, spiegelzymes, molécules antisens, molécules d'ARNsi et des molécules servant de leurres, de préférence des spiegelmères.

14. Procédé selon l'une quelconque des revendications 1 à 13, **caractérisé en ce qu'**un profil de masse spécifique d'une molécule d'acide nucléique est déterminé.

15. Procédé selon l'une quelconque des revendications 1 à 14, **caractérisé en ce que** la molécule d'acide nucléique comprend plus de 25 nucléotides ou nucléobases.
